# EUROPEAN PATENT SPECIFICATION

(11) **EP 3 705 483 B1**
(45) Date of publication and mention of the grant of the patent: **20.10.2021**
(21) Application number: 20161005.2
(22) Date of filing: 04.03.2020
(51) Int. Cl.: C07F 15/00, C09K 11/06, H01L 51/00, H01L 51/50

(54) **ORGANOMETALLIC COMPOUND, ORGANIC LIGHT-EMITTING DEVICE INCLUDING THE SAME, AND ELECTRONIC APPARATUS INCLUDING THE ORGANIC LIGHT-EMITTING DEVICE**
ORGANOMETALLISCHE VERBINDUNG, ORGANISCHE LICHTEMITTIERENDE VORRICHTUNG DAMIT UND ELEKTRONISCHE VORRICHTUNG MIT DER ORGANISCHEN LICHTEMITTIERENDEN VORRICHTUNG
COMPOSÉ ORGANOMÉTALLIQUE, DISPOSITIF ÉLECTROLUMINESCENT ORGANIQUE LE COMPRENANT ET APPAREIL ÉLECTRONIQUE COMPRENANT LE DISPOSITIF ÉLECTROLUMINESCENT ORGANIQUE

(30) Priority: 07.03.2019 KR 20190026320
(43) Date of publication of application: 09.09.2020
(73) Proprietor: Samsung Electronics Co., Ltd., Gyeonggi-do 16677 (KR)
(72) Inventor: HWANG, Kyuyoung, 16678 Gyeonggi-do (KR); KANG, Byungjoon, 16678 Gyeonggi-do (KR); KWAK, Seungyeon, 16678 Gyeonggi-do (KR); SIM, Myungsun, 16678 Gyeonggi-do (KR); LEE, Kum Hee, 16678 Gyeonggi-do (KR); JEON, Aram, 16678 Gyeonggi-do (KR); CHOI, Byoungki, 16678 Gyeonggi-do (KR); CHOI, Hyeonho, 16678 Gyeonggi-do (KR)
(74) Representative: Elkington and Fife LLP

(56) References cited:
- KR-A- 20180 020 878

## Description

### FIELD OF THE INVENTION

One or more embodiments relate to an organometallic compound, an organic light-emitting device including the organometallic compound, and an electronic apparatus including the organic light-emitting device.

### BACKGROUND OF THE INVENTION

Organic light-emitting devices are self-emission devices, which have better characteristics in terms of a viewing angle, response time, brightness, driving voltage, and response speed, and produce full-color images.

In an example, an organic light-emitting device includes an anode, a cathode, and an organic layer between the anode and the cathode, wherein the organic layer includes an emission layer. A hole transport region may be between the anode and the emission layer, and an electron transport region may be between the emission layer and the cathode. Holes provided from the anode may move toward the emission layer through the hole transport region, and electrons provided from the cathode may move toward the emission layer through the electron transport region. The holes and the electrons recombine in the emission layer to produce excitons. These excitons transit from an excited state to a ground state, thereby generating light.

KR 10-2018-0020878 discloses novel organometallic compounds, and an organic light emitting device using the same.

### SUMMARY OF THE INVENTION

Aspects of the present disclosure provide an organometallic compound, an organic light-emitting device including the organometallic compound, and an electronic apparatus including the organic light-emitting device.

Additional aspects will be set forth in part in the description which follows and, in part, will be apparent from the description, or may be learned by practice of the presented embodiments.

An aspect of the present disclosure provides an organometallic compound represented by Formula 1 below:

<Formula 1> M(L₁)n1(L₂)n2

In Formula 1,
M may be a transition metal,
L₁ may be a ligand represented by Formula 2A,
L₂ may be a ligand represented by Formula 2B,
n1 and n2 may each independently be 1 or 2, wherein, when n1 is 2, two L₁(s) may be identical to or different from each other and when n2 is 2, two L₂(s) may be identical to or different from each other,
the sum of n1 and n2 may be 2 or 3, and
L₁ to L₂ may be different from each other: 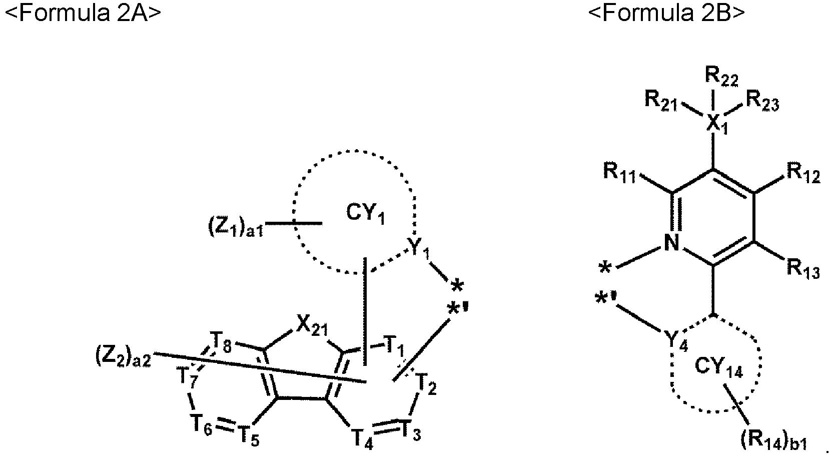
In Formulae 2A and 2B,
Y₁ and Y₄ may each independently be C or N,
X₁ may be Si or Ge,
X₂₁ may be O, S, S(=O), N(Z₂₉), C(Z₂₉)(Z₃₀), or Si(Z₂₉)(Z₃₀),
T₁ to T₄ may each independently be C, N, carbon linked to ring CY₁, or carbon linked to M in Formula 1, wherein one of T₁ to T₄ may be carbon linked to M in Formula 1, and one of the remaining T₁ to T₄ that are not linked to M in Formula 1 may be carbon linked to ring CY₁,
T₅ to T₈ may each independently be C or N,
when X₁ is Si, at least one of the remaining T₁ to T₈ that are not carbon linked to M and ring CY₁ may be N,
ring CY₁ and ring CY₁₄ may each independently be a C₅-C₃₀ carbocyclic group or a C1-C30 heterocyclic group,
R₂₁ to R₂₃ may each independently be a C₁-C₆₀ alkyl group or a C₆-C₆₀ aryl group, each unsubstituted or substituted with deuterium, -F, -CI, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₃-C₁₀ cycloalkyl group, a phenyl group, or any combination thereof,
Z₁, Z₂, and R₁₁ to R₁₄ may each independently be hydrogen, deuterium, -F, -CI, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₁-C₆₀ alkylthio group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Ge(Q₃)(Q₄)(Q₅), -B(Q₆)(Q₇), -P(=O)(Q₈)(Q₉) or - P(Q₈)(Q₉), wherein R₁₂ is neither hydrogen nor a methyl group,
a1 and b1 may each independently be an integer from 0 to 20, wherein, when a1 is 2 or more, two or more Z₁(S) may be identical or different, and when b1 is 2 or more, two or more R₁₄(s) may be identical or different,
a2 may be an integer from 0 to 6, wherein, when a2 is 2 or more, two or more Z₂(s) may be identical to or different from each other,
two or more selected from R₂₁ to R₂₃ may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
two or more selected from a plurality of Z₁(S) may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
two or more selected from a plurality of Z₂(s) may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
R₁₂ and R₁₃ may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
two or more selected from a plurality of R₁₄(S) may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
two or more selected from Z₁, Z₂ and R₁₁ to R₁₄ may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
R₁₀ₐ may be the same as defined in connection with R₁₄,
* and *' in Formulae 2A and 2B each indicate a binding site to M in Formula 1,
a substituent(s) of the substituted C₁-C₆₀ alkyl group, the substituted C₂-C₆₀ alkenyl group, the substituted C₂-C₆₀ alkynyl group, the substituted C₁-C₆₀ alkoxy group, the substituted C₁-C₆₀ alkylthio group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted monovalent non-aromatic condensed heteropolycyclic group may each independently be:
   deuterium, -F, -CI, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, or a C₁-C₆₀ alkoxy group;
   a C₁-C₆₀ alkyl group, a C₂-C₆₀ alkenyl group, a C₂-C₆₀ alkynyl group, or a C₁-C₆₀ alkoxy group, each substituted with deuterium, -F, -CI, -Br, -I, -CD₃, -CD₂H, -CDH₂, -CF₃, - CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), - Ge(Q₁₃)(Q₁₄)(Q₁₅), -B(Q₁₆)(Q₁₇), -P(=O)(Q₁₈)(Q₁₉), -P(Q₁₈)(Q₁₉), or any combination thereof;
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group, each unsubstituted or substituted with deuterium, -F, -CI, -Br, -I, -CD₃, -CD₂H, -CDH₂, -CF₃, - CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), - Ge(Q₂₃)(Q₂₄)(Q₂₅), -B(Q₂₆)(Q₂₇), -P(=O)(Q₂₈)(Q₂₉), -P(Q₂₈)(Q₂₉), or any combination thereof;
   -N(Q₃₁)(Q₃₂), -Ge(Q₃₃)(Q₃₄)(Q₃₅), -B(Q₃₆)(Q₃₇), -P(=O)(Q₃₈)(Q₃₉) or -P(Q₃₈)(Q₃₉); or any combination thereof,
   Q₁ to Q₉, Q₁₁ to Q₁₉, Q₂₁ to Q₂₉, and Q₃₁ to Q₃₉ may each independently be hydrogen; deuterium; -F; -Cl; -Br; -I; a hydroxyl group; a cyano group; a nitro group; an amino group; an amidino group; a hydrazine group; a hydrazone group; a carboxylic acid group or a salt thereof; a sulfonic acid group or a salt thereof; a phosphoric acid group or a salt thereof; a C₁-C₆₀ alkyl group unsubstituted or substituted with deuterium, a C₁-C₆₀ alkyl group, a C₆-C₆₀ aryl group, or any combination thereof; a C₂-C₆₀ alkenyl group; a C₂-C₆₀ alkynyl group; a C₁-C₆₀ alkoxy group; a C₃-C₁₀ cycloalkyl group; a C₁-C₁₀ heterocycloalkyl group; a C₃-C₁₀ cycloalkenyl group; a C₁-C₁₀ heterocycloalkenyl group; a C₆-C₆₀ aryl group unsubstituted or substituted with deuterium, a C₁-C₆₀ alkyl group, a C₆-C₆₀ aryl group, or any combination thereof; a C₆-C₆₀ aryloxy group; a C₆-C₆₀ arylthio group; a C₁-C₆₀ heteroaryl group, a monovalent non-aromatic condensed polycyclic group; or a monovalent non-aromatic condensed heteropolycyclic group.

Another aspect of the present disclosure provides an organic light-emitting device including: a first electrode, a second electrode, and an organic layer disposed between the first electrode and the second electrode and including an emission layer, wherein the organic layer including at least one of the organometallic compound.

The organometallic compound may be included in the emission layer of the organic layer and the organometallic compound included in the emission layer may act as a dopant.

Another aspect of the present disclosure provides an electronic apparatus including the organic light-emitting device.

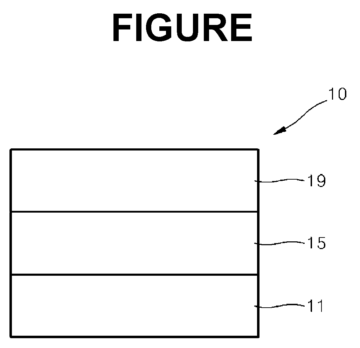

### BRIEF DESCRIPTION OF THE DRAWING

These and/or other aspects will become apparent and more readily appreciated from the following description of the embodiments, taken in conjunction with FIGURE which is a schematic view of an organic light-emitting device according to an embodiment.

### DETAILED DESCRIPTION OF THE EMBODIMENTS

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

An aspect of the present disclosure provides an organometallic compound represented by Formula 1 below:

<Formula 1> M(L₁)n1(L₂)n2

M in Formula 1 may be a transition metal.

For example, M may be a first-row transition metal, a second-row transition metal, or a third-row transition metal, of the Periodic Table of Elements.

For example, M may be iridium (Ir), platinum (Pt), osmium (Os), titanium (Ti), zirconium (Zr), hafnium (Hf), europium (Eu), terbium (Tb), thulium (Tm), or rhodium (Rh).

In one embodiment, M may be Ir, Pt, Os, or Rh.

L₁ in Formula 1 may be a ligand represented by Formula 2A, and n1 in Formula 1 indicates the number of L₁(s) in Formula 1 and may be 1 or 2. When n1 is two, two L₁(s) may be identical to or different from each other.

L₂ in Formula 1 may be a ligand represented by Formula 2B, and n2 in Formula 1 indicates the number of L₂(s) in Formula 1 and may be 1 or 2. When n2 is two, two L₂(s) may be identical to or different from each other:

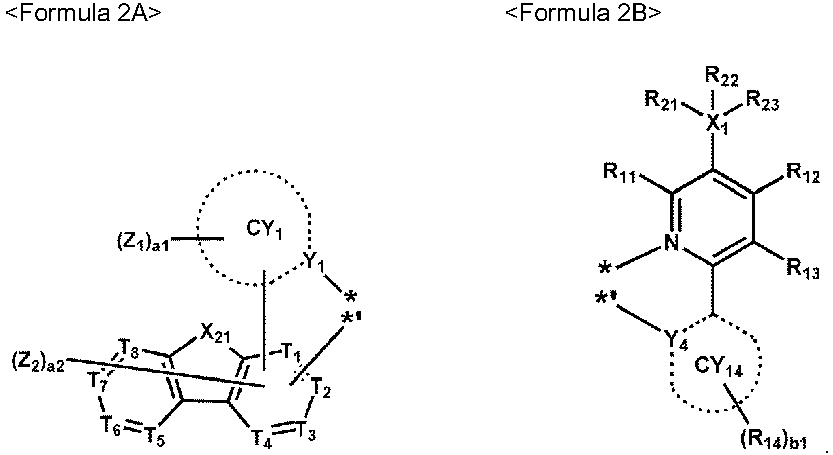

Formulae 2A and 2B will be understood by referring to a detailed description thereof to be provided later.

L₁ and L₂ in Formula 1 may be different from each other. That is, the organometallic compound represented by Formula 1 may be a heteroleptic complex.

In one embodiment, M may be Ir, and the sum of n1 and n2 may be 3; or
M may be Pt, and the sum of n1 and n2 may be 2.

Y₁ and Y₄ in Formulae 2Aand 2B may each independently be C or N.

For example, Y₁ in Formula 2A may be N and Y₄ in Formula 2B may be C.

X₁ in Formula 2B may be Si or Ge.

X₂₁ in Formula 2A may be O, S, S(=O), N(Z₂₉), C(Z₂₉)(Z₃₀), or Si(Z₂₉)(Z₃₀). Z₂₉ and Z₃₀ will be understood by referring to a detailed description thereof to be provided later.

For example, X₂₁ in Formula 2A may be O or S.

In Formula 2A, i) T₁ to T₄ may each independently be C, N, carbon linked to ring CY₁, or carbon linked to M in Formula 1, wherein one of T₁ to T₄ may be carbon linked to M in Formula 1, and one of the remaining T₁ to T₄ that are not linked to M in Formula 1 may be carbon linked to ring CY₁, and ii) T₅ to T₈ may each independently be C or N.

In Formula 1, when X₁ in Formula 2B is Si, at least one of the remaining T₁ to T₈ that are not carbon linked to M and ring CY₁ may be N.

In one embodiment, X₁ in Formula 2B may be Si, and at least one of the remaining T₁ to T₈ that are not carbon linked to M and ring CY₁ in Formula 2A may be N.

In one embodiment, X₁ in Formula 2B may be Ge, and T₁ to T₈ in Formula 2A may be C.

In one embodiment, X₁ in Formula 2B may be Ge, and at least one of the remaining T₁ to T₈ that are not carbon linked to M and ring CY₁ in Formula 2A may be N.

In one embodiment, T₈ in Formula 2A may be N.

Ring CY₁ and ring CY₁₄ in Formulae 2A and 2B may each independently be a C₅-C₃₀ carbocyclic group or a C₁-C₃₀ heterocyclic group.

For example, ring CY₁ and ring CY₁₄ may each independently be i) a first ring, ii) a second ring, iii) a condensed ring in which at least two first rings are condensed with each other, iv) a condensed ring in which at least two second rings are condensed with each other, or v) a condensed ring in which at least one first ring and at least one second ring are condensed with each other.

The first ring may be a cyclopentane group, a cyclopentadiene group, a furan group, a thiophene group, a pyrrole group, a silole group, an indene group, a benzofuran group, a benzothiophene group, an indole group, a benzosilole group, an oxazole group, an isoxazole group, an oxadiazole group, an isoxadiazole group, an oxatriazole group, an isoxatriazole group, a thiazole group, an isothiazole group, a thiadiazole group, an isothiadiazole group, a thiatriazole group, an isothiatriazole group, a pyrazole group, an imidazole group, a triazole group, a tetrazole group, an azasilole group, a diazasilole group, or a triazasilole group.

The second ring may be an admantane group, a norbornane group, a norbornene group, a cyclohexane group, a cyclohexene group, a benzene group, a pyridine group, a pyrimidine group, a pyrazine group, a pyridazine group, or a triazine group.

In one embodiment, in Formulae 2A and 2B, ring CY₁ and ring CY₁₄ may each independently be a cyclopentane group, a cyclohexane group, a cycloheptane group, a cyclopentene group, a cyclohexene group, a cycloheptene group, a benzene group, a naphthalene group, an anthracene group, a phenanthrene group, a triphenylene group, a pyrene group, a chrysene group, a cyclopentadiene group, a 1,2,3,4-tetrahydronaphthalene group, a thiophene group, a furan group, an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, a 5,6,7,8-tetrahydroquinoline group, an admantane group, a norbornane group, or a norbornene group.

In one or more embodiments, ring CY₁ and ring CY₁₄ may each independently be a benzene group, a naphthalene group, 1,2,3,4-tetrahydronaphthalene group, a phenanthrene group, a pyridine group, a pyrimidine group, a pyrazine group, a triazine group, a benzofuran group, a benzothiophene group, a fluorene group, a carbazole group, a dibenzofuran group, a dibenzothiophene group, a dibenzosilole group, an azafluorene group, an azacarbazole group, an azadibenzofuran group, an azadibenzothiophene group, or an azadibenzosilole group.

In one or more embodiments, ring CY₁ in Formula 2A may be a pyridine group, a 5,6,7,8-tetrahydroisoquinoline group, or a 5,6,7,8-tetrahydroquinoline group.

In one or more embodiments, ring CY₁₄ in Formula 2B may be a benzene group, a naphthalene group, 1,2,3,4-tetrahydronaphthalene group, a dibenzothiophene group, a dibenzofuran group, or a pyridine group.

In Formula 2B, R₂₁ to R₂₃ may each independently be a C₁-C₆₀ alkyl group or a C₆-C₆₀ aryl group, each unsubstituted or substituted with deuterium, -F, -CI, -Br, -I, -CD₃, - CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₃-C₁₀ cycloalkyl group, a phenyl group, or any combination thereof.

For example, R₂₁ to R₂₃ in Formula 2B may each independently be a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a secnonyl group, a tert-nonyl group, an n-decyl group, an isodecyl group, a sec-decyl group, a tert-decyl group, a phenyl group, a biphenyl group, or a naphthyl group, each unsubstituted or substituted with deuterium, -F, -CI, -Br, -I, -CD₃, -CD₂H, -CDH₂, -CF₃, - CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₃-C₁₀ cycloalkyl group, a phenyl group, or any combination thereof.

In one embodiment, R₂₁ to R₂₃ may each independently be -CH₃, -CH₂CH₃, -CD₃, - CD₂H, -CDH₂, -CH₂CD₃, or -CD₂CH₃.

In one embodiment, R₂₁ to R₂₃ in Formula 2B may be identical to each other.

In one embodiment, at least two of R₂₁ to R₂₃ may be different from each other.

Z₁, Z₂, and R₁₁ to R₁₄ in Formulae 2Aand 2B may each independently be hydrogen, deuterium, -F, -CI, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₁-C₆₀ alkylthio group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Ge(Q₃)(Q₄)(Q₅), -B(Q₆)(Q₇), -P(=O)(Q₈)(Q₉) or - P(Q₈)(Q₉), and R₁₂ may be neither hydrogen nor a methyl group. Q₁ to Q₉ may be the same as described in this disclosure.

In Formulae 2Aand 2B, a1 and b1 indicate the number of Z₁(S) and the number of R₁₄(S), respectively, and may each independently be an integer from 0 to 20. When a1 is two or more, two or more Z₁(S) may be identical to or different from each other, and when b1 is two or more, two or more R₁₄(S) may be identical to or different from each other. For example, a1 and b1 may each independently be an integer from 0 to 10.

In Formula 2A, a2 indicates the number of Z₂(S)and may each independently be an integer from 0 to 6. When a2 is two or more, two or more Z₂(S)may be identical to or different from each other. For example, a2 may each independently be 0, 1, 2 or 3.

In one embodiment, Z₁ in Formula 2A may be hydrogen, deuterium, -F, -CI, -Br, -I, a cyano group, a substituted or unsubstituted C₁-C₆₀ alkyl group, or a substituted or unsubstituted C₃-C₁₀ cycloalkyl group.

In one embodiment, in Formula 2A,

Z₁ may be:
hydrogen, deuterium, -F, or a cyano group;
a C₁-C₂₀ alkyl group unsubstituted or substituted with deuterium, -F, a cyano group, a C₃-C₁₀ cycloalkyl group, a deuterated C₃-C₁₀ cycloalkyl group, a (C₁-C₂₀ alkyl)C₃-C₁₀ cycloalkyl group, a C₁-C₁₀ heterocycloalkyl group, a deuterated C₁-C₁₀ heterocycloalkyl group, a (C₁-C₂₀ alkyl)C₁-C₁₀ heterocycloalkyl group, or any combination thereof; or
a C₃-C₁₀ cycloalkyl group or a C₁-C₁₀ heterocycloalkyl group, unsubstituted or substituted with deuterium, -F, a cyano group, a C₁-C₂₀ alkyl group, a deuterated C₁-C₂₀ alkyl group, or any combination thereof.

In one or more embodiments, in Formula 2A,

Z₁ may be:
hydrogen, deuterium, -F, or a cyano group;
a C₁-C₂₀ alkyl group unsubstituted or substituted with deuterium, -F, a cyano group, a cyclopentyl group, a cyclohexyl group, a cycloheptyl group, a cycloctyl group, an adamantanyl group, a norbornanyl group, a norbornenyl group, a bicyclo[1.1.1]pentyl group, a bicyclo[2.1,1]hexyl group, a bicyclo[2.2.2]octyl group, a (C₁-C₂₀ alkyl)cyclopentyl group, a (C₁-C₂₀ alkyl)cyclohexyl group, a (C₁-C₂₀ alkyl)cycloheptyl group, a (C₁-C₂₀ alkyl)cycloctyl group, a (C₁-C₂₀ alkyl)adamantanyl group, a (C₁-C₂₀ alkyl)norbornanyl group, a (C₁-C₂₀ alkyl)norbornenyl group, a (C₁-C₂₀ alkyl)bicyclo[1.1.1]pentyl group, a (C₁-C₂₀ alkyl)bicyclo[2.1.1]hexyl group, a (C₁-C₂₀ alkyl)bicyclo[2.2.2]octyl group, or any combination thereof; or
a cyclopentyl group, a cyclohexyl group, a cycloheptyl group, a cycloctyl group, an adamantanyl group, a norbornanyl group, a norbornenyl group, a bicyclo[1.1.1]pentyl group, a bicyclo[2.1,1]hexyl group, or a bicyclo[2.2.2]octyl group, each unsubstituted or substituted with deuterium, -F, a cyano group, a C₁-C₂₀ alkyl group, or any combination thereof.

In one or more embodiments, Z₂ and R₁₁ to R₁₄ in Formulae 2A and 2B may each independently be:
hydrogen, deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, C₁-C₂₀ alkyl group, a C₁-C₂₀ alkoxy group or a C₁-C₂₀ alkylthio group;
a C₁-C₂₀ alkyl group, a C₁-C₂₀ alkoxy group or a C₁-C₂₀ alkylthio group, each substituted with deuterium, -F, -CI, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1,1]hexyl group, a bicyclo[2.2.2]octyl group, a (C₁-C₂₀ alkyl)cyclopentyl group, a (C₁-C₂₀ alkyl)cyclohexyl group, a (C₁-C₂₀ alkyl)cycloheptyl group, a (C₁-C₂₀ alkyl)cycloctyl group, a (C₁-C₂₀ alkyl)adamantanyl group, a (C₁-C₂₀ alkyl)norbornanyl group, a (C₁-C₂₀ alkyl)norbornenyl group, a (C₁-C₂₀ alkyl)cyclopentenyl group, a (C₁-C₂₀ alkyl)cyclohexenyl group, a (C₁-C₂₀ alkyl)cycloheptenyl group, a (C₁-C₂₀ alkyl)bicyclo[1.1.1]pentyl group, a (C₁-C₂₀ alkyl)bicyclo[2.1.1]hexyl group, a (C₁-C₂₀ alkyl)bicyclo[2.2.2]octyl group, a phenyl group, a (C₁-C₂₀ alkyl)phenyl group, a biphenyl group, a terphenyl group, a naphthyl group, a pyridinyl group, a pyrimidinyl group, or any combination thereof;
a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.2]octyl group, a phenyl group, a (C₁-C₂₀ alkyl)phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, a benzoisothiazolyl group, a benzoxazolyl group, a benzoisoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, or an azadibenzothiophenyl group, each unsubstituted or substituted with deuterium, -F, -CI, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1,1]hexyl group, a bicyclo[2.2.2]octyl group, a (C₁-C₂₀ alkyl)cyclopentyl group, a (C₁-C₂₀ alkyl)cyclohexyl group, a (C₁-C₂₀ alkyl)cycloheptyl group, a (C₁-C₂₀ alkyl)cycloctyl group, a (C₁-C₂₀ alkyl)adamantanyl group, a (C₁-C₂₀ alkyl)norbornanyl group, a (C₁-C₂₀ alkyl)norbornenyl group, a (C₁-C₂₀ alkyl)cyclopentenyl group, a (C₁-C₂₀ alkyl)cyclohexenyl group, a (C₁-C₂₀ alkyl)cycloheptenyl group, a (C₁-C₂₀ alkyl)bicyclo[1.1.1]pentyl group, a (C₁-C₂₀ alkyl)bicyclo[2.1.1]hexyl group, a (C₁-C₂₀ alkyl)bicyclo[2.2.2]octyl group, a phenyl group, a (C₁-C₂₀ alkyl)phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, a benzoisothiazolyl group, a benzoxazolyl group, a benzoisoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, an azadibenzothiophenyl group, or any combination thereof; or
   -N(Q₁)(Q₂), -Ge(Q₃)(Q₄)(Q₅), -B(Q₆)(Q₇), -P(=O)(Q₈)(Q₉), or -P(Q₈)(Q₉),
   R₁₂ may not be hydrogen nor a methyl group, and
   Q₁ to Q₉ may each independently be:
      -CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, or -CD₂CDH₂; or
      an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, a phenyl group, a biphenyl group, or a naphthyl group, each unsubstituted or substituted with deuterium, a C₁-C₁₀ alkyl group, a phenyl group, or any combination thereof.

In one or more embodiments, a number of carbon included in R₁₂ of Formula 2B may be at least two.

In one or more embodiments, R₁₂ in Formula 2B may be:
a C₂-C₂₀ alkyl group or a C₂-C₂₀ alkoxy group;
a methyl group or a methoxy group, each substituted with a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₁₄ aryl group, a C₁-C₁₄ heteroaryl group, a monovalent non-aromatic condensed polycyclic group, a monovalent non-aromatic condensed heteropolycyclic group, or any combination thereof;
a C₂-C₂₀ alkyl group or a C₂-C₂₀ alkoxy group, each substituted with deuterium, -F, -CI, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a C₁-C₁₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₁₄ aryl group, a C₁-C₁₄ heteroaryl group, a monovalent non-aromatic condensed polycyclic group, a monovalent non-aromatic condensed heteropolycyclic group, or any combination thereof;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₁₄ aryl group, a C₁-C₁₄ heteroaryl group, a monovalent non-aromatic condensed polycyclic group, or a monovalent non-aromatic condensed heteropolycyclic group, each unsubstituted or substituted with deuterium, -F, - CI, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₁₄ aryl group, a C₁-C₁₄ heteroaryl group, a monovalent non-aromatic condensed polycyclic group, a monovalent non-aromatic condensed heteropolycyclic group, or any combination thereof; or
-N(Q₁)(Q₂), -Ge(Q₃)(Q₄)(Q₅), -B(Q₆)(Q₇), or -P(=O)(Q₈)(Q₉)

In one embodiment, R₁₂ in Formula 2B may be:
a methyl group or a methoxy group, each substituted with a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1,1]hexyl group, a bicyclo[2.2.2]octyl group, a (C₁-C₂₀ alkyl)cyclopentyl group, a (C₁-C₂₀ alkyl)cyclohexyl group, a (C₁-C₂₀ alkyl)cycloheptyl group, a (C₁-C₂₀ alkyl)cycloctyl group, a (C₁-C₂₀ alkyl)adamantanyl group, a (C₁-C₂₀ alkyl)norbornanyl group, a (C₁-C₂₀ alkyl)norbornenyl group, a (C₁-C₂₀ alkyl)cyclopentenyl group, a (C₁-C₂₀ alkyl)cyclohexenyl group, a (C₁-C₂₀ alkyl)cycloheptenyl group, a (C₁-C₂₀ alkyl)bicyclo[1.1.1]pentyl group, a (C₁-C₂₀ alkyl)bicyclo[2.1.1]hexyl group, a (C₁-C₂₀ alkyl)bicyclo[2.2.2]octyl group, a phenyl group, a (C₁-C₂₀ alkyl)phenyl group, a naphthyl group, a pyridinyl group, a pyrimidinyl group, or any combination thereof;
a methyl group or a methoxy group, each substituted with i) cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1,1]hexyl group, a bicyclo[2.2.2]octyl group, a (C₁-C₂₀ alkyl)cyclopentyl group, a (C₁-C₂₀ alkyl)cyclohexyl group, a (C₁-C₂₀ alkyl)cycloheptyl group, a (C₁-C₂₀ alkyl)cycloctyl group, a (C₁-C₂₀ alkyl)adamantanyl group, a (C₁-C₂₀ alkyl)norbornanyl group, a (C₁-C₂₀ alkyl)norbornenyl group, a (C₁-C₂₀ alkyl)cyclopentenyl group, a (C₁-C₂₀ alkyl)cyclohexenyl group, a (C₁-C₂₀ alkyl)cycloheptenyl group, a (C₁-C₂₀ alkyl)bicyclo[1.1.1]pentyl group, a (C₁-C₂₀ alkyl)bicyclo[2.1.1]hexyl group, a (C₁-C₂₀ alkyl)bicyclo[2.2.2]octyl group, a phenyl group, a (C₁-C₂₀ alkyl)phenyl group, a naphthyl group, a pyridinyl group, a pyrimidinyl group, or any combination thereof, and ii) at least one deuterium;
a C₂-C₂₀ alkyl group or a C₂-C₂₀ alkoxy group, each unsubstituted or substituted with deuterium, -F, -CI, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1,1]hexyl group, a bicyclo[2.2.2]octyl group, a (C₁-C₂₀ alkyl)cyclopentyl group, a (C₁-C₂₀ alkyl)cyclohexyl group, a (C₁-C₂₀ alkyl)cycloheptyl group, a (C₁-C₂₀ alkyl)cycloctyl group, a (C₁-C₂₀ alkyl)adamantanyl group, a (C₁-C₂₀ alkyl)norbornanyl group, a (C₁-C₂₀ alkyl)norbornenyl group, a (C₁-C₂₀ alkyl)cyclopentenyl group, a (C₁-C₂₀ alkyl)cyclohexenyl group, a (C₁-C₂₀ alkyl)cycloheptenyl group, a (C₁-C₂₀ alkyl)bicyclo[1.1.1]pentyl group, a (C₁-C₂₀ alkyl)bicyclo[2.1.1]hexyl group, a (C₁-C₂₀ alkyl)bicyclo[2.2.2]octyl group, a phenyl group, a (C₁-C₂₀ alkyl)phenyl group, a naphthyl group, a pyridinyl group, a pyrimidinyl group, or any combination thereof; or
a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1,1]hexyl group, a bicyclo[2.2.2]octyl group, a phenyl group, a (C₁-C₂₀ alkyl)phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, a benzoisothiazolyl group, a benzoxazolyl group, a benzoisoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, or an azadibenzothiophenyl group, each unsubstituted or substituted with deuterium, -F, -CI, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1,1]hexyl group, a bicyclo[2.2.2]octyl group, a (C₁-C₂₀ alkyl)cyclopentyl group, a (C₁-C₂₀ alkyl)cyclohexyl group, a (C₁-C₂₀ alkyl)cycloheptyl group, a (C₁-C₂₀ alkyl)cycloctyl group, a (C₁-C₂₀ alkyl)adamantanyl group, a (C₁-C₂₀ alkyl)norbornanyl group, a (C₁-C₂₀ alkyl)norbornenyl group, a (C₁-C₂₀ alkyl)cyclopentenyl group, a (C₁-C₂₀ alkyl)cyclohexenyl group, a (C₁-C₂₀ alkyl)cycloheptenyl group, a (C₁-C₂₀ alkyl)bicyclo[1.1.1]pentyl group, a (C₁-C₂₀ alkyl)bicyclo[2.1.1]hexyl group, a (C₁-C₂₀ alkyl)bicyclo[2.2.2]octyl group, a phenyl group, a (C₁-C₂₀ alkyl)phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, a benzoisothiazolyl group, a benzoxazolyl group, a benzoisoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, an azadibenzothiophenyl group, or any combination thereof.

In one or more embodiments, the organometallic compound represented by Formula 1 may satisfy at least one of <Condition (1)> to <Condition (3)> below:
<Condition (1)>
   In Formula 2A, Z₁ is not hydrogen, and a1 is an integer of 1 to 20.
<Condition (2)>
   In Formula 2B, R₁₄ is not hydrogen, and b1 is an integer of 1 to 20.
<Condition (3)>
   In Formula 2A, Z₂ is not hydrogen, and a2 is an integer of 1 to 6.

In one or more embodiments, the organometallic compound represented by Formula 1 may include at least one deuterium, at least one fluoro group (-F), at least one cyano group (-CN), or any combination thereof.

In one or more embodiments, the organometallic compound represented by Formula 1 may include at least one deuterium.

In one or more embodiments, the organometallic compound represented by Formula 1 may satisfy at least one of <Condition A> to <Condition l> below:
<Condition A>
   In Formula 2A, Z₁ is not hydrogen, a1 is an integer of 1 to 20, and at least one of Z₁(S) in the number of a1 includes deuterium.
<Condition B>
   In Formula 2A, Z₂ is not hydrogen, a2 is an integer of 1 to 6, and at least one of Z₂(s) in the number of a2 includes deuterium.
<Condition C>
   In Formula 2A, Z₂ is not hydrogen, a2 is an integer of 1 to 6, and at least one of Z₂(s) in the number of a2 includes -F.
<Condition D>
   In Formula 2A, Z₂ is not hydrogen, a2 is an integer of 1 to 6, and at least one of Z₂(s) in the number of a2 includes -CN.
<Condition E>
   In Formula 2A, Z₂ is not hydrogen, a2 is an integer of 1 to 6, and at least one of Z₂(s) in the number of a2 is a substituted or unsubstituted C₆-C₆₀ aryl group, a substituted or unsubstituted C₁-C₆₀ heteroaryl group, a substituted or unsubstituted monovalent non-aromatic condensed polycyclic group, or a substituted or unsubstituted monovalent non-aromatic condensed heteropolycyclic group.
<Condition F>
   In Formula 2B, at least one of R₂₁ to R₂₃ includes deuterium.
<Condition G>
   In Formula 2B, R₁₂ includes deuterium.
<Condition H>
   In Formula 2B, R₁₄ is not hydrogen, b1 is an integer of 1 to 20, and at least one of R₁₄(S) in the number of b1 includes deuterium.
<Condition I>
   In Formula 2B, R₁₄ is not hydrogen, b1 is an integer of 1 to 20, and at least one of R₁₄(S) in the number of b1 includes -F.

In one or more embodiments, Z₂ in Formula 2A may not be hydrogen, a2 may be an integer from 1 to 3, and at least one of Z₂(s) in number of a2 may each independently be a substituted or unsubstituted C₁-C₆₀ alkyl group, a substituted or unsubstituted C₁-C₆₀ alkoxy group, a substituted or unsubstituted C₁-C₆₀ alkylthio group, a substituted or unsubstituted C₃-C₁₀ cycloalkyl group, a substituted or unsubstituted C₁-C₁₀ heterocycloalkyl group, a substituted or unsubstituted C₆-C₆₀ aryl group, a substituted or unsubstituted C₁-C₆₀ heteroaryl group, a substituted or unsubstituted monovalent non-aromatic condensed polycyclic group, or a substituted or unsubstituted monovalent non-aromatic condensed heteropolycyclic group.

In one or more embodiments, Z₂ in Formula 2A may not be hydrogen, a2 may be an integer from 1 to 3, and at least one of Z₂(s) in number of a2 may each independently be a substituted or unsubstituted C₆-C₆₀ aryl group, a substituted or unsubstituted C₁-C₆₀ heteroaryl group, a substituted or unsubstituted monovalent non-aromatic condensed polycyclic group, or a substituted or unsubstituted monovalent non-aromatic condensed heteropolycyclic group.

In one embodiment, Z₁ in Formula 2A may be hydrogen, deuterium, -F, a cyano group, a nitro group, -SF₅, -CH₃, -CD₃, -CD₂H, -CDH₂, -CF₃, -CF₂H, -CFH₂, -OCH₃, -OCDH₂, - OCD₂H, -OCD₃, -SCH₃, -SCDH₂, -SCD₂H, -SCD₃, one of groups represented by Formulae 9-1 to 9-39, one of groups represented by Formulae 9-1 to 9-39 in which at least one hydrogen is substituted with deuterium, one of groups represented by Formulae 9-1 to 9-39 in which at least one hydrogen is substituted with -F, one of groups represented by Formulae 9-201 to 9-233, one of groups represented by Formulae 9-201 to 9-233 in which at least one hydrogen is substituted with deuterium, one of groups represented by Formulae 9-201 to 9-233 in which at least one hydrogen is substituted with -F, one of groups represented by Formulae 10-1 to 10-11, one of groups represented by Formulae 10-1 to 10-11 in which at least one hydrogen is substituted with deuterium, or one of groups represented by Formulae 10-1 to 10-11 in which at least one hydrogen is substituted with -F.

In one embodiment, Z₂ and R₁₁ to R₁₄ in Formulae 2A and 2B may each independently be hydrogen, deuterium, -F, a cyano group, a nitro group, -SF₅, -CH₃, -CD₃, -CD₂H, -CDH₂, - CF₃, -CF₂H, -CFH₂, -OCH₃, -OCDH₂, -OCD₂H, -OCD₃, -SCH₃, -SCDH₂, -SCD₂H, -SCD₃, one of groups represented by Formulae 9-1 to 9-39, one of groups represented by Formulae 9-1 to 9-39 in which at least one hydrogen is substituted with deuterium, one of groups represented by Formulae 9-1 to 9-39 in which at least one hydrogen is substituted with -F, one of groups represented by Formulae 9-201 to 9-233, one of groups represented by Formulae 9-201 to 9-233 in which at least one hydrogen is substituted with deuterium, one of groups represented by Formulae 9-201 to 9-233 in which at least one hydrogen is substituted with -F, one of groups represented by Formulae 10-1 to 10-132, one of groups represented by Formulae 10-1 to 10-132 in which at least one hydrogen is substituted with deuterium, or one of groups represented by Formulae 10-1 to 10-132 in which at least one hydrogen is substituted with -F, one of groups represented by Formulae 10-201 to 10-353, one of groups represented by Formulae 10-201 to 10-353 in which at least one hydrogen is substituted with deuterium, one of groups represented by Formulae 10-201 to 10-353 in which at least one hydrogen is substituted with -F, -N(Q₁)(Q₂), or - Ge(Q₃)(Q₄)(Q₅) (wherein Q₁ to Q₅ are the same as described above).

In one embodiment, R₁₂ in Formula 2B may be one of groups represented by Formulae 9-1 to 9-39, one of groups represented by Formulae 9-1 to 9-39 in which at least one hydrogen is substituted with deuterium, one of groups represented by Formulae 9-1 to 9-39 in which at least one hydrogen is substituted with -F, one of groups represented by Formulae 9-201 to 9-233, one of groups represented by Formulae 9-201 to 9-233 in which at least one hydrogen is substituted with deuterium, one of groups represented by Formulae 9-201 to 9-233 in which at least one hydrogen is substituted with -F, one of groups represented by Formulae 10-1 to 10-132, one of groups represented by Formulae 10-1 to 10-132 in which at least one hydrogen is substituted with deuterium, or one of groups represented by Formulae 10-1 to 10-132 in which at least one hydrogen is substituted with -F, one of groups represented by Formulae 10-201 to 10-353, one of groups represented by Formulae 10-201 to 10-353 in which at least one hydrogen is substituted with deuterium, or one of groups represented by Formulae 10-201 to 10-353 in which at least one hydrogen is substituted with -F:

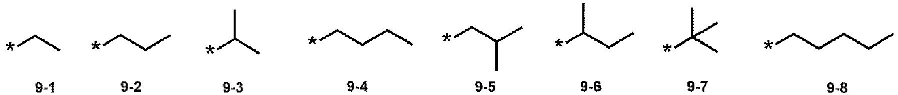

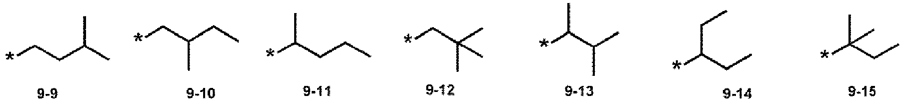

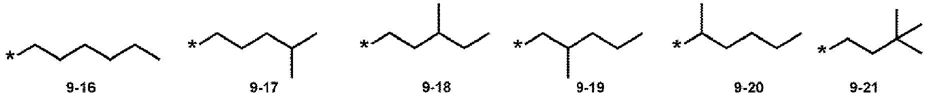

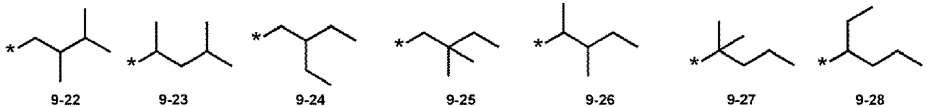

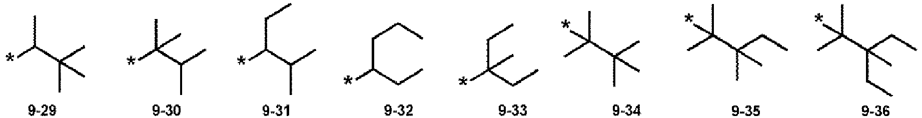

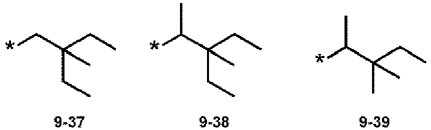

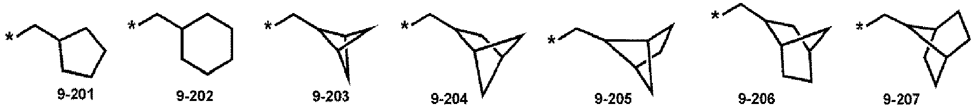

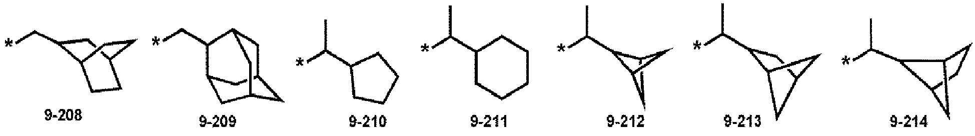

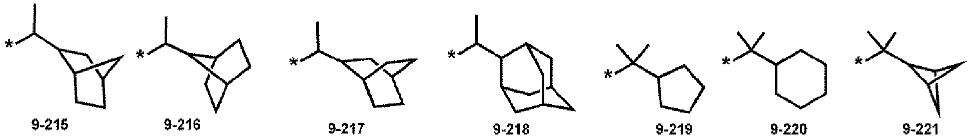

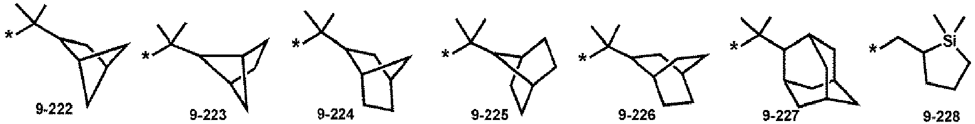

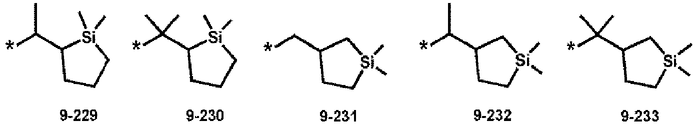

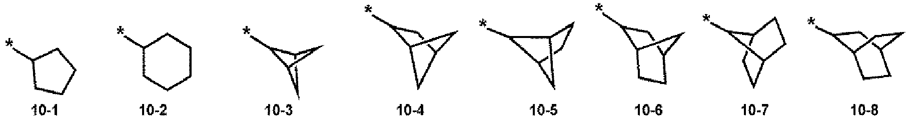

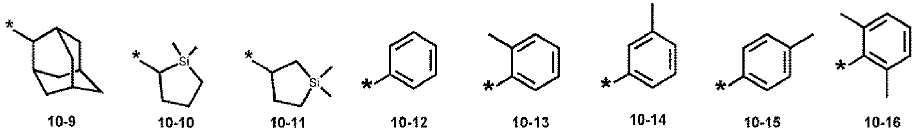

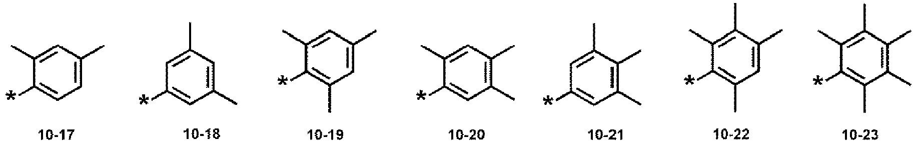

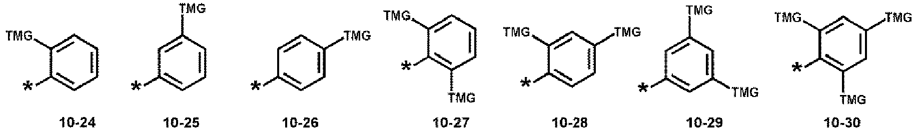

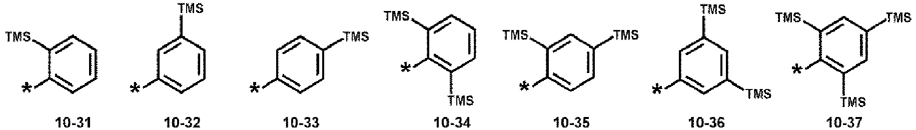

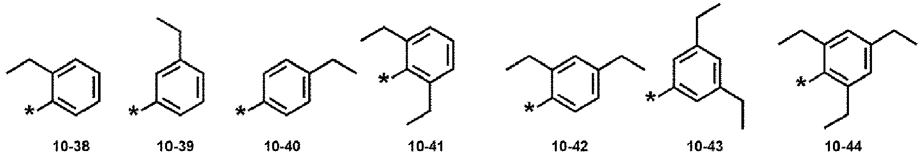

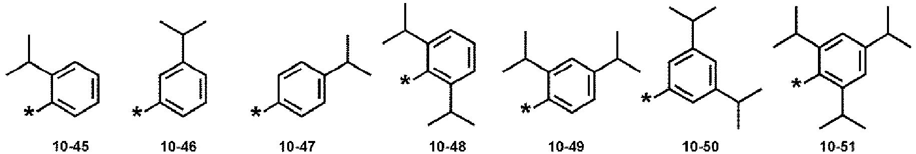

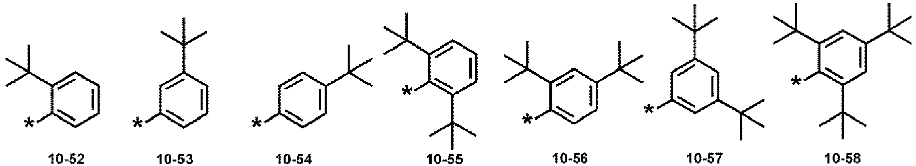

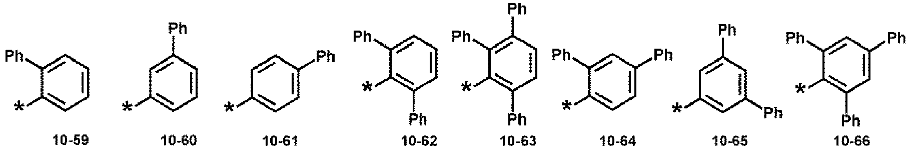

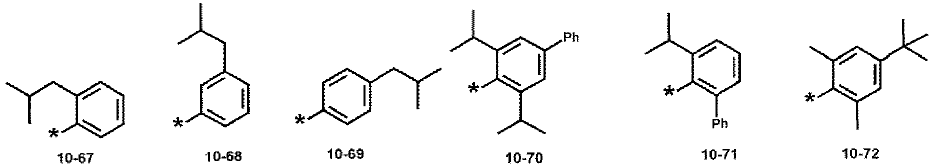

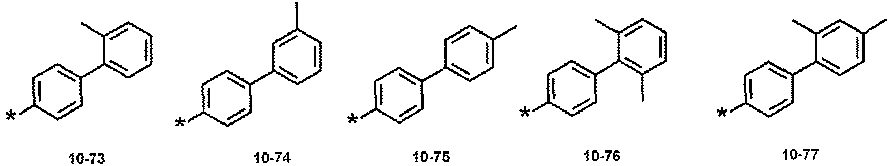

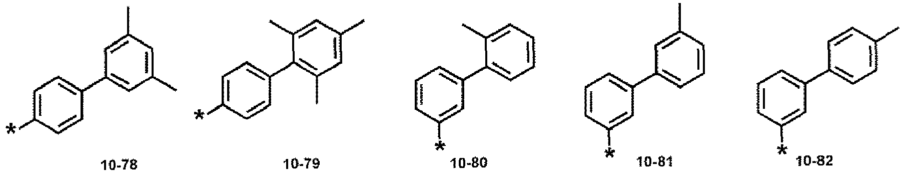

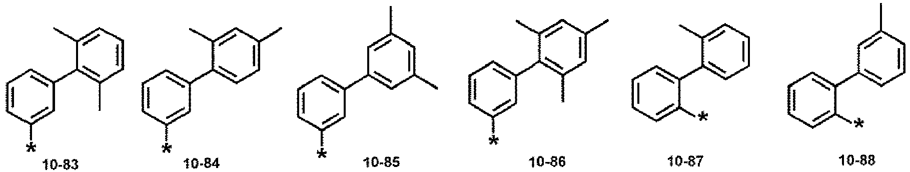

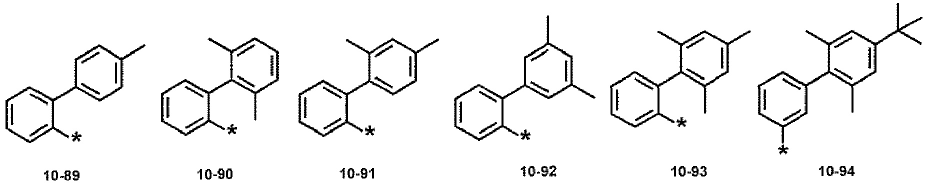

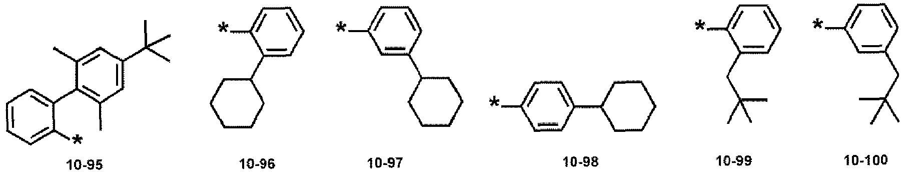

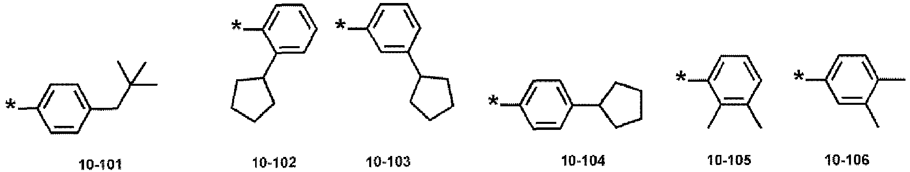

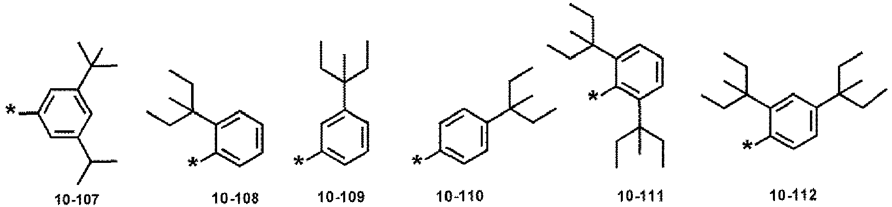

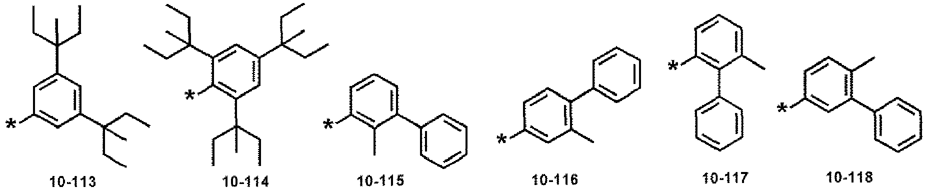

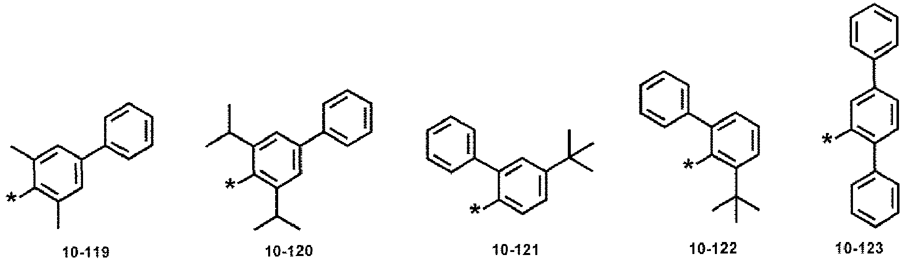

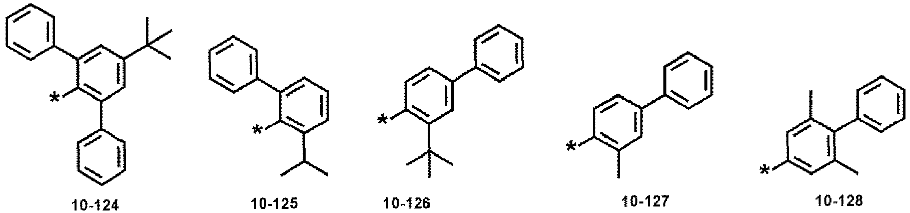

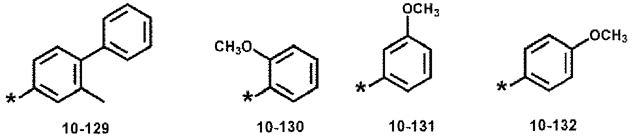

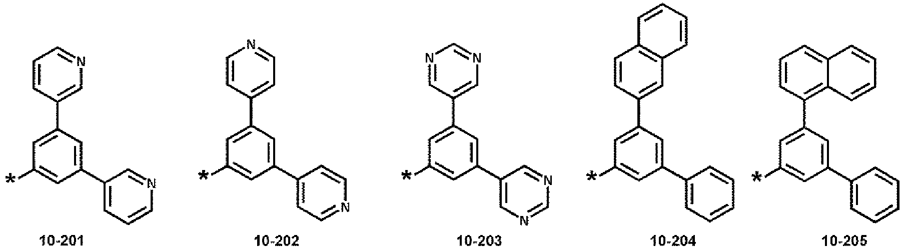

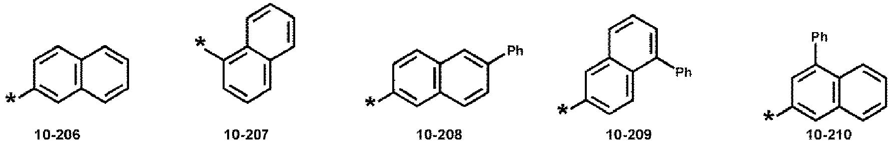

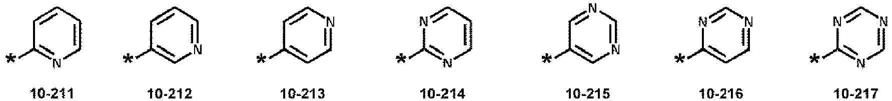

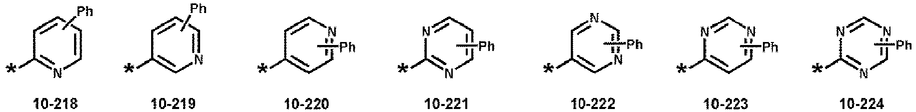

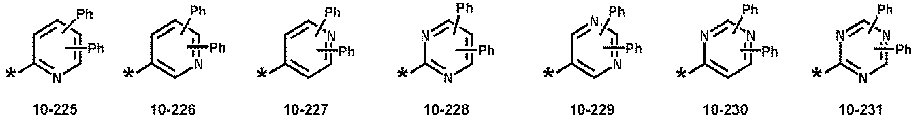

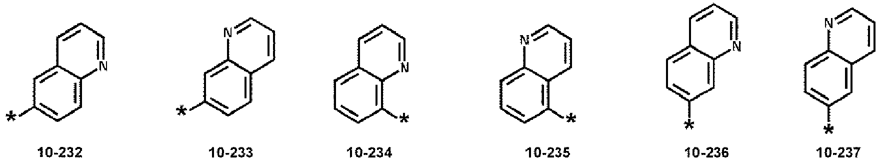

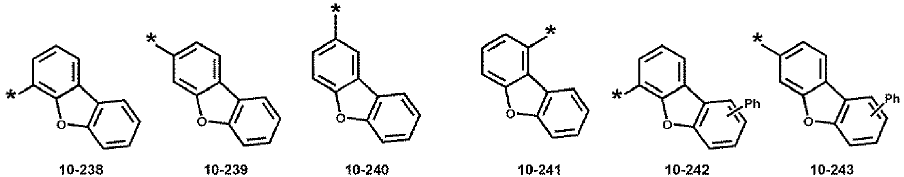

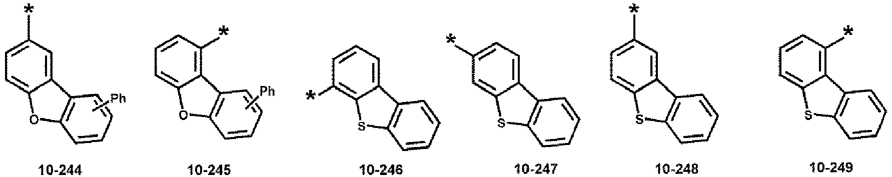

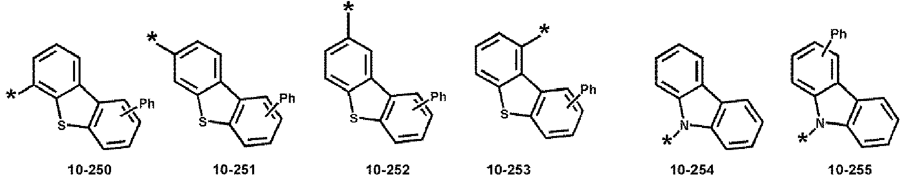

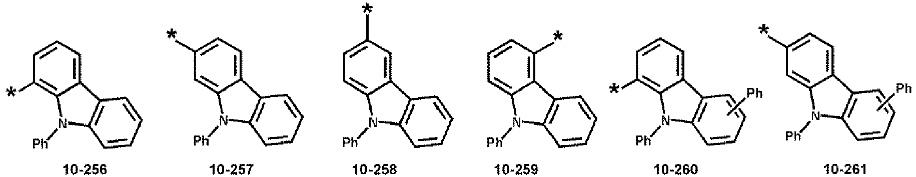

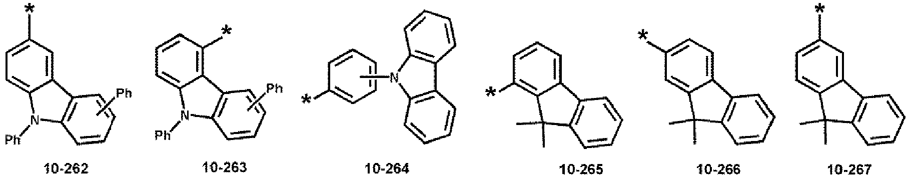

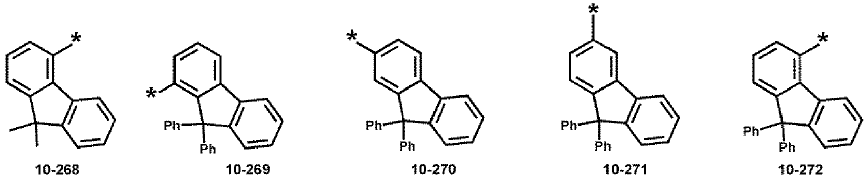

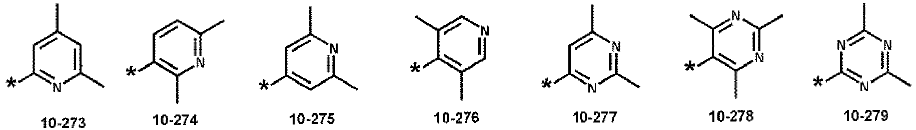

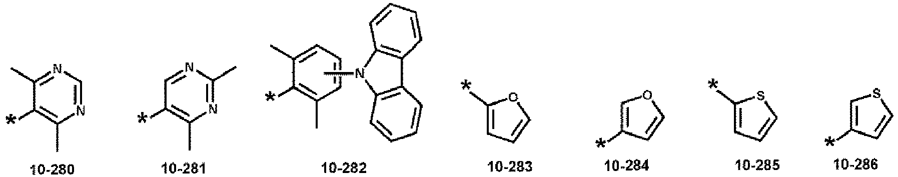

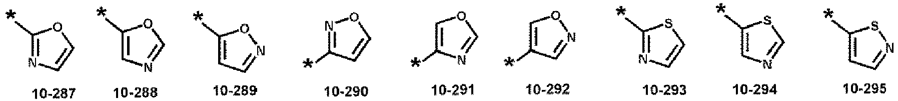

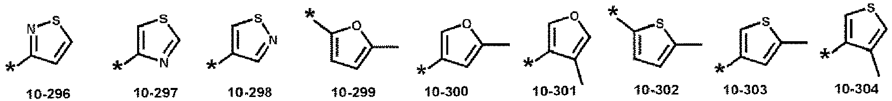

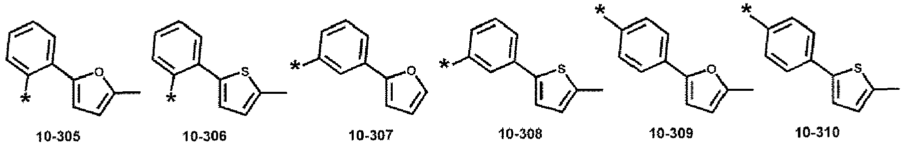

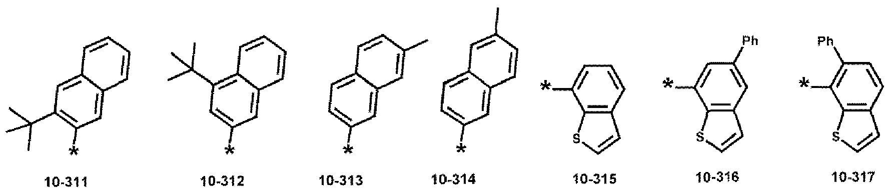

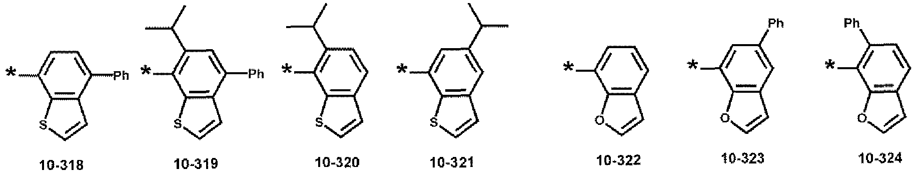

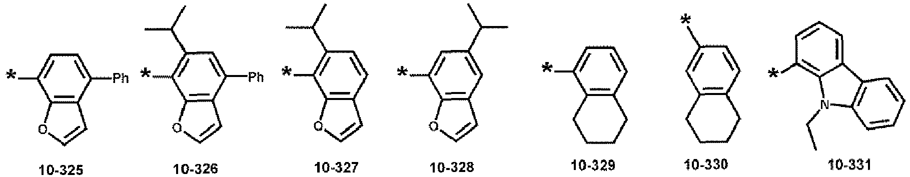

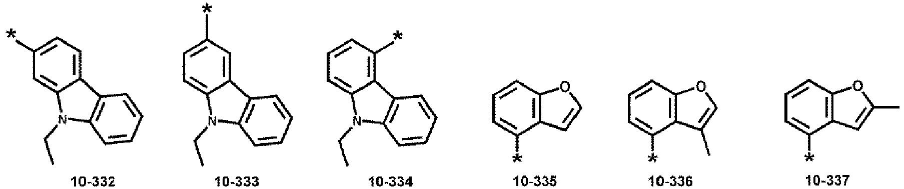

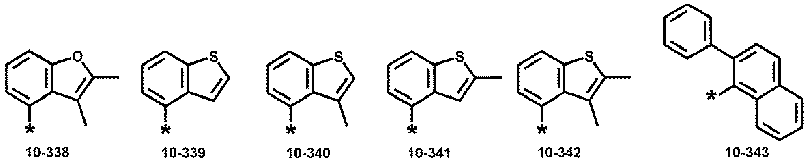

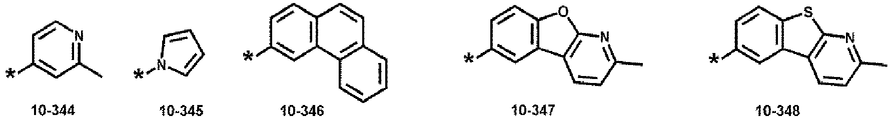

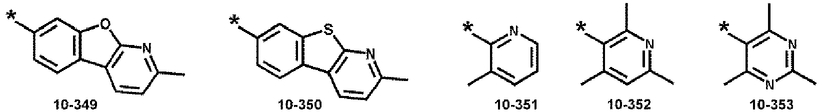

In Formulae 9-1 to 9-39, 9-201 to 9-233, 10-1 to 10-132 and 10-201 to 10-353,
* indicates a binding site to a neighboring atom,
Ph indicates a phenyl group,
TMS indicates a trimethylsilyl group,
TMG indicates a trimethylgermyl group, and
OMe indicates a methoxy group.

The term "groups represented by Formulae 9-1 to 9-39 in which at least one hydrogen is substituted with deuterium" and "groups represented by Formulae 9-201 to 9-233 in which at least one hydrogen is substituted with deuterium" as used herein may be, for example, groups represented by Formulae 9-501 to 9-514 and 9-601 to 9-635:

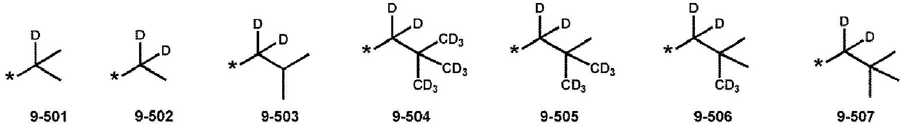

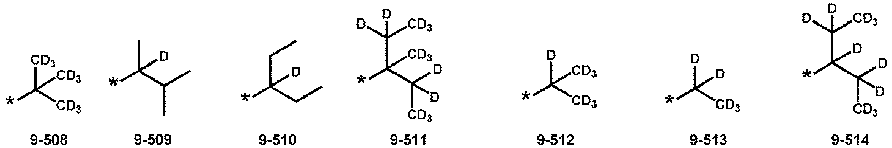

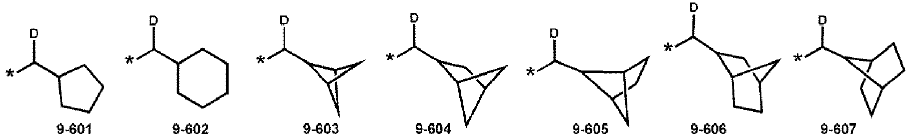

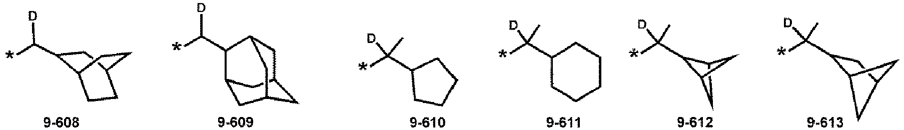

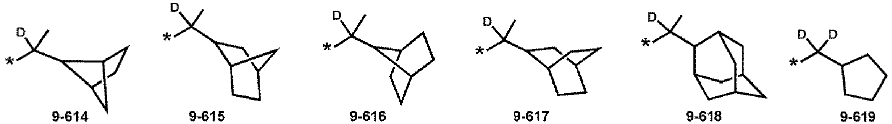

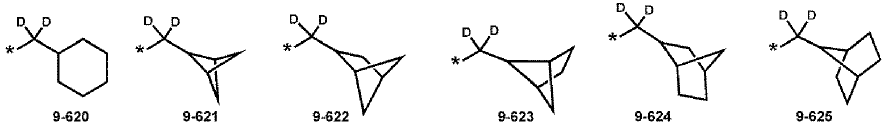

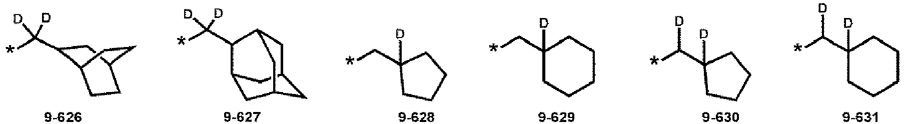

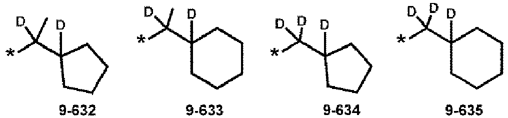

The term "groups represented by Formulae 9-1 to 9-39 in which at least one hydrogen is substituted with -F" and "groups represented by Formulae 9-201 to 9-233 in which at least one hydrogen is substituted with -F" as used herein may be, for example, groups represented by Formulae 9-701 to 9-710:

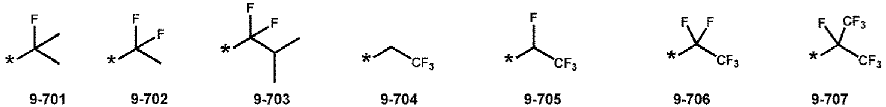

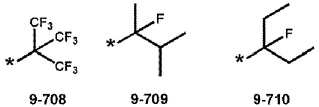

The term "groups represented by Formulae 10-1 to 10-132 in which at least one hydrogen is substituted with deuterium" and "groups represented by Formulae 10-201 to 10-353 in which at least one hydrogen is substituted with deuterium" as used herein may be, for example, groups represented by Formulae 10-501 to 10-553:

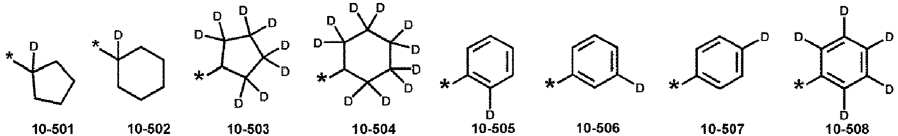

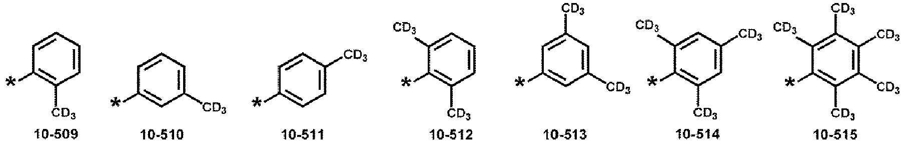

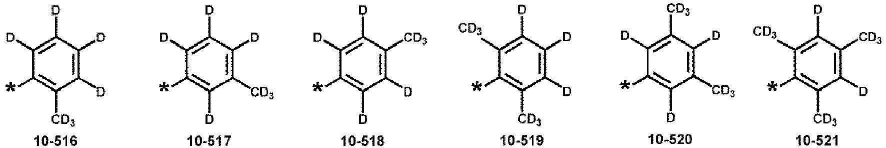

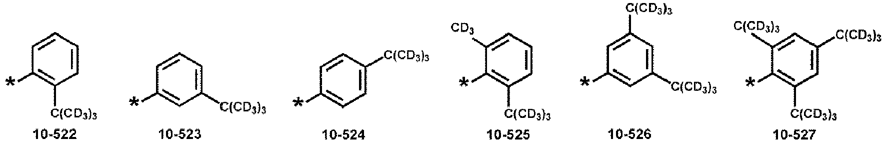

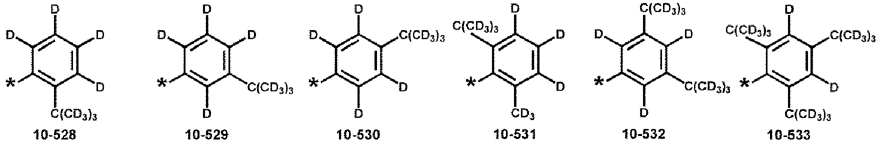

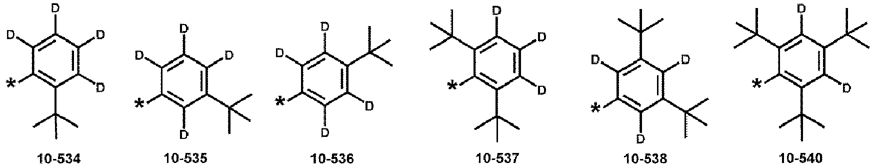

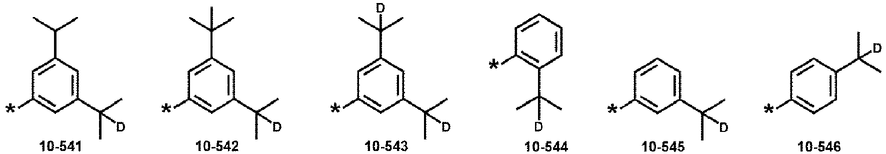

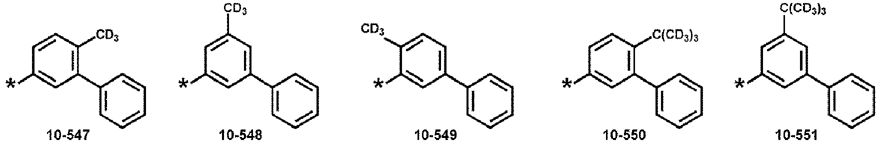

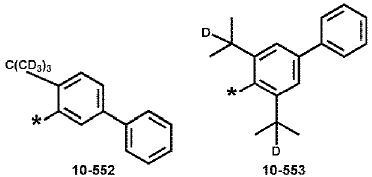

The term "groups represented by Formulae 10-1 to 10-132 in which at least one hydrogen is substituted with -F" and "groups represented by Formulae 10-201 to 10-353 in which at least one hydrogen is substituted with -F" as used herein may be, for example, groups represented by Formulae 10-601 to 10-620:

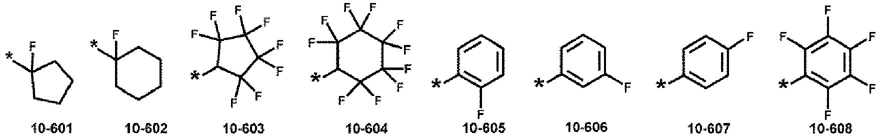

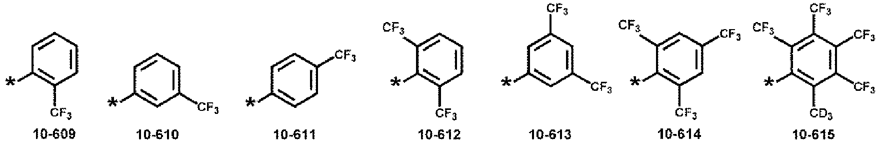

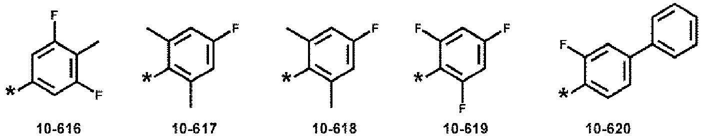

In Formulae 2A and 2B, i) two or more selected from R₂₁ to R₂₃ may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,ii) two or more selected from a plurality of Z₁(S) may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,iii) two or more selected from a plurality of Z₂(s) may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,iv) R₁₂ and R₁₃ may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ, v) two or more selected from a plurality of R₁₄(S) may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ, and vi) two or more selected from Z₁, Z₂ and R₁₁ to R₁₄ may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ.

R₁₀ₐ may be the same as defined in connection with R₁₄ in the present specification. In Formulae 2A and 2B, * and *' each indicate a binding site to M in Formula 1.

In one or more embodiments, the group represented by 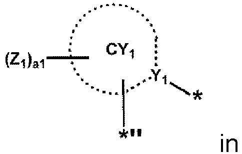 Formula 2A may be a group represented by one of Formulae CY1-1 to CY1-28:

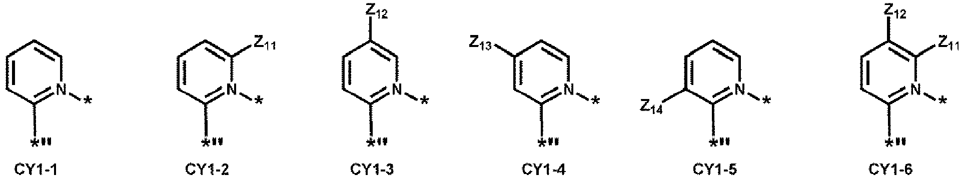

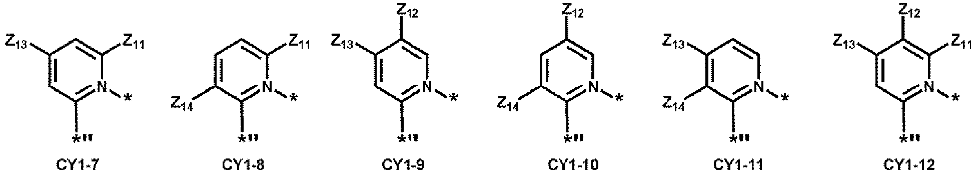

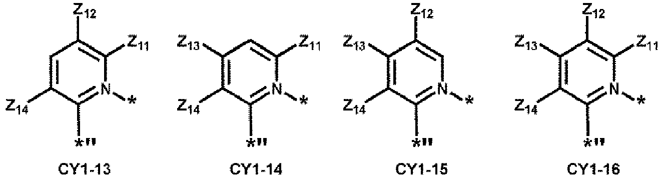

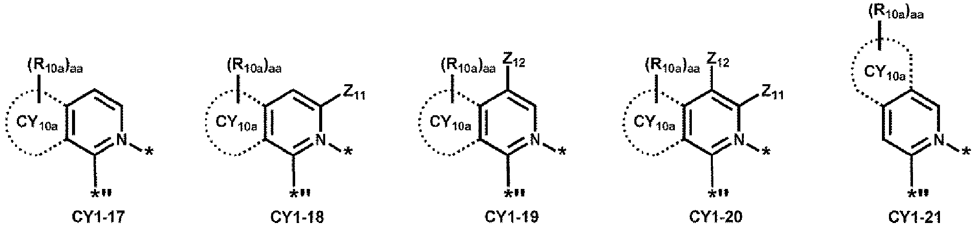

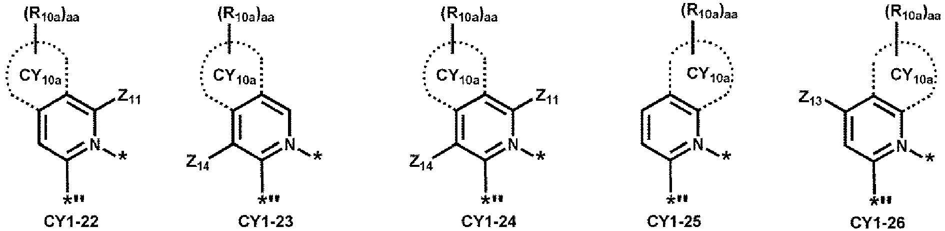

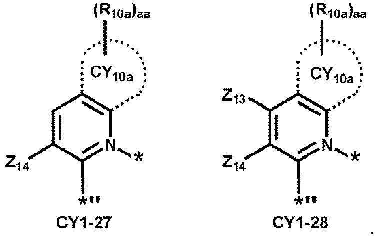

In Formulae CY1-1 to CY1-28,
Z₁₁ to Z₁₄ may each be the same as defined in connection with Z₁, provided that each of Z₁₁ to Z₁₄ are not hydrogen,
ring CY₁₀ₐ may be a C₅-C₃₀ carbocyclic group or a C₁-C₃₀ heterocyclic group,
R₁₀ₐ may be the same as described above,
aa may be an integer from 0 to 10,
* indicates a binding site to M in Formula 1, and
*" indicates a binding site to one of T₁ to T₄ in Formula 2A.

In one or more embodiments, the group represented by

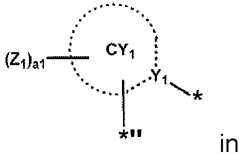

Formula 2A may be a group represented by one of Formulae CY1-4, CY1-7, CY1-9, CY₁-11, CY1-12, and CY1-14 to CY1-24.

In one or more embodiments, the group represented by 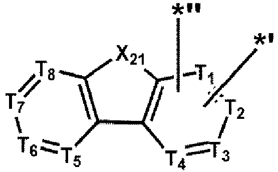 in Formula 2A may be a group represented by one of Formulae CY2-1 to CY2-6:

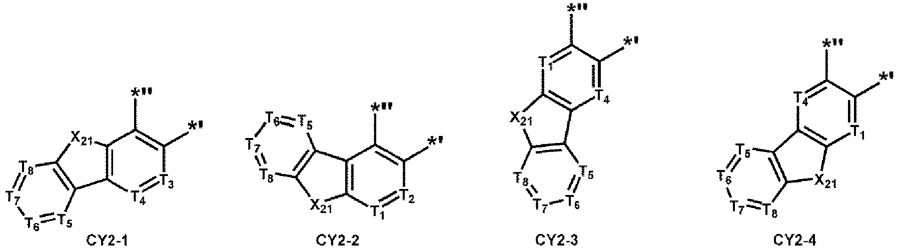

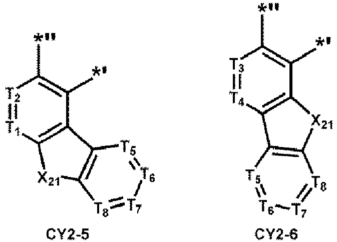

In Formulae CY2-1 to CY2-6,
T₁ to T₈ may each independently be C or N,
X₂₁ may be the same as described above,
*" indicates a binding site to ring CY₁ in Formula 2A, and
*' indicates a binding site to M in Formula 1.

For example,
a) X₁ in Formula 2B may be Ge, and each of T₁ to T₈ in Formulae CY2-1 to CY2-6 may be C, (and/or)
b) X₁ in Formula 2B may be Si or Ge, and at least one of T₃ to T₈ (for example, one or two of T₃ to Ts) in Formulae CY2-1 and CY2-6 may be N, (and/or)
c) X₁ in Formula 2B may be Si or Ge, and at least one of T₁, T₂, and T₅ to T₈ (for example, one or two of T₁, T₂, and T₅ to Ts) in Formulae CY2-2 and CY2-5 may be N, (and/or)
d) X₁ in Formula 2B may be Si or Ge, and at least one of T₁ and T₄ to T₈ (for example, one or two T₁ and T₄ to Ts) in Formulae CY2-3 and CY2-4 may be N.

In one or more embodiments,
1) T₁ to T₈ in Formulae CY2-1 to CY2-6 may be C;
2) one of T₃ to T₈ in Formula CY2-1 may be N, and the remaining T₃ to T₈ that are not N in Formula CY2-1 may be C;
3) T₃ and T₈ in Formula CY2-1 may be N, and T₄ to T₇ in Formula CY2-1 may be C;
4) T₆ and T₈ in Formula CY2-1 may be N, and T₃ to T₅ and T₇ in Formula CY2-1 may be C;
5) one of T₁, T₂ and T₈ in Formula CY2-2 may be N, and the remaining T₁, T₂ and T₅ to T₈ that are not N in Formula CY2-2 may be C;
6) T₁ and T₈ in Formula CY2-2 may be N, and T₂ and T₅ to T₇ in Formula CY2-2 may be C;
7) T₂ and T₈ in Formula CY2-2 may be N, and T₁ and T₅ to T₇ in Formula CY2-2 may be C;
8) one of T₁, T₄ and T₈ in Formulae CY2-3 and CY2-4 may be N, and the remaining T₁, T₄ and T₅ to T₈ that are not N in Formulae CY2-3 and CY2-4 may be C;
9) T₁ and T₈ in Formulae CY2-3 and CY2-4 may be N, and T₄ and T₅ to T₇ in Formulae CY2-3 and CY2-4 may be C;
10) T₄ and T₈ in Formulae CY2-3 and CY2-4 may be N, and T₁ and T₅ to T₇ in Formulae CY2-3 and CY2-4 may be C;
11) one of T₁ and T₈ in Formula CY2-5 may be N, and the remaining T₁, T₂ and T₅ to T₈ that are not N in Formula CY2-5 may be C;
12) T₁ and T₈ in Formula CY2-5 may be N, and T₂ and T₅ to T₇ in Formula CY2-5 may be C;
13) one of T₄ and T₈ in Formula CY2-6 may be N, and the remaining T₃ to T₈ that are not N in Formula CY2-6 may be C; or
14) T₄ and T₈ in Formula CY2-6 may be N, and T₃ and T₅ to T₇ in Formula CY2-6 may be C.

In one or more embodiments, a group represented by 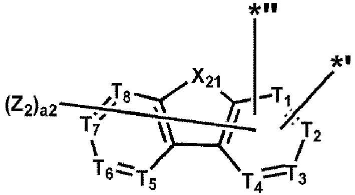 in Formula 2A may be a group represented by one of Formulae CY2-1001 to CY2-1141, CY2-2001 to CY2-2092, CY2-3001 to CY2-3092, CY2-4001 to CY2-4092, CY2-5001 to CY2-5065 and CY2-6001 to CY2-6065:

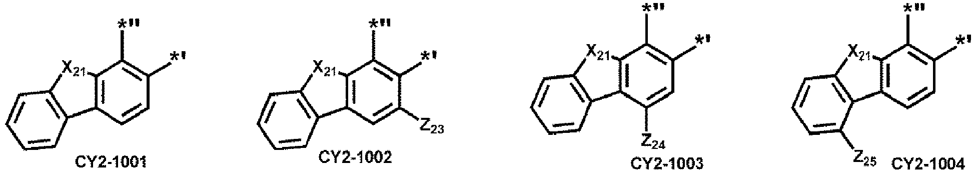

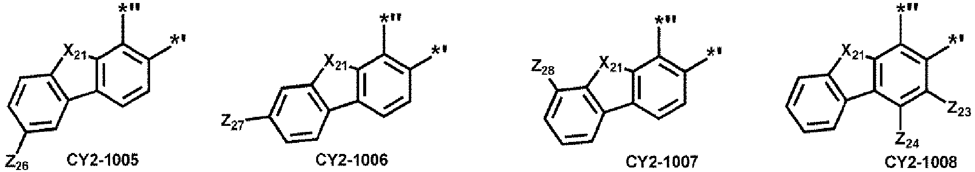

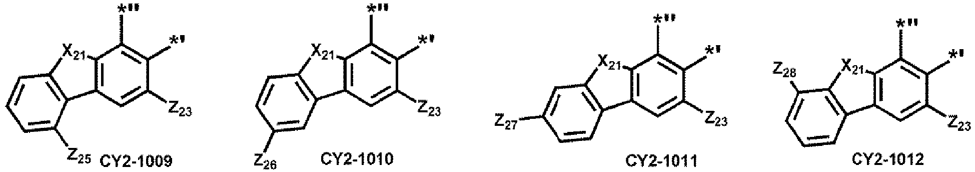

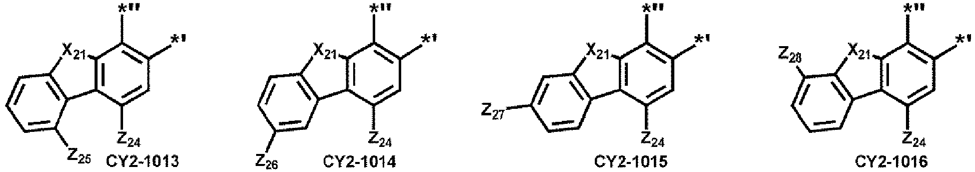

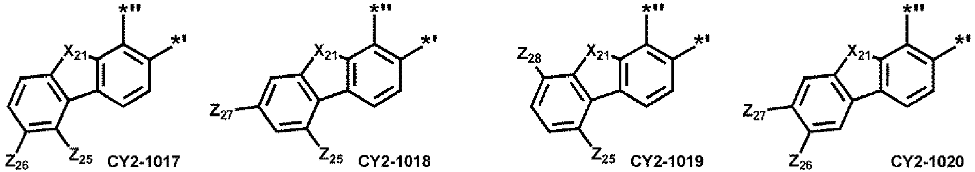

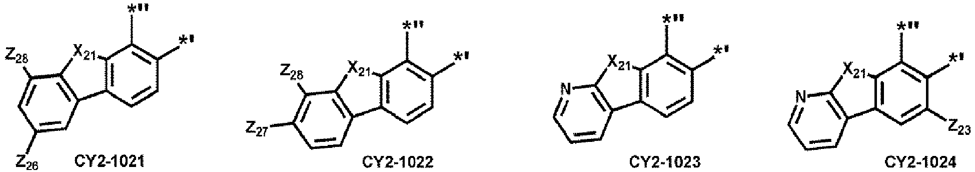

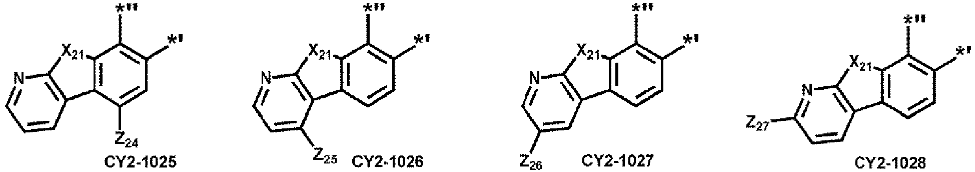

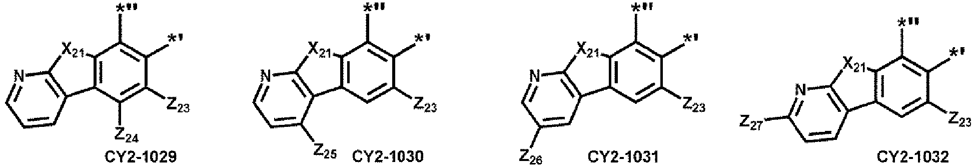

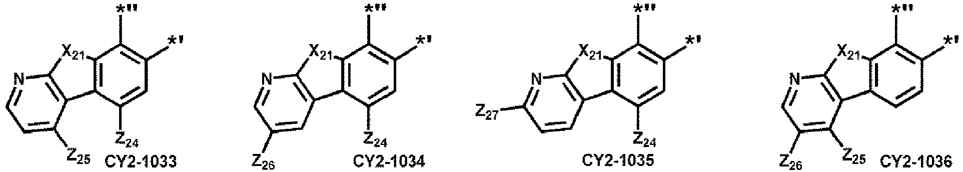

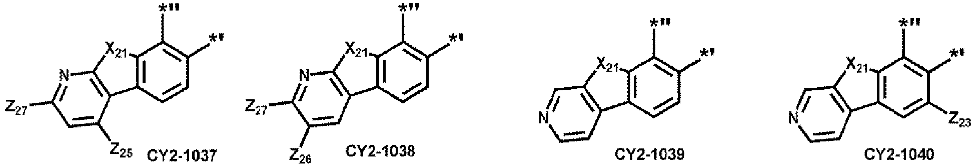

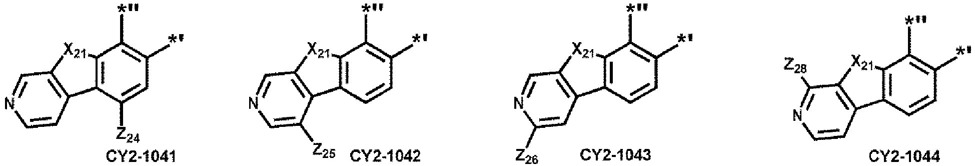

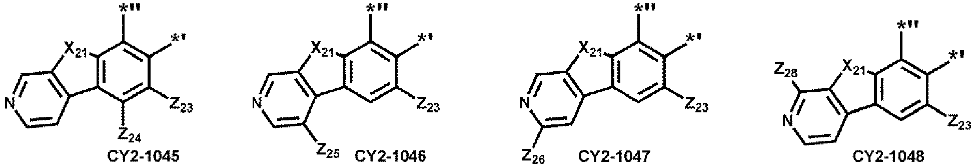

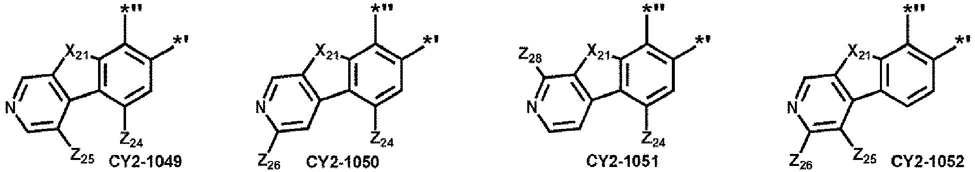

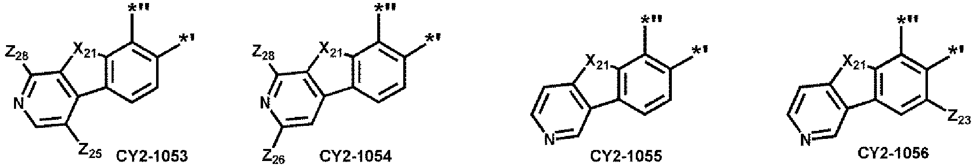

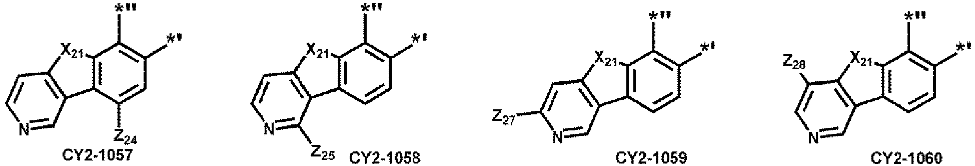

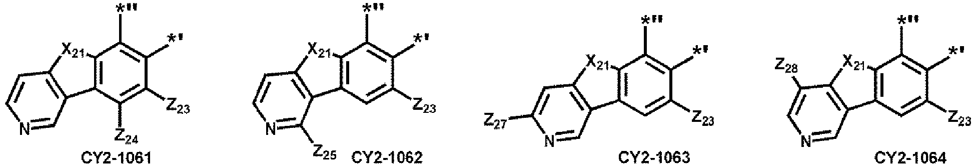

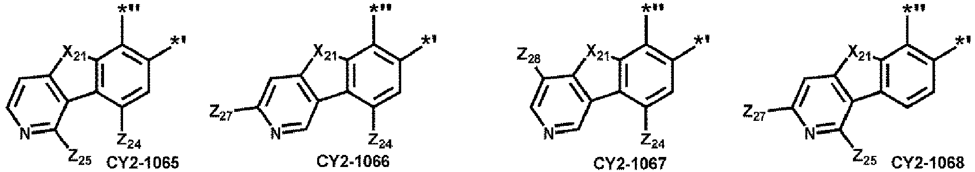

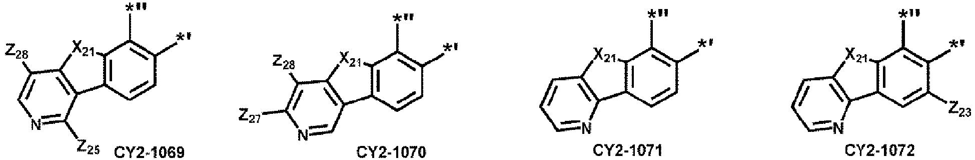

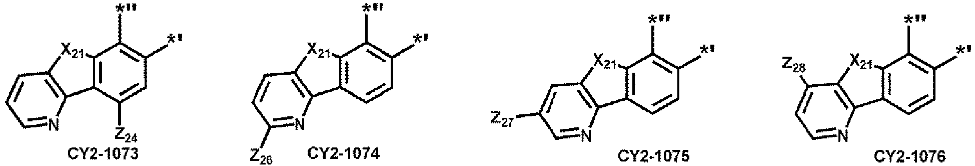

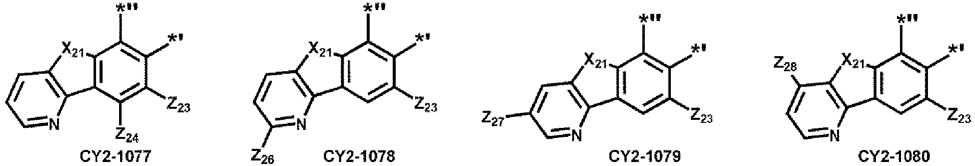

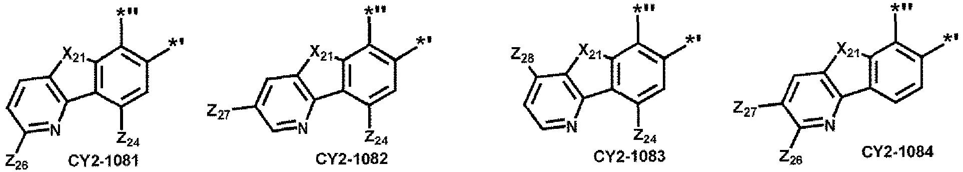

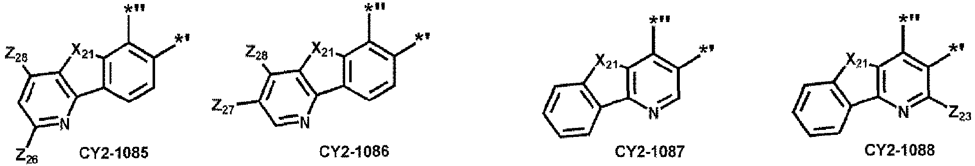

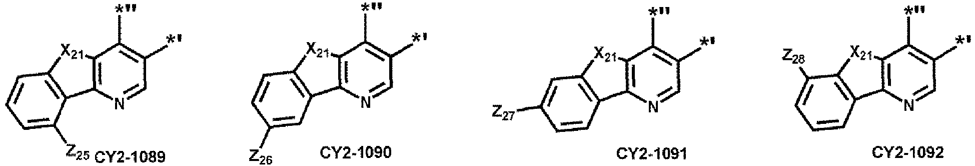

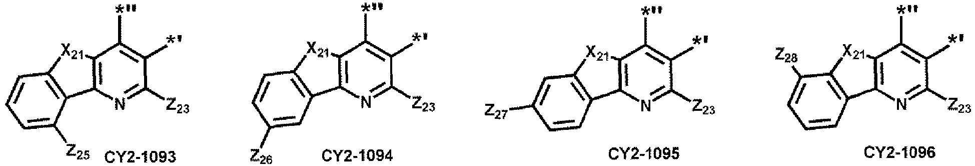

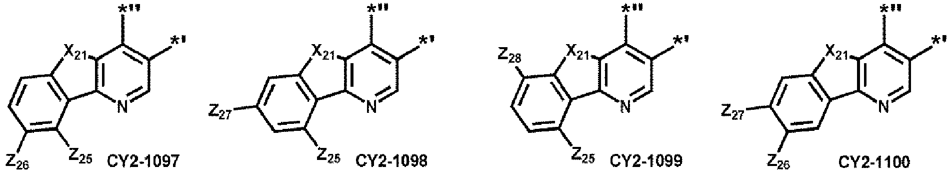

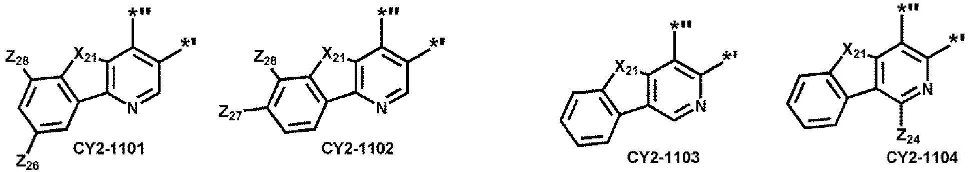

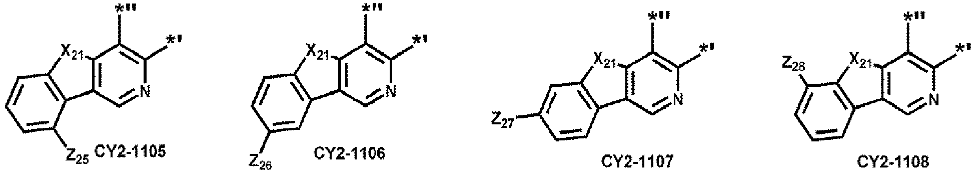

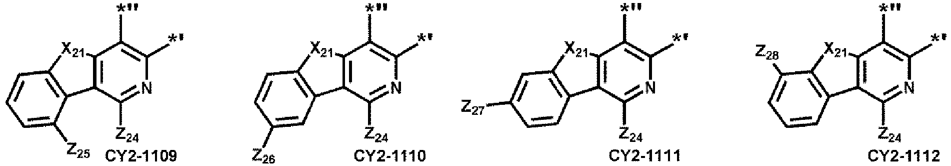

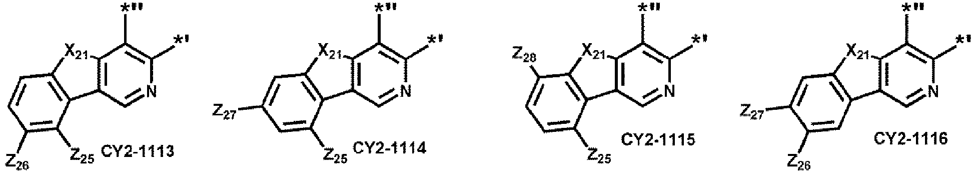

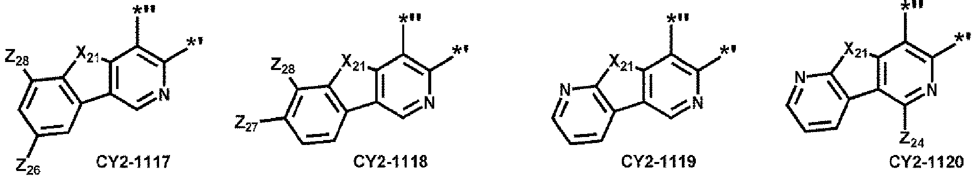

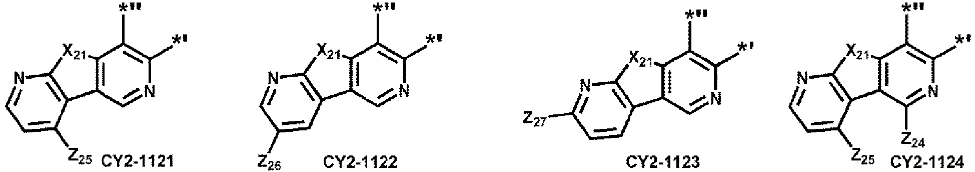

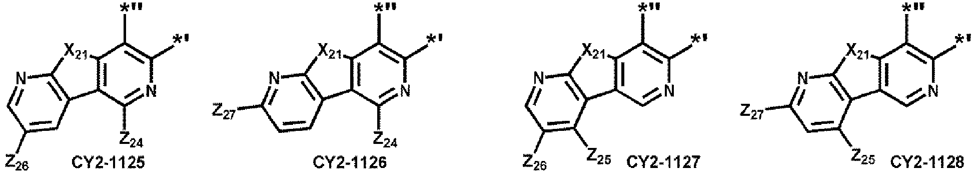

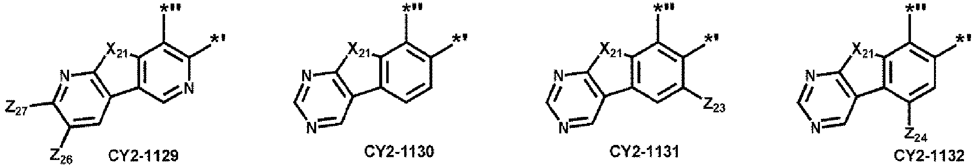

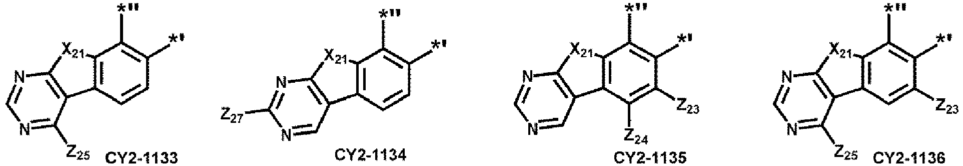

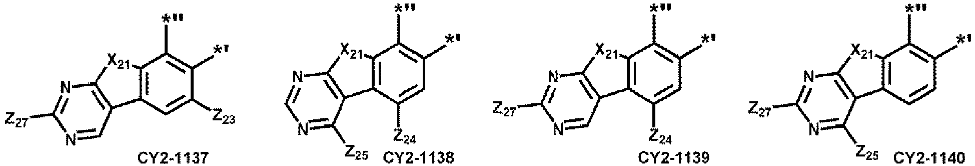

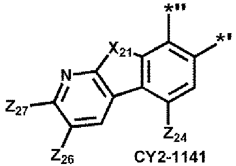

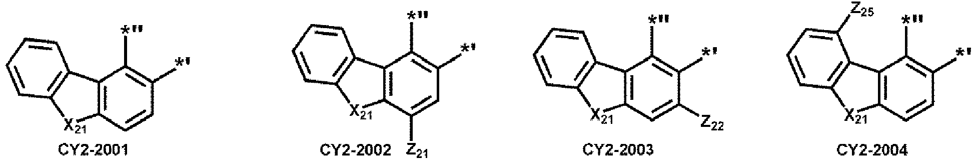

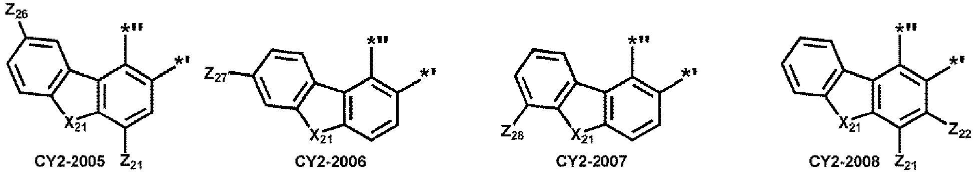

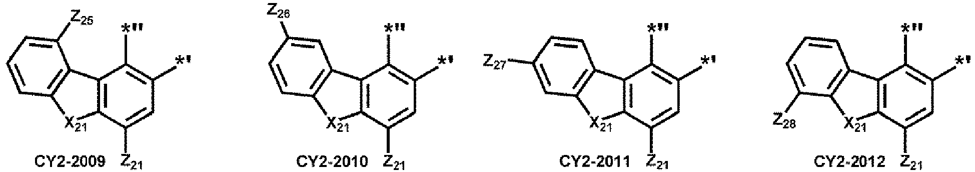

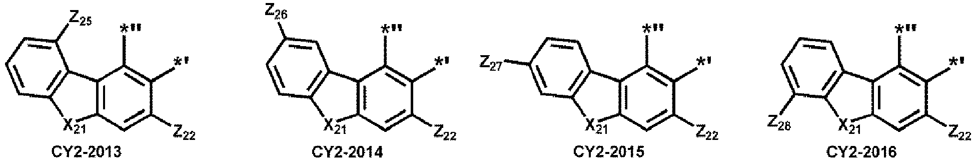

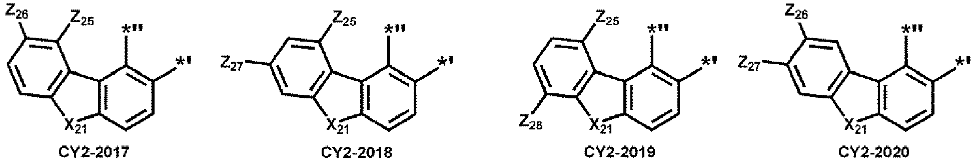

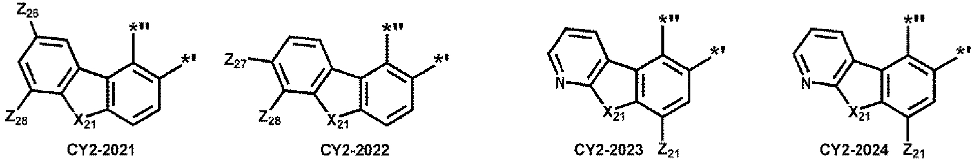

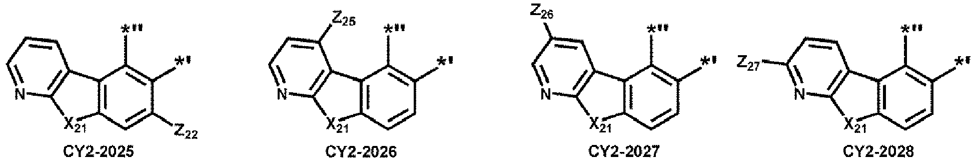

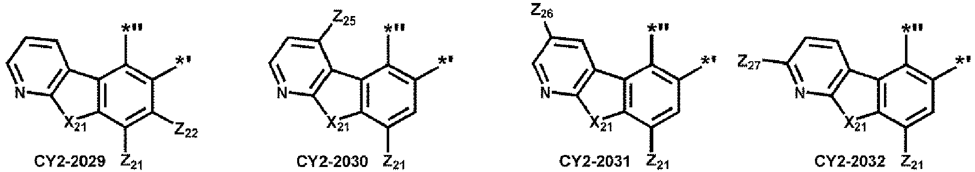

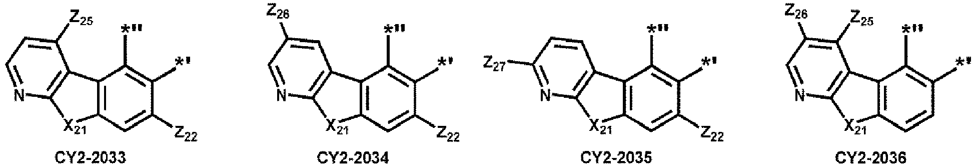

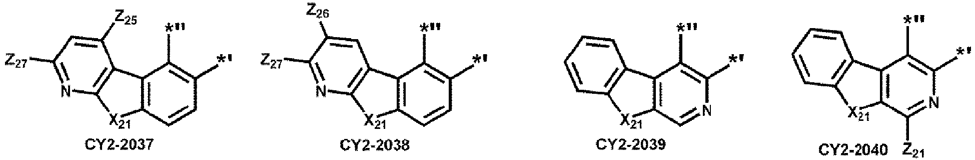

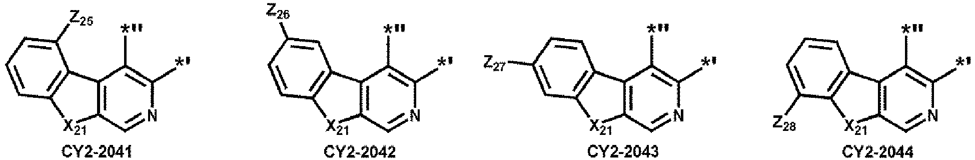

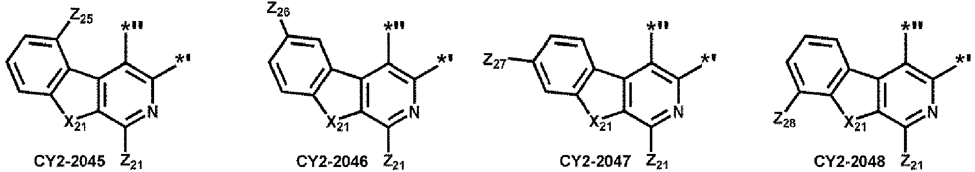

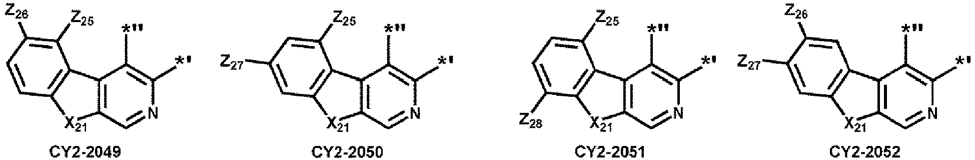

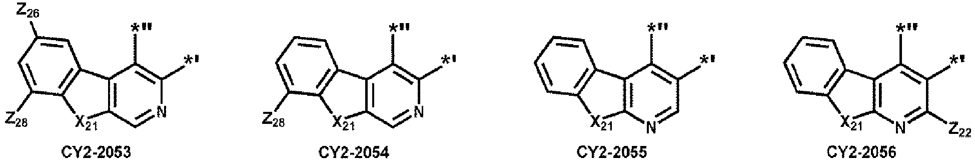

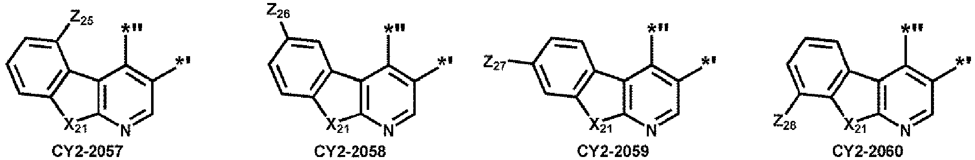

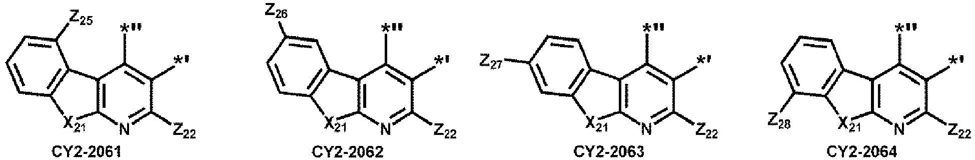

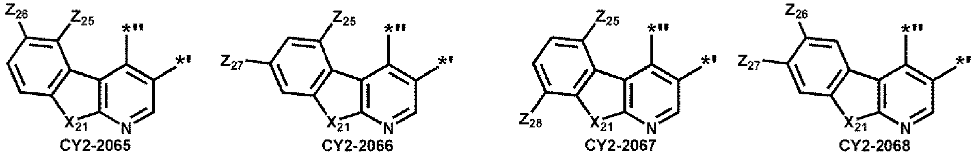

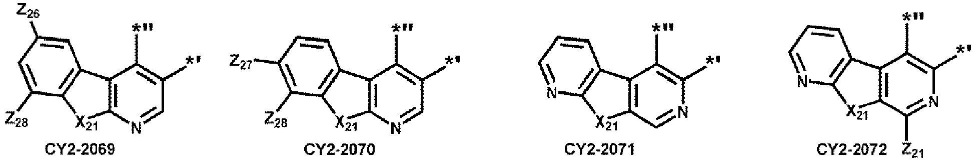

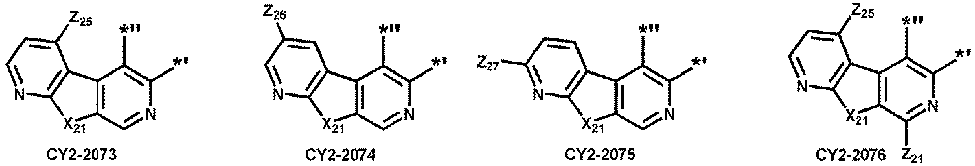

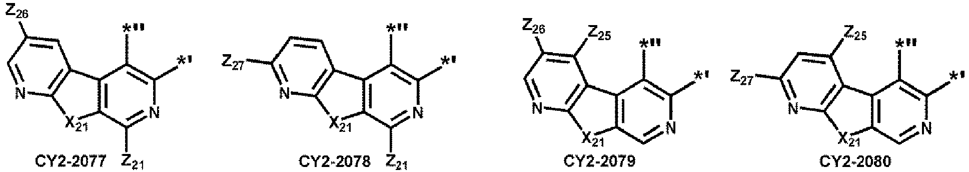

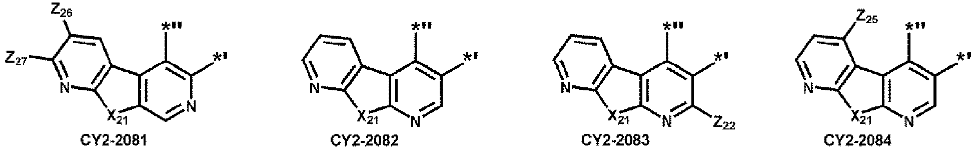

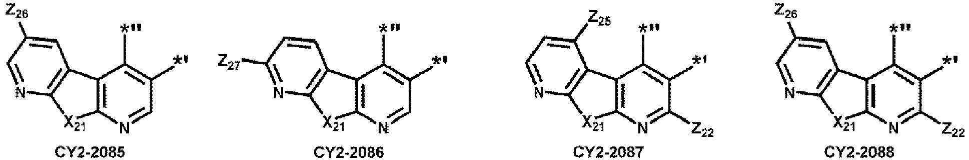

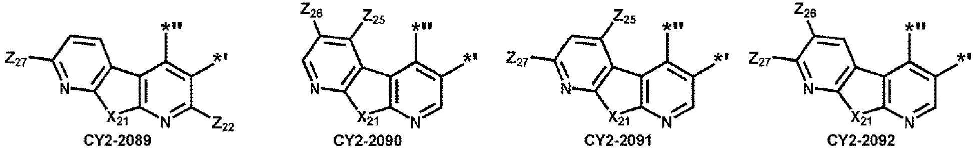

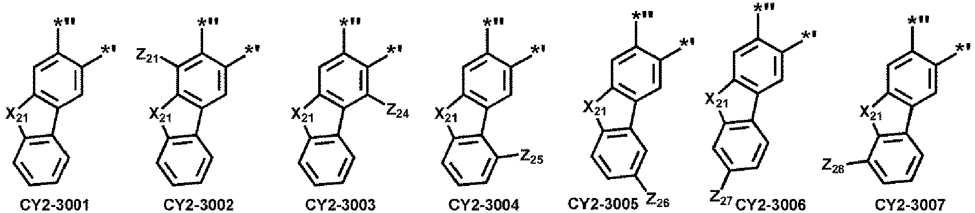

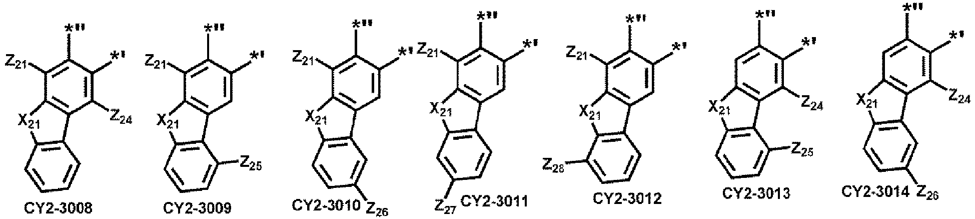

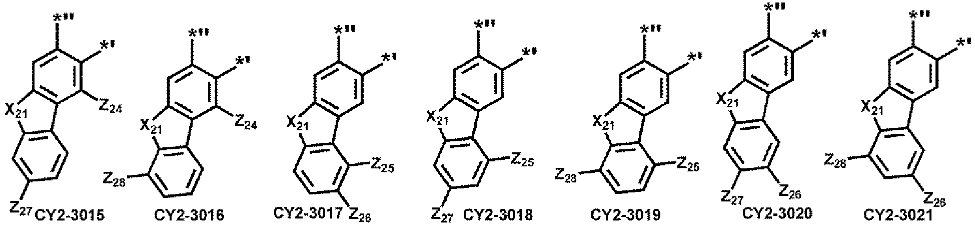

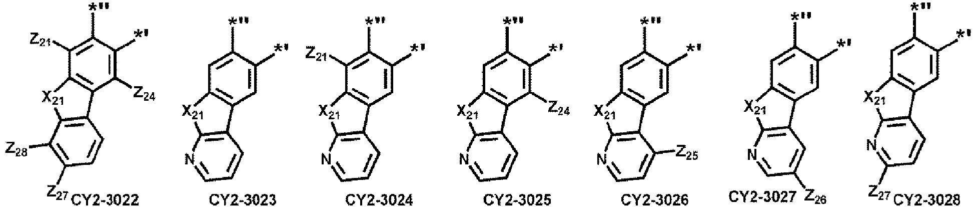

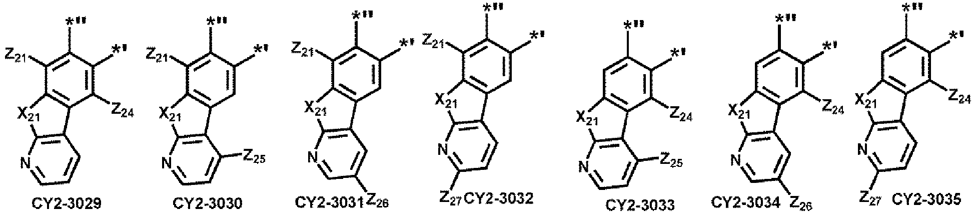

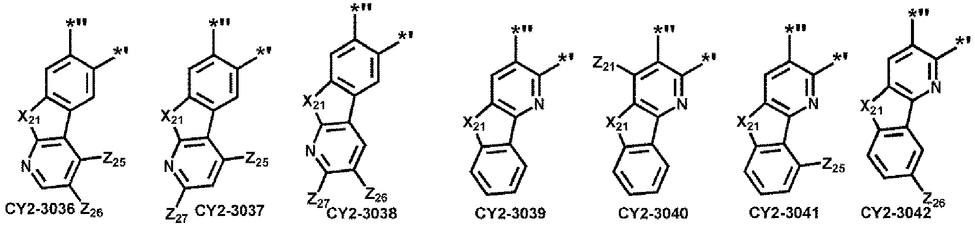

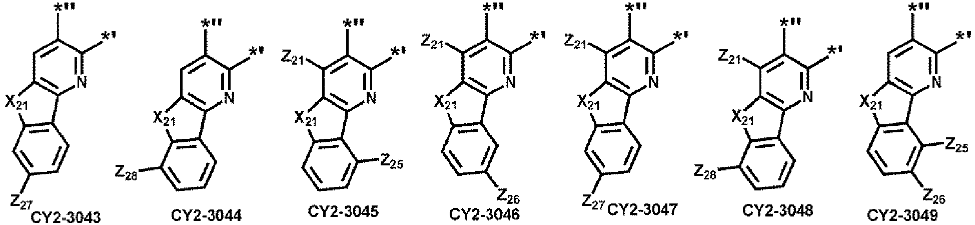

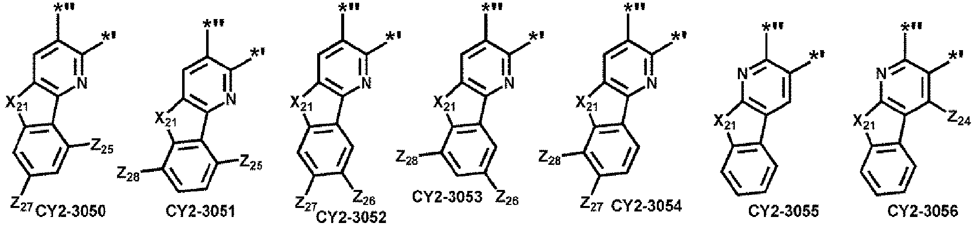

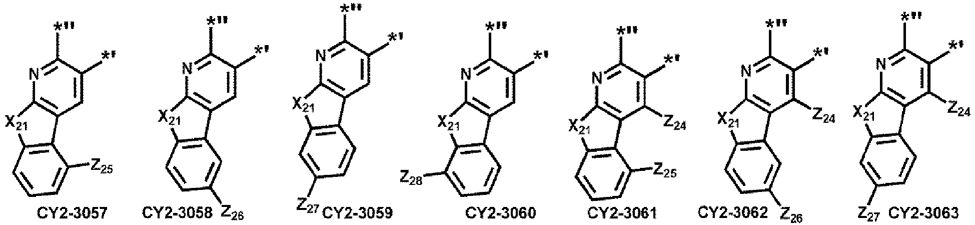

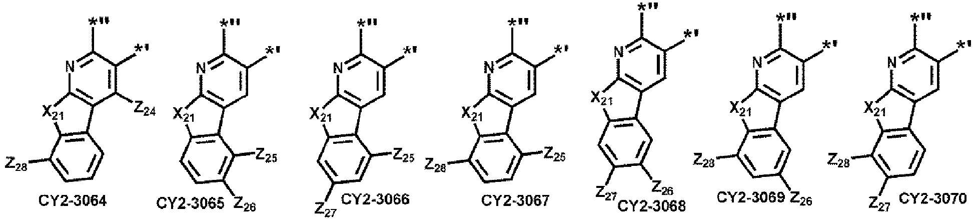

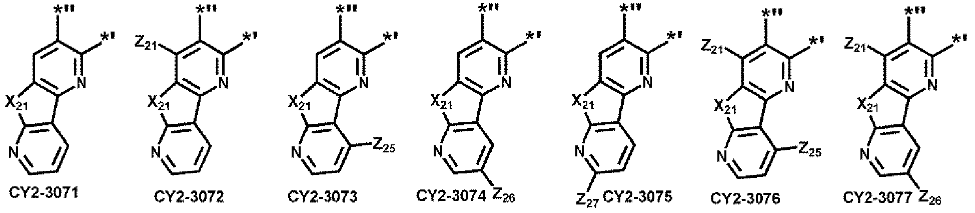

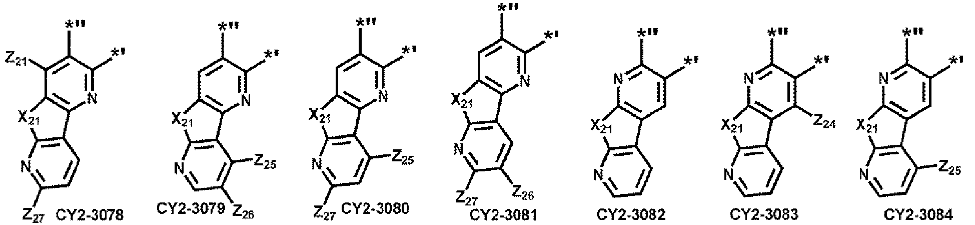

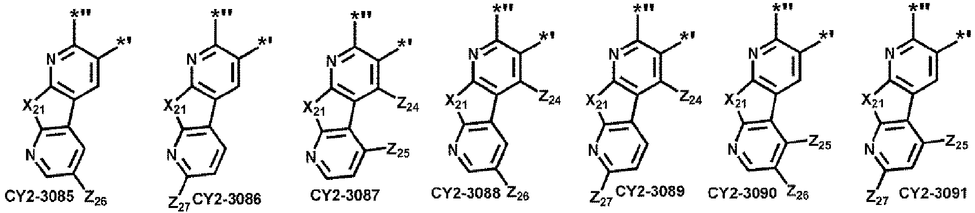

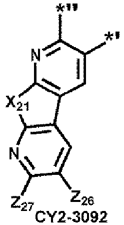

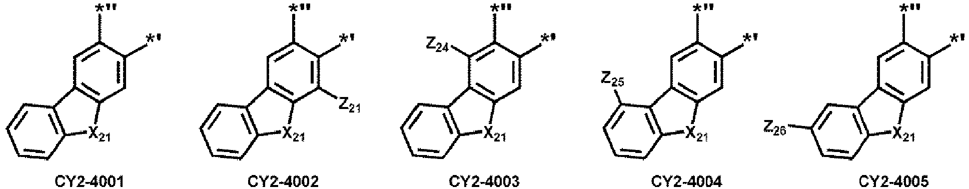

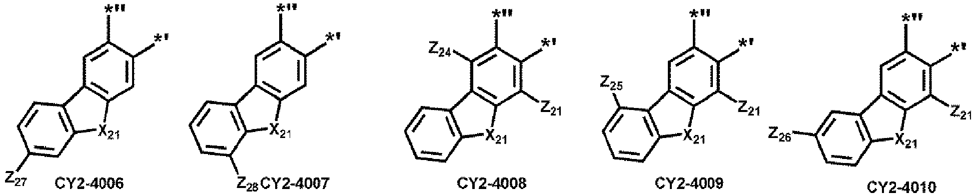

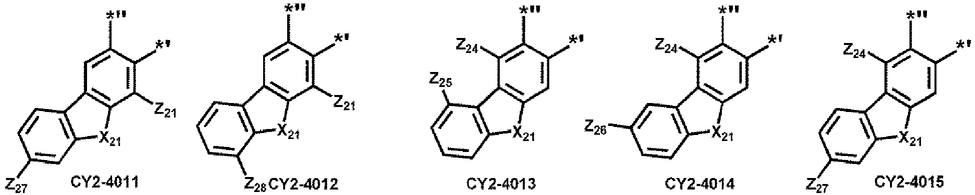

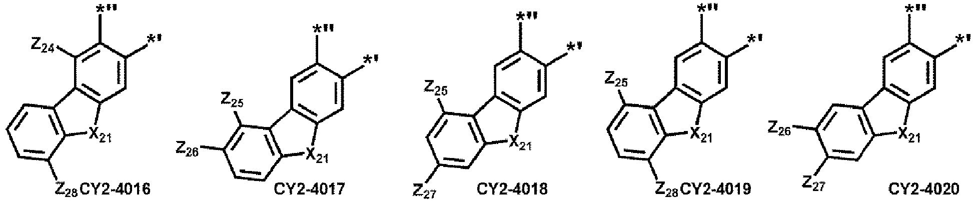

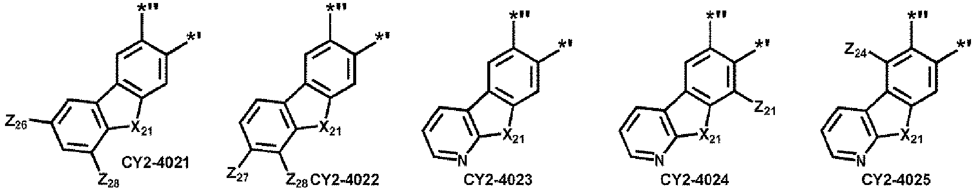

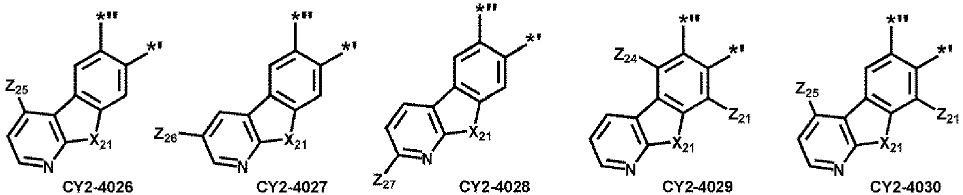

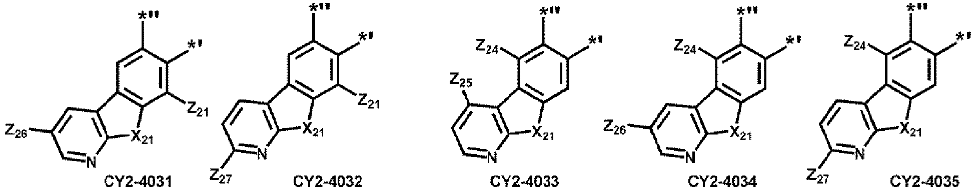

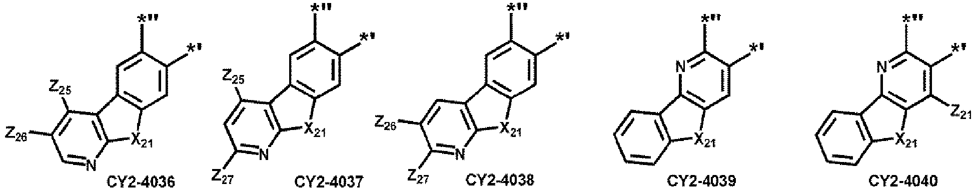

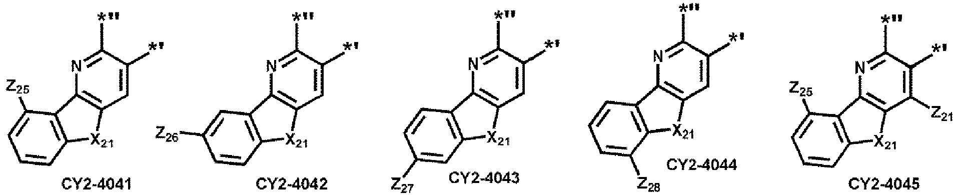

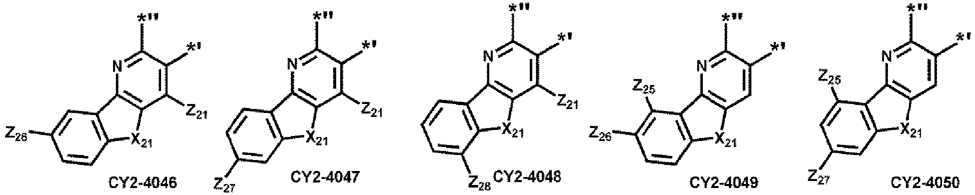

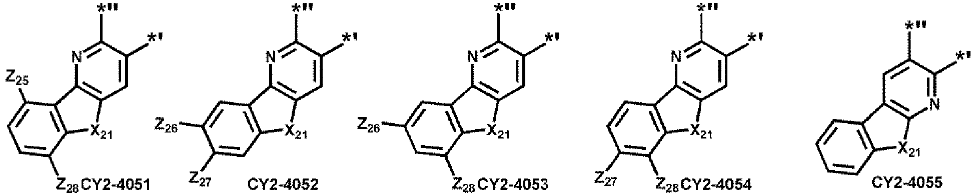

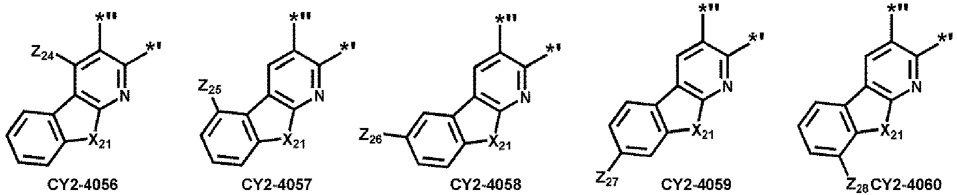

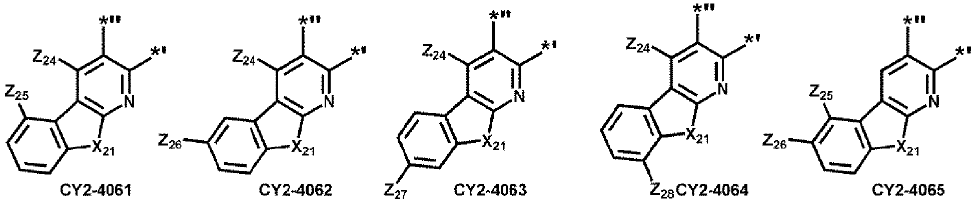

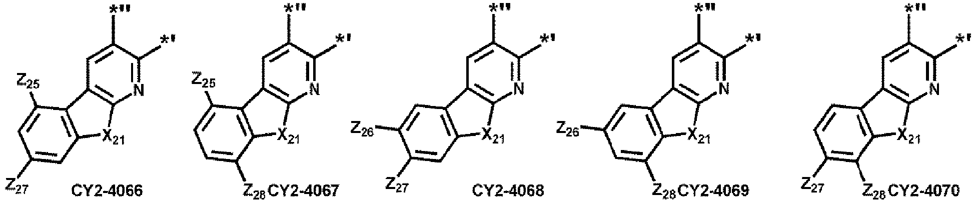

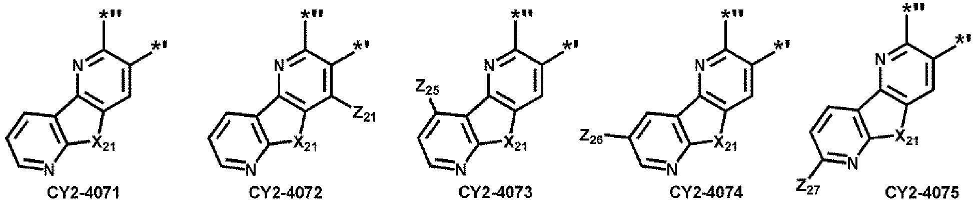

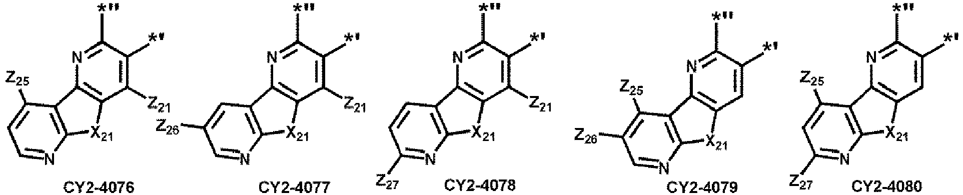

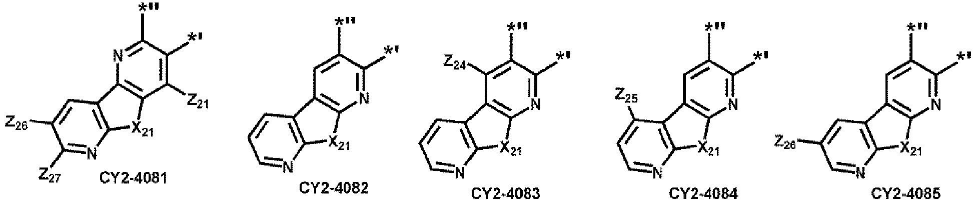

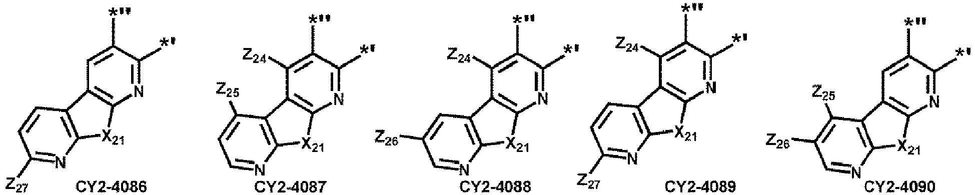

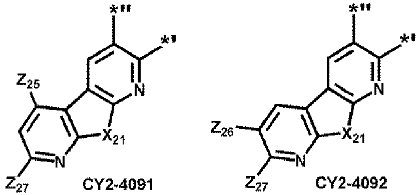

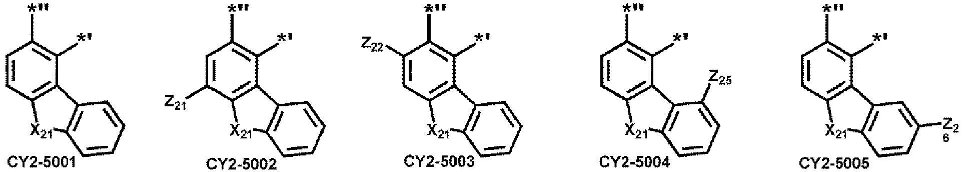

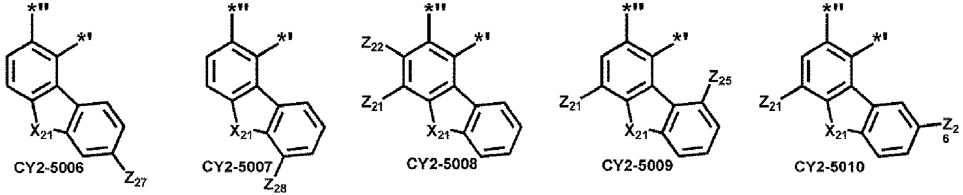

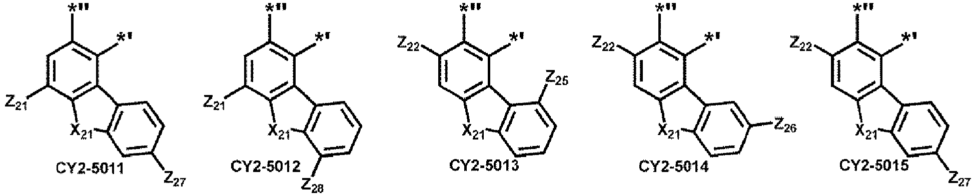

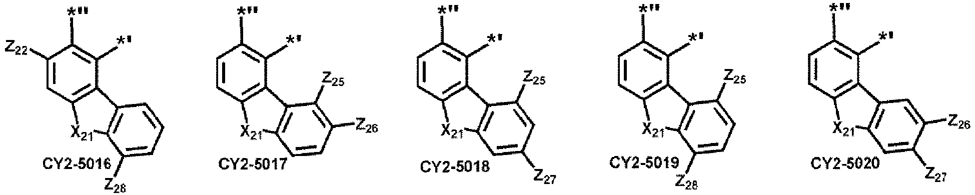

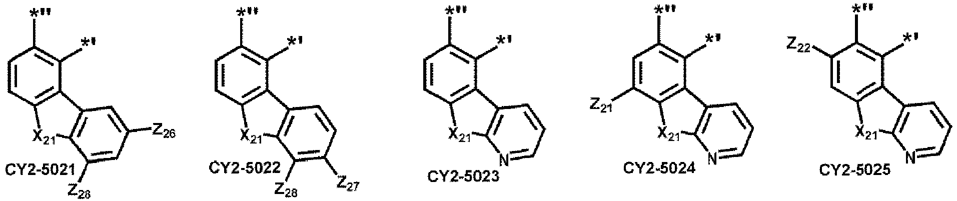

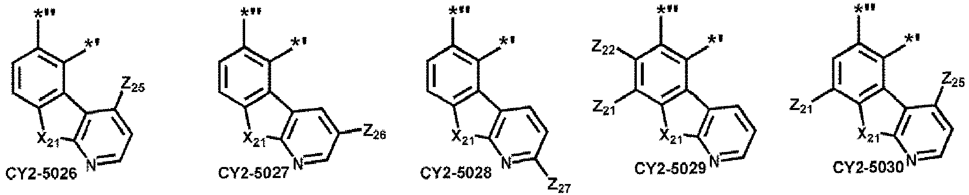

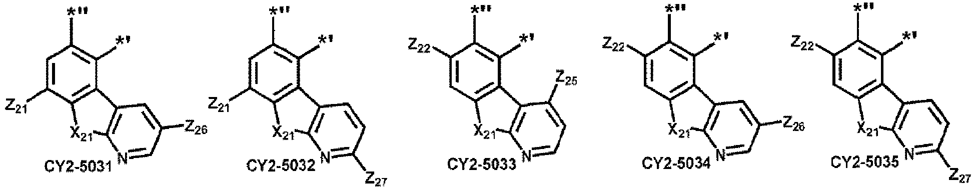

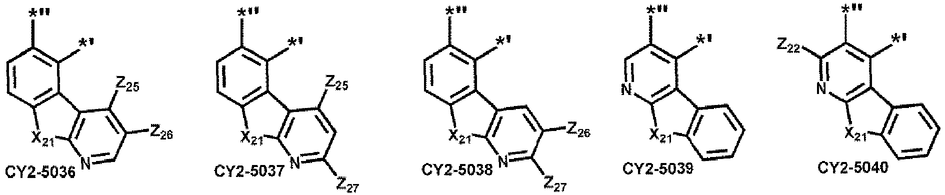

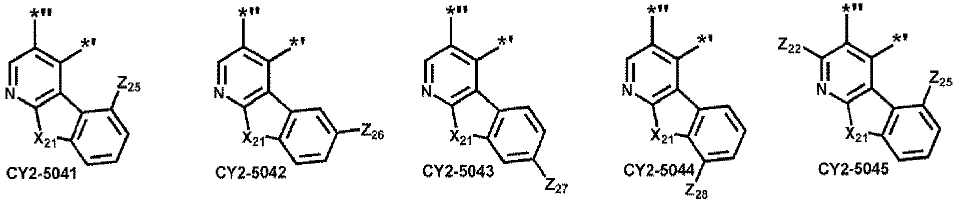

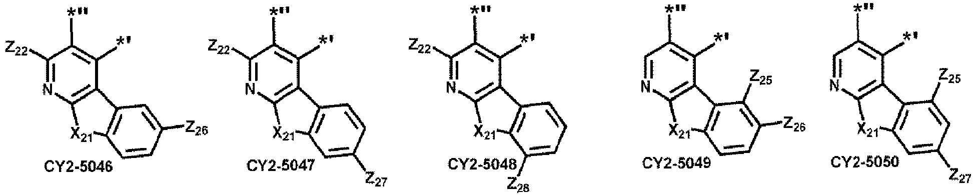

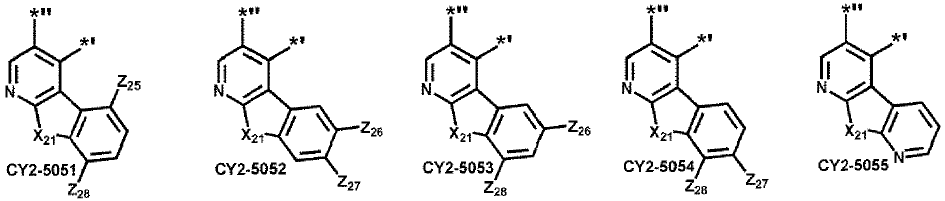

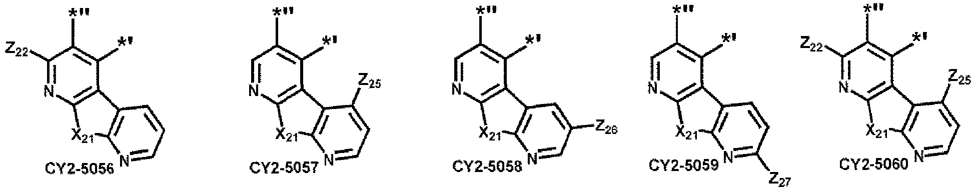

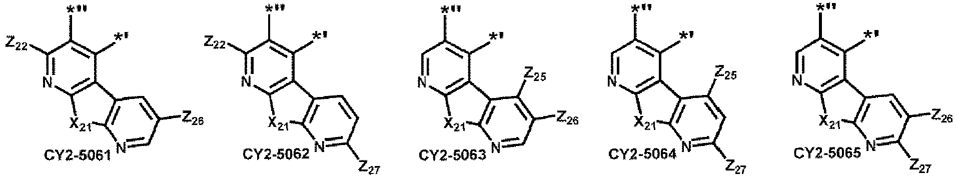

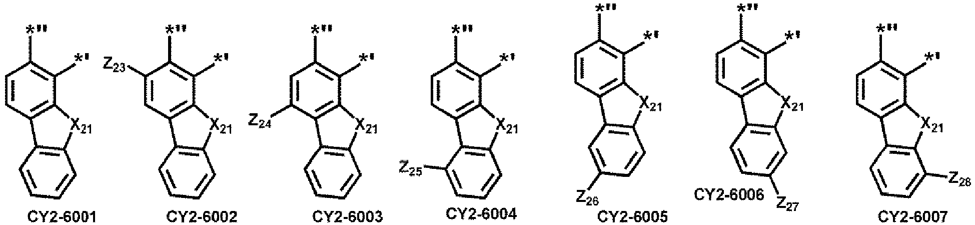

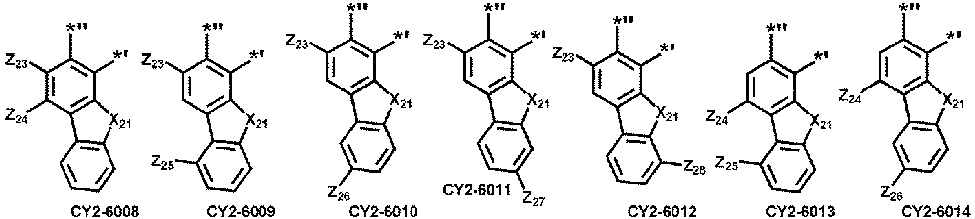

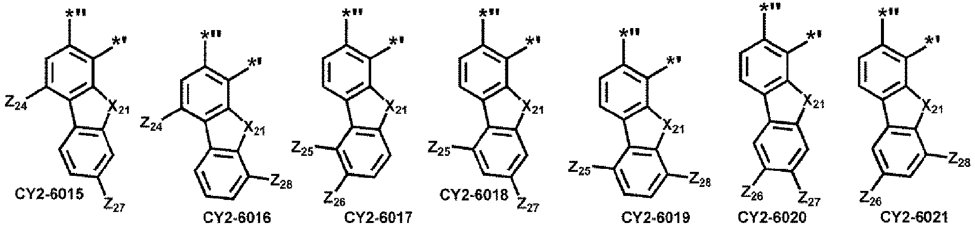

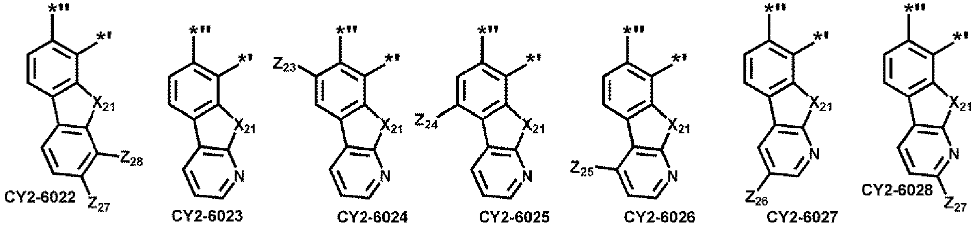

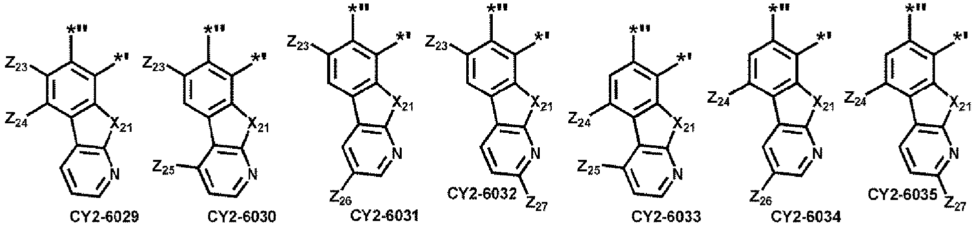

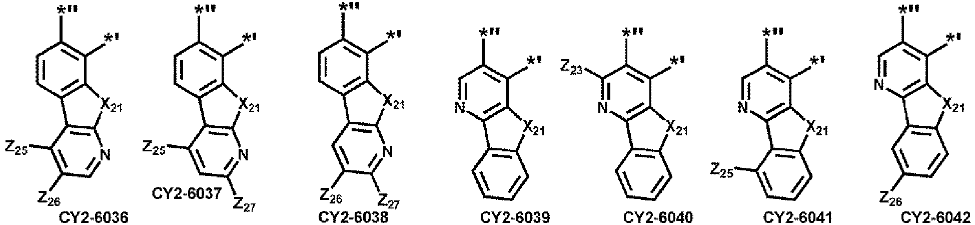

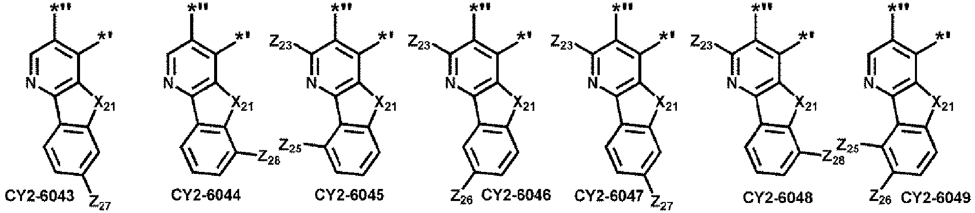

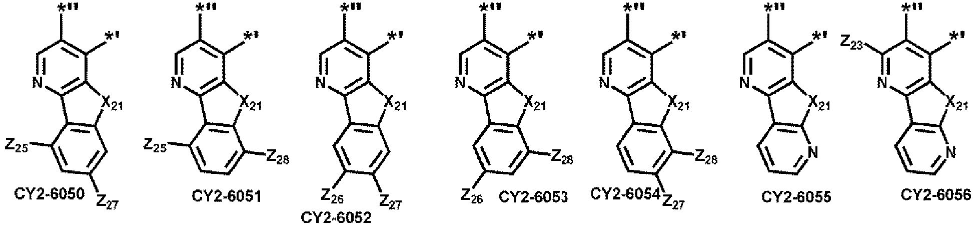

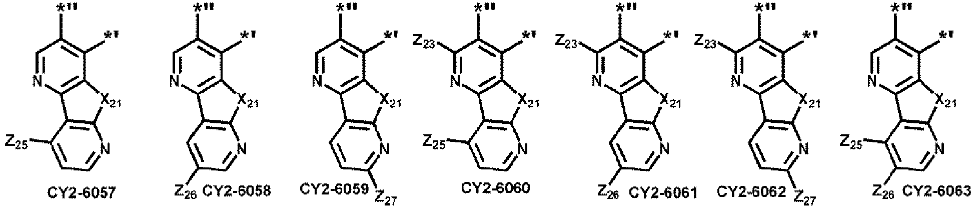

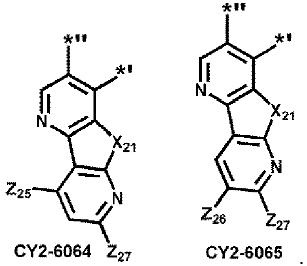

In Formulae CY2-1001 to CY2-1141, CY2-2001 to CY2-2092, CY2-3001 to CY2-3092, CY2-4001 to CY2-4092, CY2-5001 to CY2-5065 and CY2-6001 to CY2-6065,
X₂₁ may each be the same as described above,
Z₂₁ to Z₂₈ may each be the same as defined in connection with Z₂, provided that each of Z₂₁ to Z₂₈ are not hydrogen,
*" indicates a binding site to ring CY₁ in Formula 2A, and
*' indicates a binding site to M in Formula 1.

In one or more embodiments, a group represented by 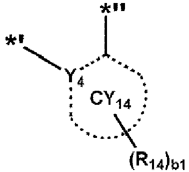 in Formula 2B may be a group represented by one of Formulae CY14-1 to CY14-64:

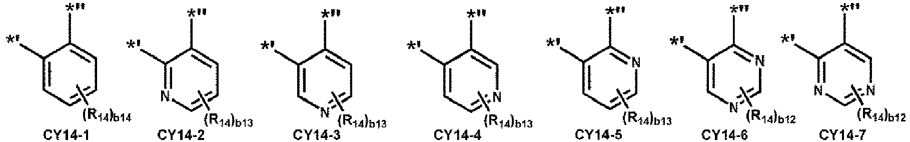

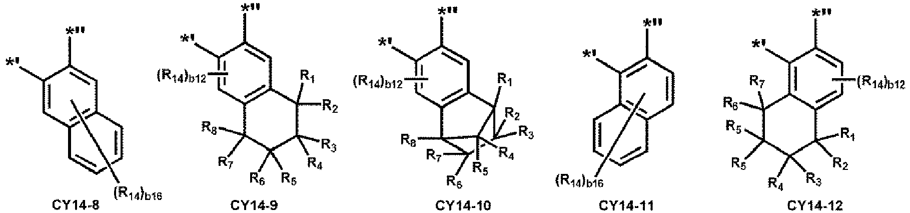

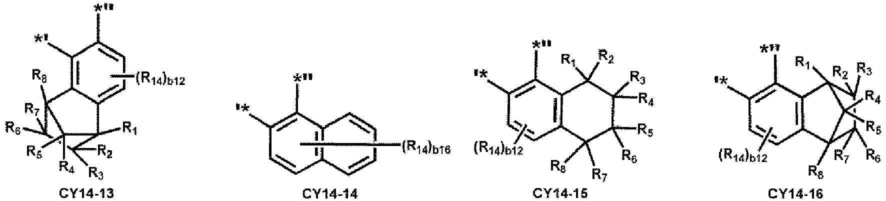

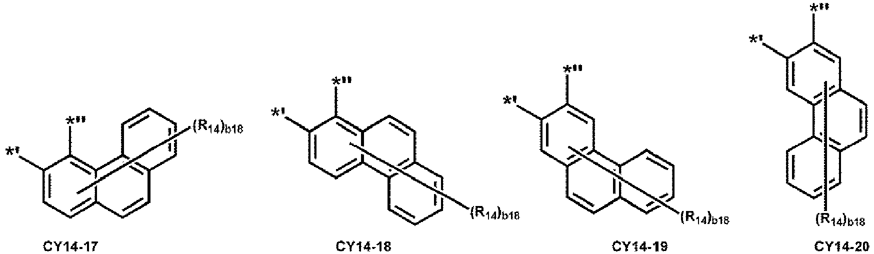

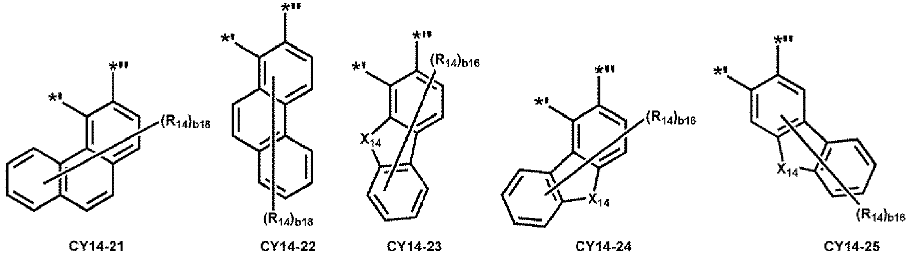

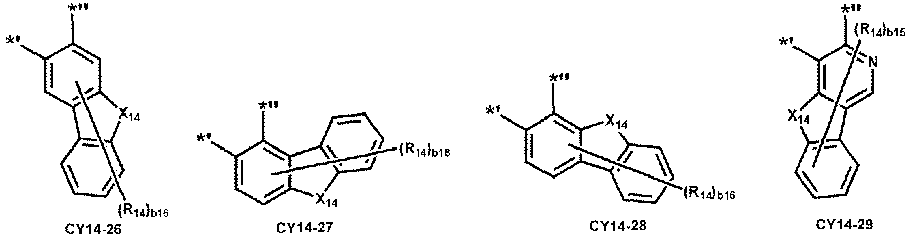

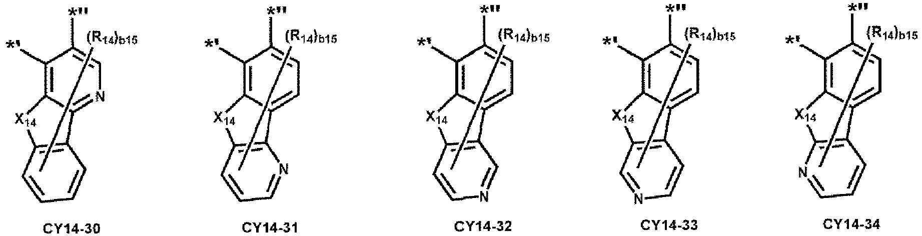

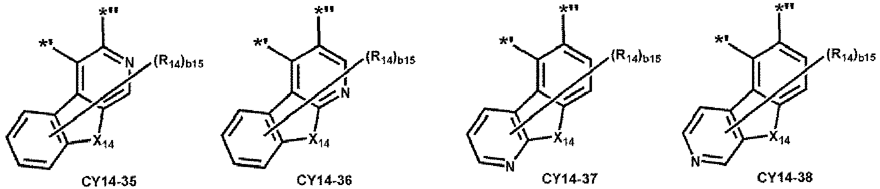

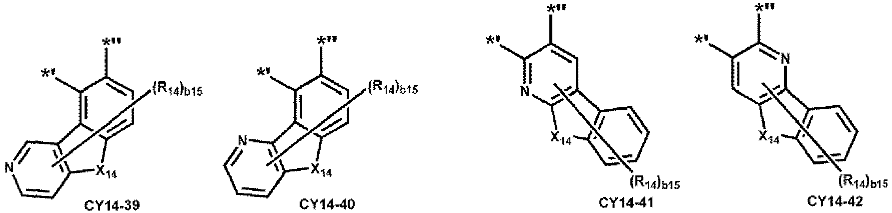

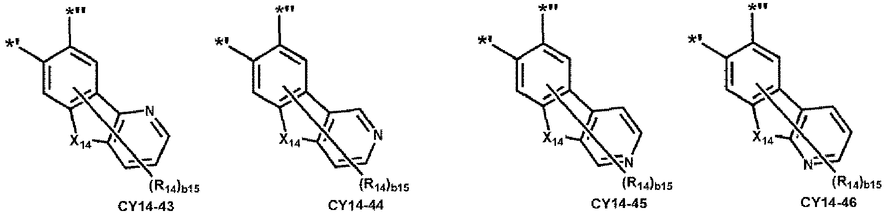

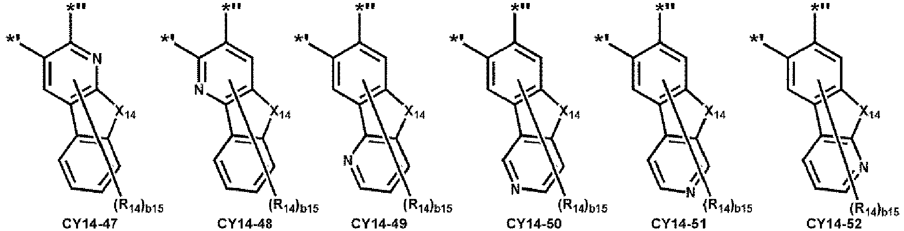

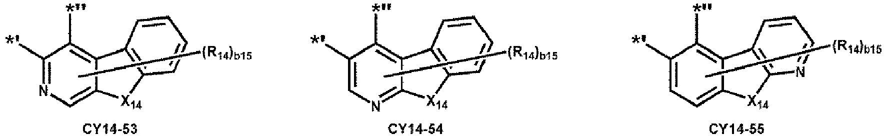

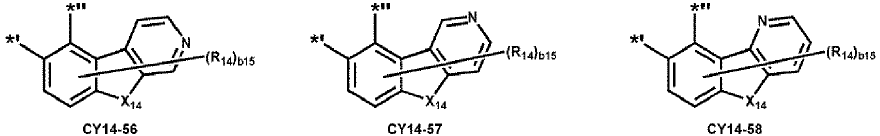

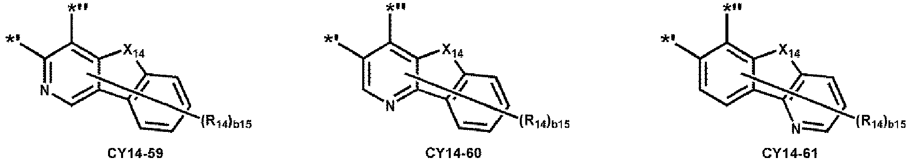

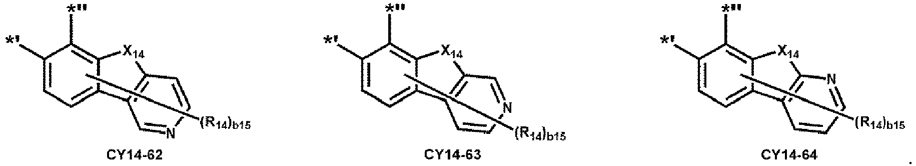

In Formulae CY14-1 to CY14-64,
R₁₄ may be the same as described above,
X₁₄ may be C(R₁)(R₂), N(R₁), O, S, or Si(R₁)(R₂),
R₁ to R₈ may each be the same as defined in connection with R₁₄ in the present specification,
b18 may be an integer from 0 to 8,
b16 may be an integer from 0 to 6,
b15 may be an integer from 0 to 5,
b14 may be an integer from 0 to 4,
b13 may be an integer from 0 to 3,
b12 may be an integer from 0 to 2,
*" indicates a binding site to a carbon atom of a neighboring pyridine ring in Formula 2B, and
*' indicates a binding site to M in Formula 1.

In one or more embodiments, a group represented by 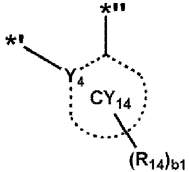 in Formula 2B may be a group represented by one of Formulae CY14(1) to CY14(63):

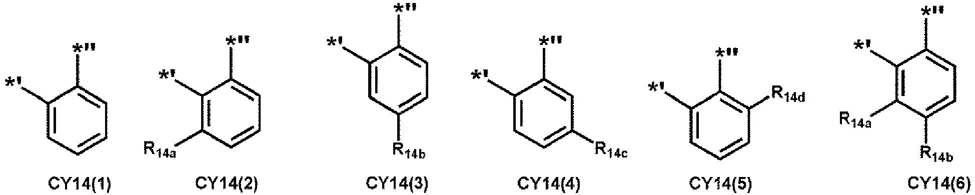

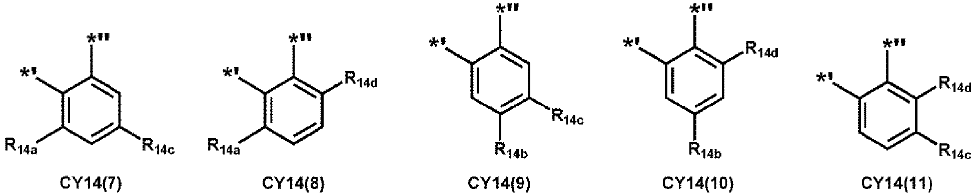

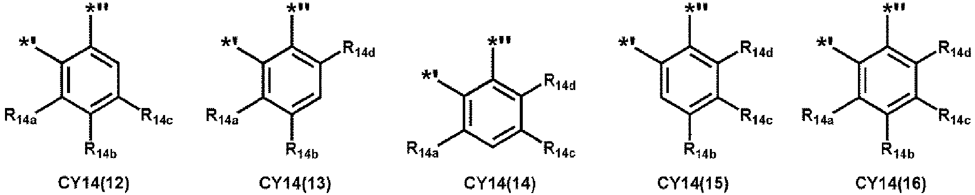

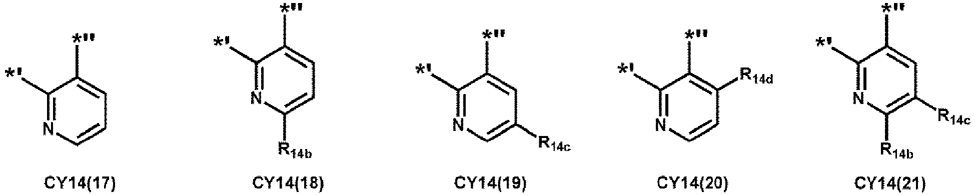

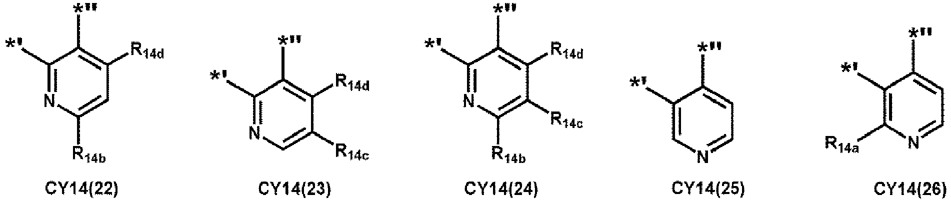

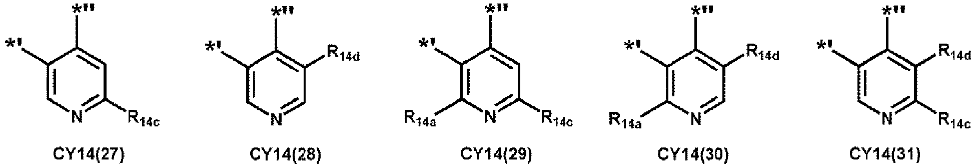

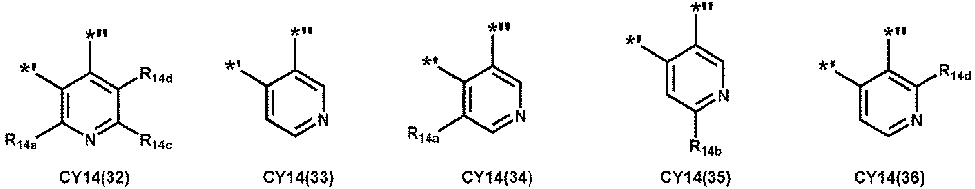

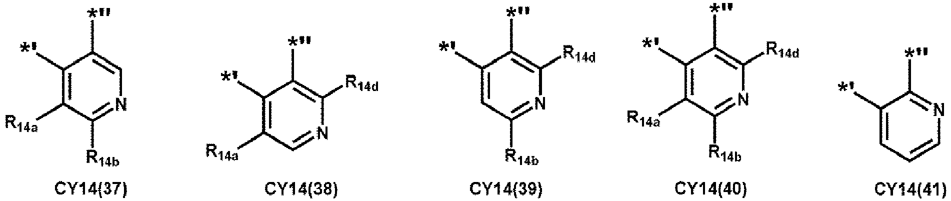

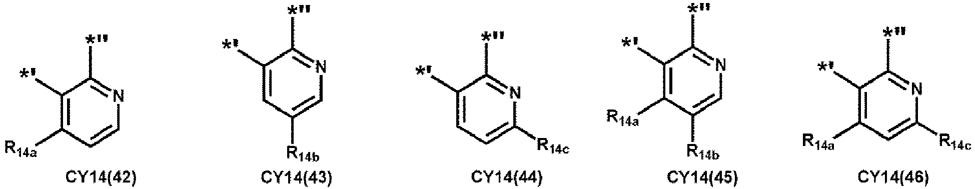

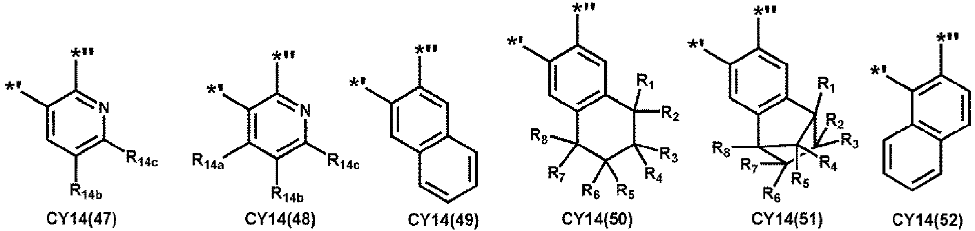

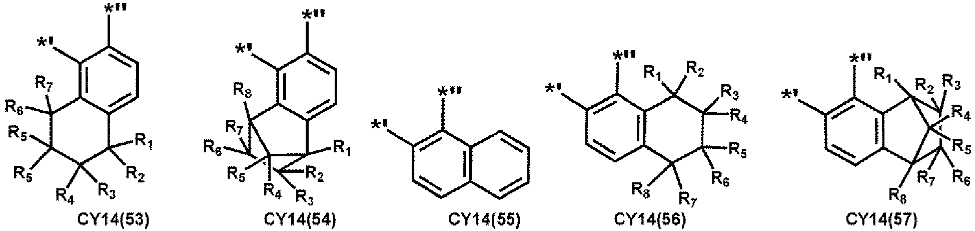

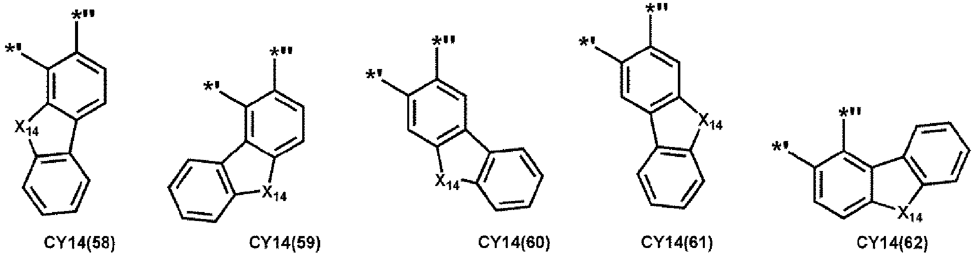

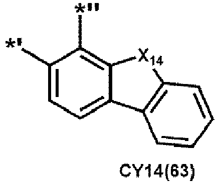

In Formulae CY14(1) to CY14(63),
R₁₄ₐ to R_{14d} may each the same as defined in connection with R₁₄, wherein each of R₁₄ₐ to R_{14d} may not be hydrogen,
X₁₄ may be C(R₁)(R₂), N(R₁), O, S or Si(R₁)(R₂),
R₁ to R₈ may each the same as defined in connection with R₁₄, wherein
*" indicates a binding site to a carbon atom of a neighboring pyridine ring in Formula 2B, and
*' indicates a binding site to M in Formula 1.

In one or more embodiments, the organometallic compound may be represented by Formula 1A:

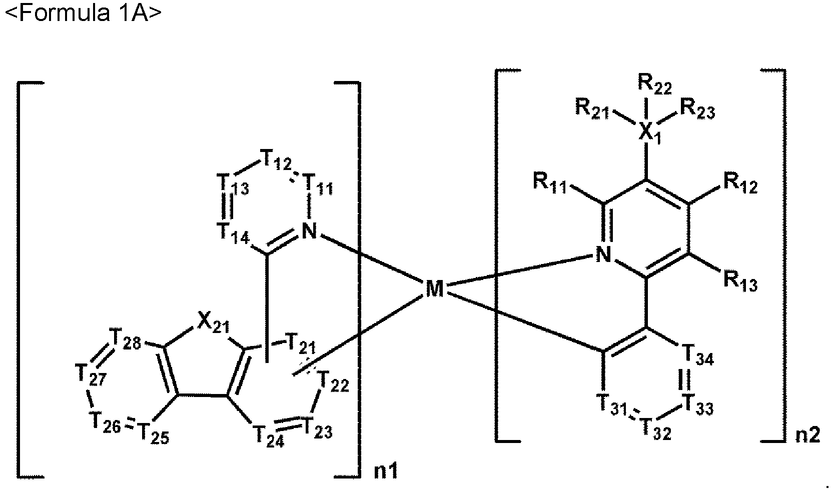

In Formula 1A,
M, n1, n2, X₁, X₂₁, R₂₁ to R₂₃, and R₁₁ to R₁₃ may each be the same as described above,
T₁₁ may be N or C(Z₁₁), T₁₂ may be N or C(Z₁₂), T₁₃ may be N or C(Z₁₃), and T₁₄ may be N or C(Z₁₄), wherein Z₁₁ to Z₁₄ may each be the same as defined in connection with Z₁,
T₂₁ may be N, C(Z₂₁), carbon linked to a neighboring 6-membered ring, or carbon linked to M in Formula 1, T₂₂ may be N, C(Z₂₂), carbon linked to a neighboring 6-membered ring, or carbon linked to M in Formula 1, T₂₃ may be N, C(Z₂₃), carbon linked to a neighboring 6-membered ring, or carbon linked to M in Formula 1, T₂₄ may be N, C(Z₂₄), carbon linked to a neighboring 6-membered ring, or carbon linked to M in Formula 1, T₂₅ may be N or C(Z₂₅), T₂₆ may be N or C(Z₂₆), T₂₇ may be N or C(Z₂₇), and T₂₈ may be N or C(Z₂₈), wherein one of T₂₁ to T₂₄ may be carbon linked to M in Formula 1, one of the remaining T₂₁ to T₂₄ that are not linked to M in Formula 1 may be carbon linked to a neighboring 6-membered ring, and Z₂₁ to Z₂₄ may each be the same as defined in connection with Z₂,
T₃₁ may be N or C(R₁₄ₐ), T₃₂ may be N or C(R_{14b}), T₃₃ may be N or C(R_{14c}), and T₂₄ may be N or C(R_{14d}), wherein R₁₄ₐ to R_{14d} may each be the same as defined in connection with R₁₄,
two or more selected from Z₁₁ to Z₁₄ may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
two or more selected from Z₂₁ to Z₂₈ may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
R₁₂ and R₁₃ may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
two or more selected from R₁₄ₐ to R_{14d} may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,and
R₁₀ₐ may be the same as defined in connection with R₁₄.

Descriptions for Formula 1A may refer to descriptions for Formula 1 in this disclosure.

For example, T₁₃ in Formula 1A may be C(Z₁₃) and Z₁₃ may not be a hydrogen.

In one or more embodiments, the number of silicon (Si) atoms in the organometallic compound represented by Formula 1 may be 1 or 2.

In one or more embodiments, the organometallic compound may be one of Compounds 1 to 1620:

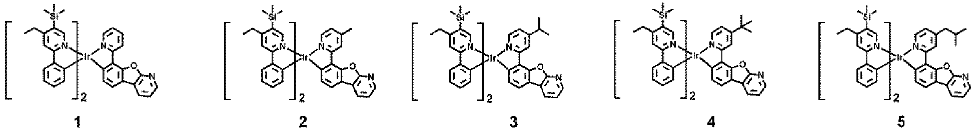

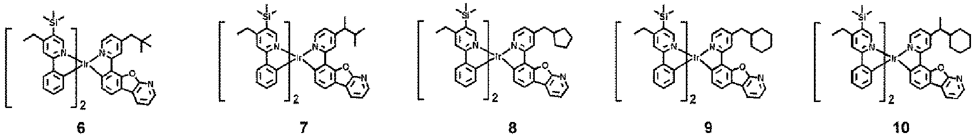

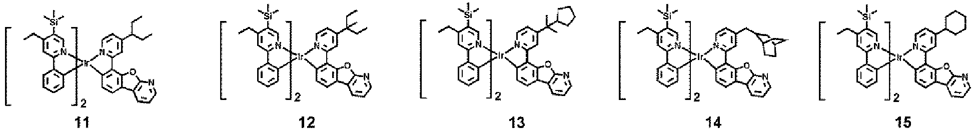

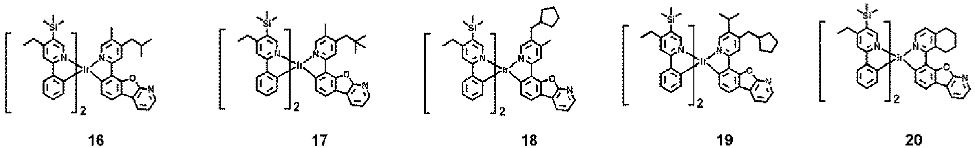

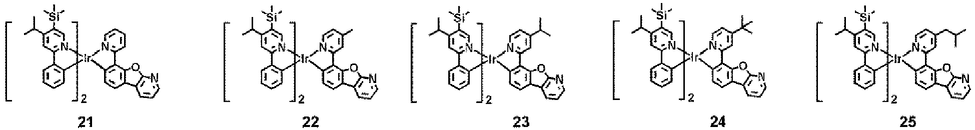

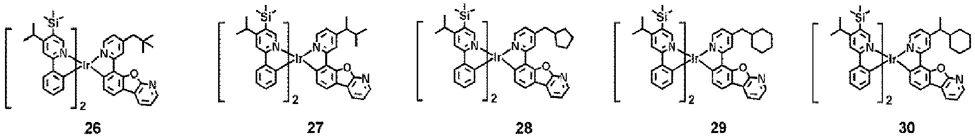

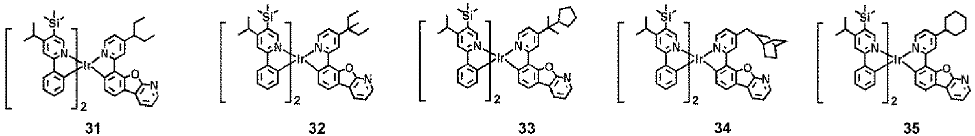

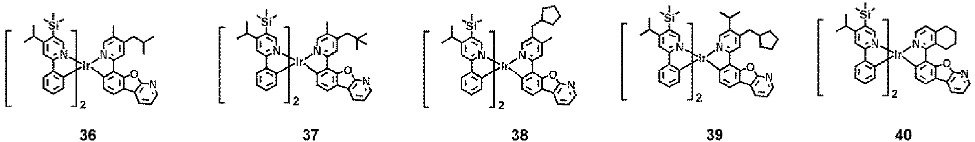

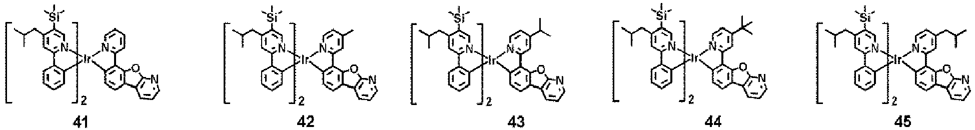

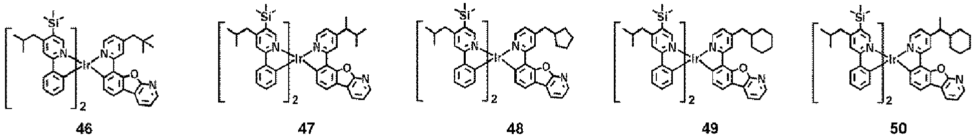

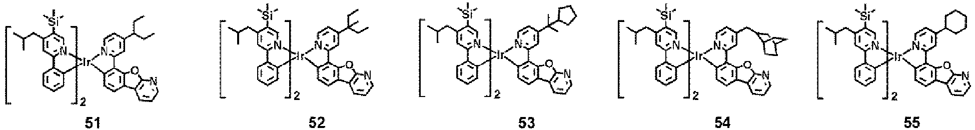

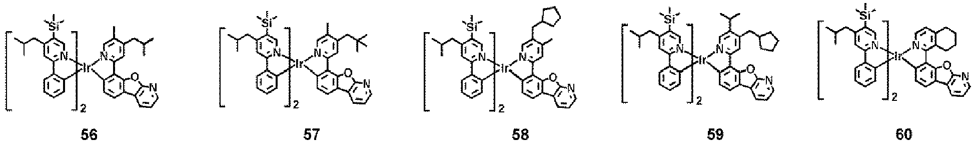

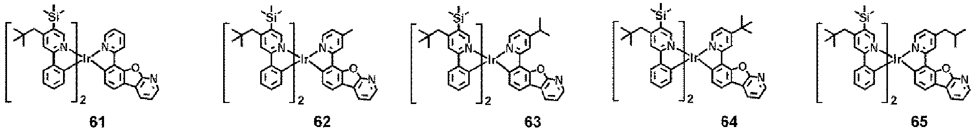

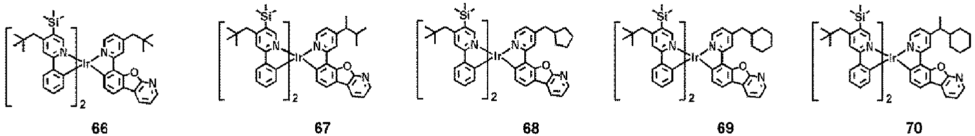

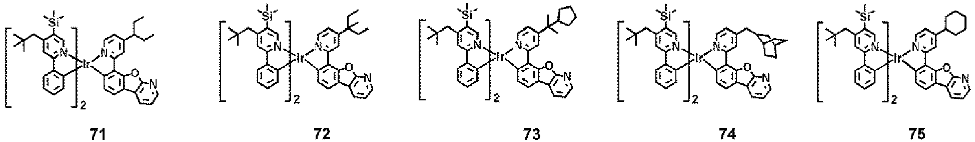

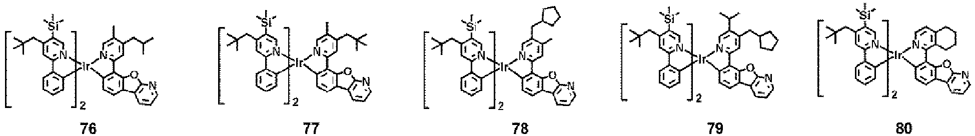

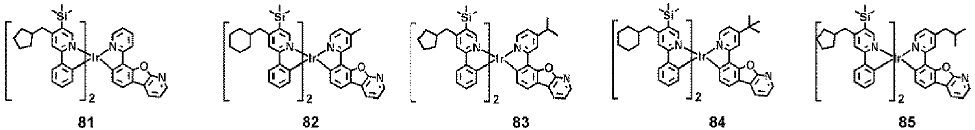

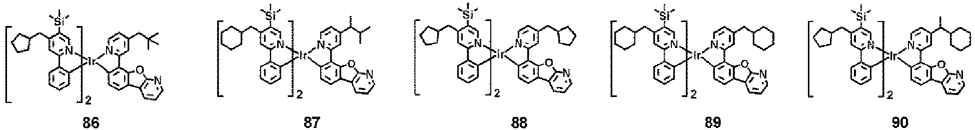

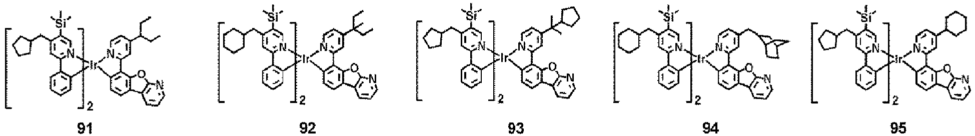

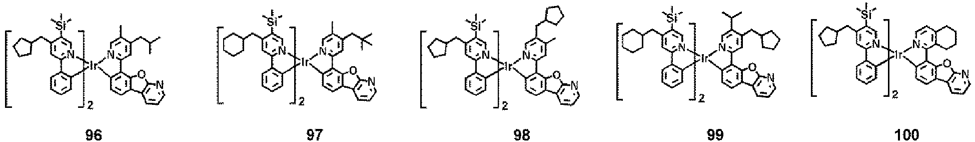

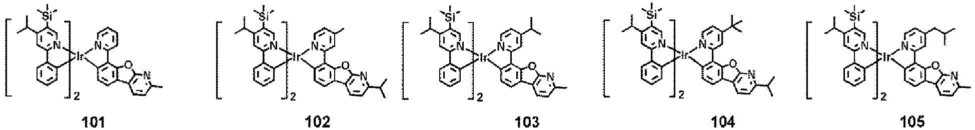

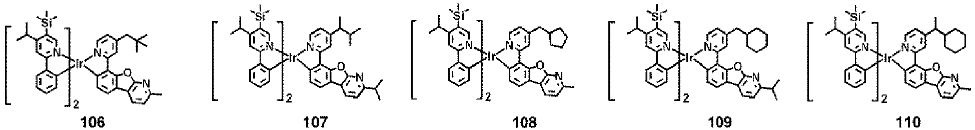

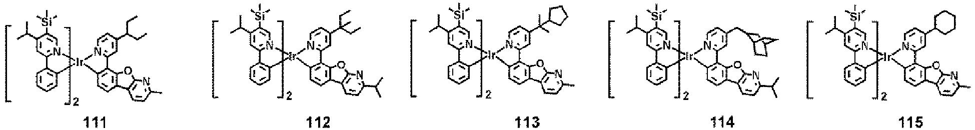

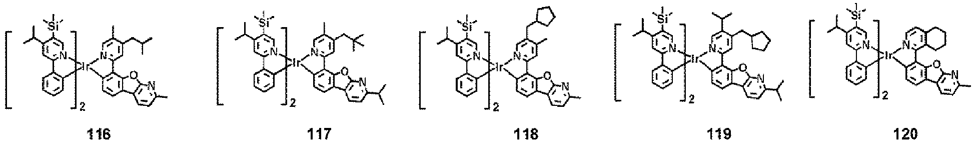

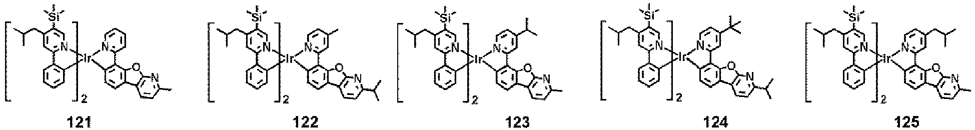

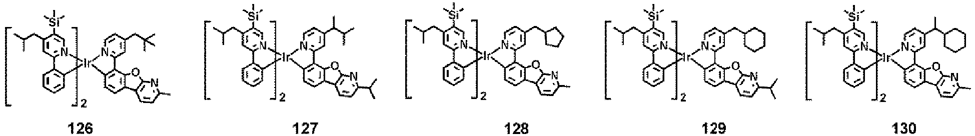

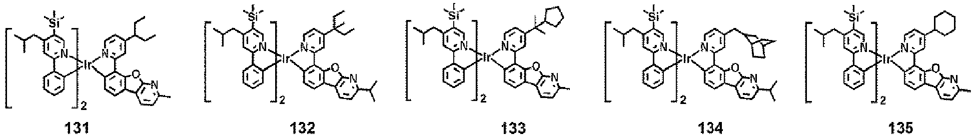

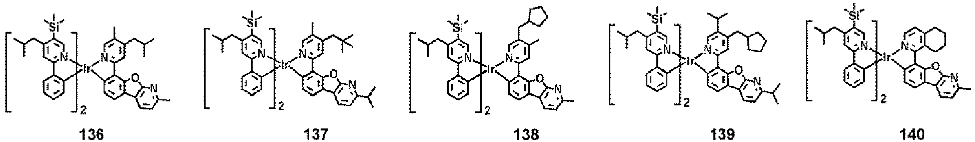

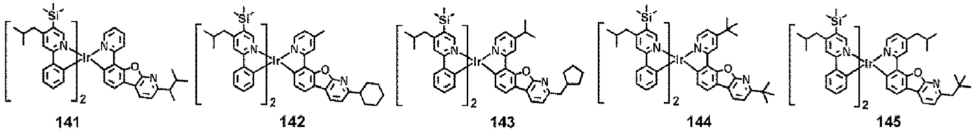

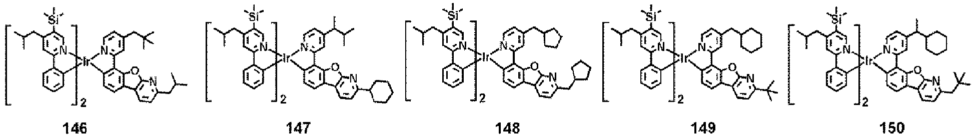

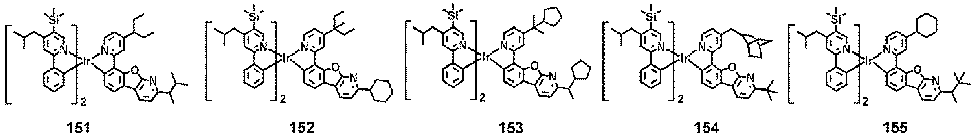

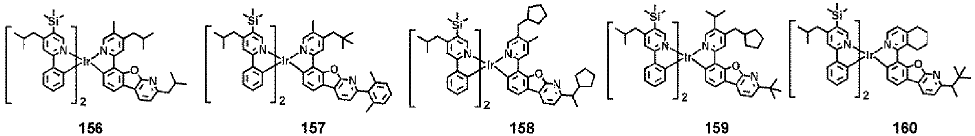

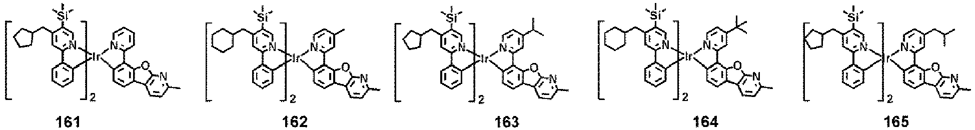

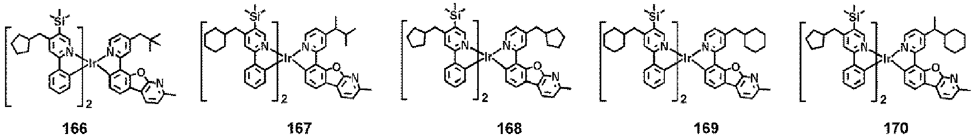

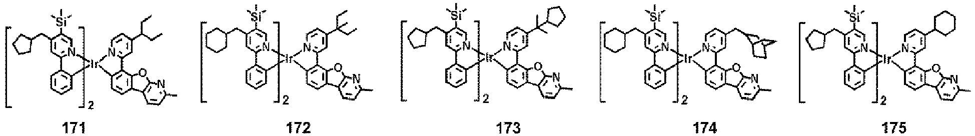

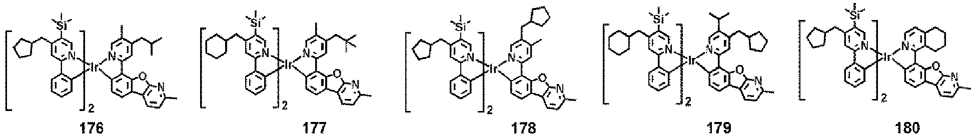

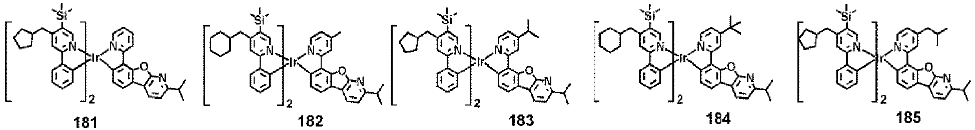

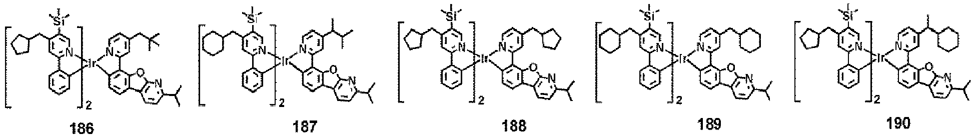

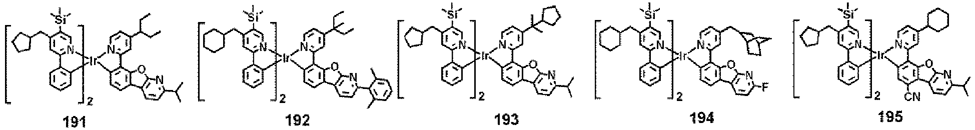

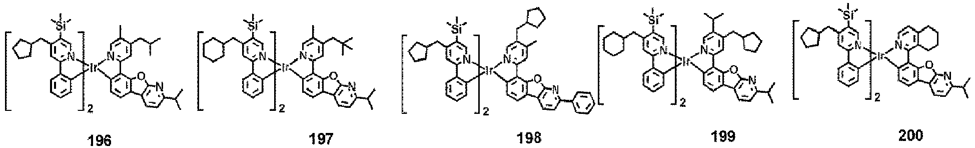

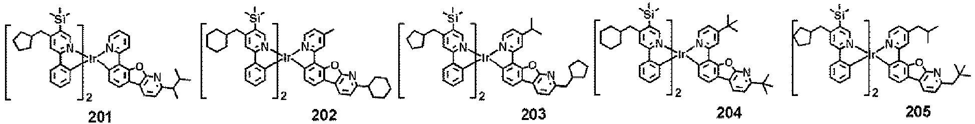

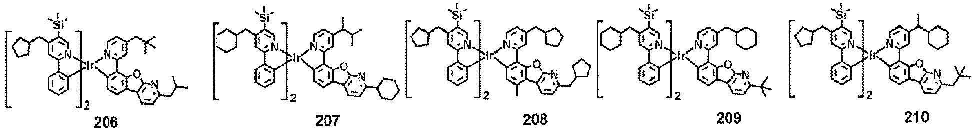

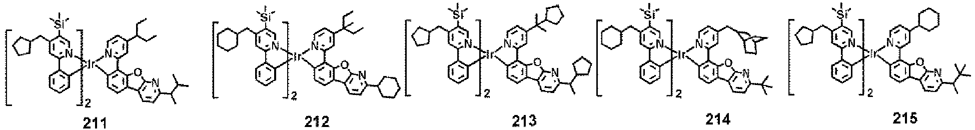

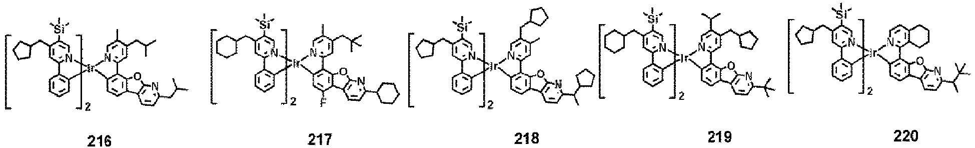

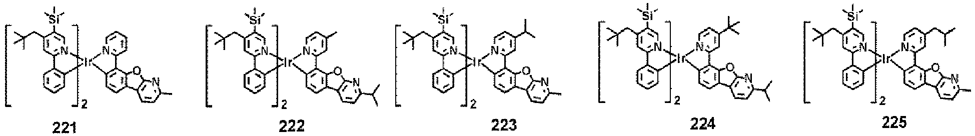

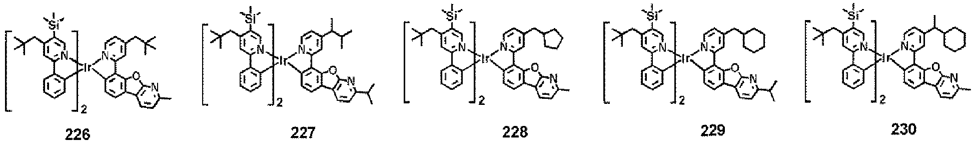

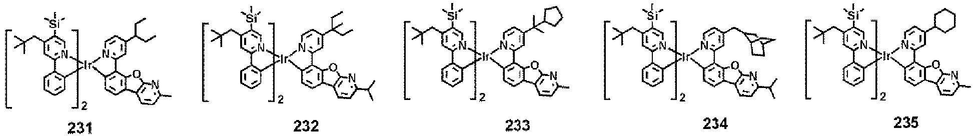

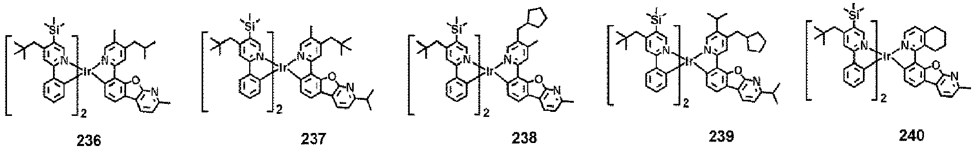

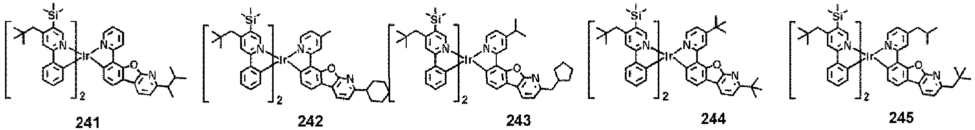

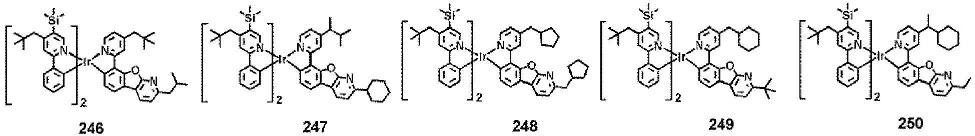

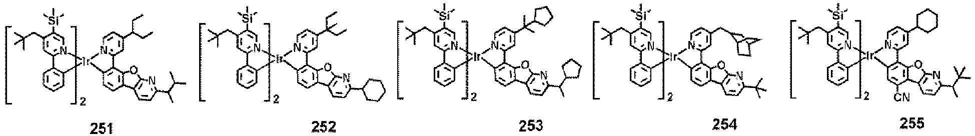

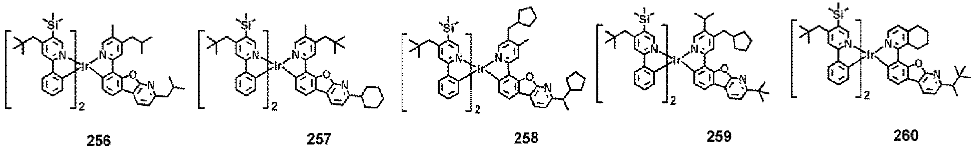

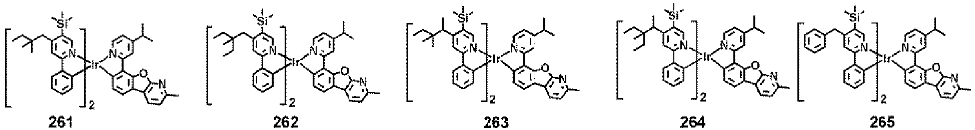

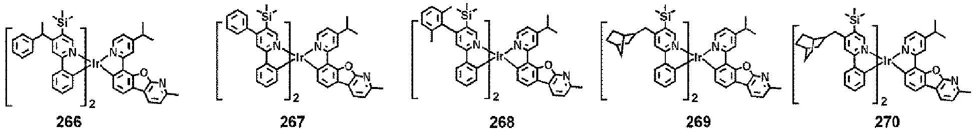

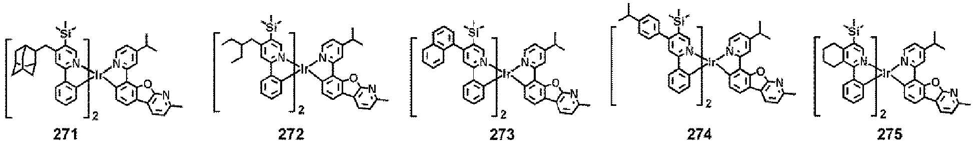

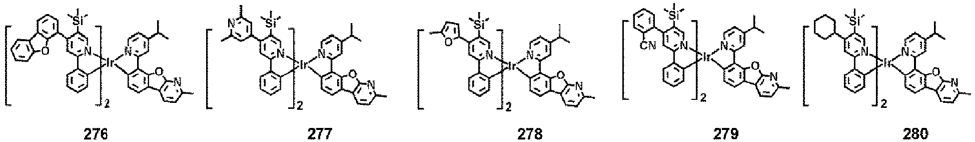

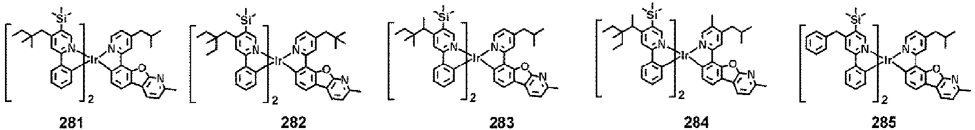

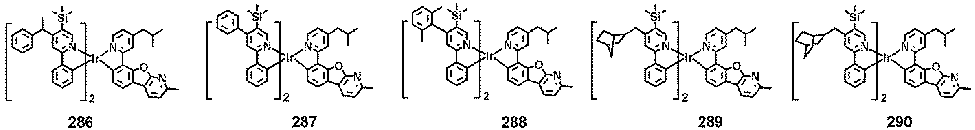

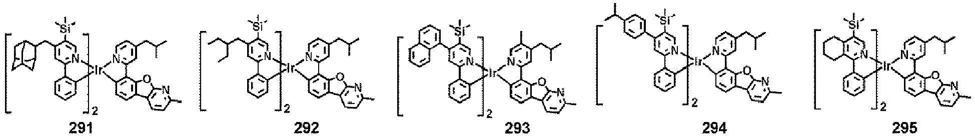

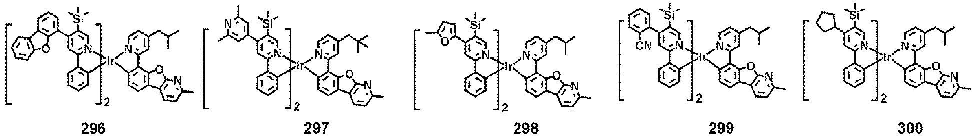

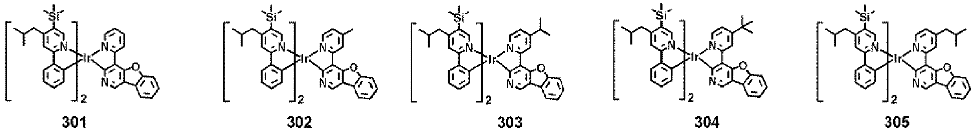

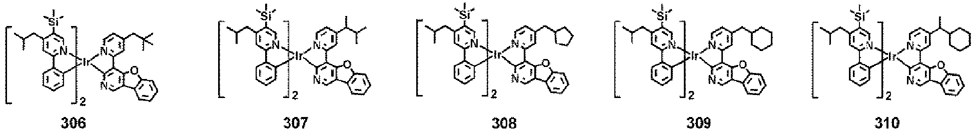

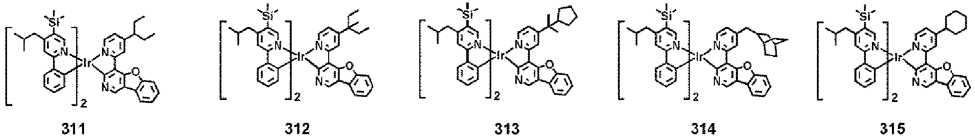

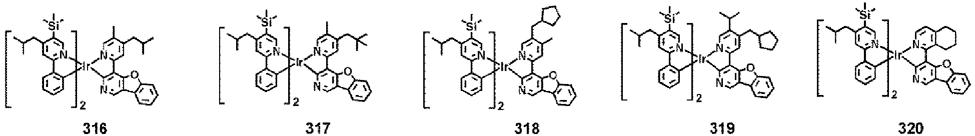

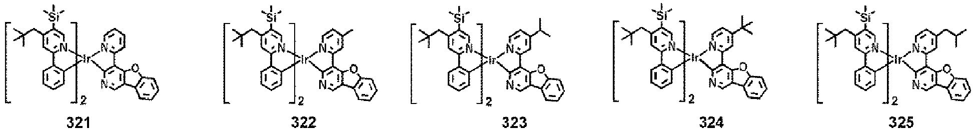

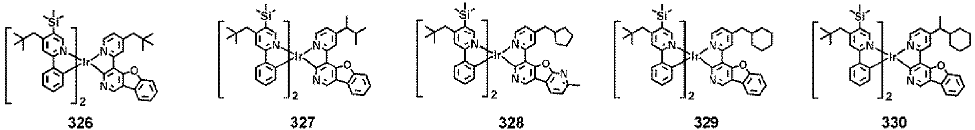

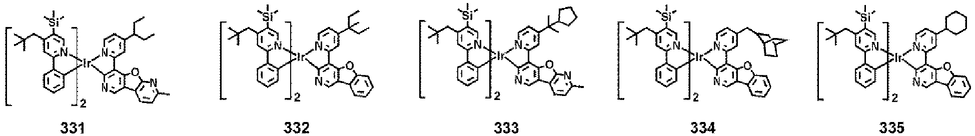

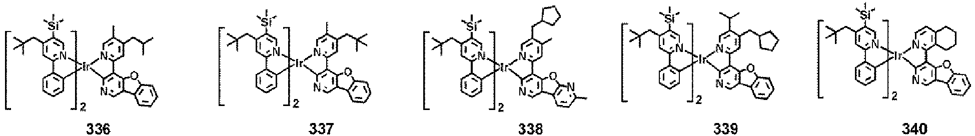

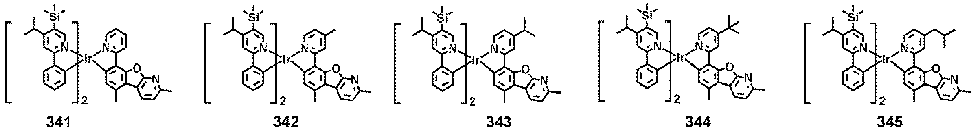

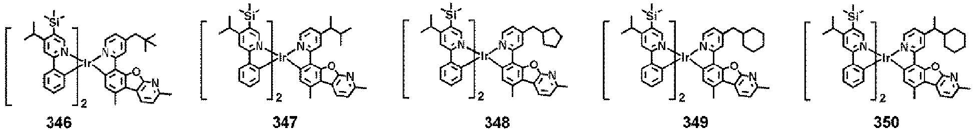

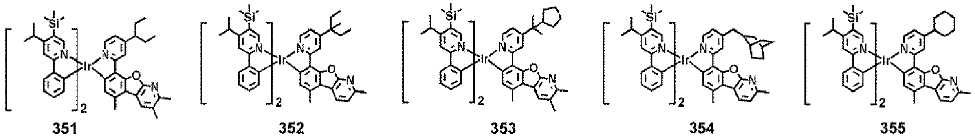

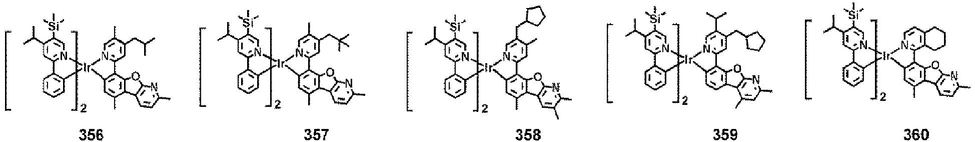

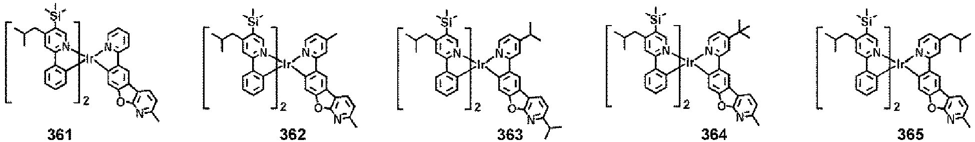

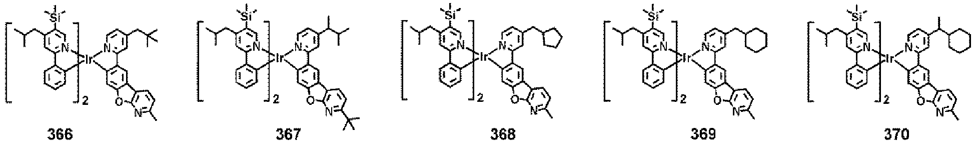

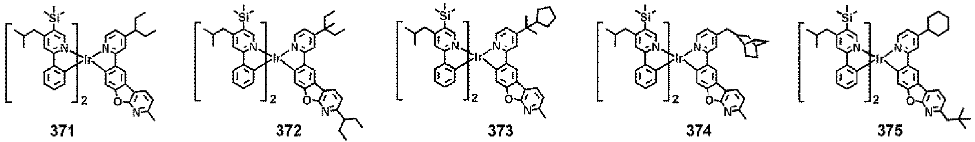

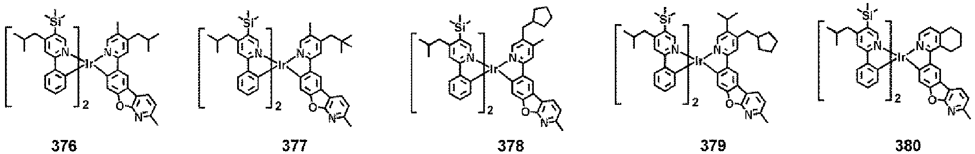

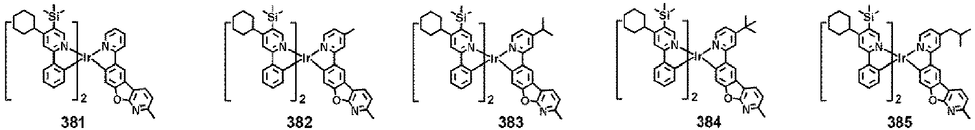

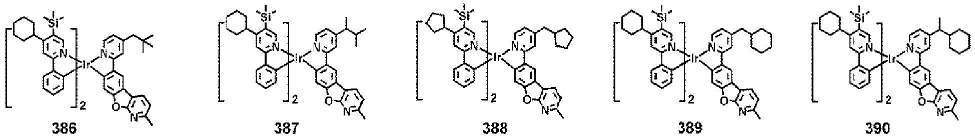

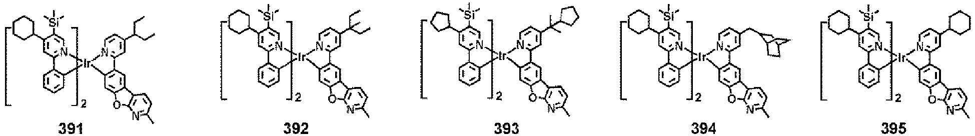

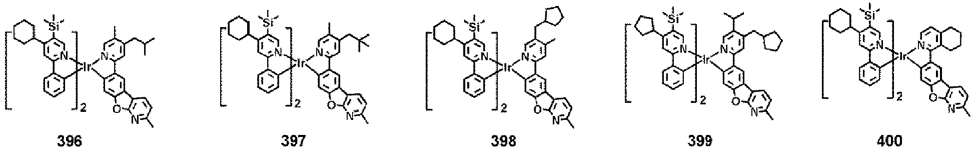

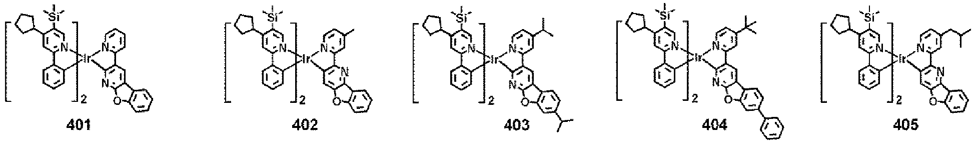

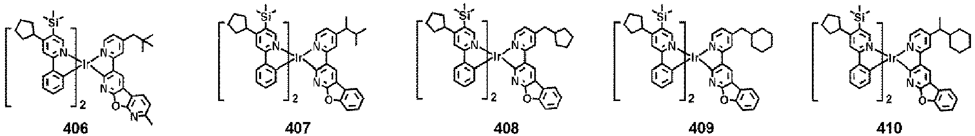

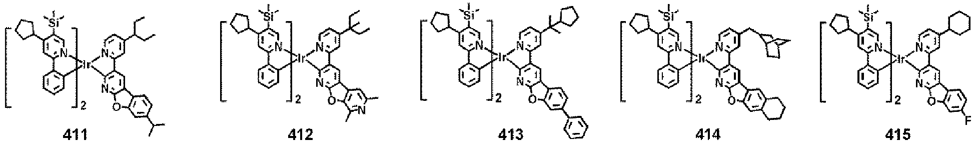

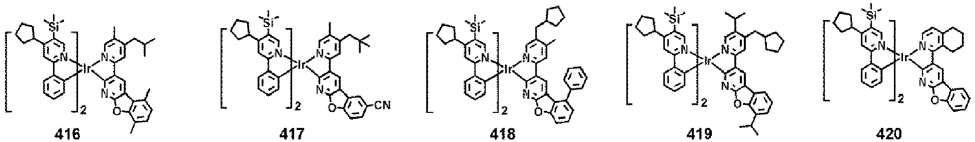

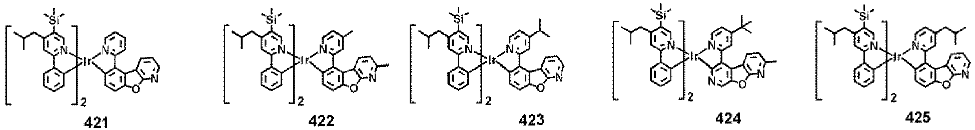

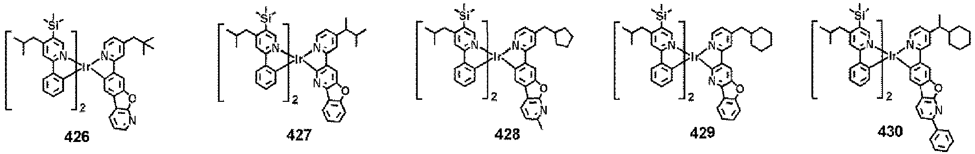

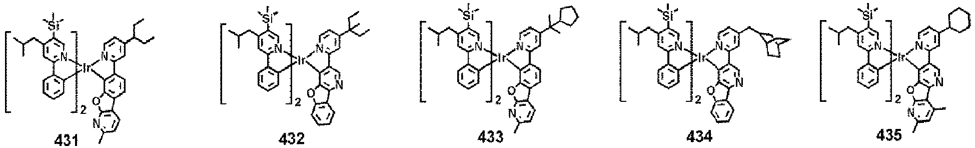

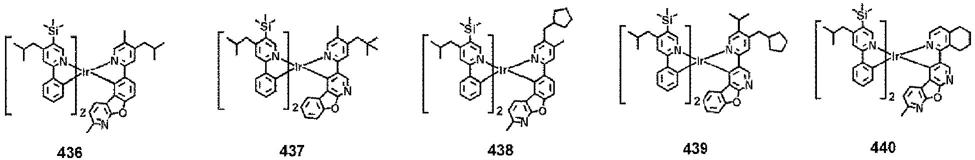

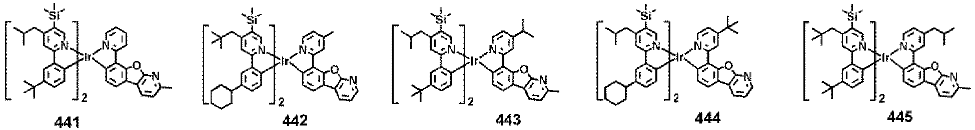

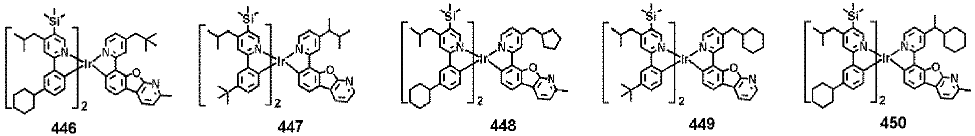

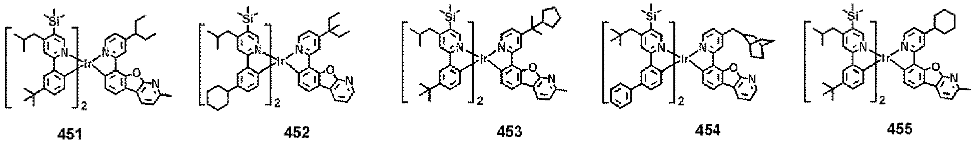

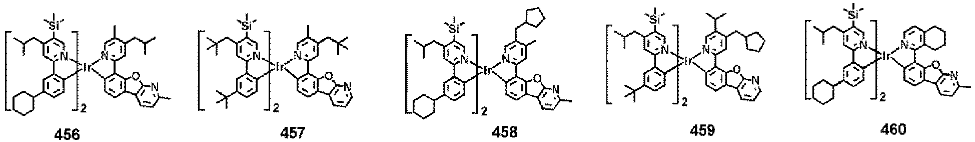

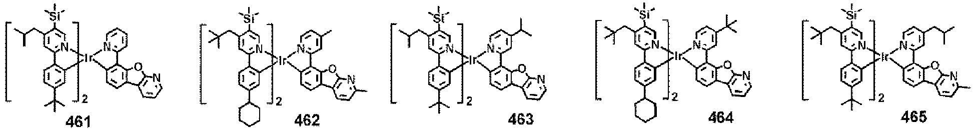

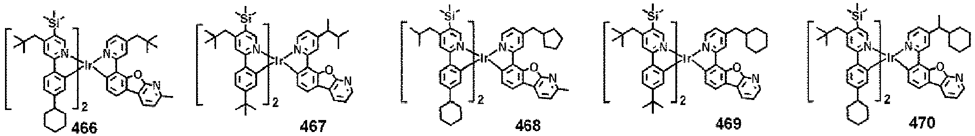

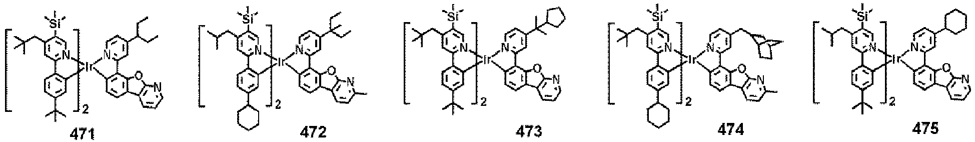

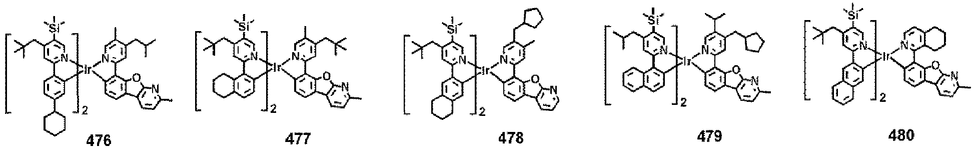

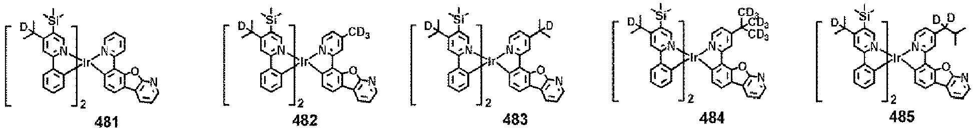

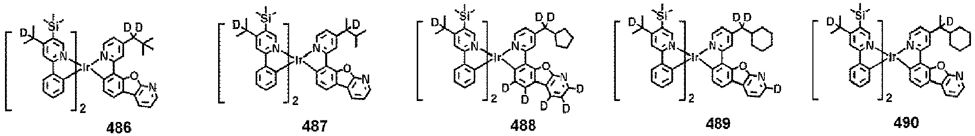

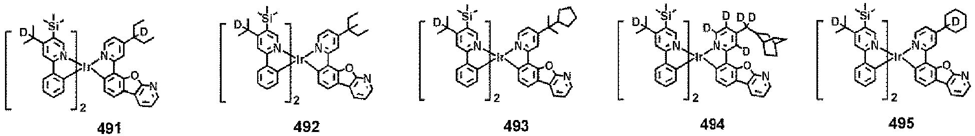

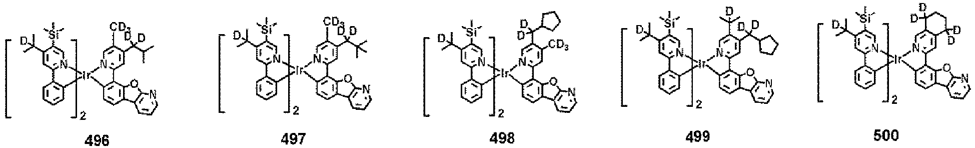

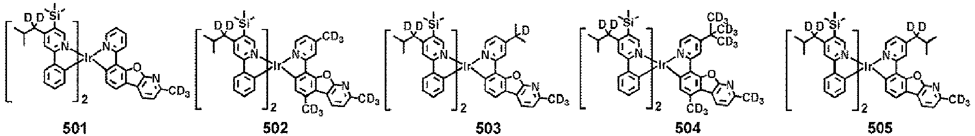

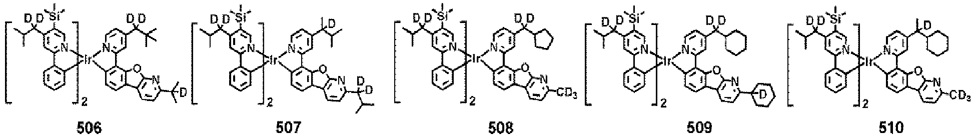

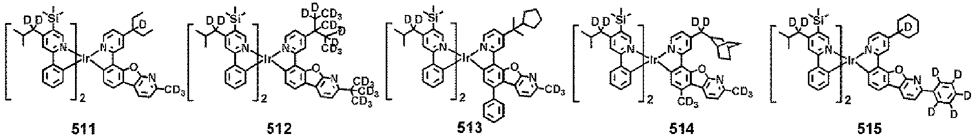

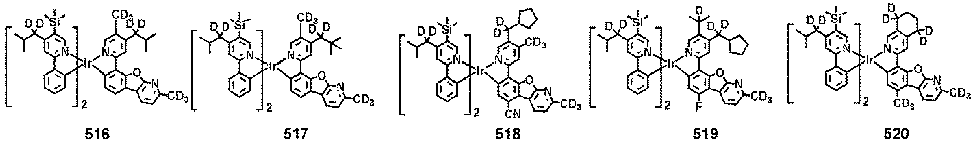

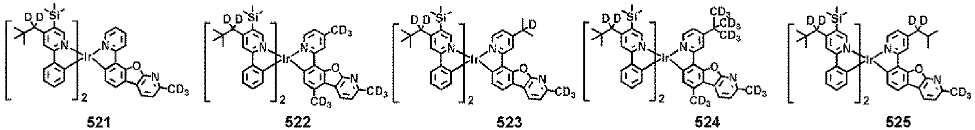

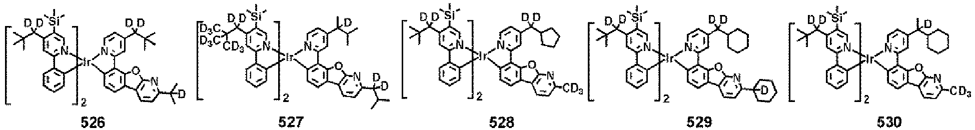

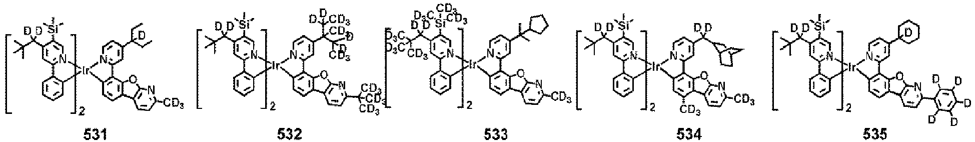

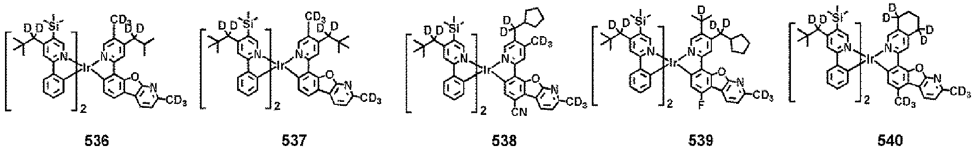

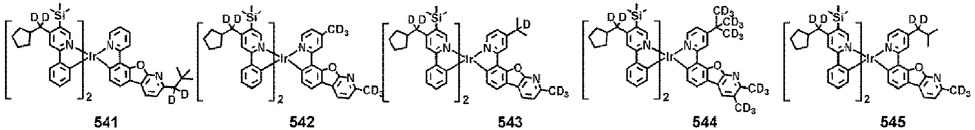

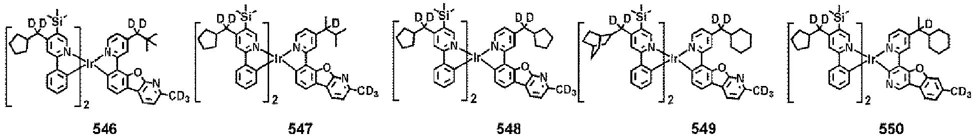

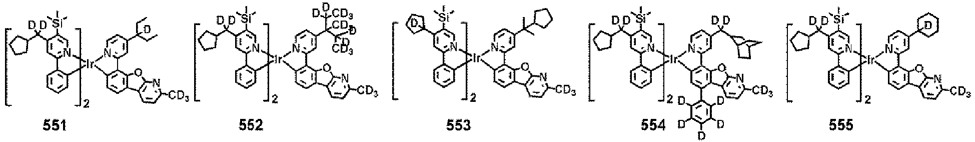

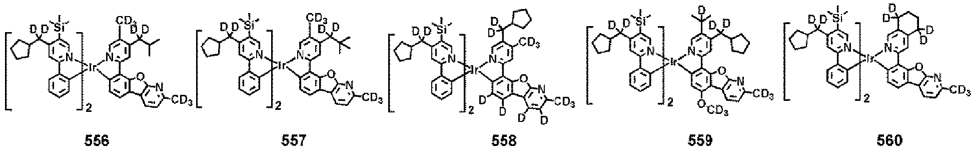

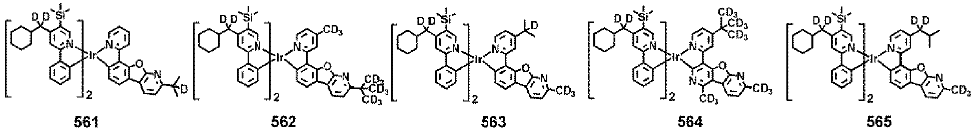

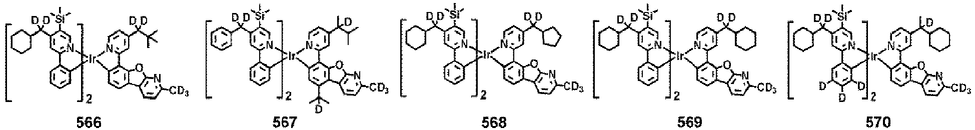

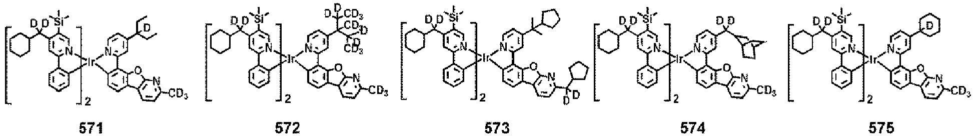

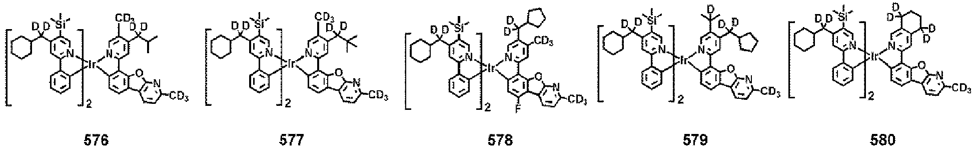

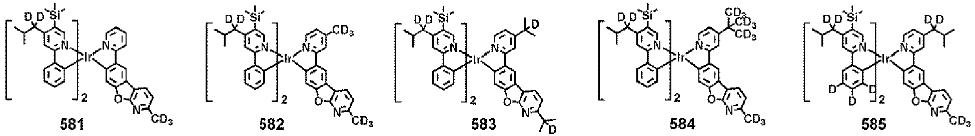

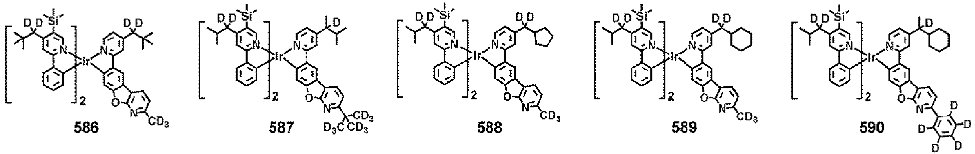

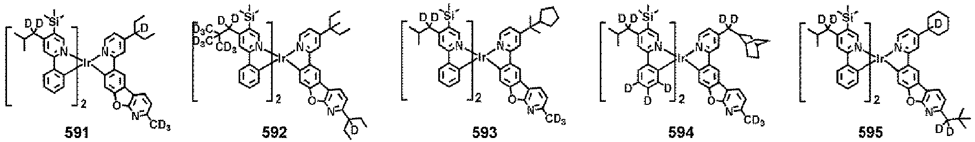

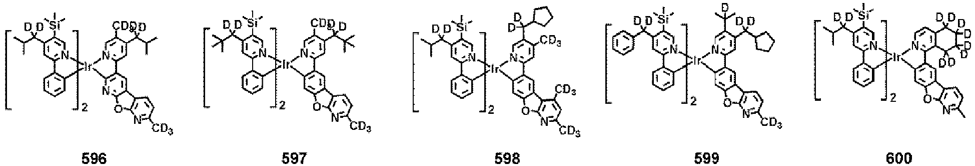

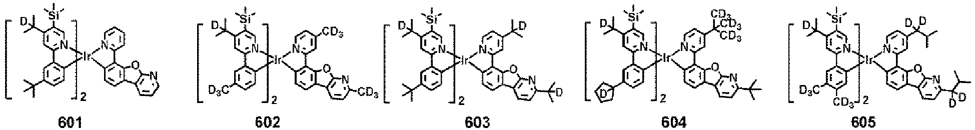

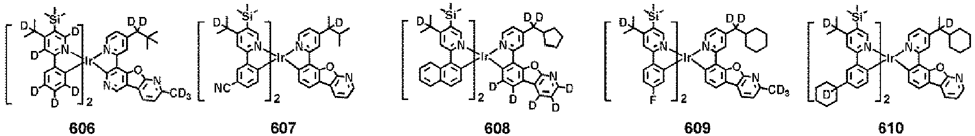

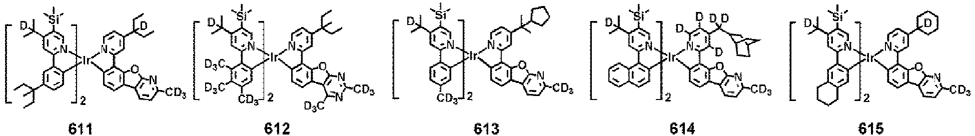

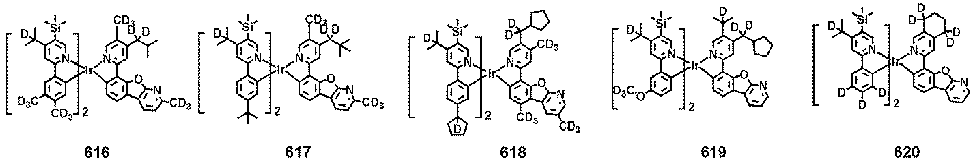

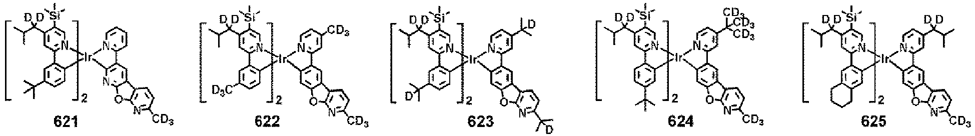

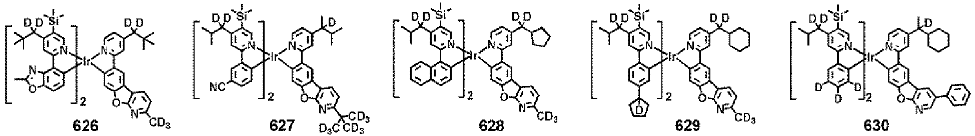

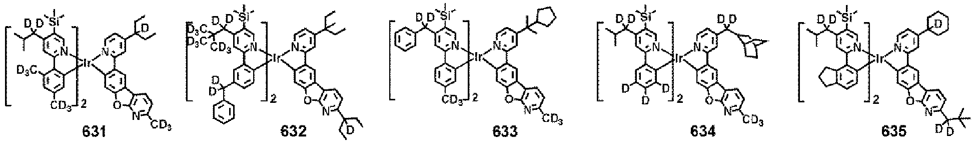

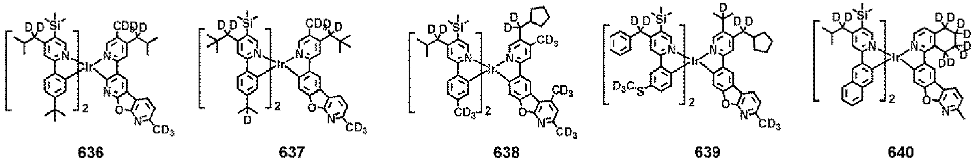

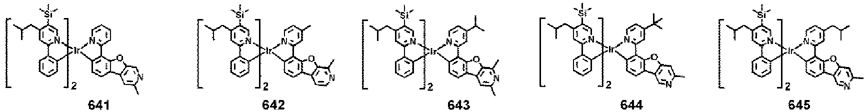

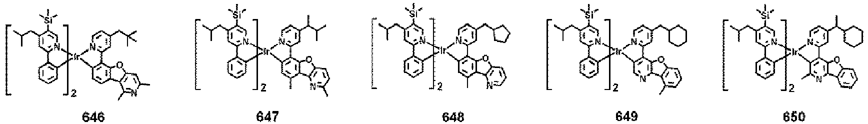

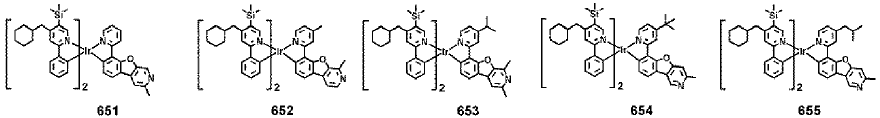

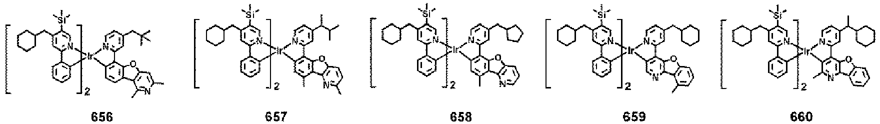

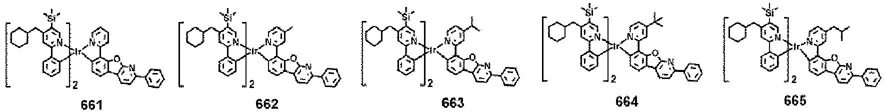

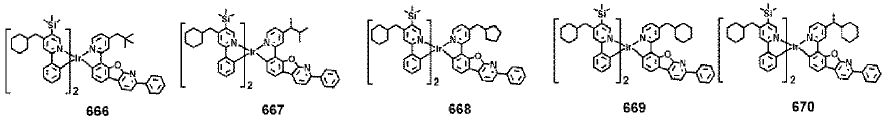

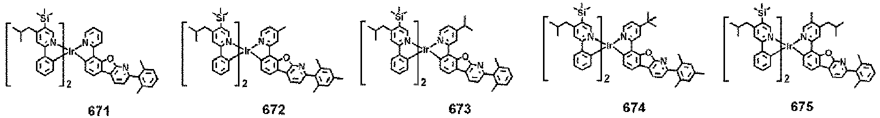

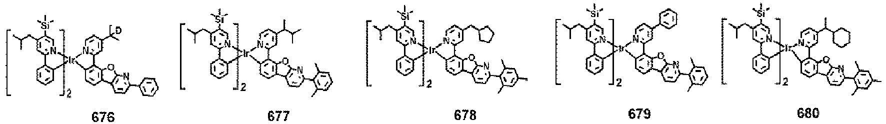

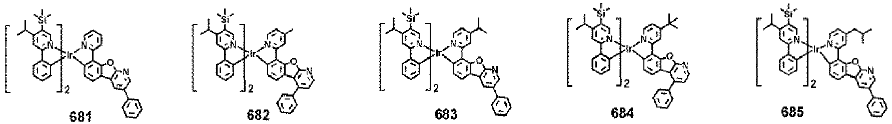

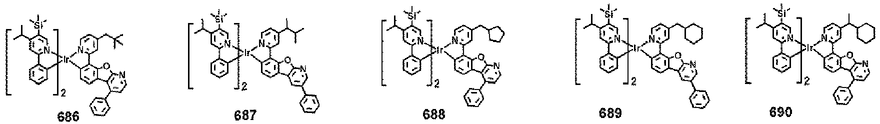

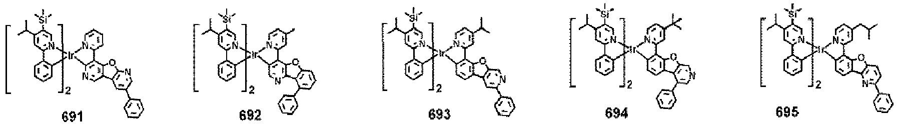

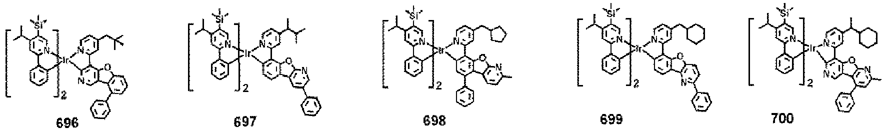

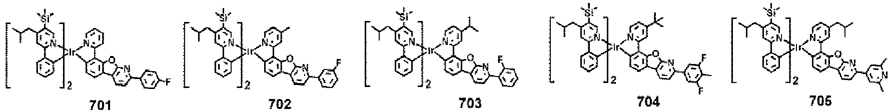

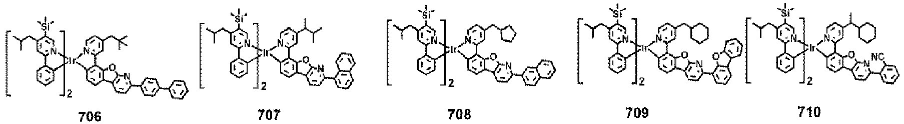

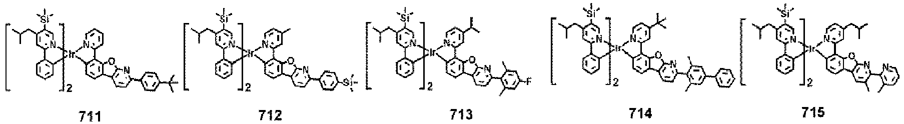

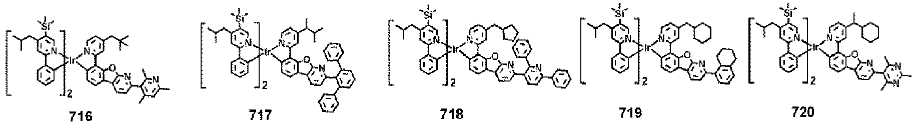

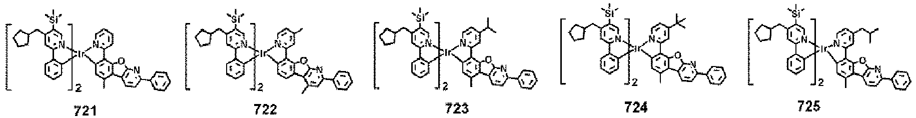

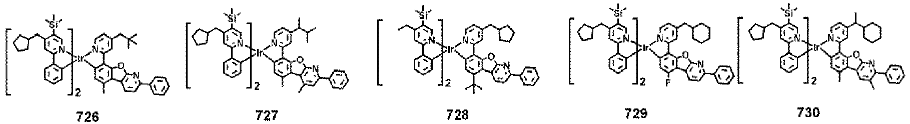

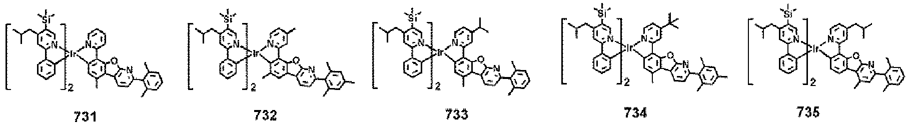

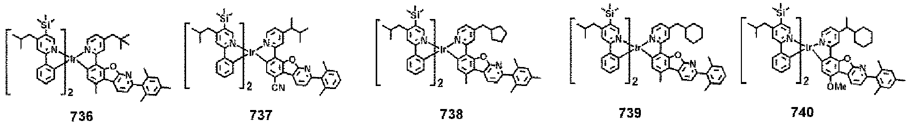

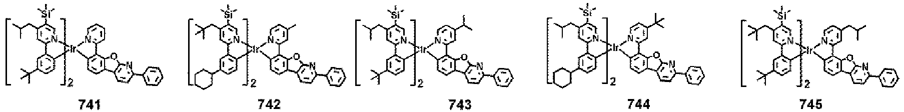

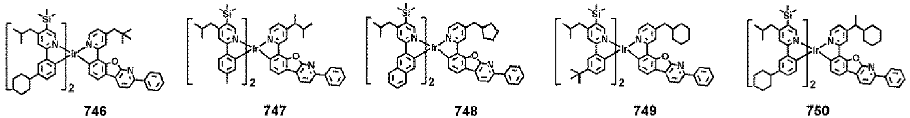

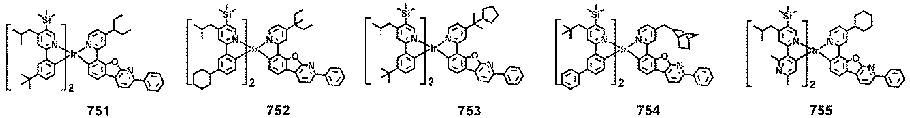

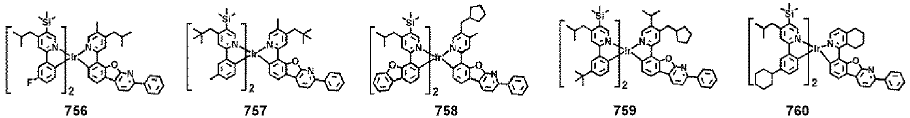

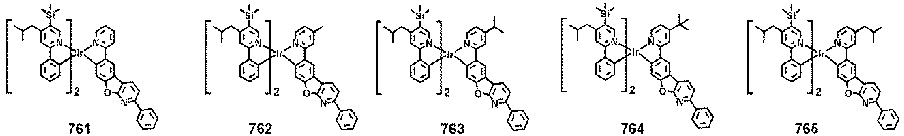

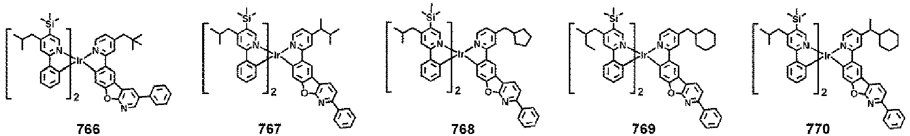

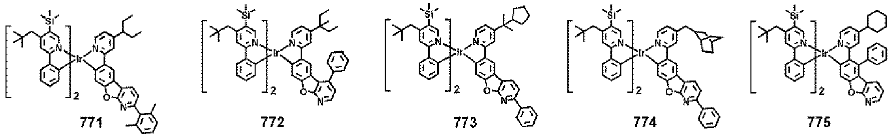

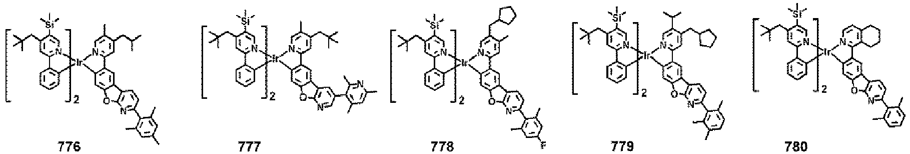

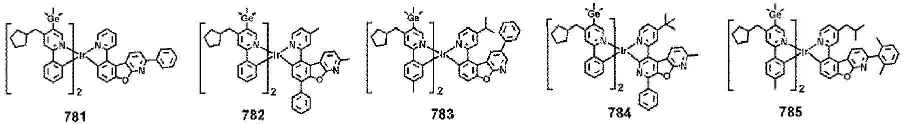

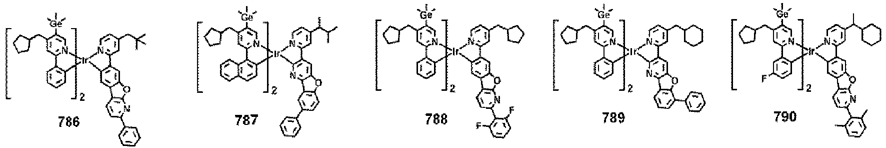

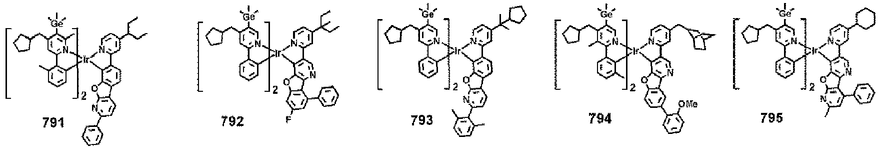

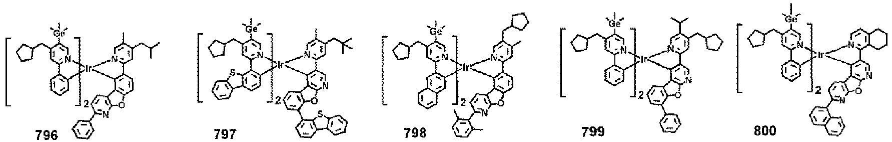

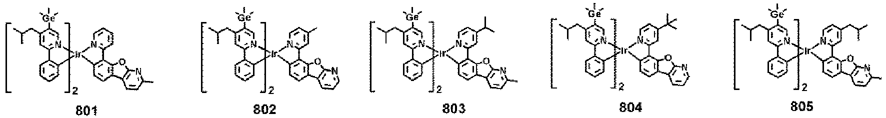

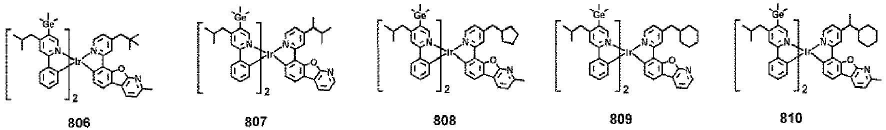

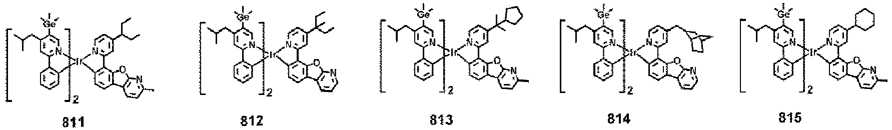

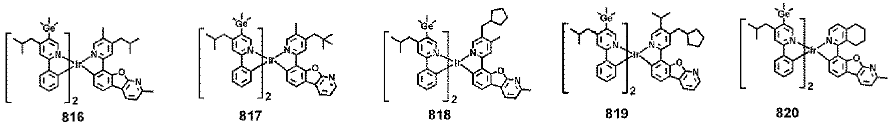

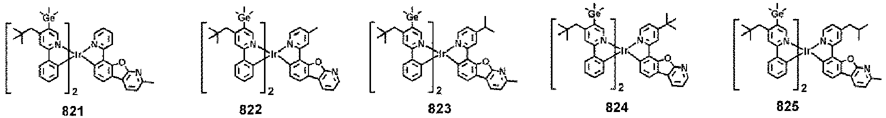

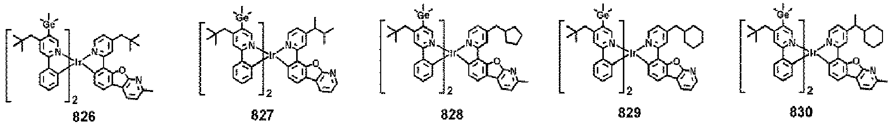

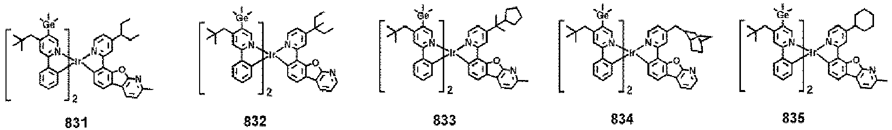

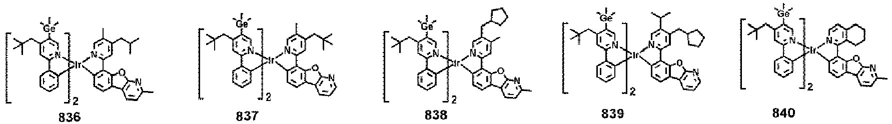

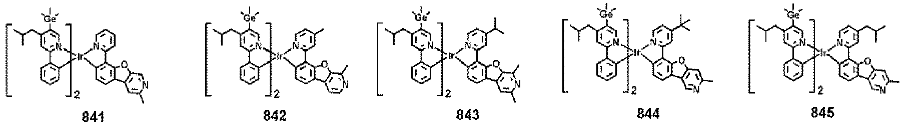

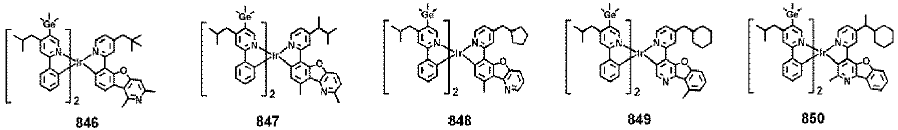

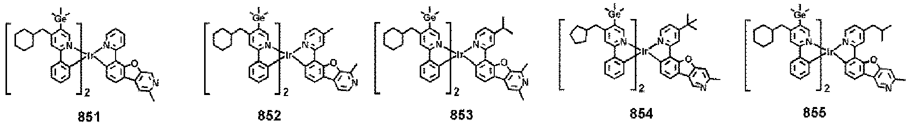

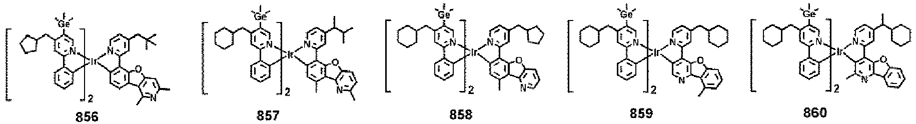

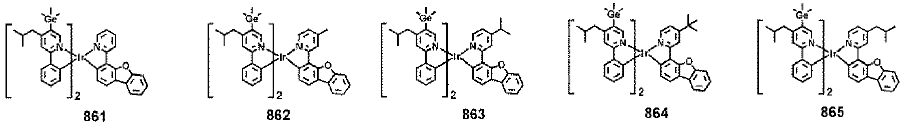

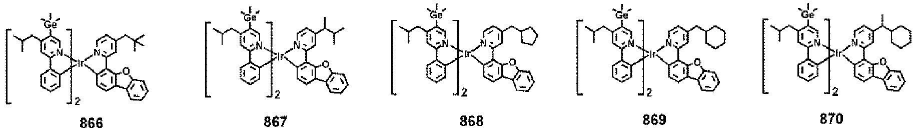

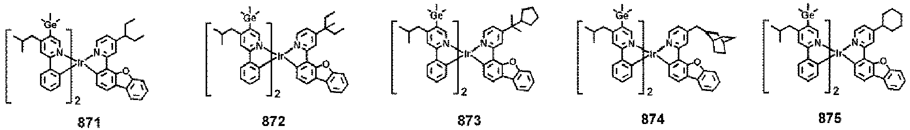

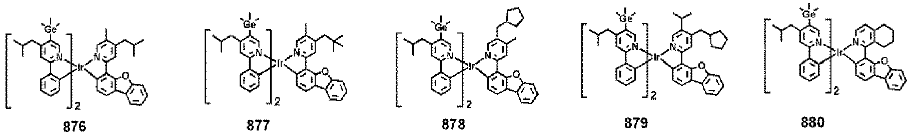

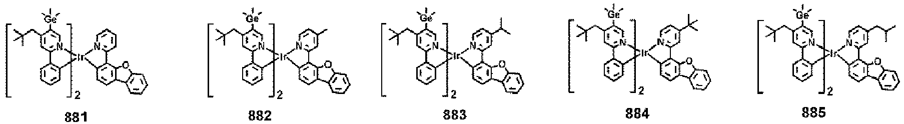

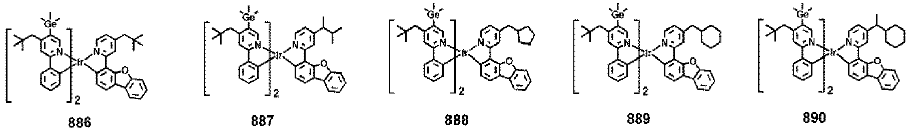

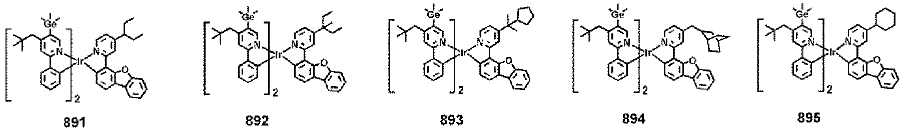

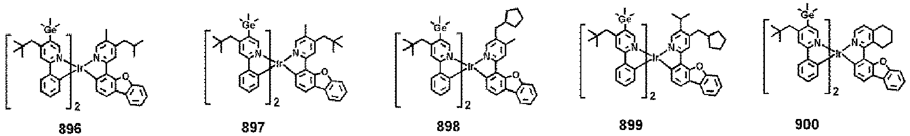

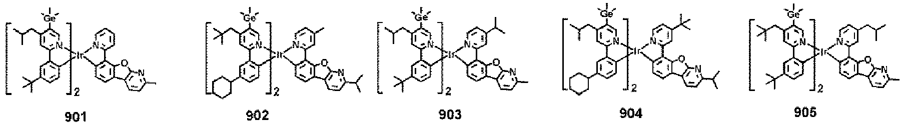

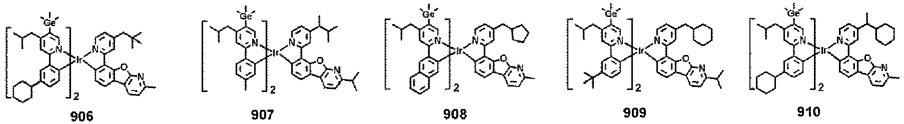

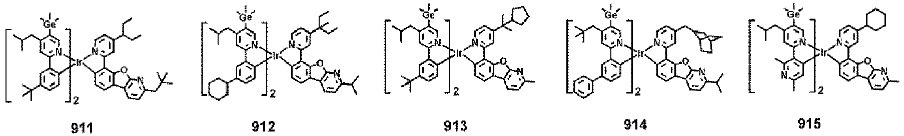

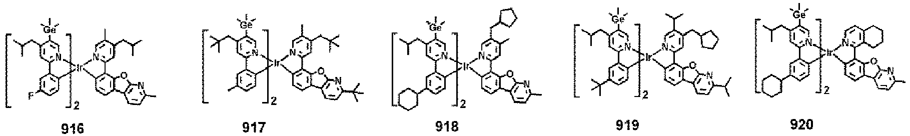

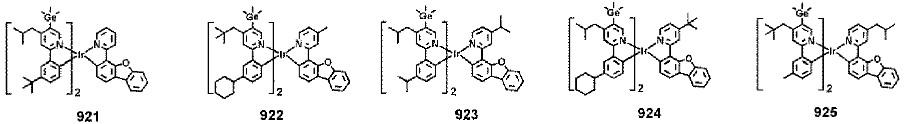

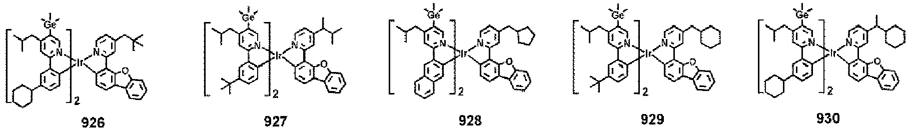

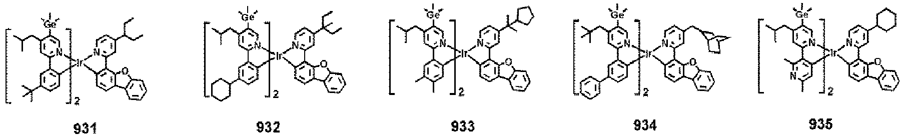

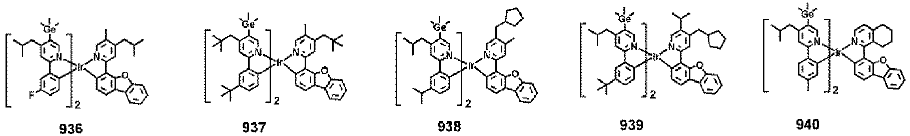

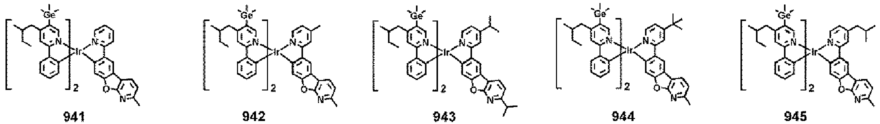

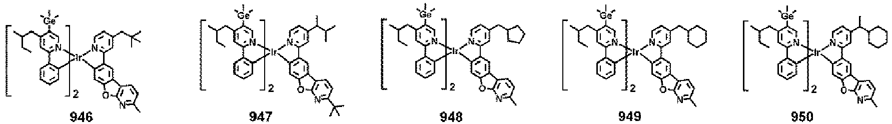

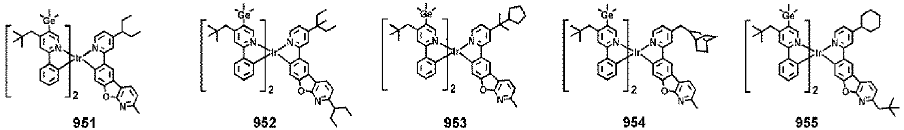

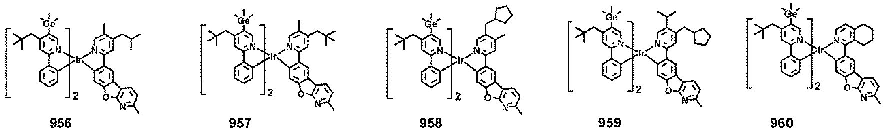

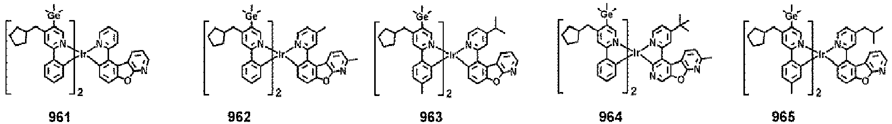

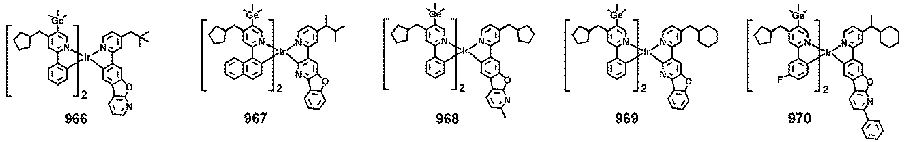

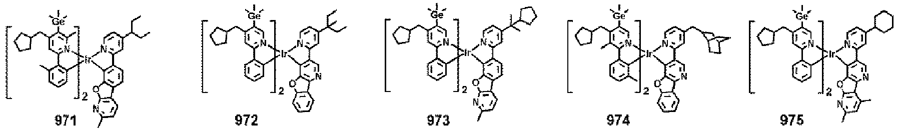

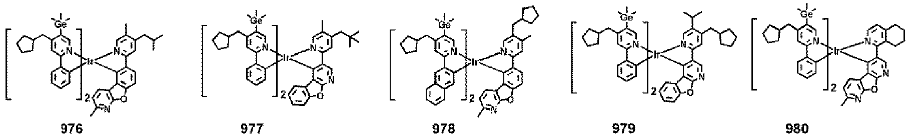

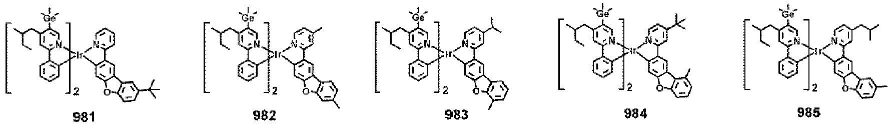

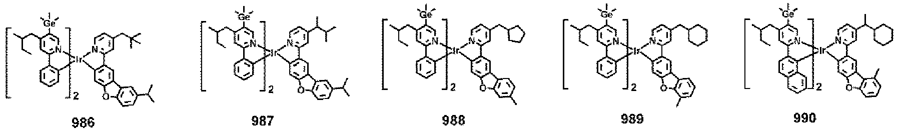

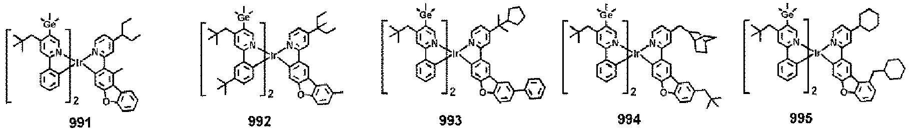

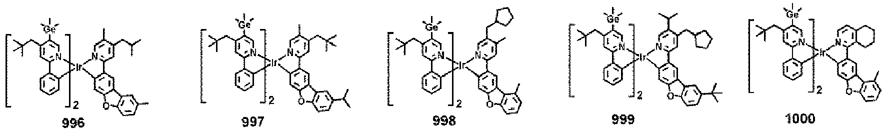

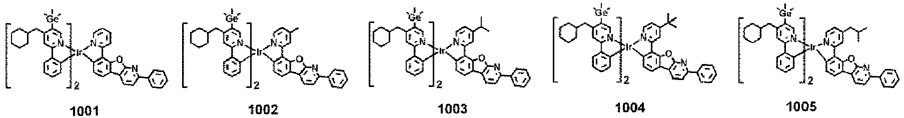

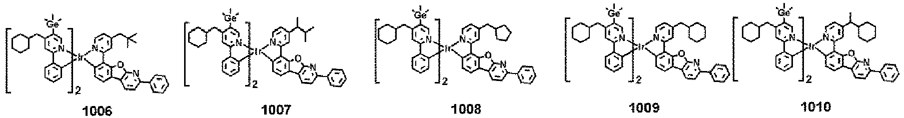

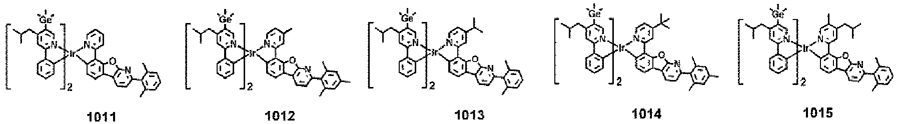

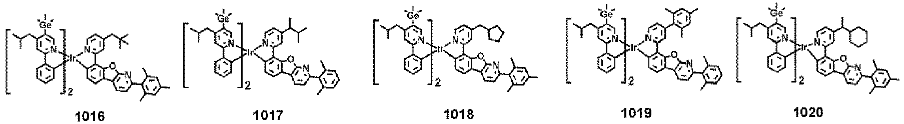

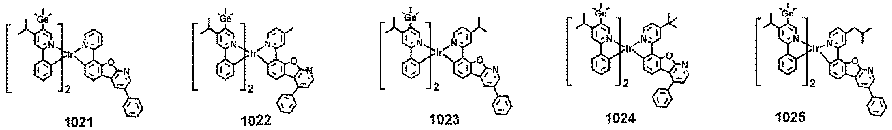

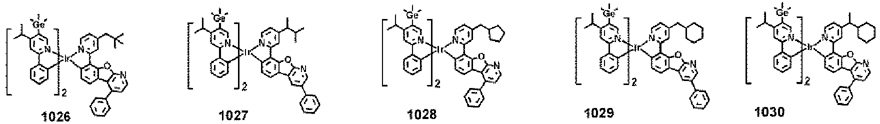

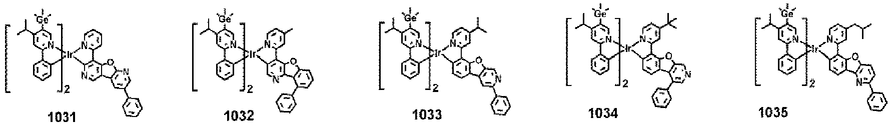

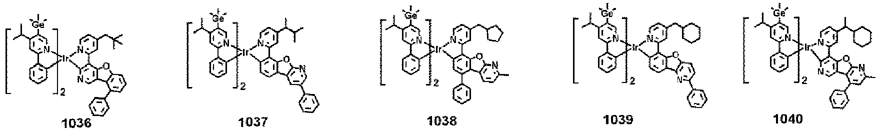

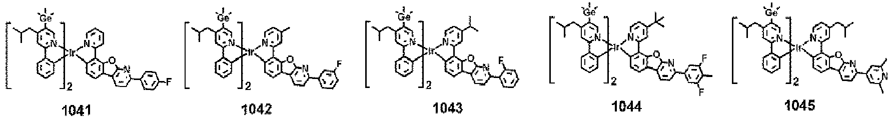

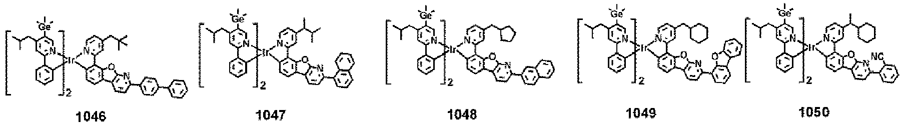

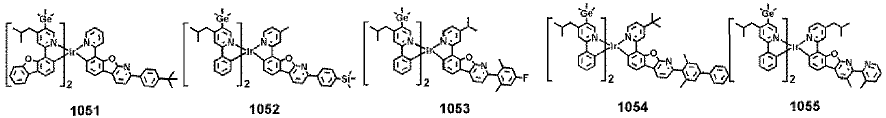

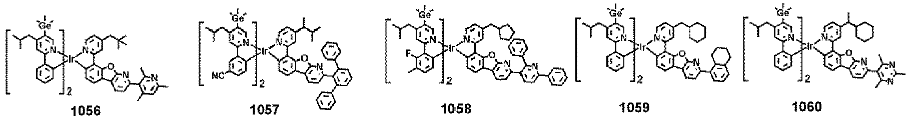

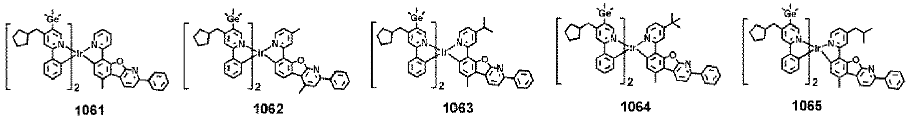

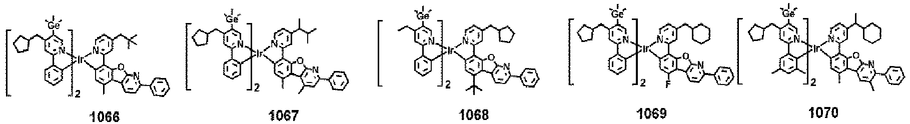

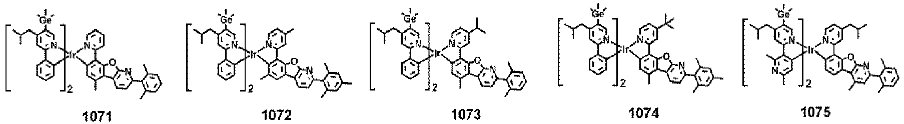

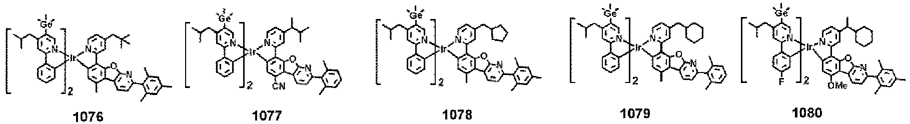

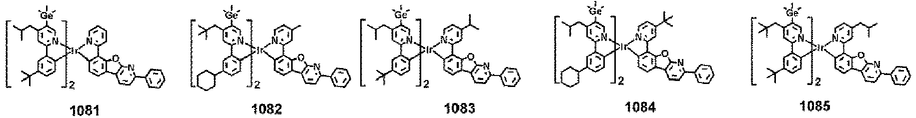

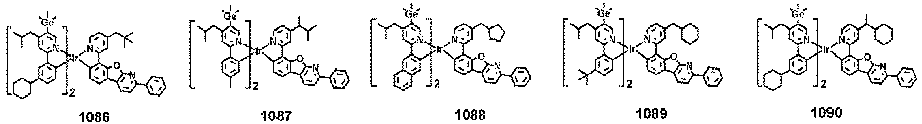

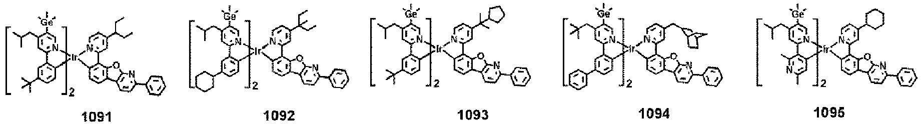

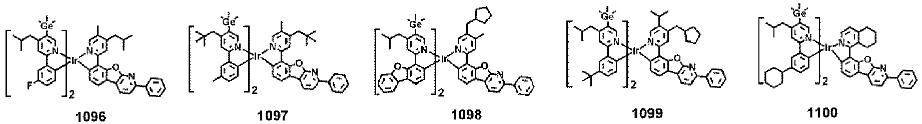

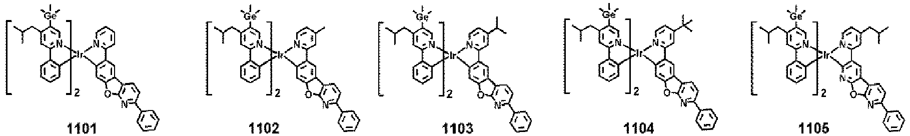

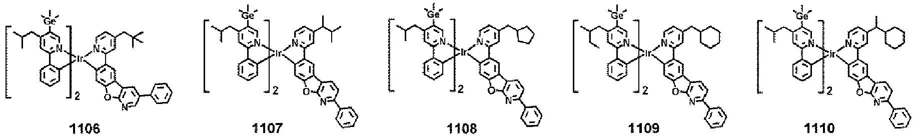

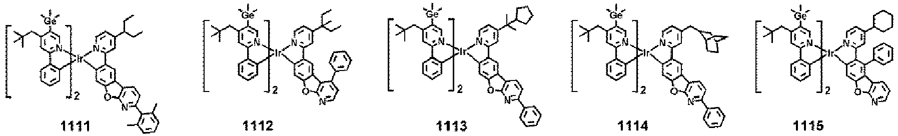

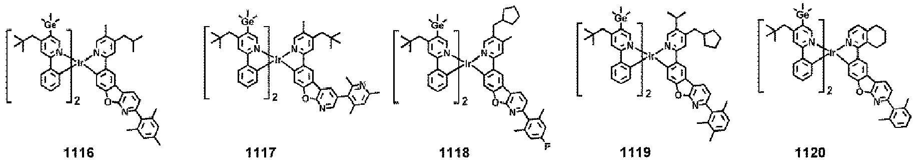

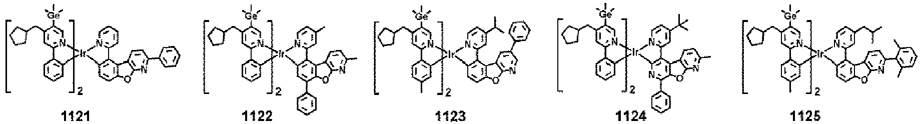

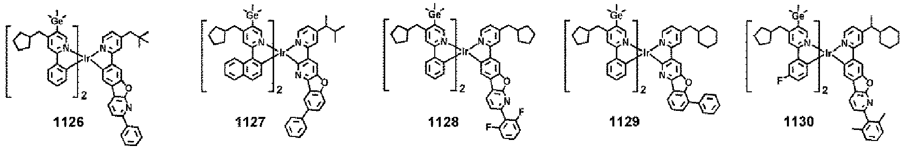

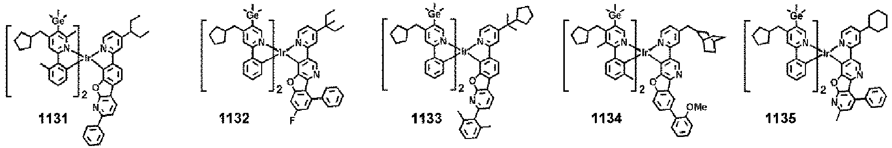

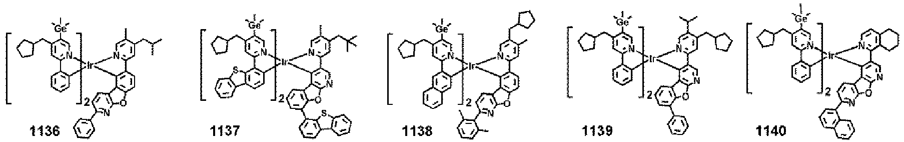

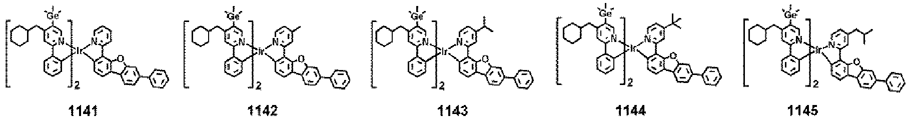

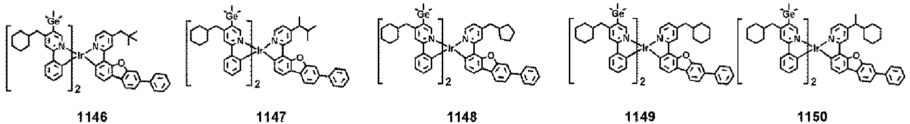

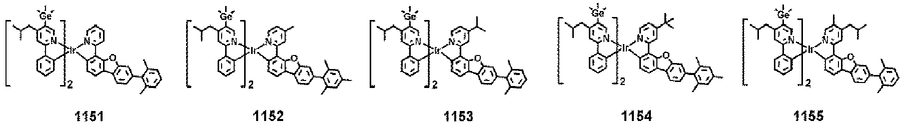

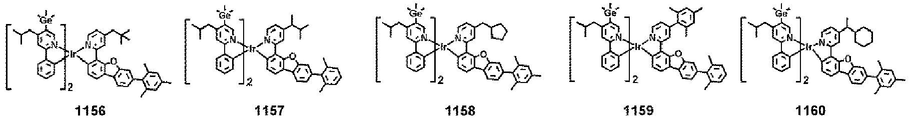

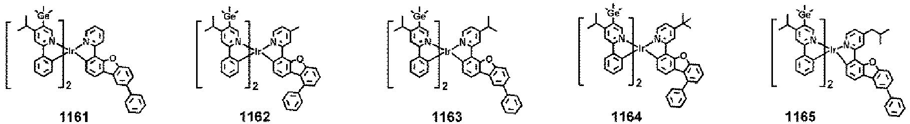

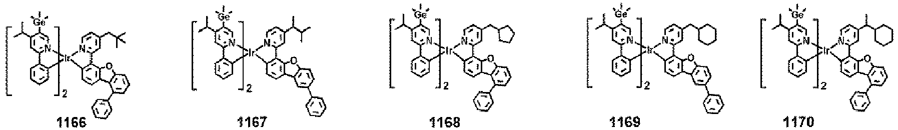

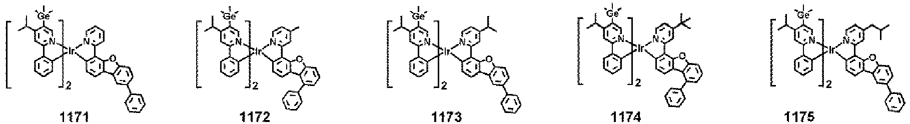

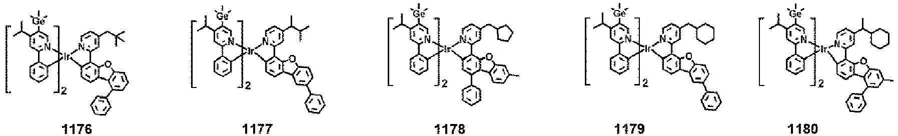

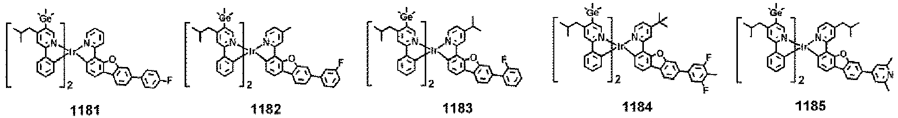

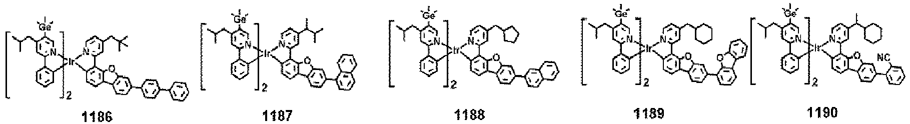

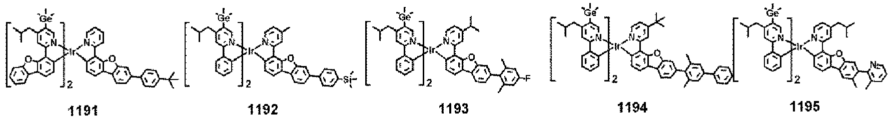

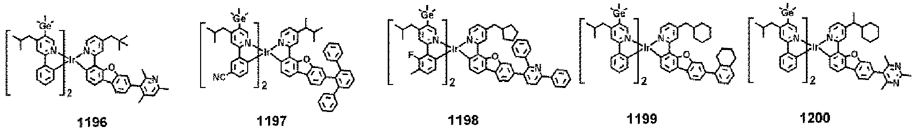

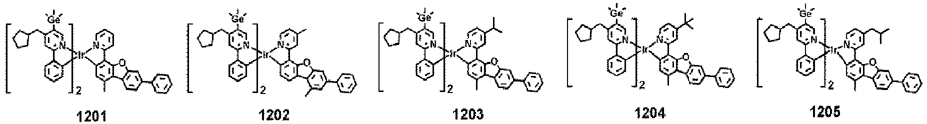

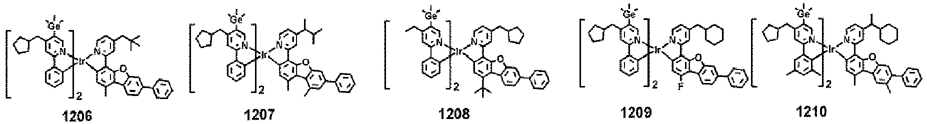

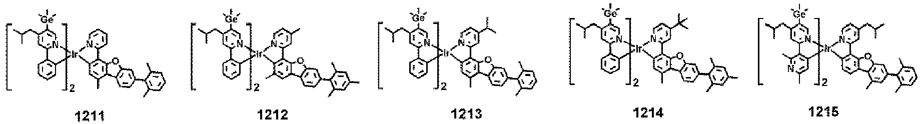

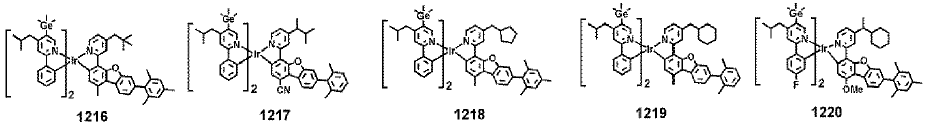

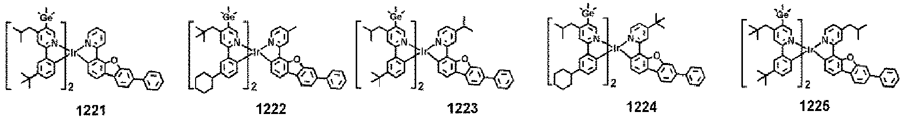

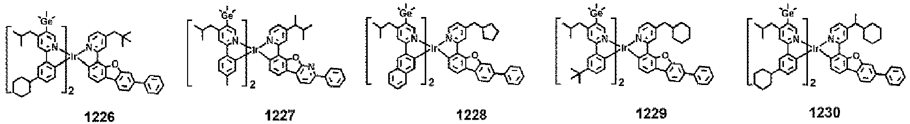

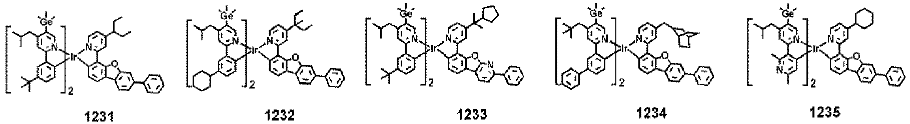

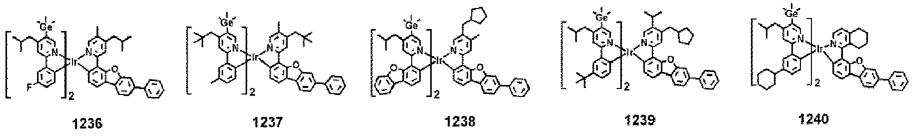

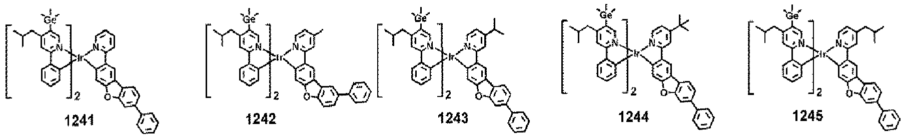

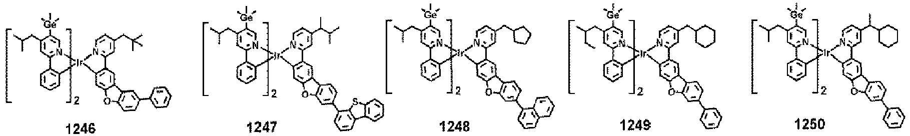

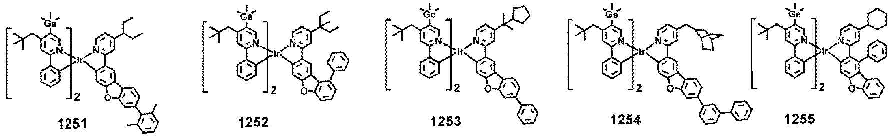

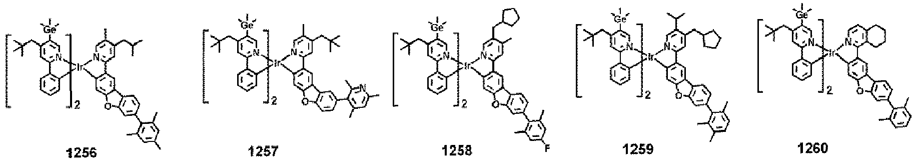

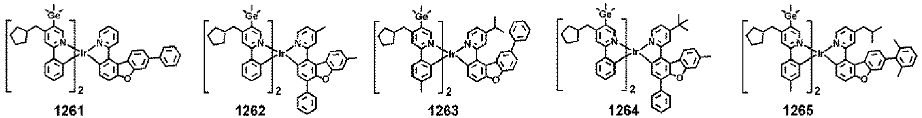

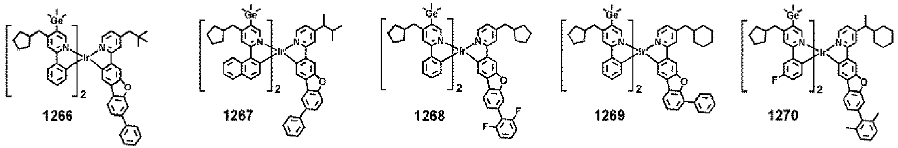

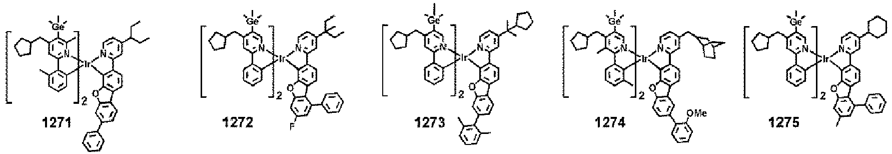

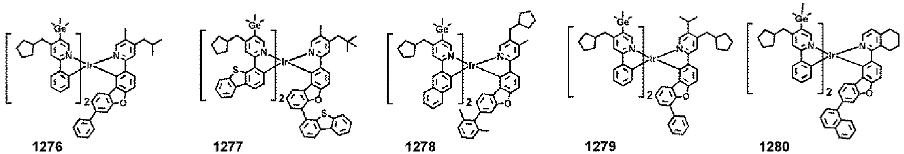

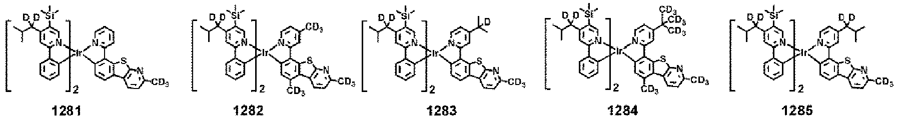

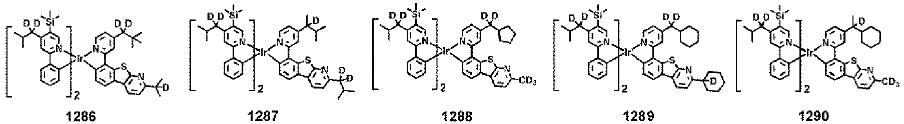

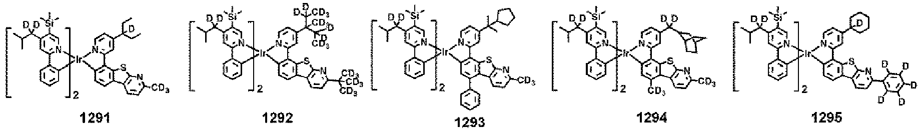

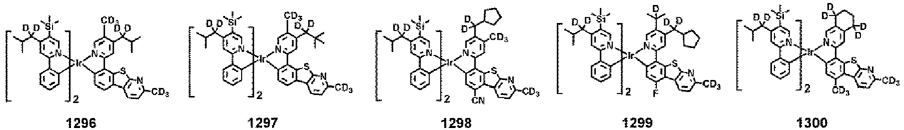

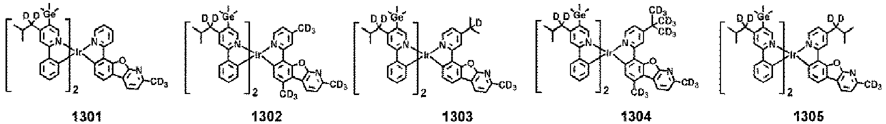

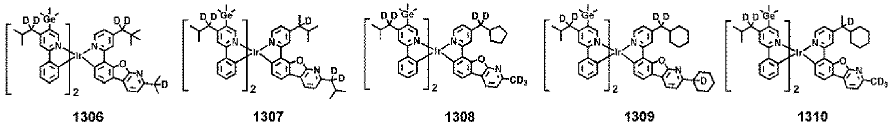

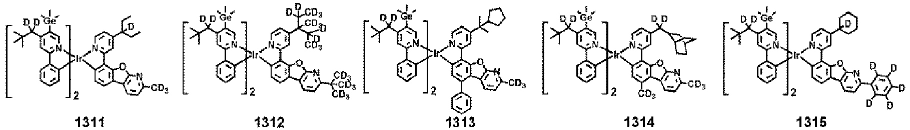

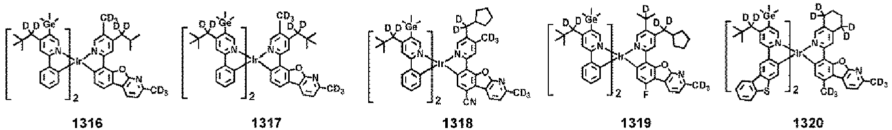

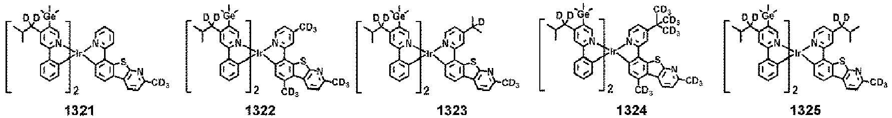

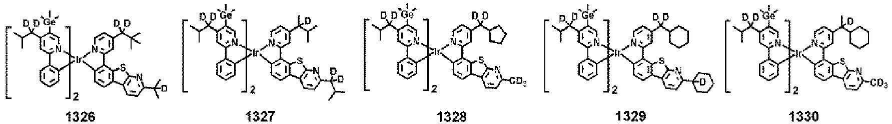

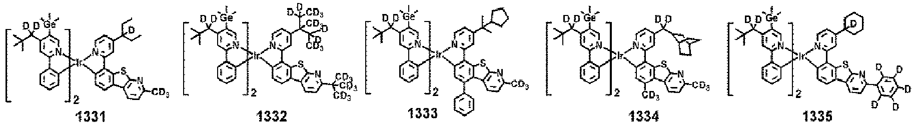

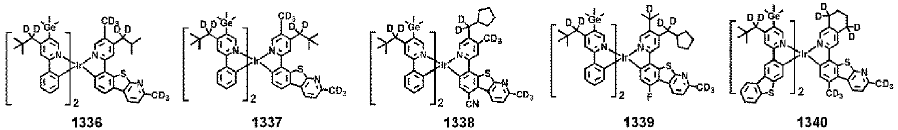

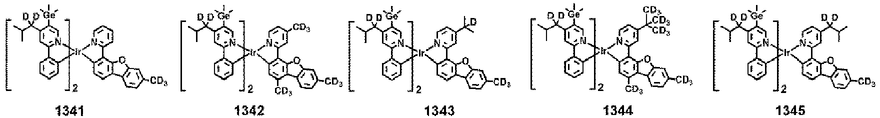

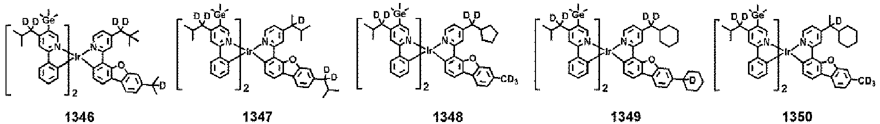

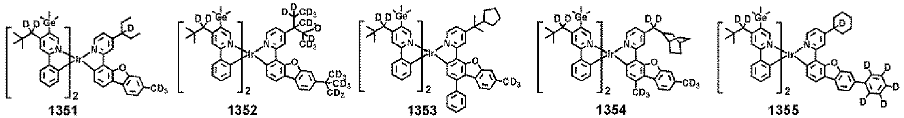

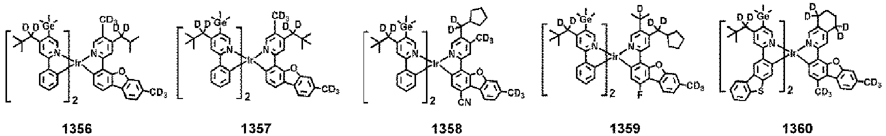

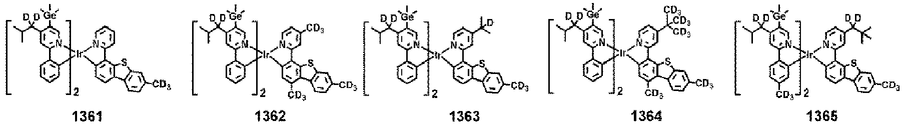

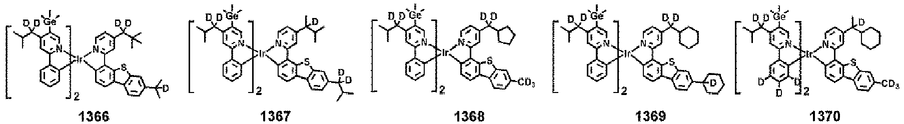

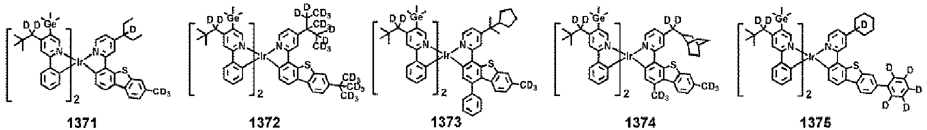

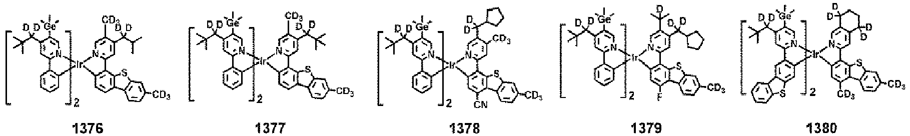

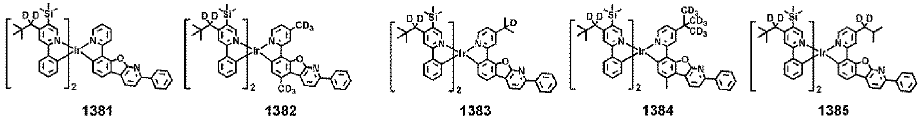

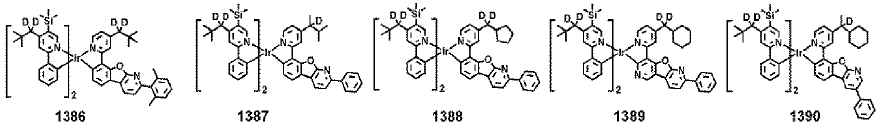

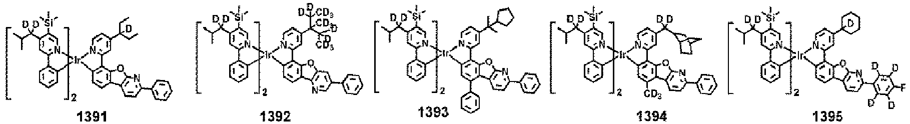

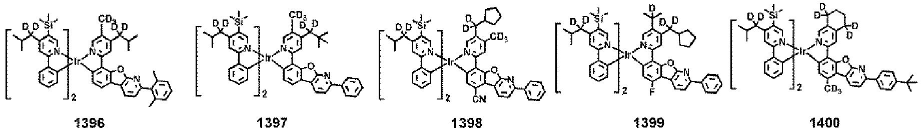

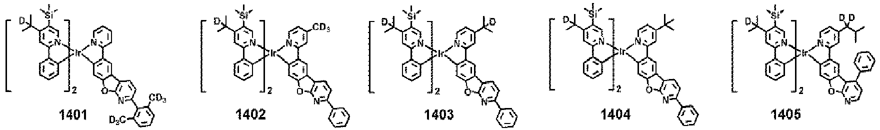

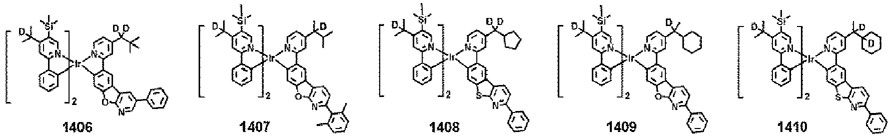

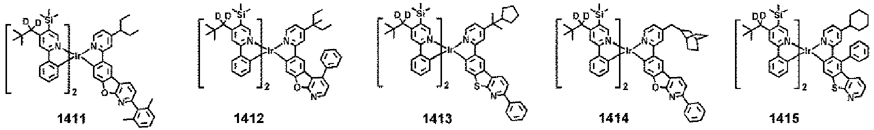

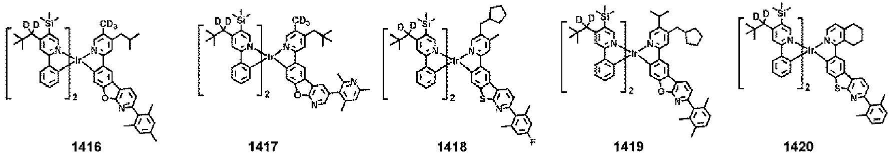

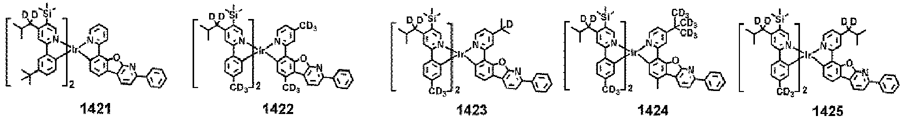

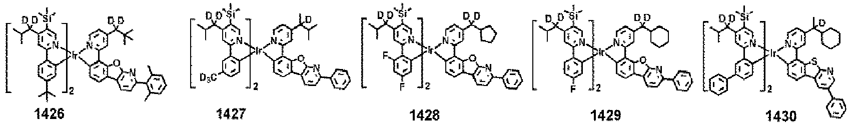

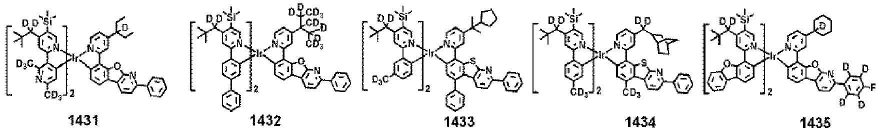

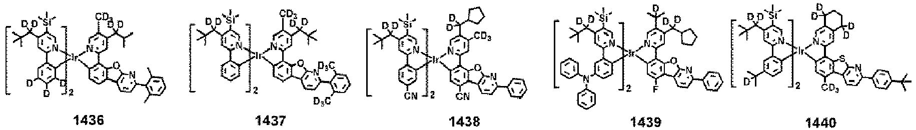

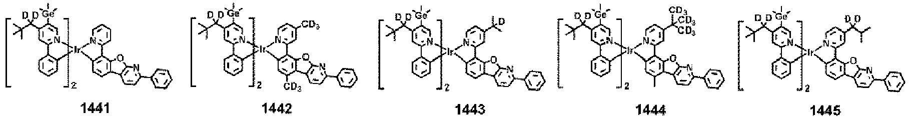

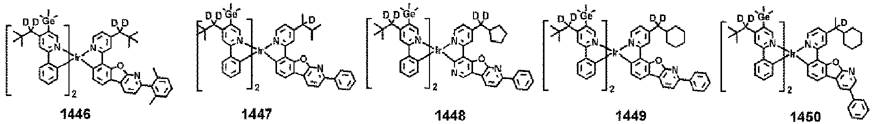

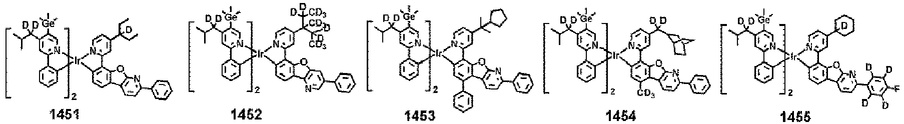

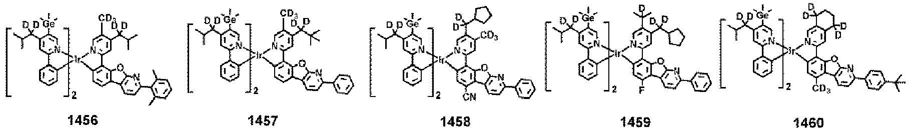

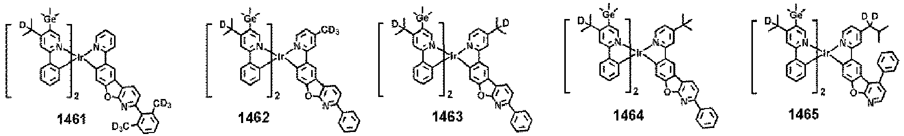

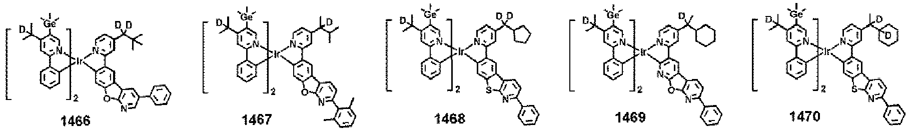

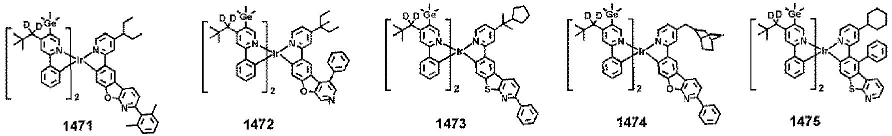

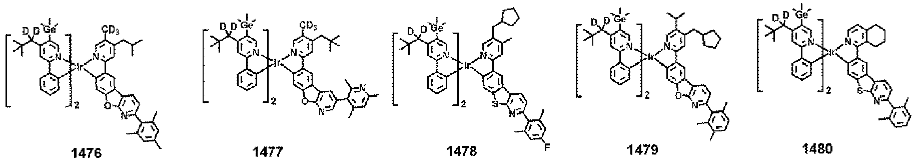

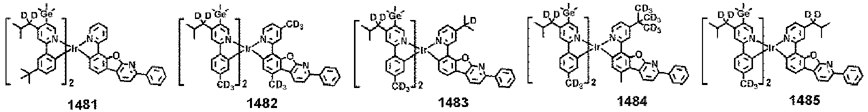

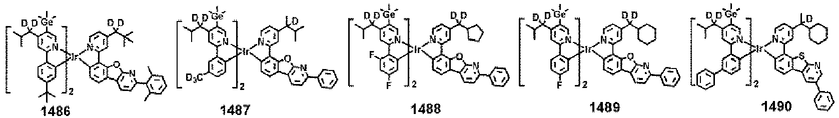

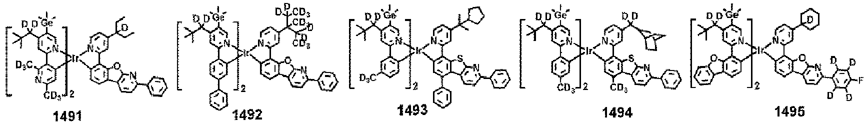

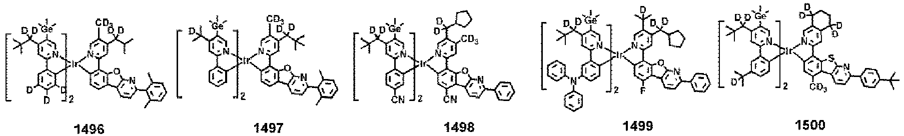

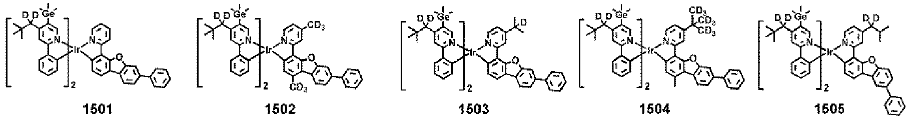

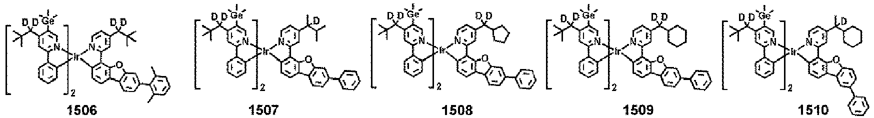

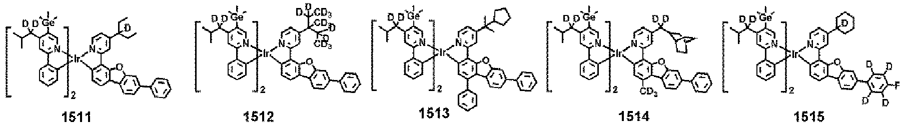

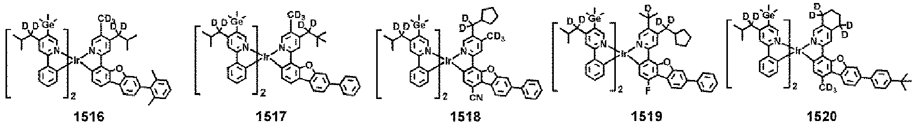

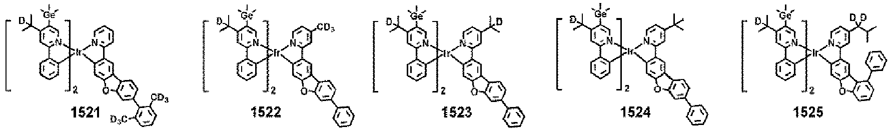

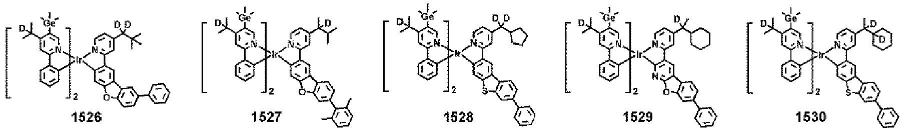

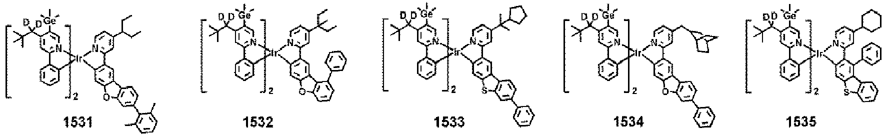

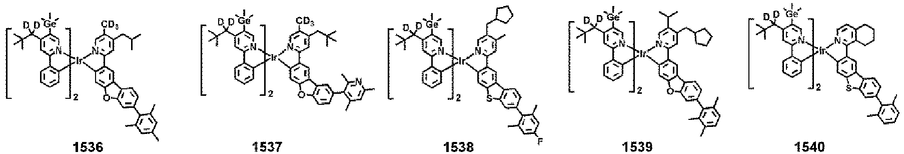

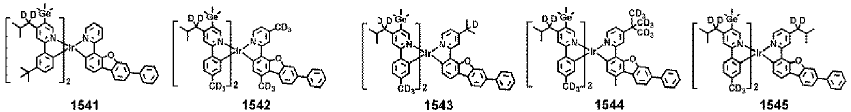

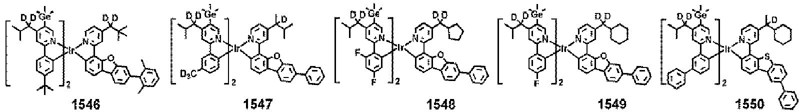

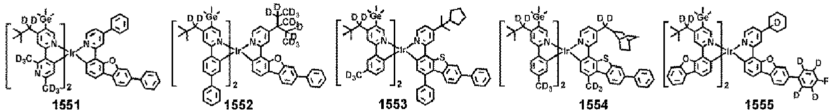

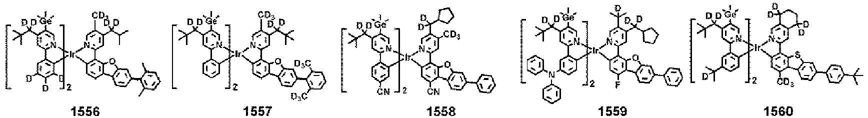

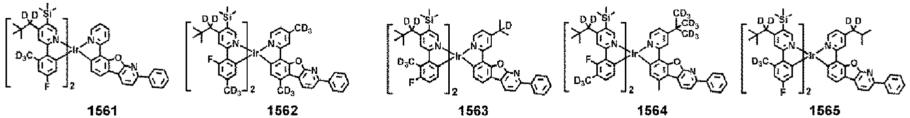

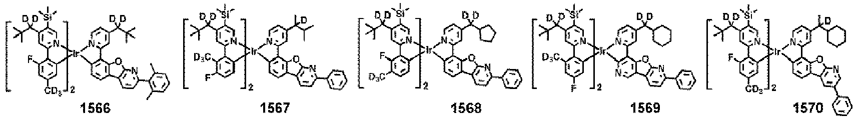

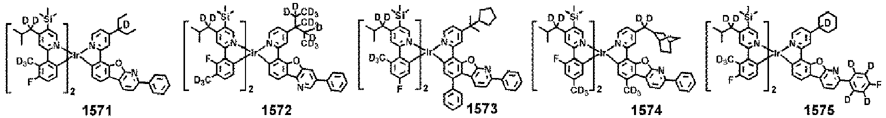

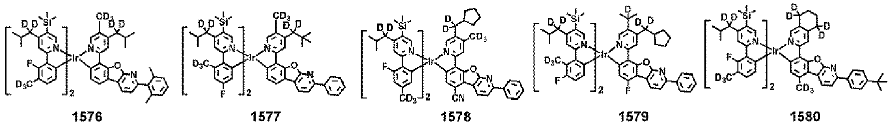

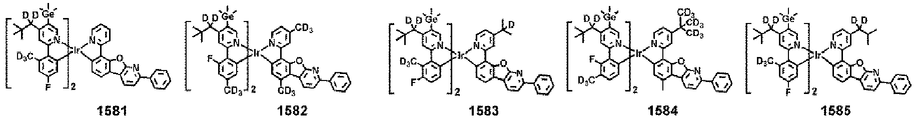

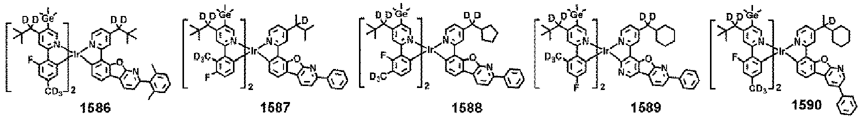

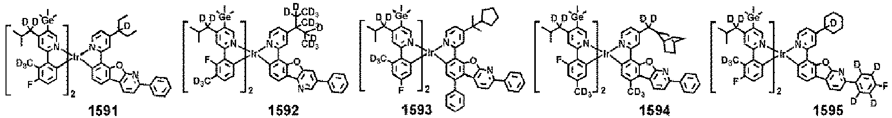

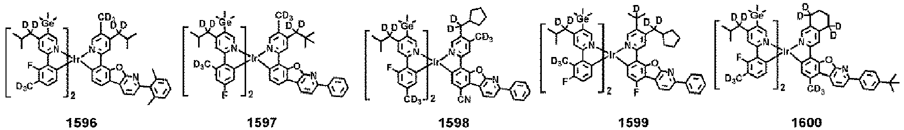

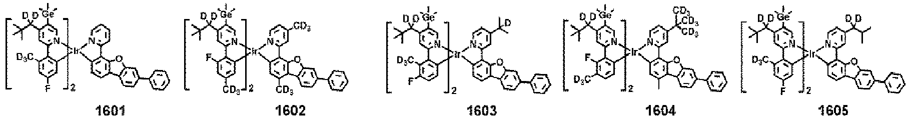

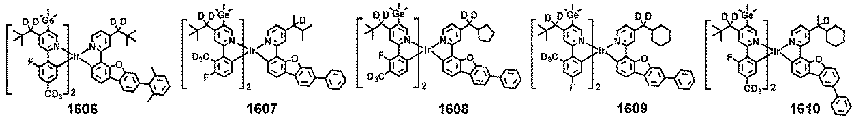

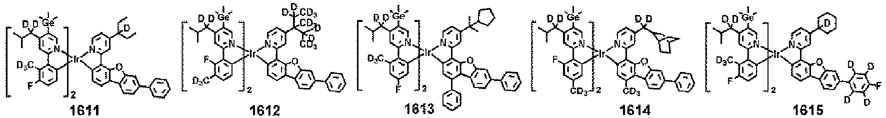

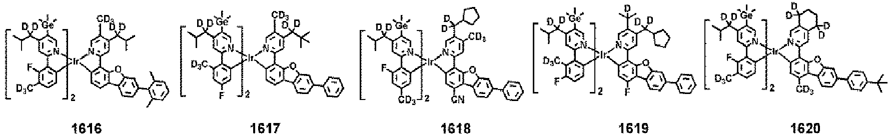

In Compounds 1 to 1620, OMe indicates a methoxy group.

L₁ of the organometallic compound represented by Formula 1 may be a ligand represented by Formula 2A, and n1 which indicates the number of L₁(s) may be 1 or 2. L₂ of the organometallic compound represented by Formula 1 may be a ligand represented by Formula 2B, and n2 which indicates the number of L₂(s) may be 1 or 2. Here, L₁ and L₂ are different from each other. That is, the organometallic compound may be a heteropeltic complex essentially including, as ligands linked to metal M, at least one ligand represented by Formula 2A and at least one ligand represented by Formula 2B.

A group represented by *-X₁(R₂₁)(R₂₂)(R₂₃) in Formula 1 may be linked to the fifth position of a pyridine ring in a ligand represented by Formula 2B (see Formula 2B). Accordingly, the organometallic compound including the ligand represented by Formula 2B may have excellent heat resistance and degradation resistance so that an electronic device, for example, an organic light-emitting device, including the organometallic compound may have high stability and long lifespan in production, storage, and/or operation.

Furthermore, when X₁ is Si, at least one of T₁ to T₈ which are not linked to M and ring CY₁ in Formula 2A may be N. Accordingly, an electronic device, for example, an organic light-emitting device, including the organometallic compound represented by Formula 1 may have improved driving voltage and roll-off ratio.

In one embodiment, in Formulae 2A and 2B, R₂₁ to R₂₃, Z₁, Z₂, and R₁₁ to R₁₄ do not each include a silicon (Si). Accordingly, an electronic device, for example, an organic light-emitting device, including the organometallic compound represented by Formula 1 may have improved out-coupling characteristics.

In addition, R₁₂ in Formula 2B is not hydrogen nor a methyl group. As such, the organometallic compound represented by Formula 1 may emit light that is shifted toward relatively shorter wavelengths, for example, blue light, green light, or greenish blue light, and an electronic device, for example, an organic light-emitting device, including the organometallic compound may have an excellent out-coupling effect, thereby having high luminescence efficiency.

A highest occupied molecular orbital (HOMO) energy level, a lowest unoccupied molecular orbital (LUMO) energy level, a singlet (S₁) energy level, and a triplet (T₁) energy level of some compounds of the organometallic compound represented by Formula 1 are evaluated by a density functional theory (DFT) of Gaussian program with molecular structure optimization based on B3LYP, and results are shown in Table 1.

**[Table 1]**

| Compound No. | HOMO (eV) | LUMO (eV) | S₁ (eV) | T₁ (eV) |
|---|---|---|---|---|
| 123 | -4.773 | -1.203 | 2.880 | 2.546 |
| 301 | -4.787 | -1.314 | 2.738 | 2.476 |
| 226 | -4.780 | -1.208 | 2.876 | 2.529 |
| 545 | -4.767 | -1.196 | 2.879 | 2.526 |

Referring to Table 1, it is confirmed that the organometallic compound represented by Formula 1 has such electrical characteristics that are suitable for use in an electronic device, for example, for use as a dopant for an organic light-emitting device.

Synthesis methods of the organometallic compound represented by Formula 1 may be recognizable by one of ordinary skill in the art by referring to Synthesis Examples provided below.

Therefore, the organometallic compound represented by Formula 1 may be suitable for use in an organic layer of an organic light-emitting device, for example, for use as a dopant in an emission layer of the organic layer. Another aspect of the present disclosure provides an organic light-emitting device including: a first electrode, a second electrode, and an organic layer disposed between the first electrode and the second electrode and including an emission layer, wherein the organic layer may include at least one organometallic compound represented by Formula 1.

The organic light-emitting device may have, due to the inclusion of an organic layer including the organometallic compound represented by Formula 1, a low driving voltage, high external quantum efficiency, a long lifespan, a low roll-off ratio, and excellent color purity.

The organometallic compound represented by Formula 1 may be used between a pair of electrodes of an organic light-emitting device. For example, the organometallic compound represented by Formula 1 may be included in the emission layer. In this regard, the organometallic compound may act as a dopant, and the emission layer may further include a host (that is, an amount of the organometallic compound represented by Formula 1 is smaller than an amount of the host). The emission layer may emit, for example, green light or blue light.

The expression "(an organic layer) includes at least one of the organometallic compound" as used herein may include a case in which "(an organic layer) includes identical organometallic compounds represented by Formula 1" and a case in which "(an organic layer) includes two or more different organometallic compounds represented by Formula 1".

For example, the organic layer may include, as the organometallic compound, only Compound 1. In this regard, Compound 1 may exist only in the emission layer of the organic light-emitting device. In one or more embodiments, the organic layer may include, as the organometallic compound, Compound 1 and Compound 2. In this regard, Compound 1 and Compound 2 may exist in an identical layer (for example, Compound 1 and Compound 2 all may exist in an emission layer).

The first electrode may be an anode, which is a hole injection electrode, and the second electrode may be a cathode, which is an electron injection electrode; or the first electrode may be a cathode, which is an electron injection electrode, and the second electrode may be an anode, which is a hole injection electrode.

For example, in the organic light-emitting device, the first electrode is an anode, and the second electrode is a cathode, and the organic layer further includes a hole transport region between the first electrode and the emission layer and an electron transport region between the emission layer and the second electrode, and the hole transport region includes a hole injection layer, a hole transport layer, an electron blocking layer, or any combination thereof, and the electron transport region includes a hole blocking layer, an electron transport layer, an electron injection layer, or any combination thereof.

The term "organic layer" as used herein refers to a single layer and/or a plurality of layers disposed between the first electrode and the second electrode of an organic light-emitting device. The "organic layer" may include, in addition to an organic compound, an organometallic complex including metal.

FIGURE is a schematic cross-sectional view of an organic light-emitting device 10 according to an embodiment. Hereinafter, the structure of an organic light-emitting device according to an embodiment and a method of manufacturing an organic light-emitting device according to an embodiment will be described in connection with the FIGURE. The organic light-emitting device 10 includes a first electrode 11, an organic layer 15, and a second electrode 19, which are sequentially stacked.

A substrate may be additionally disposed under the first electrode 11 or above the second electrode 19. For use as the substrate, any substrate that is used in general organic light-emitting devices may be used, and the substrate may be a glass substrate or a transparent plastic substrate, each having excellent mechanical strength, thermal stability, transparency, surface smoothness, ease of handling, and water resistance.

The first electrode 11 may be formed by depositing or sputtering a material for forming the first electrode 11 on the substrate. The first electrode 11 may be an anode. The material for forming the first electrode 11 may include a material(s) with a high work function to facilitate hole injection. The first electrode 11 may be a reflective electrode, a semi-reflective electrode, or a transmissive electrode. The material for forming the first electrode may be, for example, indium tin oxide (ITO), indium zinc oxide (IZO), tin oxide (SnO₂), and zinc oxide (ZnO). In one or more embodiments, the material for forming the first electrode 11 may be metal, such as magnesium (Mg), aluminum (Al), aluminum-lithium (Al-Li), calcium (Ca), magnesium-indium (Mg-In), or magnesium-silver (Mg-Ag).

The first electrode 11 may have a single-layered structure or a multi-layered structure including two or more layers. For example, the first electrode 11 may have a three-layered structure of ITO/Ag/ITO.

The organic layer 15 is disposed on the first electrode 11.

The organic layer 15 may include a hole transport region, an emission layer, and an electron transport region.

The hole transport region may be disposed between the first electrode 11 and the emission layer.

The hole transport region may include a hole injection layer, a hole transport layer, an electron blocking layer, a buffer layer, or any combination thereof.

The hole transport region may include only either a hole injection layer or a hole transport layer. In one or more embodiments, the hole transport region may have a hole injection layer/hole transport layer structure or a hole injection layer/hole transport layer/electron blocking layer structure, which are sequentially stacked in this stated order from the first electrode 11.

When the hole transport region includes a hole injection layer (HIL), the hole injection layer may be formed on the first electrode 11 by using one or more suitable methods, for example, vacuum deposition, spin coating, casting, and/or Langmuir-Blodgett (LB) deposition.

When a hole injection layer is formed by vacuum deposition, the deposition conditions may vary according to a material that is used to form the hole injection layer, and the structure and thermal characteristics of the hole injection layer. For example, the deposition conditions may include a deposition temperature of about 100°C to about 500 °C, a vacuum pressure of about 10⁻⁸ to about 10⁻³ torr (wherein 1 torr = 133.322 Pa), and a deposition rate of about 0.01 Å/sec to about 100 Å/sec. However, the deposition conditions are not limited thereto. When the hole injection layer is formed using spin coating, coating conditions may vary according to the material used to form the hole injection layer, and the structure and thermal properties of the hole injection layer. For example, a coating speed may be from about 2,000 rpm to about 5,000 rpm (wherein 1 rpm = 1/60 Hz), and a temperature at which a heat treatment is performed to remove a solvent after coating may be from about 80 °C to about 200 °C. However, the coating conditions are not limited thereto.

Conditions for forming a hole transport layer and an electron blocking layer may be understood by referring to conditions for forming the hole injection layer.

The hole transport region may include m-MTDATA, TDATA, 2-TNATA, NPB, β-NPB, TPD, spiro-TPD, spiro-NPB, methylated-NPB, TAPC, HMTPD, 4,4',4"-tris(N-carbazolyl)triphenylamine (TCTA), polyaniline/dodecylbenzenesulfonic acid (PANI/DBSA), poly(3,4-ethylenedioxythiophene)/poly(4-styrenesulfonate) (PEDOT/PSS), polyaniline/camphor sulfonicacid (PANI/CSA), polyaniline/poly(4-styrenesulfonate) (PANI/PSS), a compound represented by Formula 201 below, a compound represented by Formula 202 below, or any combination thereof:

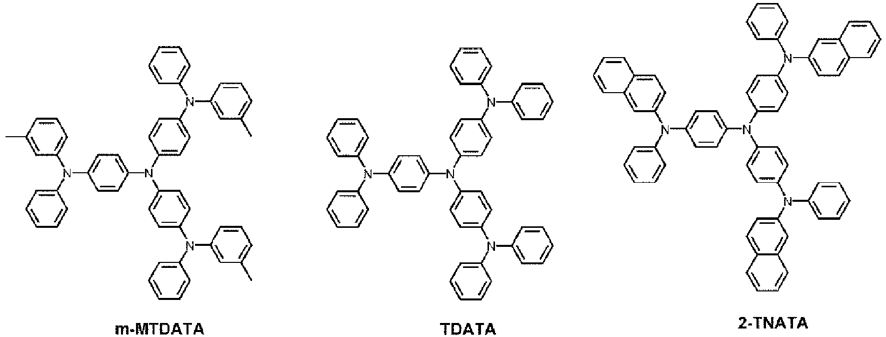

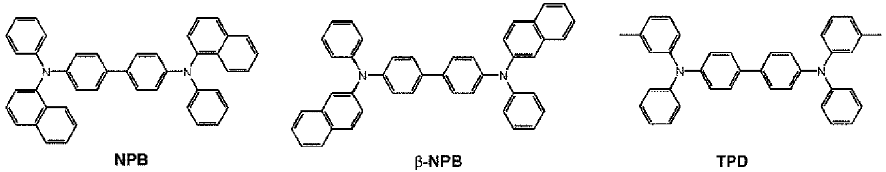

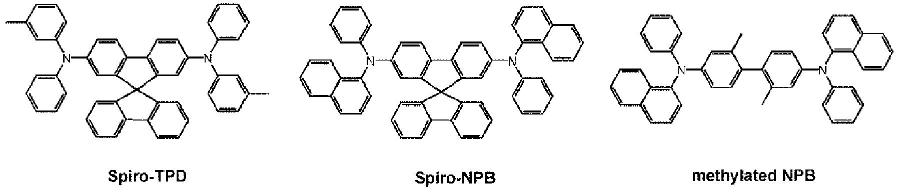

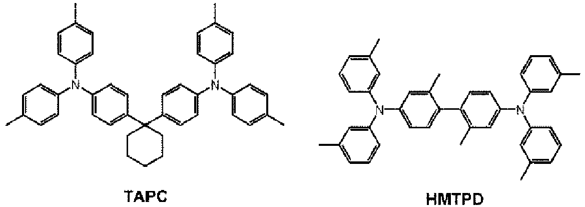

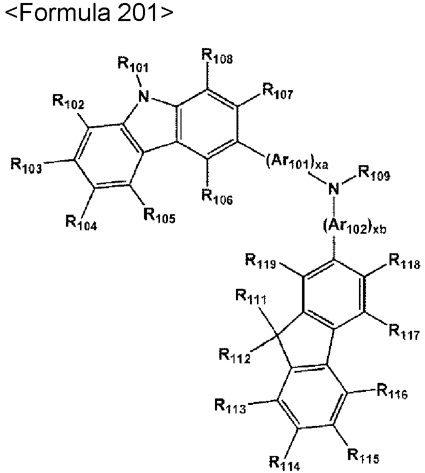

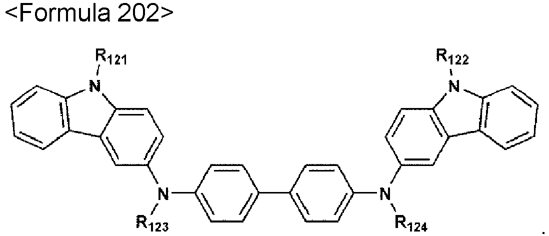

Ar₁₀₁ to Ar₁₀₂ in Formula 201 may each independently be:
a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, or a perylenylene group, each unsubstituted or substituted with deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ cycloalkenyl group, a C₁-C₁₀ heterocycloalkyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, or any combination thereof.

xa and xb in Formula 201 may each independently be an integer from 0 to 5, or may be 0, 1 or 2. For example, xa may be 1 and xb may be 0.

R₁₀₁ to R₁₀₈, R₁₁₁ to R₁₁₉, and R₁₂₁ to R₁₂₄ in Formulae 201 and 202 may each independently be:
hydrogen, deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group (for example, a methyl group, an ethyl group, a propyl group, a butyl group, pentyl group, a hexyl group, and the like), or a C₁-C₁₀ alkoxy group (for example, a methoxy group, an ethoxy group, a propoxy group, a butoxy group, a pentoxy group, and the like);
a C₁-C₁₀ alkyl group or a C₁-C₁₀ alkoxy group, each substituted with deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, or any combination thereof;
a phenyl group, a naphthyl group, an anthracenyl group, a fluorenyl group, or a pyrenyl group, each unsubstituted or substituted with deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a C₁-C₁₀ alkoxy group, or any combination thereof

R₁₀₉ in Formula 201 may be:
a phenyl group, a naphthyl group, an anthracenyl group, or a pyridinyl group, each unsubstituted or substituted with deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, a naphthyl group, an anthracenyl group, a pyridinyl group, or any combination thereof.

In one embodiment, the compound represented by Formula 201 may be represented by Formula 201A below:

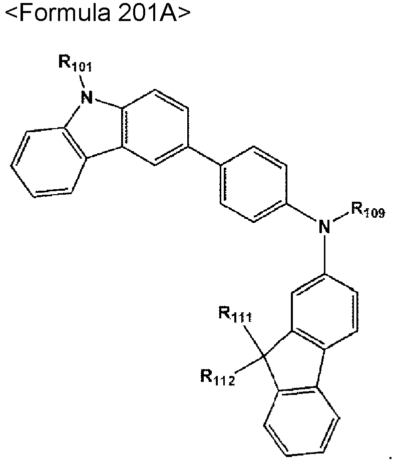

Detailed descriptions of R₁₀₁, R₁₁₁, R₁₁₂, and R₁₀₉ in Formula 201A are the same as described above.

For example, the hole transport region may include at least one of compounds HT1 to HT20 illustrated below:

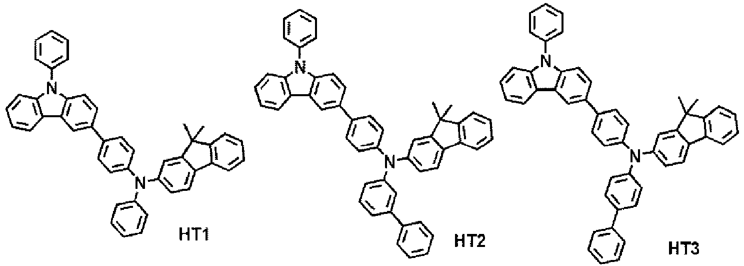

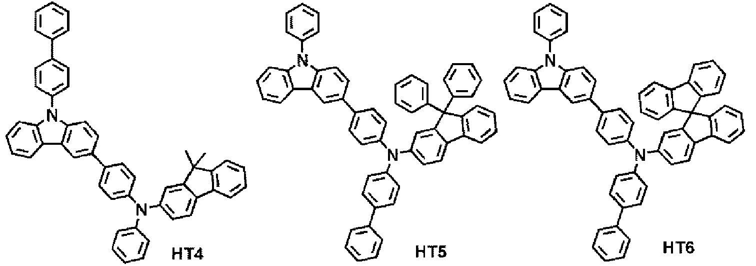

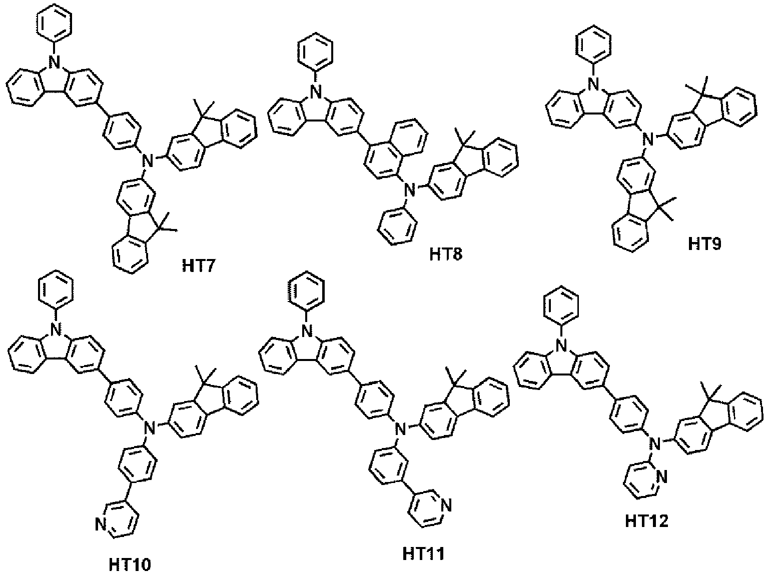

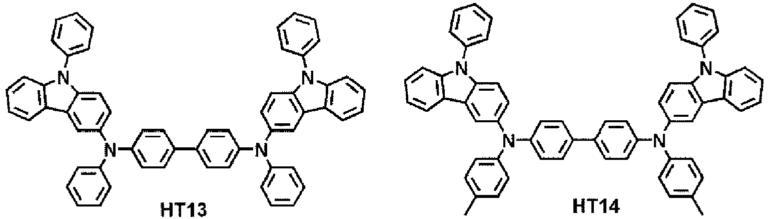

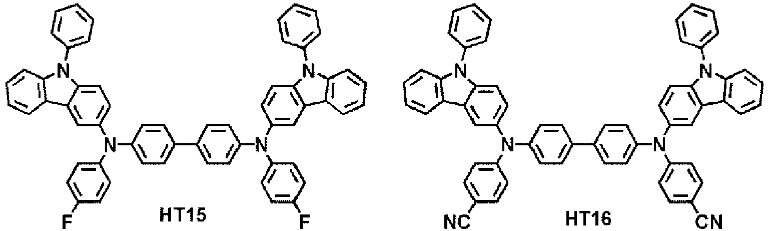

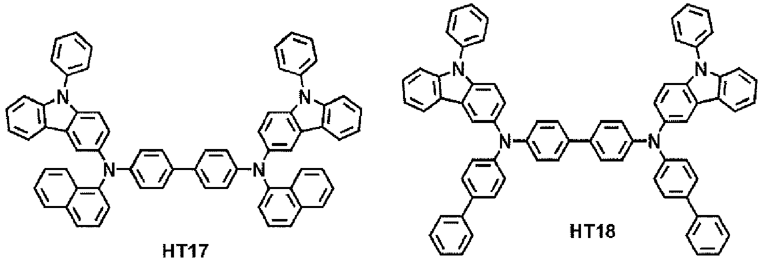

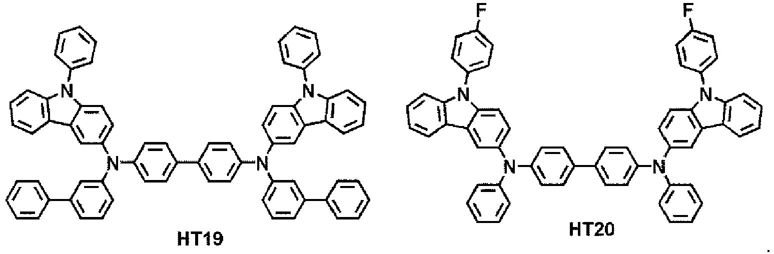

A thickness of the hole transport region may be from about 100Å to about 10,000 Å, for example, about 100 Å to about 1,000 Å. When the hole transport region includes a hole injection layer, a hole transport layer, an electron blocking layer or combination thereof, a thickness of the hole injection layer may be in a range of about 100 Å to about 10000 Å, for example, about 100 Å to about 1,000 Å,and a thickness of the hole transport layer may be in a range of about 50 Å to about 2,000 Å,for example about 100 Å to about 1,500 Å. When the thicknesses of the hole transport region, the hole injection layer, and the hole transport layer are within these ranges, satisfactory hole transporting characteristics may be obtained without a substantial increase in driving voltage.

The hole transport region may further include, in addition to these materials, a charge-generation material for the improvement of conductive properties. The charge-generation material may be homogeneously or non-homogeneously dispersed in the hole transport region.

The charge-generation material may be, for example, a p-dopant. The p-dopant may be a quinone derivative, a metal oxide, a cyano group-containing compound, or any combination thereof. Non-limiting examples of the p-dopant are a quinone derivative, such as tetracyanoquinonedimethane (TCNQ), 2,3,5,6-tetrafluoro-tetracyano-1,4-benzoquinonedimethane (F4-TCNQ) and F6-TCNNQ; a metal oxide, such as a tungsten oxide or a molybdenium oxide; and a cyano group-containing compound, such as Compound HT-D1 below:

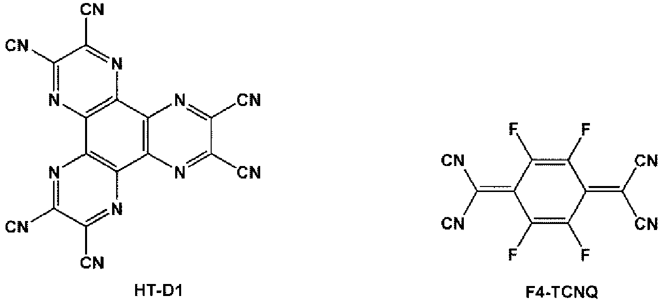

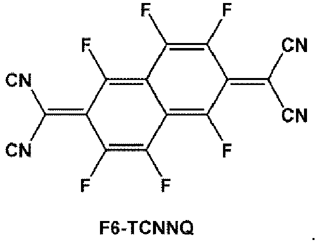

The hole transport region may include a buffer layer.

Also, the buffer layer may compensate for an optical resonance distance according to a wavelength of light emitted from the emission layer, and thus, efficiency of a formed organic light-emitting device may be improved.

Meanwhile, when the hole transport region includes an electron blocking layer, a material for the electron blocking layer may be materials for the hole transport region described above, materials for a host to be explained later, or any combination thereof. However, the material for the electron blocking layer is not limited thereto. For example, when the hole transport region includes an electron blocking layer, a material for the electron blocking layer may be mCP, which will be explained later.

Then, an emission layer may be formed on the hole transport region by vacuum deposition, spin coating, casting, LB deposition, or the like. When the emission layer is formed by vacuum deposition or spin coating, the deposition or coating conditions may be similar to those applied in forming the hole injection layer although the deposition or coating conditions may vary according to a compound that is used to form the emission layer.

The emission layer may include a host and a dopant, and the dopant may include the organometallic compound represented by Formula 1.

The host may include TPBi, TBADN, ADN (also referred to as "DNA"), CBP, CDBP, TCP, mCP, Compounds H50, Compound H51, Compound H52, or any combination thereof:

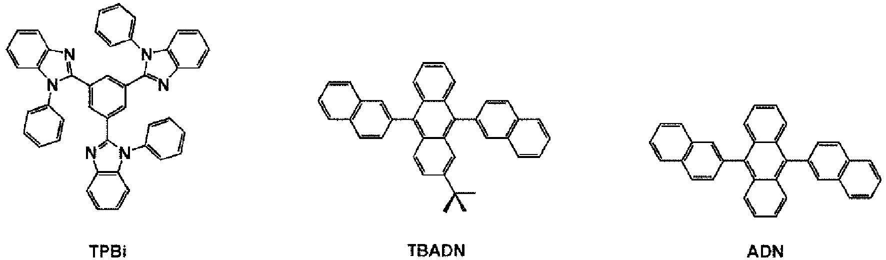

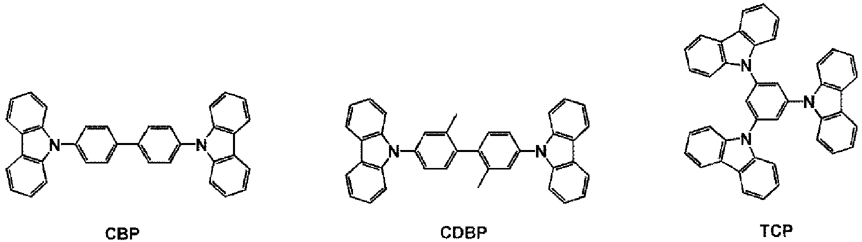

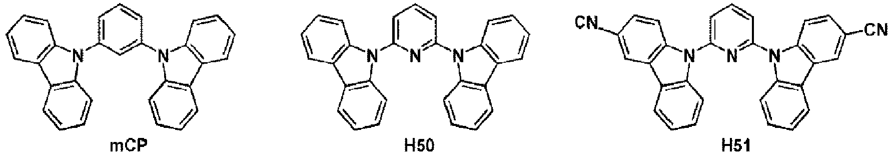

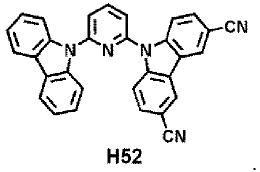

When the organic light-emitting device is a full-color organic light-emitting device, the emission layer may be patterned into a red emission layer, a green emission layer, and/or a blue emission layer. In one or more embodiments, due to a stacked structure including a red emission layer, a green emission layer, and/or a blue emission layer, the emission layer may emit white light.

When the emission layer includes a host and a dopant, an amount of the dopant may be in a range of about 0.01 parts by weight to about 15 parts by weight based on 100 parts by weight of the host.

A thickness of the emission layer may be in a range of about 100 Å to about 1,000 Å,for example, about 200 Å to about 600 Å. When the thickness of the emission layer is within this range, excellent light-emission characteristics may be obtained without a substantial increase in driving voltage.

Then, an electron transport region may be disposed on the emission layer.

The electron transport region may include a hole blocking layer, an electron transport layer, an electron injection layer, or any combination thereof.

For example, the electron transport region may have a hole blocking layer/electron transport layer/electron injection layer structure or an electron transport layer/electron injection layer structure. The electron transport layer may have a single-layered structure or a multi-layered structure including two or more different materials.

Conditions for forming the hole blocking layer, the electron transport layer, and the electron injection layer which constitute the electron transport region may be understood by referring to the conditions for forming the hole injection layer.

When the electron transport region includes a hole blocking layer, the hole blocking layer may include, for example, BCP, Bphen, BAIq, or any combination thereof:

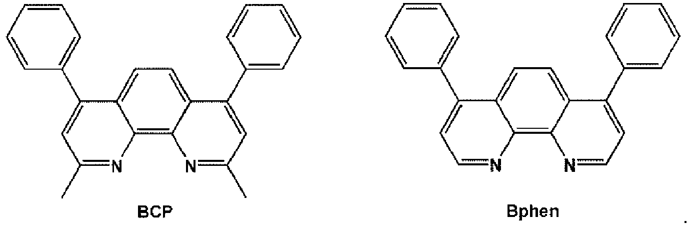

A thickness of the hole blocking layer may be from about 20 Å to about 1,000 Å,for example, about 30 Å to about 600 Å. When the thickness of the hole blocking layer is within these ranges, the hole blocking layer may have excellent hole blocking characteristics without a substantial increase in driving voltage.

The electron transport layer may include BCP, Bphen, Alq₃, BAlq, TAZ, NTAZ, or any combination thereof:

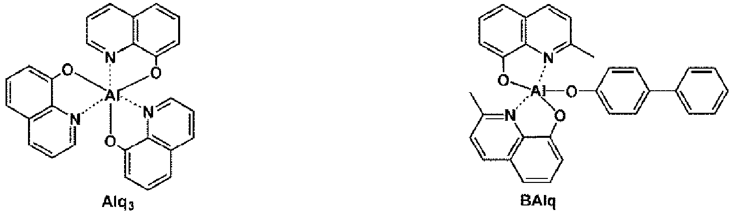

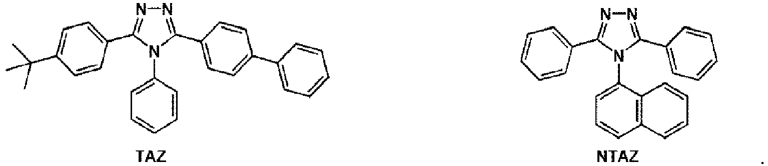

In one or more embodiments, the electron transport layer may include at least one of ET1 to ET25:

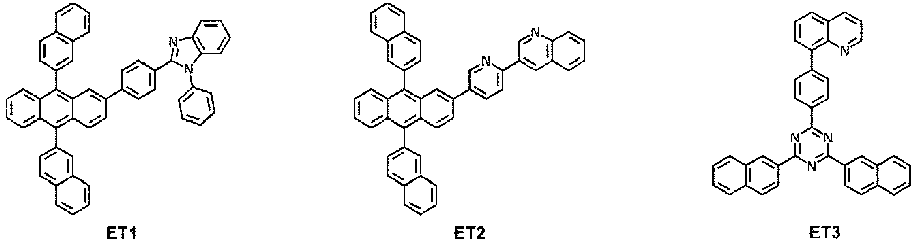

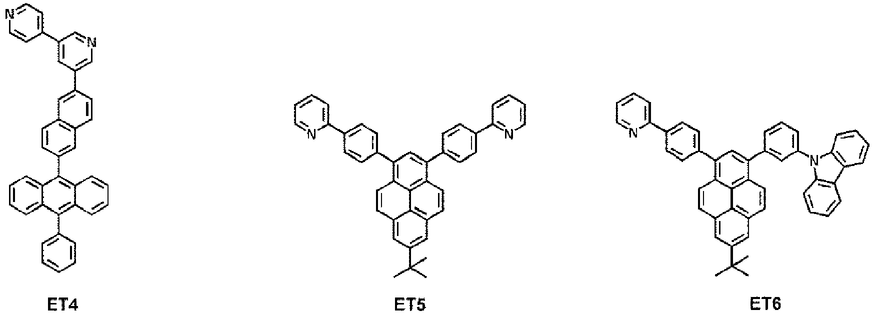

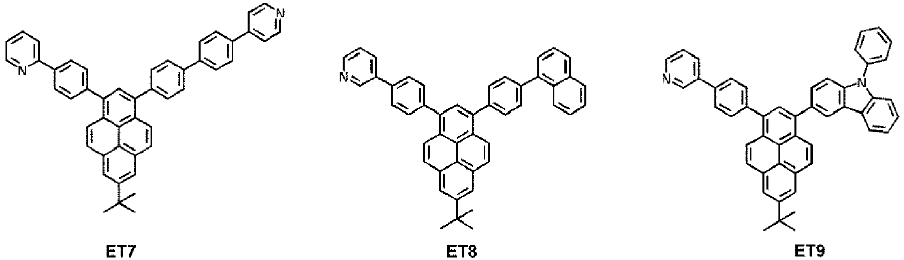

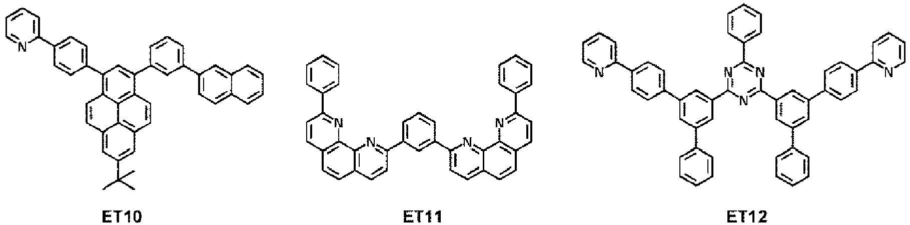

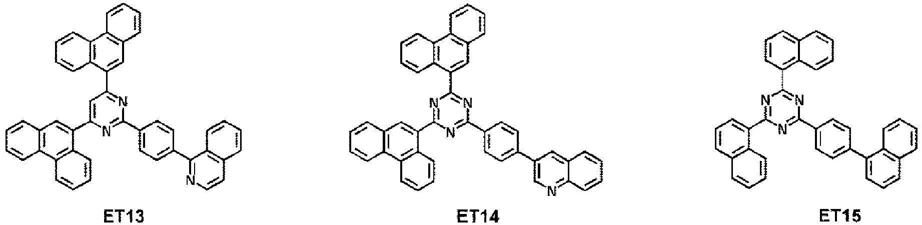

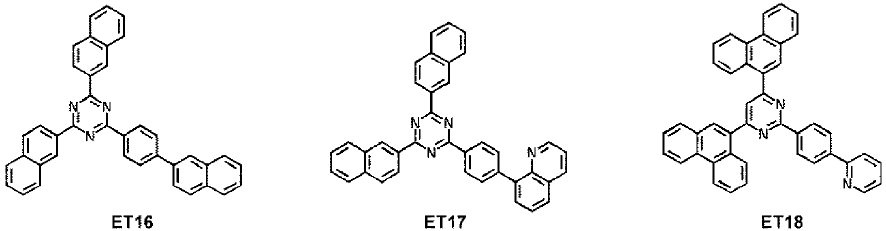

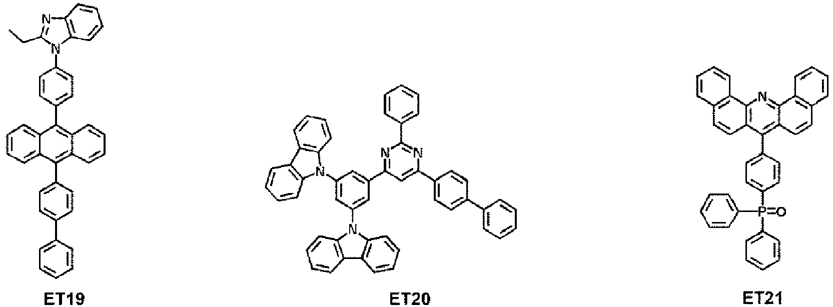

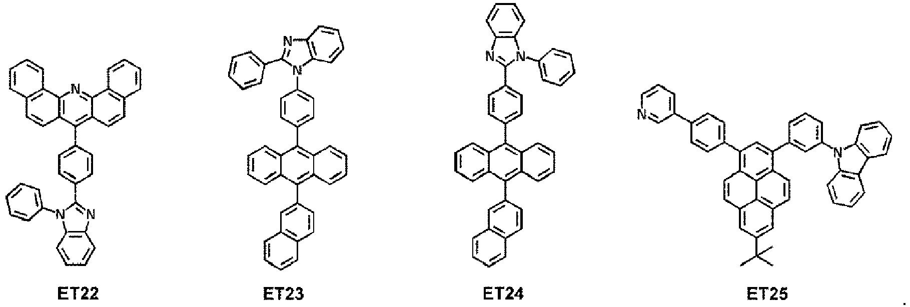

A thickness of the electron transport layer may be from about 100 Å to about 1,000 Å,for example, about 150 Å to about 500 Å. When the thickness of the electron transport layer is within the range described above, the electron transport layer may have satisfactory electron transport characteristics without a substantial increase in driving voltage.

Also, the electron transport layer may further include, in addition to the materials described above, a metal-containing material.

The metal-containing material may include a Li complex. The Li complex may include, for example, Compound ET-D1 (LiQ), Compound ET-D2, or combination thereof:

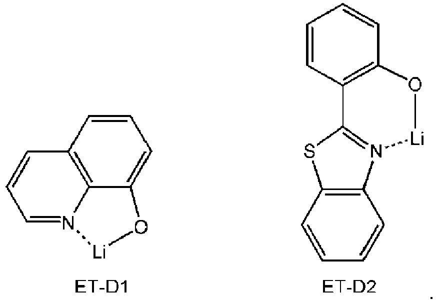

The electron transport region may include an electron injection layer that promotes flow of electrons from the second electrode 19 thereinto.

The electron injection layer may include LiF, NaCl, CsF, Li₂O, BaO, or any combination thereof.

A thickness of the electron injection layer may be from about 1 Å to about 100 Å, for example, about 3 Å to about 90 Å. When a thickness of the electron injection layer is within these ranges, satisfactory electron injection characteristics may be obtained without substantial increase in driving voltage.

The second electrode 19 is disposed on the organic layer 15. The second electrode 19 may be a cathode. A material for forming the second electrode 19 may be a metal, an alloy, an electrically conductive compound, and a combination thereof, which have a relatively low work function. For example, lithium (Li), magnesium (Mg), aluminum (Al), aluminum-lithium (Al-Li), calcium (Ca), magnesium-indium (Mg-In), or magnesium-silver (Mg-Ag) may be formed as the material for forming the second electrode 19. To manufacture a top-emission type light-emitting device, a transmissive electrode formed using ITO or IZO may be used as the second electrode 19.

Hereinbefore, the organic light-emitting device according to an embodiment has been described in connection with the FIGURE.

In one or more embodiments, the organic light-emitting device may be included in an electronic apparatus. Accordingly, provided is an electronic apparatus including the organic light-emitting device. The electronic apparatus may include, for example, a display, an illuminator, and a sensor.

Another aspect of the present disclosure provides a diagnostic composition including at least one of the organometallic compound represented by Formula 1.

The organometallic compound represented by Formula 1 provides high luminescent efficiency. Accordingly, a diagnostic composition including the organometallic compound may have high diagnostic efficiency.

The diagnostic composition may be used in various applications including a diagnosis kit, a diagnosis reagent, a biosensor, and a biomarker.

The term "C₁-C₆₀ alkyl group" as used herein refers to a linear or branched saturated aliphatic hydrocarbon monovalent group having 1 to 60 carbon atoms, and the term "C₁-C₆₀ alkylene group" as used herein refers to a divalent group having the same structure as the C₁-C₆₀ alkyl group.

Non-limiting examples of the C₁-C₆₀ alkyl group, the C₁-C₂₀ alkyl group, and/or the C₁-C₁₀ alkyl group are a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sechexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, and a tert-decyl group, each unsubstituted or substituted with a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sechexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, a tert-decyl group, or any combination thereof. For example, Formula 9-33 may be a branched C₆ alkyl group, and may be a tert-butyl group that is substituted with two methyl groups.

The term "C₁-C₆₀ alkoxy group" used herein refers to a monovalent group represented by -OA₁₀₁ (wherein A₁₀₁ is the C₁-C₆₀ alkyl group). Non-limiting examples of the C₁-C₆₀ alkoxy group, the C₁-C₂₀ alkoxy group, or the C₁-C₁₀ alkoxy group are a methoxy group, an ethoxy group, a propoxy group, a butoxy group, and a pentoxy group.

The term "C₂-C₆₀ alkenyl group" as used herein refers to a hydrocarbon group having at least one carbon-carbon double bond in the middle or at the terminus of the C₂-C₆₀ alkyl group, and examples thereof include an ethenyl group, a propenyl group, and a butenyl group. The term "C₂-C₆₀ alkenylene group" as used herein refers to a divalent group having the same structure as the C₂-C₆₀ alkenyl group.

The term "C₂-C₆₀ alkynyl group" as used herein refers to a hydrocarbon group having at least one carbon-carbon triple bond in the middle or at the terminus of the C₂-C₆₀ alkyl group, and examples thereof include an ethynyl group, and a propynyl group. The term "C₂-C₆₀ alkynylene group" as used herein refers to a divalent group having the same structure as the C₂-C₆₀ alkynyl group.

The term "C₃-C₁₀ cycloalkyl group" as used herein refers to a monovalent saturated hydrocarbon monocyclic group having 3 to 10 carbon atoms, and the term "C₃-C₁₀ cycloalkylene group" as used herein refers to a divalent group having the same structure as the C₃-C₁₀ cycloalkyl group.

Non-limiting examples of the C₃-C₁₀ cycloalkyl group are a cyclopropyl group, a cyclobutyl group, a cyclopentyl group, a cyclohexyl group, a cycloheptyl group, a cyclooctyl group, an adamantyl group, a bicyclo[1.1.1]pentyl group, a bicyclo[2.1.1]hexyl group, a bicyclo[2.2.1]heptyl group(a norbornyl group), and a bicyclo[2.2.2]octyl group.

The term "C₁-C₁₀ heterocycloalkyl group" as used herein refers to a monovalent saturated monocyclic group having at least one heteroatom selected from N, O, P, Si and S as a ring-forming atom and 1 to 10 carbon atoms, and the term "C₁-C₁₀ heterocycloalkylene group" as used herein refers to a divalent group having the same structure as the C₁-C₁₀ heterocycloalkyl group.

Non-limiting examples of the C₁-C₁₀ heterocycloalkyl group are a silolanyl group, a silinanyl group, a tetrahydrofuranyl group, a tetrahydro-2H-pyranyl group, and a tetrahydrothiophenyl group.

The term "C₃-C₁₀ cycloalkenyl group" as used herein refers to a monovalent monocyclic group that has 3 to 10 carbon atoms and at least one carbon-carbon double bond in the ring thereof and no aromaticity, and non-limiting examples thereof include a cyclopentenyl group, a cyclohexenyl group, and a cycloheptenyl group. The term "C₃-C₁₀ cycloalkenylene group" as used herein refers to a divalent group having the same structure as the C₃-C₁₀ cycloalkenyl group.

The term "C₁-C₁₀ heterocycloalkenyl group" as used herein refers to a monovalent monocyclic group that has at least one heteroatom selected from N, O, P, Si, and S as a ring-forming atom, 1 to 10 carbon atoms, and at least one double bond in its ring. Examples of the C₁-C₁₀ heterocycloalkenyl group are a 2,3-dihydrofuranyl group, and a 2,3-dihydrothiophenyl group. The term "C₁-C₁₀ heterocycloalkenylene group" as used herein refers to a divalent group having the same structure as the C₁-C₁₀ heterocycloalkenyl group.

The term "C₆-C₆₀ aryl group" as used herein refers to a monovalent group having a carbocyclic aromatic system having 6 to 60 carbon atoms, and the term "C₆-C₆₀ arylene group" as used herein refers to a divalent group having a carbocyclic aromatic system having 6 to 60 carbon atoms. Non-limiting examples of the C₆-C₆₀ aryl group include a phenyl group, a naphthyl group, an anthracenyl group, a phenanthrenyl group, a pyrenyl group, and a chrysenyl group. When the C₆-C₆₀ aryl group and the C₆-C₆₀ arylene group each include two or more rings, the rings may be fused to each other.

The term "C₇-C₆₀ alkylaryl group" used herein refers to a C₆-C₅₉ aryl group substituted with at least one C₁-C₅₄ alkyl group.

The term "C₁-C₆₀ heteroaryl group" as used herein refers to a monovalent group having a heterocyclic aromatic system that has at least one heteroatom selected from N, O, P, Si, and S as a ring-forming atom, in addition to 1 to 60 carbon atoms. The term "C₁-C₆₀ heteroarylene group" as used herein refers to a divalent group having a heterocyclic aromatic system that has at least one heteroatom selected from N, O, P, Si, and S as a ring-forming atom, in addition to 1 to 60 carbon atoms. Non-limiting examples of the C₁-C₆₀ heteroaryl group include a pyridinyl group, a pyrimidinyl group, a pyrazinyl group, a pyridazinyl group, a triazinyl group, a quinolinyl group, and an isoquinolinyl group. When the C₁-C₆₀ heteroaryl group and the C₁-C₆₀ heteroarylene group each include two or more rings, the rings may be fused to each other.

The term "C₂-C₆₀ alkylheteroaryl group" used herein refers to a C₁-C₅₉ heteroaryl group substituted with at least one C₁-C₅₉ alkyl group.

The term "C₆-C₆₀ aryloxy group" used herein indicates -OA₁₀₂ (wherein A₁₀₂ is the C₆-C₆₀ aryl group), a C₆-C₆₀ arylthio group used herein indicates -SA₁₀₃ (wherein A₁₀₃ is the C₆-C₆₀ aryl group), and a C₁-C₆₀ alkylthio group used herein indicates -SA₁₀₄ (wherein A₁₀₄ is the C₁-C₆₀ alkyl group).

The term "monovalent non-aromatic condensed polycyclic group" as used herein refers to a monovalent group (for example, having 8 to 60 carbon atoms) having two or more rings condensed to each other, only carbon atoms as ring-forming atoms, and no aromaticity in its entire molecular structure. Examples of the monovalent non-aromatic condensed polycyclic group include a fluorenyl group. The term "divalent non-aromatic condensed polycyclic group" as used herein refers to a divalent group having the same structure as the monovalent non-aromatic condensed polycyclic group.

The term "monovalent non-aromatic condensed heteropolycyclic group" as used herein refers to a monovalent group (for example, having 2 to 60 carbon atoms) having two or more rings condensed to each other, a heteroatom selected from N, O, P, Si, and S, other than carbon atoms, as a ring-forming atom, and no aromaticity in its entire molecular structure. Non-limiting examples of the monovalent non-aromatic condensed heteropolycyclic group include a carbazolyl group. The term "divalent non-aromatic condensed heteropolycyclic group" as used herein refers to a divalent group having the same structure as the monovalent non-aromatic condensed heteropolycyclic group.

The term "C₅-C₃₀ carbocyclic group" as used herein refers to a saturated or unsaturated cyclic group having, as a ring-forming atom, 5 to 30 carbon atoms only. The C₅-C₃₀ carbocyclic group may be a monocyclic group or a polycyclic group. The term " C₅-C₃₀ carbocyclic group (which is unsubstituted or substituted with at least one R₁ₐ)" may include, for example, an adamantane group, a norbornene group, a bicyclo[1.1.1]pentane group, a bicyclo[2.1.1]hexane group, a bicyclo[2.2.1]heptane group (a norbornane group), a bicyclo[2.2.2]octane group, a cyclopentane group, a cyclohexane group, a cyclohexene group, a benzene group, a naphthalene group, an anthracene group, a phenanthrene group, a triphenylene group, a pyrene group, a chrysene group, a 1,2,3,4-tetrahydronaphthalene group, cyclopentadiene group, and a fluorene group, each being unsubstituted or substituted with at least one R₁ₐ.

The term "C₁-C₃₀ heterocyclic group" as used herein refers to a saturated or unsaturated cyclic group having, as a ring forming atom, at least one heteroatom selected from N, O, P, Si, Se, Ge, B and S other than 1 to 30 carbon atoms, The C₁-C₃₀ heterocyclic group may be a monocyclic group or a polycyclic group. The C₁-C₃₀ heterocyclic group (which is unsubstituted or substituted with at least one R₁ₐ)" may include, for example, a thiophene group, a furan group, a pyrrole group, a a silole group, a borole group, a phosphole group, a selenophene group, a germole group, a benzothiophene group, a benzofuran group, an indole group, an indene group, a benzosilole group, a benzoborole group, a benzophosphole group, a benzoselenophene group, a benzogermole group, a dibenzothiophene group, a dibenzofuran group, a carbazole group, a dibenzosilole group, a dibenzoborole group, a dibenzophosphole group, a dibenzoselenophene group, a dibenzogermole group, a dibenzothiophene 5-oxide group, 9H-fluorene-9-one group, a dibenzothiophene 5,5-dioxide group, an azabenzothiophene group, an azabenzofuran group, an azaindole group, an azaindene group, an azabenzosilole group, an azabenzoborole group, an azabenzophosphole group, an azabenzoselenophene group, an azabenzogermole group, an azadibenzothiophene group, an azadibenzofuran group, an azacarbazole group, an azafluorene group, an azadibenzosilole group, an azadibenzoborole group, an azadibenzophosphole group, an azadibenzoselenophene group, an azadibenzogermole group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrazole group, an imidazole group, a triazole group, an oxazole group, an isooxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, and a 5,6,7,8-tetrahydroquinoline group, each being unsubstituted or substituted with at least one R₁ₐ.

The terms "fluorinated C₁-C₆₀ alkyl group (or a fluorinated C₁-C₂₀ alkyl group, or the like)", "fluorinated C₃-C₁₀ cycloalkyl group", "fluorinated C₂-C₁₀ heterocycloalkyl group", "or "fluorinated phenyl group" as used herein refer to a C₁-C₆₀ alkyl group (or, C₁-C₂₀ alkyl group, or the like) substituted with at least one fluoro group (-F), a C₃-C₁₀ cycloalkyl group substituted with at least one fluoro group (-F), a C₂-C₁₀ heterocycloalkyl group substituted with at least one fluoro group (-F), and a phenyl substituted with at least one fluoro group (-F), respectively. For example, the term "the fluorinated C₁ alkyl group (that is, the fluorinated methyl group)" includes -CF₃, -CF₂H, and -CFH₂. The "fluorinated C₁-C₆₀ alkyl group (or the fluorinated C₁-C₂₀ alkyl group, or the like)", "the fluorinated C₃-C₁₀ cycloalkyl group", "the fluorinated C₂-C₁₀ heterocycloalkyl group" and "the fluorinated phenyl group" may be i) a fully fluorinated C₁-C₆₀ alkyl group (or, fully fluorinated C₁-C₂₀ alkyl group, or the like), a fully fluorinated C₃-C₁₀ cycloalkyl group, a fully fluorinated C₂-C₁₀ heterocycloalkyl group, or a fully fluorinated phenyl group, each group in which all hydrogen are substituted with a fluoro group, or ii) a partially fluorinated C₁-C₆₀ alkyl group (or, a partially fluorinated C₁-C₂₀ alkyl group, or the like), a partially fluorinated C₃-C₁₀ cycloalkyl group, a partially fluorinated C₂-C₁₀ heterocycloalkyl group, or, a partially fluorinated phenyl group, each group in which some hydrogen are substituted with a fluoro group.

The terms "deuterated C₁-C₆₀ alkyl group (or a deuterated C₁-C₂₀ alkyl group, or the like)", "deuterated C₃-C₁₀ cycloalkyl group", "deuterated C₂-C₁₀ heterocycloalkyl group", "or "deuterated phenyl group" as used herein refer to a C₁-C₆₀ alkyl group (or, C₁-C₂₀ alkyl group, or the like) substituted with at least one deuterium, a C₃-C₁₀ cycloalkyl group substituted with at least one deuterium, a C₂-C₁₀ heterocycloalkyl group substituted with at least one deuterium, and a phenyl substituted with at least one deuterium, respectively. For example, the term "the deuterated C₁ alkyl group (that is, the deuterated methyl group)" includes -CD₃, -CD₂H, and -CDH₂ and the term "deuterated C₃-C₁₀ cycloalkyl group" refer to Formula 10-501. The "deuterated C₁-C₆₀ alkyl group (or the deuterated C₁-C₂₀ alkyl group, or the like)", "the deuterated C₃-C₁₀ cycloalkyl group", "the deuterated C₂-C₁₀ heterocycloalkyl group" and "the deuterated phenyl group" may be i) a fully deuterated C₁-C₆₀ alkyl group (or, fully deuterated C₁-C₂₀ alkyl group, or the like), a fully deuterated C₃-C₁₀ cycloalkyl group, a fully deuterated C₂-C₁₀ heterocycloalkyl group, or a fully deuterated phenyl group, each group in which all hydrogen are substituted with deuterium, or ii) a partially deuterated C₁-C₆₀ alkyl group (or, a partially deuterated C₁-C₂₀ alkyl group, or the like), a partially deuterated C₃-C₁₀ cycloalkyl group, a partially deuterated C₂-C₁₀ heterocycloalkyl group, or, a partially deuterated phenyl group, each group in which some hydrogen are substituted with deuterium.

The term "(C₁-C₂₀ alkyl)'X' group" as used herein refers to a 'X' group substituted with at least one C₁-C₂₀ alkyl group. For example, the term "(C₁-C₂₀ alkyl)C₃-C₁₀ cycloalkyl group" as used herein refers to a C₃-C₁₀ cycloalkyl group substituted with at least one C₁-C₂₀ alkyl group and the term "(C₁-C₂₀ alkyl)phenyl group" as used herein refers to a phenyl group substituted with at least one C₁-C₂₀ alkyl group. An example of a (C₁ alkyl)phenyl group is a toluyl group.

The terms "an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, and an azadibenzothiophene 5,5-dioxide group" respectively refer to a heterocyclic group having the same backbone as "an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, 9H-fluorene-9-one group, and a dibenzothiophene 5,5-dioxide group" in which at least one of the carbon atoms constituting the cyclic groups is substituted with a nitrogen.

A subsittutnet(s) of the substituted C₅-C₃₀ carbocyclic group, the substituted C₁-C₃₀ heterocyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₁-C₆₀ alkylthio group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₇-C₆₀ alkylaryl group, the substituted C₆-C₆₀ aryloxy group, the substituted C₆-C₆₀ arylthio group, the substituted C₁-C₆₀ heteroaryl group, the substituted C₂-C₆₀ alkylheteroaryl group, the substituted monovalent non-aromatic condensed polycyclic group, and the substituted monovalent non-aromatic condensed heteropolycyclic group may each independently be:
deuterium, -F, -CI, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, or a C₁-C₆₀ alkylthio group;
a C₁-C₆₀ alkyl group, a C₂-C₆₀ alkenyl group, a C₂-C₆₀ alkynyl group, a C₁-C₆₀ alkoxy group, or a C₁-C₆₀ alkylthio group, each substituted with deuterium, -F, -CI, -Br, -I, - CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₇-C₆₀ alkylaryl group, a C₆-C₆₀ aryloxy group, a C₆-C₆₀ arylthio group, a C₁-C₆₀ heteroaryl group, a C₂-C₆₀ alkylheteroaryl group, a monovalent non-aromatic condensed polycyclic group, a monovalent non-aromatic condensed heteropolycyclic group, -N(Q₁₁)(Q₁₂), - Ge(Q₁₃)(Q₁₄)(Q₁₅), -B(Q₁₆)(Q₁₇), -P(=O)(Q₁₈)(Q₁₉), -P(Q₁₈)(Q₁₉), or any combination thereof;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group, each substituted with deuterium, -F, -CI, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₁-C₆₀ alkylthio group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₇-C₆₀ alkylaryl group, a C₆-C₆₀ aryloxy group, a C₆-C₆₀ arylthio group, a C₁-C₆₀ heteroaryl group, a C₂-C₆₀ alkylheteroaryl group, a monovalent non-aromatic condensed polycyclic group, a monovalent non-aromatic condensed heteropolycyclic group, -N(Q₂₁)(Q₂₂), - Ge(Q₂₃)(Q₂₄)(Q₂₅), -B(Q₂₆)(Q₂₇), -P(=O)(Q₂₆)(Q₂₉), -P(Q₂₈)(Q₂₉), or any combination thereof; -N(Q₃₁)(Q₃₂), -Ge(Q₃₃)(Q₃₄)(Q₃₅), -B(Q₃₆)(Q₃₇), -P(=O)(Q₃₆)(Q₃₉) or -P(Q₃₈)(Q₃₉); or any combination thereof.

In the present specification, Q₁ to Q₉, Q₁₁ to Q₁₉, Q₂₁ to Q₂₉, and Q₃₁ to Q₃₉ may each independently be hydrogen; deuterium; -F; -CI; -Br; -I; a hydroxyl group; a cyano group; a nitro group; an amino group; an amidino group; a hydrazine group; a hydrazone group; a carboxylic acid group or a salt thereof; a sulfonic acid group or a salt thereof; a phosphoric acid group or a salt thereof; a C₁-C₆₀ alkyl group unsubstituted or substituted with deuterium, a C₁-C₆₀ alkyl group, a C₆-C₆₀ aryl group, or any combination thereof; a C₂-C₆₀ alkenyl group; a C₂-C₆₀ alkynyl group; a C₁-C₆₀ alkoxy group; a C₃-C₁₀ cycloalkyl group; a C₁-C₁₀ heterocycloalkyl group; a C₃-C₁₀ cycloalkenyl group; a C₁-C₁₀ heterocycloalkenyl group; a C₆-C₆₀ aryl group unsubstituted or substituted with deuterium, a C₁-C₆₀ alkyl group, a C₆-C₆₀ aryl group, or any combination thereof; a C₆-C₆₀ aryloxy group; a C₆-C₆₀ arylthio group; a C₁-C₆₀ heteroaryl group, a monovalent non-aromatic condensed polycyclic group; or a monovalent non-aromatic condensed heteropolycyclic group.

For example, in the present specification, Q₁ to Q₉, Q₁₁ to Q₁₉, Q₂₁ to Q₂₉, and Q₃₁ to Q₃₉ may each independently be
-CH₃, -CD₃, -CD₂H, -CDH₂, -CH₂CH₃, -CH₂CD₃, -CH₂CD₂H, -CH₂CDH₂, - CHDCH₃, -CHDCD₂H, -CHDCDH₂, -CHDCD₃, -CD₂CD₃, -CD₂CD₂H, or -CD₂CDH₂; or
an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, a phenyl group, a biphenyl group, or a naphthyl group, each unsubstituted or substituted with deuterium, a C₁-C₁₀ alkyl group, a phenyl group, or any combination thereof.

Hereinafter, a compound and an organic light-emitting device according to embodiments are described in detail with reference to Synthesis Example and Examples. However, the organic light-emitting device is not limited thereto. The wording "B was used instead of A" used in describing Synthesis Examples means that an amount of A used was identical to an amount of B used, in terms of a molar equivalent.

### [Examples]

### Synthesis Example 1 (Compound 125)

### Synthesis of Compound 125A

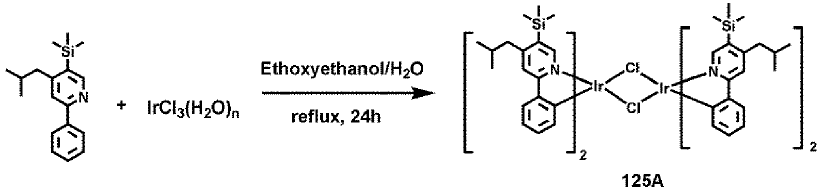

4-isobutyl-2-phenyl-5-(trimethylsilyl)pyridine (8.0 g, 28.4 mmol) and iridium chloride (4.4 g, 12.6 mmol) were mixed with 120 mL of ethoxyethanol and 40 mL of distilled water. The mixed solution was stirred under reflux for 24 hours, and the temperature was lowered to room temperature. A solid produced therefrom was separated by filtration, and then, washed thoroughly with water/methanol/hexane in the stated order. The resulting solid was then dried in a vacuum oven, thereby obtaining 7.5 g of Compound 125A (yield of 75 %).

### Synthesis of Compound 125B

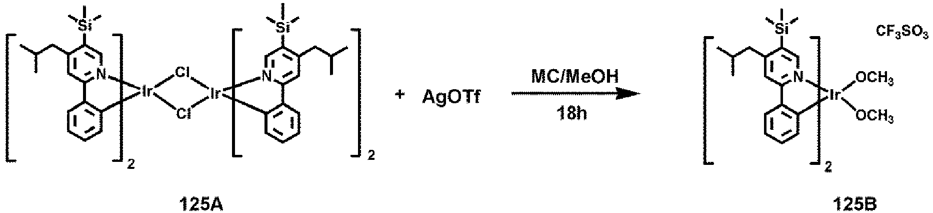

Compound 125A (1.6 g, 1.0 mmol) was mixed with 45 ml of methylenechloride, and a mixture of AgOTf (Silver trifluoromethanesulfonate) (0.5 g, 2.1 mmol) and 15 ml of methanol was added thereto. Afterwards, the mixed solution was stirred at room temperature for 18 hours while blocking the light with aluminum foil. The resulting solution was then filtered through celite to remove a solid produced therefrom, and the solvent was removed from the filtrate under reduced pressure, and a solid (Compound 125B) produced therefrom was used in the next reaction without additional purification.

### Synthesis of Compound 125

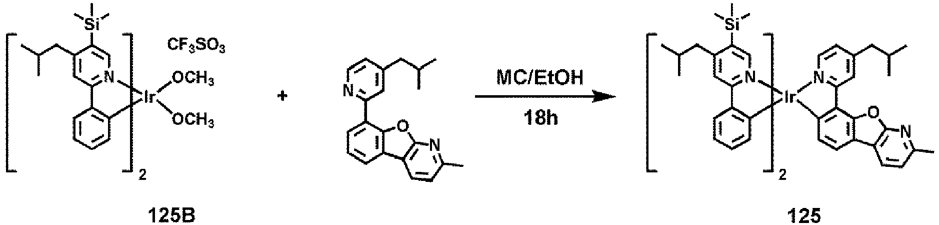

Compound 125B (2.0 g, 2.1 mmol) and 8-(4-isobutylpyridin-2-yl)-2-methylbenzofuro[2,3-b]pyridine (0.8 g, 2.4 mmol) were mixed with 40 ml of a mixture of MC and ethanol. The mixed solution was stirred under reflux for 18 hours, and the temperature was lowered down. The resulting solution was filtered, and a solid obtained therefrom was washed thoroughly with ethanol and hexane. The resulting product was subjected to column chromatography under the MC: hexane conditions, thereby obtaining 0.8 g of Compound 125 (yield of 36 %). Substances of the compound were identified by the Mass Spectrum and HPLC analysis.
HRMS(MALDI) calcd for C₅₇H₆₇IrN₄OSi₂: m/z 1072.4483 Found: 1072.4490

### Synthesis Example 2 (Compound 128)

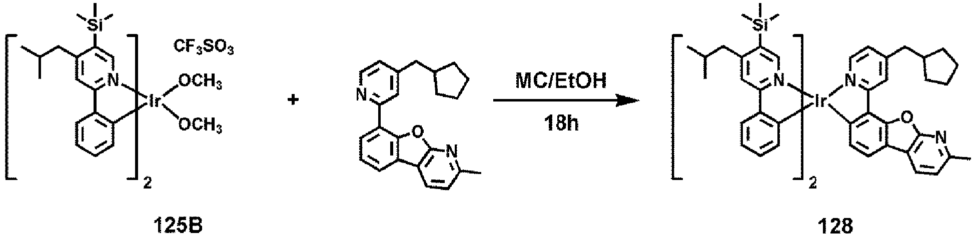

0.9 g of Compound 128 (yield of 40 %) was obtained in the same manner as in the synthesis of Compound 125 according to Synthesis Example 1, except that 8-(4-(cyclopentylmethyl)pyridin-2-yl)-2-methylbenzofuro[2,3-b]pyridine was used instead of 8-(4-isobutylpyridin-2-yl)-2-methylbenzofuro[2,3-b]pyridine. Substances of the compound were identified by the Mass Spectrum and HPLC analysis.
HRMS(MALDI) calcd for Cs₉H₆₉IrN₄OSi₂: m/z 1098.4639, Found: 1098.4631

### Synthesis Example 3 (Compound 163)

### Synthesis of Compound 163A

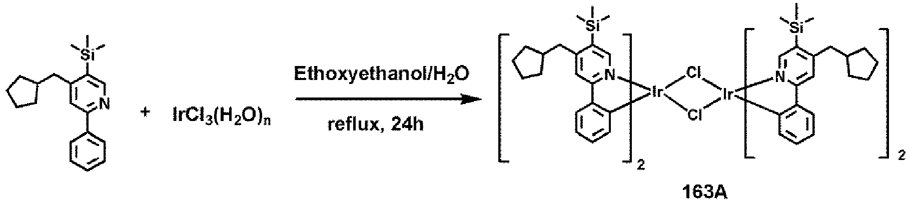

6.5 g of Compound 163A (yield of 65 %) was obtained in the same manner as in the synthesis of Compound 125A according to Synthesis Example 1, except that 4-(cyclopentylmethyl)-2-phenyl-5-(trimethylsilyl)pyridine was used instead of 4-isobutyl-2-phenyl-5-(trimethylsilyl)pyridine.

### Synthesis of Compound 163B

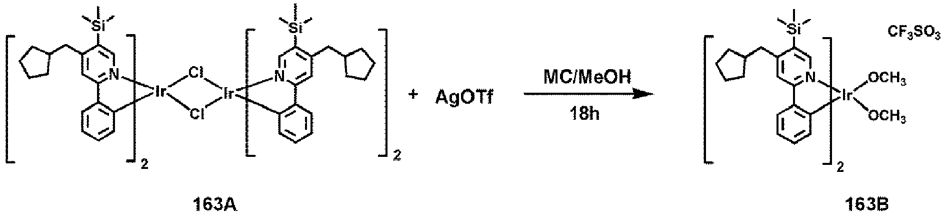

Compound 163B was obtained in the same manner as in the synthesis of Compound 125B according to Synthesis Example 1, except that Compound 163A was used instead of Compound 125A. Compound 163B thus obtained was used in the next reaction without additional purification.

### Synthesis of Compound 163

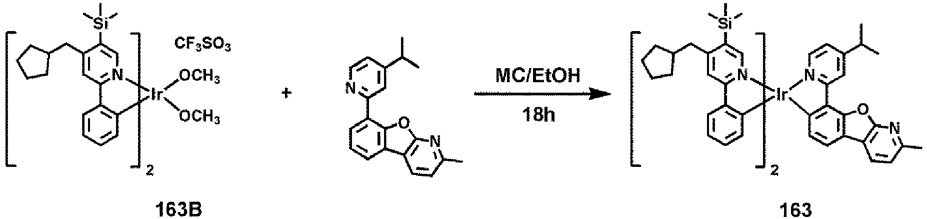

0.65 g of Compound 163 (yield of 29 %) was obtained in the same manner as in the synthesis of Compound 125 according to Synthesis Example 1, except that Compound 163B was used instead of Compound 125B and 8-(4-isopropylpyridin-2-yl)-2-methylbenzofuro[2,3-b]pyridine was used instead of 8-(4-isobutylpyridin-2-yl)-2-methylbenzofuro[2,3-b]pyridine. Substances of the compound were identified by the Mass Spectrum and HPLC analysis.
HRMS(MALDI) calcd for C₆₀H₆₉IrN₄OSi₂: m/z 1110.4639, Found: 1110.4644

### Synthesis Example 4 (Compound 365)

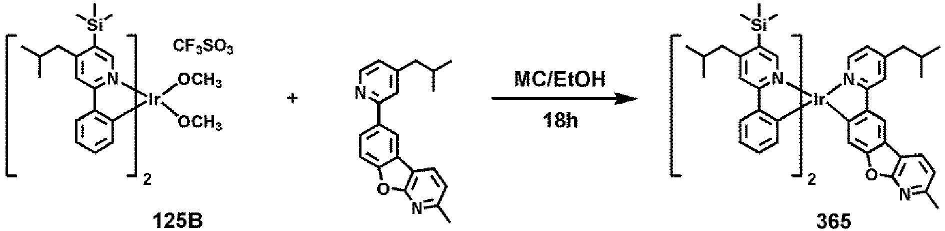

0.85 g of Compound 365 (yield of 37 %) was obtained in the same manner as in the synthesis of Compound 125 according to Synthesis Example 1, except that 6-(4-isobutylpyridin-2-yl)-2-methylbenzofuro[2,3-b]pyridine was used instead of 8-(4-isobutylpyridin-2-yl)-2-methylbenzofuro[2,3-b]pyridine. Substances of the compound were identified by the Mass Spectrum and HPLC analysis.
HRMS(MALDI) calcd for C₅₇H₆₇IrN₄OSi₂: m/z 1072.4483 Found: 1072.4488

### Synthesis Example 5 (Compound 505)

### Synthesis of Compound 505A

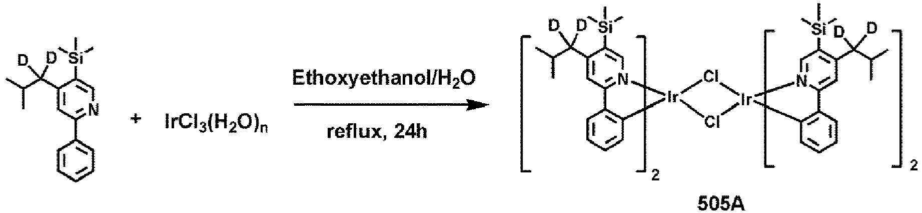

7.1 g of Compound 505A (yield of 71 %) was obtained in the same manner as in the synthesis of Compound 125A according to Synthesis Example 1, except that 4-isobutyl-D₂-2-phenyl-5-(trimethylsilyl)pyridine was used instead of 4-isobutyl-2-phenyl-5-(trimethylsilyl)pyridine.

### Synthesis of Compound 505B

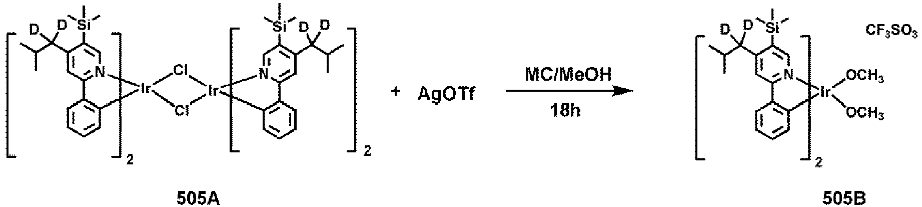

Compound 505B was obtained in the same manner as in the synthesis of Compound 125B according to Synthesis Example 1, except that Compound 505A was used instead of Compound 125A. Compound 505B thus obtained was used in the next reaction without additional purification.

### Synthesis of Compound 505

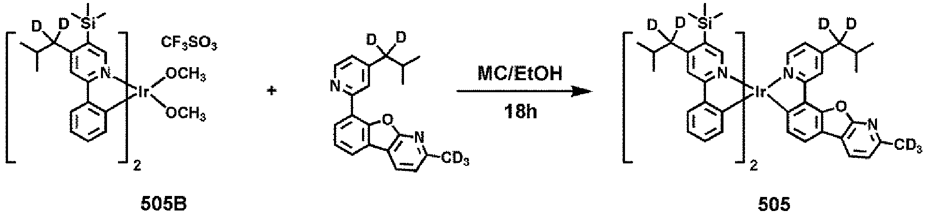

0.5 g of Compound 505 (yield of 22%) was obtained in the same manner as in the synthesis of Compound 125 according to Synthesis Example 1, except that Compound 505B was used instead of Compound 125B and 8-(4-isobutylpyridin-2-yl-D₂)-2-methyl(D₃)benzofuro[2,3-b]pyridine was used instead of 8-(4-isobutylpyridin-2-yl)-2-methylbenzofuro[2,3-b]pyridine. Substances of the compound were identified by the Mass Spectrum and HPLC analysis.
HRMS(MALDI) calcd for C₅₇H₅₈D₉IrN₄OSi₂: m/z 1081.5048, Found: 1081.5052

### Synthesis Example 6 (Compound 526)

### Synthesis of Compound 526A

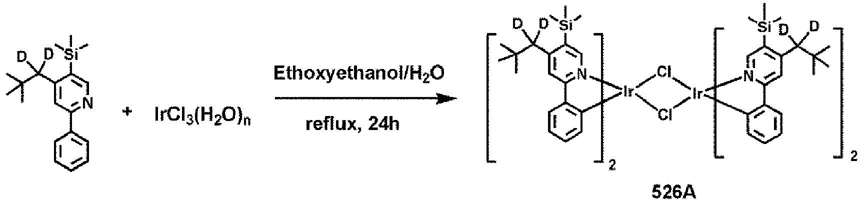

6.6 g of Compound 526A (yield of 66 %) was obtained in the same manner as in the synthesis of Compound 125A according to Synthesis Example 1, except that 4-neopentyl(D₂)-2-phenyl-5-(trimethylsilyl)pyridine was used instead of 4-isobutyl-2-phenyl-5-(trimethylsilyl)pyridine.

### Synthesis of Compound 526B

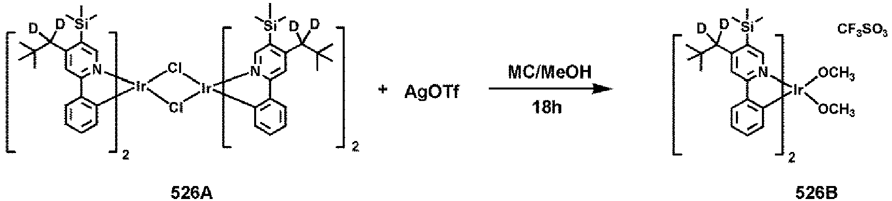

Compound 526B was obtained in the same manner as in the synthesis of Compound 125B according to Synthesis Example 1, except that Compound 526A was used instead of Compound 125A. Compound 526B thus obtained was used in the next reaction without additional purification.

### Synthesis of Compound 526

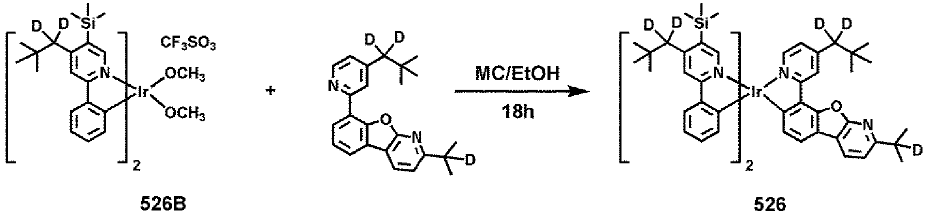

1.0 g of Compound 526 (yield of 44 %) was obtained in the same manner as in the synthesis of Compound 125 according to Synthesis Example 1, except that Compound 526B was used instead of Compound 125B and 2-isopropyl(D)-8-(4-neopentyl(D₂)pyridin-2-yl)benzofuro[2,3-b]pyridine was used instead of 8-(4-isobutylpyridin-2-yl)-2-methylbenzofuro[2,3-b]pyridine. Substances of the compound were identified by the Mass Spectrum and HPLC analysis.
HRMS(MALDI) calcd for C₆₂H₇₀D₇IrN₄OSi₂: m/z 1149.5705, Found: 1149.5700

### Synthesis Example 7 (Compound 676)

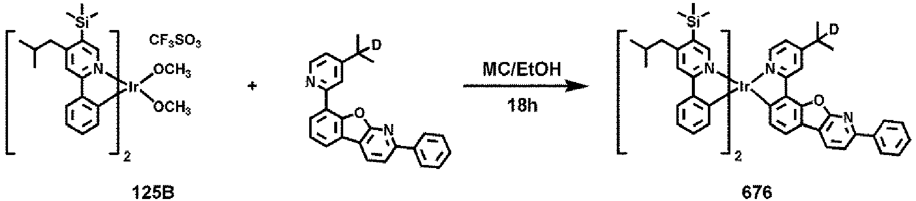

0.8 g of Compound 676 (yield of 41 %) was obtained in the same manner as in the synthesis of Compound 125 according to Synthesis Example 1, except that 2-phenyl-8-(4-(propan-2-yl-2-d)pyridin-2-yl)benzofuro[2,3-b]pyridine was used instead of 8-(4-isobutylpyridin-2-yl)-2-methylbenzofuro[2,3-b]pyridine. Substances of the compound were identified by the Mass Spectrum and HPLC analysis.
HRMS(MALDI) calcd for C₆₁H₆₆IrN₄OSi₂: m/z 1121.4545, Found: 1121.4549

### Synthesis Example 8 (Compound 806)

### Synthesis of Compound 806A

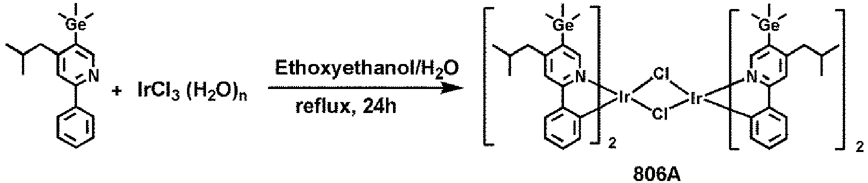

3.7 g of Compound 806A (yield of 74 %) was obtained in the same manner as in the synthesis of Compound 125A according to Synthesis Example 1, except that 4-isobutyl -2-phenyl-5-(trimethylgermyl)pyridine was used instead of 4-isobutyl-2-phenyl-5-(trimethylsilyl)pyridine.

### Synthesis of Compound 806B

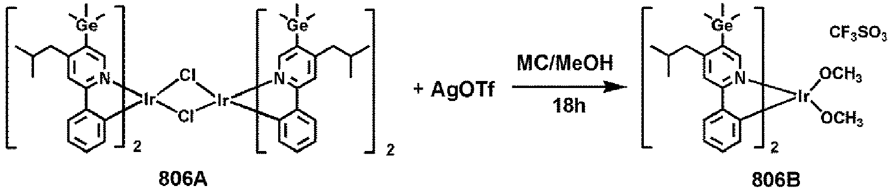

Compound 806B was obtained in the same manner as in the synthesis of Compound 125B according to Synthesis Example 1, except that Compound 806A was used instead of Compound 125A. Compound 806B thus obtained was used in the next reaction without additional purification.

### Synthesis of Compound 806

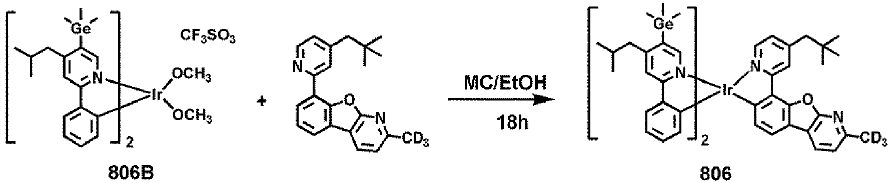

0.53 g of Compound 806 (yield of 35 %) was obtained in the same manner as in the synthesis of Compound 125 according to Synthesis Example 1, except that Compound 806B was used instead of Compound 125B and 2-methyl(D₃)-8-(4-neopentylpyridin-2-yl)benzofuro[2,3-b]pyridine was used instead of 8-(4-isobutylpyridin-2-yl)-2-methylbenzofuro[2,3-b]pyridine. Substances of the compound were identified by the Mass Spectrum and HPLC analysis.
HRMS(MALDI) calcd for C₅₈H₆₆D₃Ge₂IrN₄O: m/z 1181.3712, Found: 1181.3706

### Synthesis Example 9 (Compound 865)

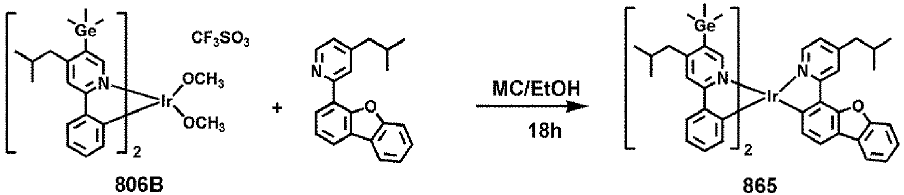

0.69 g of Compound 865 (yield of 31 %) was obtained in the same manner as in the synthesis of Compound 125 according to Synthesis Example 1, except that Compound 806B was used instead of Compound 125B and 2-(dibenzo[b,d]furan-4-yl)-4-isobutylpyridine was used instead of 8-(4-isobutylpyridin-2-yl)-2-methylbenzofuro[2,3-b]pyridine. Substances of the compound were identified by the Mass Spectrum and HPLC analysis.
HRMS(MALDI) calcd for C₅₇H₆₆Ge₂IrN₃O: m/z 1149.3259, Found: 1149.3251

### Synthesis Example 10 (Compound 1365)

### Synthesis of Compound 1365A

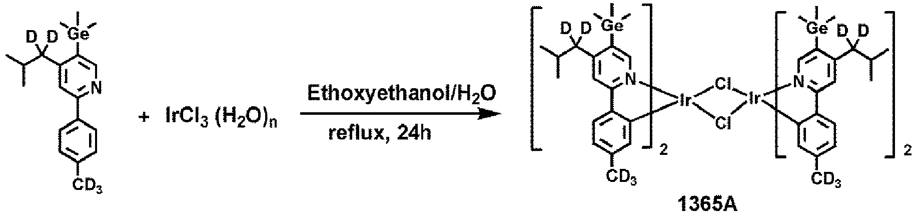

4.6 g of Compound 1365A (yield of 62 %) was obtained in the same manner as in the synthesis of Compound 125A according to Synthesis Example 1, except that 4-isobutyl(D₂)-2-(p-tolyl(D₃))-5-(trimethylgermyl)pyridine was used instead of 4-isobutyl-2-phenyl-5-(trimethylsilyl)pyridine.

### Synthesis of Compound 1365B

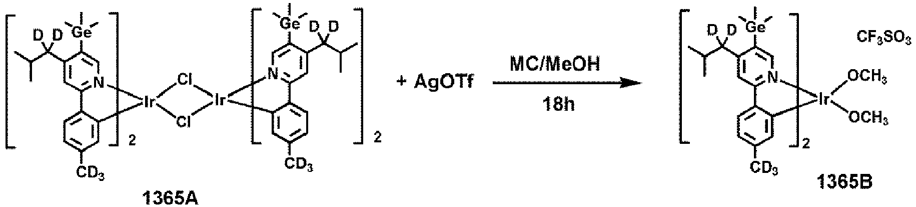

Compound 1365B was obtained in the same manner as in the synthesis of Compound 125B according to Synthesis Example 1, except that Compound 1365A was used instead of Compound 125A. Compound 1365B thus obtained was used in the next reaction without additional purification.

### Synthesis of Compound 1365

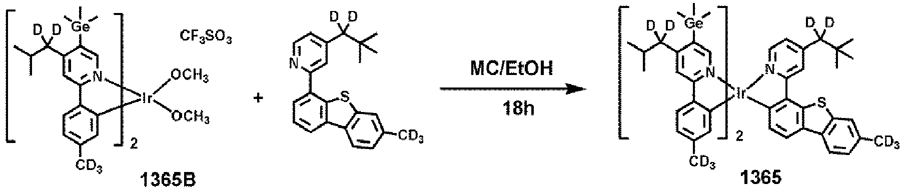

0.43 g of Compound 1365 (yield of 28 %) was obtained in the same manner as in the synthesis of Compound 125 according to Synthesis Example 1, except that Compound 1365B was used instead of Compound 125B and 2-(7-methyl(D₃)dibenzo[b,d]thiophen-4-yl)-4-neopentyl(D₂)pyridine was used instead of 8-(4-isobutylpyridin-2-yl)-2-methylbenzofuro[2,3-b]pyridine. Substances of the compound were identified by the Mass Spectrum and HPLC analysis.
HRMS(MALDI) calcd for C₆₁H₅₉D₁₅Ge₂IrN₃O: m/z 1236.4598, Found: 1236.4591

### Synthesis Example 11 (Compound 1497)

### Synthesis of Compound 1497A

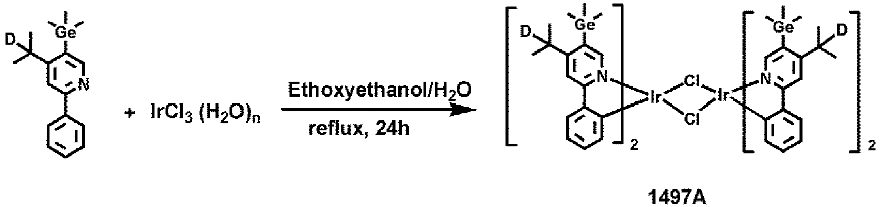

4.2 g of Compound 1497A (yield of 58 %) was obtained in the same manner as in the synthesis of Compound 125A according to Synthesis Example 1, except that 2-phenyl-4-(propan-2-yl-2-d)-5-(trimethylgermyl)pyridine was used instead of 4-isobutyl-2-phenyl-5-(trimethylsilyl)pyridine.

### Synthesis of Compound 1497B

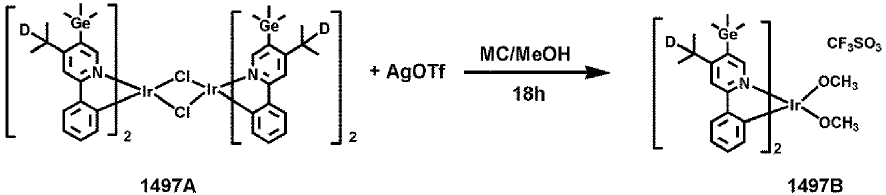

Compound 1497B was obtained in the same manner as in the synthesis of Compound 125B according to Synthesis Example 1, except that Compound 1497A was used instead of Compound 125A. Compound 1497B thus obtained was used in the next reaction without additional purification.

### Synthesis of Compound 1497

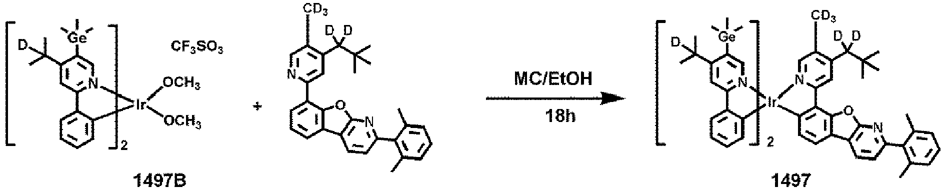

0.53 g of Compound 1497 (yield of 34 %) was obtained in the same manner as in the synthesis of Compound 125 according to Synthesis Example 1, except that Compound 1497B was used instead of Compound 125B and 2-(2,6-dimethylphenyl)-8-(4-(2,2-dimethylpropyl-1,1-d2)-5-(methyl-d3)pyridin-2-yl)benzofuro[2,3-b]pyridine was used instead of 8-(4-isobutylpyridin-2-yl)-2-methylbenzofuro[2,3-b]pyridine Substances of the compound were identified by the Mass Spectrum and HPLC analysis.
HRMS(MALDI) calcd for C₆₄H₆₆D₇Ge₂IrN₄O: m/z 1261.4277, Found: 1261.4271

### Synthesis Example 12 (Compound 1505)

### Synthesis of Compound 1505A

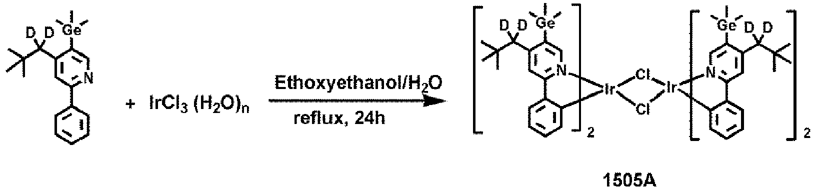

4.3 g of Compound 1505A (yield of 49 %) was obtained in the same manner as in the synthesis of Compound 125A according to Synthesis Example 1, except that 4-(2,2-dimethylpropyl-1,1-d2)-2-phenyl-5-(trimethylgermyl)pyridine was used instead of 4-isobutyl-2-phenyl-5-(trimethylsilyl)pyridine.

### Synthesis of Compound 1505B

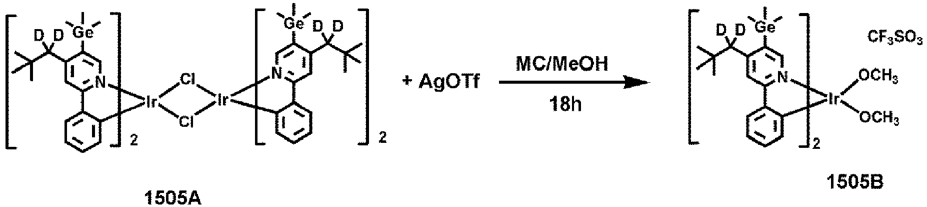

Compound 1505B was obtained in the same manner as in the synthesis of Compound 125B according to Synthesis Example 1, except that Compound 1505A was used instead of Compound 125A. Compound 1505B thus obtained was used in the next reaction without additional purification.

### Synthesis of Compound 1505

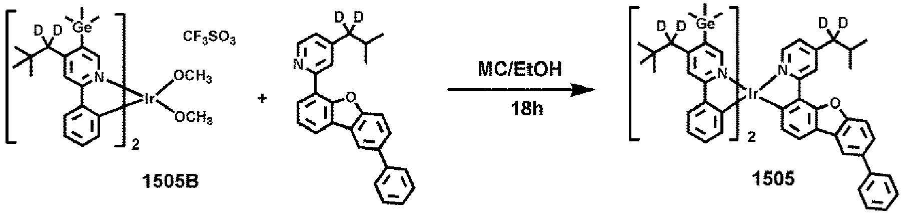

0.32 g of Compound 1505 (yield of 27 %) was obtained in the same manner as in the synthesis of Compound 125 according to Synthesis Example 1, except that Compound 1505B was used instead of Compound 125B and 4-(2-methylpropyl-1,1-d2)-2-(8-phenyldibenzo[b,d]furan-4-yl)pyridine was used instead of 8-(4-isobutylpyridin-2-yl)-2-methylbenzofuro[2,3-b]pyridine Substances of the compound were identified by the Mass Spectrum and HPLC analysis.
HRMS(MALDI) calcd for C₆₅H₆₈D₆Ge₂IrN₃O. m/z 1259.4261, Found: 1259.4255

### Example 1

As an anode, a glass substrate on which ITO/Ag/ITO was formed to a thickness of 70 Å /1,000 Å /70 Å was cut to a size of 50 mm x 50 mm x 0.5 mm, sonicated with isopropyl alcohol and pure water each for 5 minutes, and then, cleaned by exposure to ultraviolet rays and ozone for 30 minutes. Then, the anode was provided to a vacuum deposition apparatus.

2-TNATA was vacuum-deposited on the anode to form a hole injection layer having a thickness of 600 Å,and 4,4'-bis[N-(1-naphthyl)-N-phenylamino]biphenyl (hereinafter, referred to as NPB) was vacuum-deposited on the hole injection layer to form a hole transport layer having a thickness of 1,350 Å.

Next, CBP (as a host) and Compound 125 (as a dopant) were co-deposited at a weight ratio of 98 : 2 on the hole transport layer to form an emission layer having a thickness of 400 Å.

Then, BCP was vacuum-deposited on the emission layer to form a hole blocking layer having a thickness of 50 Å,and Alq₃ was vacuum-deposited on the hole blocking layer to form an electron transport layer having a thickness of 350 Å. LiF was vacuum-deposited on the electron transport layer to form an electron injection layer having a thickness of 10 Å,and Mg and Ag were co-deposited at a weight ratio of 90 : 10 on the electron injection layer to form a cathode having a thickness of 120 Å,thereby completing the manufacture of an organic light-emitting device.

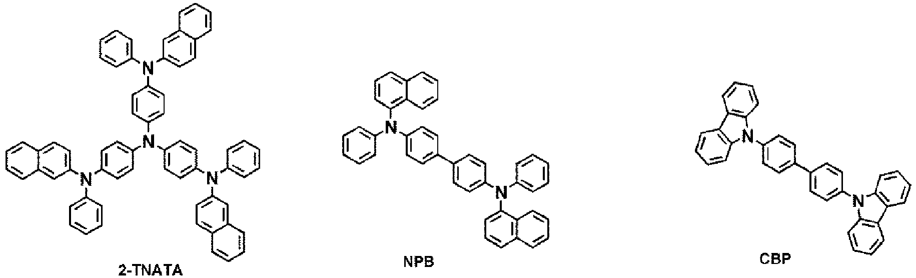

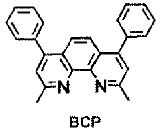

### Examples 2 to 12 and Comparative Examples A and B

Organic light-emitting devices were manufactured in the same manner as in Example 1, except that compounds listed in Table 2 below were each used as a dopant instead of Compound 125 in forming an emission layer.

### Evaluation Example 1: Evaluation of characteristics of organic light-emitting device

The driving voltage, maximum external quantum efficiency (Max EQE) value (%), and lifespan (LT₉₇, hr) of the organic light-emitting devices manufactured according to Examples 1 to 12 and Comparative Examples A and B were evaluated, and results thereof are shown in Table 2. Here, as a device used for the evaluation, a current-voltage meter (Keithley 2400) and a luminance meter (Minolta Cs-1000A) were used. The lifespan (LT₉₇) (at 3,500 nit) obtained by evaluating time (hr) that lapsed when luminance was 97 % of initial luminance (100 %), and was indicated in a relative value (%).

**[Table 2]**

| | Compound No. of dopant in emission layer | Driving voltage(V) | Max EQE(%) | LT₉₇ (relative value, %) (at 3,500 nit) |
|---|---|---|---|---|
| Example 1 | 125 | 4.43 | 23.3 | 100 |
| Example 2 | 128 | 4.41 | 23.4 | 87 |
| Example 3 | 163 | 4.42 | 24.1 | 93 |
| Example 4 | 365 | 4.16 | 22.3 | 110 |
| Example 5 | 505 | 4.43 | 23.6 | 130 |
| Example 6 | 526 | 4.47 | 25.4 | 150 |
| Example 7 | 676 | 4.33 | 25.8 | 180 |
| Example 8 | 806 | 4.45 | 23.2 | 120 |
| Example 9 | 865 | 4.10 | 23.0 | 100 |
| Example 10 | 1365 | 4.38 | 22.5 | 110 |
| Example 11 | 1497 | 4.35 | 25.1 | 165 |
| Example 12 | 1505 | 4.48 | 22.8 | 170 |
| Comparative Example A | A | 4.35 | 19.8 | 27 |
| Comparative Example B | B | 4.23 | 20.5 | 43 |

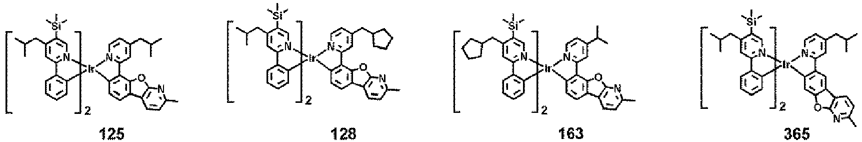

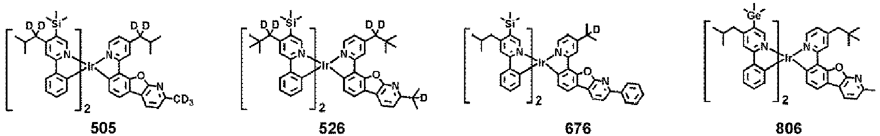

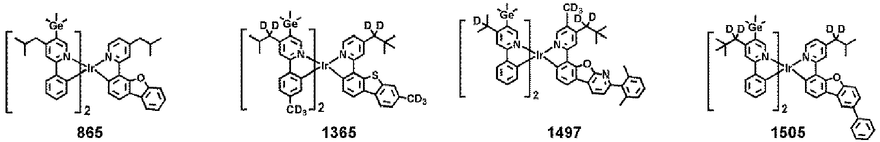

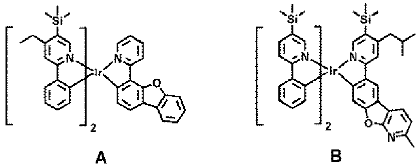

Referring to Table 2, it was confirmed that the organic light-emitting device manufactured according to Examples 1 to 12 had a comparable value of driving voltage and improved external quantum efficiency and longer lifespan characteristics, as compared with the organic light-emitting device manufactured according to Comparative Examples A and B.

According to the one or more embodiments, the organometallic compound has excellent electronic characteristics and heat resistance, and thus, an electronic device, for example, an organic light-emitting device, including the organometallic compound may have good driving voltage, good external quantum efficiency, and good lifespan characteristics. In addition, since the organometallic compound has excellent phosphorescence characteristics, a diagnostic composition including the organometallic compound may be provided with a high diagnosis efficiency.

It should be understood that embodiments described herein should be considered in a descriptive sense only and not for purposes of limitation. Descriptions of features or aspects within each embodiment should typically be considered as available for other similar features or aspects in other embodiments.

While one or more embodiments have been described with reference to the figures, it will be understood by those of ordinary skill in the art that various changes in form and details may be made therein without departing from the scope as defined by the following claims.

## Claims

1. An organometallic compound represented by Formula 1:
<Formula 1> M(L₁)ₙ₁(L₂)ₙ₂
wherein, in Formula 1,
M is a transition metal,
L₁ is a ligand represented by Formula 2A,
L₂ is a ligand represented by Formula 2B,
n1 and n2 are each independently 1 or 2, wherein, when n1 is 2, two L₁(s) are identical to or different from each other and when n2 is 2, two L₂(s) are identical to or different from each other,
the sum of n1 and n2 is 2 or 3, and
L₁ and L₂ are different from each other: 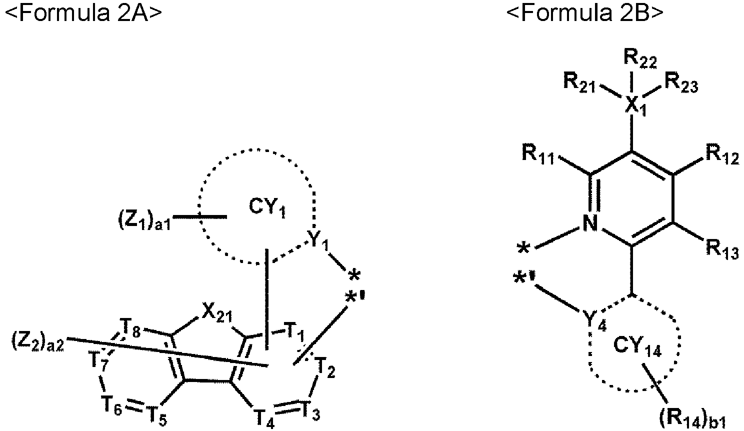 wherein, in Formulae 2A and 2B,
Y₁ and Y₄ are each independently C or N,
X₁ is Si or Ge,
X₂₁ is O, S, S(=O), N(Z₂₉), C(Z₂₉)(Z₃₀), or Si(Z₂₉)(Z₃₀),
T₁ to T₄ are each independently C, N, carbon linked to ring CY₁, or carbon linked to M in Formula 1, wherein one of T₁ to T₄ is carbon linked to M in Formula 1, and one of the remaining T₁ to T₄ that are not linked to M in Formula 1 is carbon linked to ring CY₁,
T₅ to T₈ are each independently C or N,
when X₁ is Si, at least one of the remaining T₁ to T₈ that are not carbon linked to M and ring CY₁ is N,
ring CY₁ and ring CY₁₄ are each independently a C₅-C₃₀ carbocyclic group or a C₁-C₃₀ heterocyclic group,
R₂₁ to R₂₃ are each independently a C₁-C₆₀ alkyl group or a C₆-C₆₀ aryl group, each unsubstituted or substituted with deuterium, -F, -CI, -Br, -I, -CD₃, -CD₂H, -CDH₂, -CF₃, - CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₃-C₁₀ cycloalkyl group, a phenyl group or any combination thereof,
Z₁, Z₂, and R₁₁ to R₁₄ are each independently hydrogen, deuterium, -F, -CI, -Br, -I, - SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₁-C₆₀ alkylthio group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Ge(Q₃)(Q₄)(Q₅), -B(Q₆)(Q₇), -P(=O)(Q₈)(Q₉), or - P(Q₈)(Q₉), wherein R₁₂ is neither hydrogen nor a methyl group,
a1 and b1 are each independently an integer from 0 to 20, wherein, when a1 is 2 or more, two or more Z₁(s) are identical to or different from each other, and when b1 is 2 or more, two or more R₁₄(s) are identical to or different from each other,
a2 is an integer from 0 to 6, wherein, when a2 is 2 or more, two or more Z₂(s) are identical to or different from each other,
two or more selected from R₂₁ to R₂₃ are optionally linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
two or more selected from a plurality of Z₁(s) are optionally linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
two or more selected from a plurality of Z₂(s) are optionally linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
R₁₂ and R₁₃ are optionally linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
two or more selected from a plurality of R₁₄(s) are optionally linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
two or more selected from Z₁, Z₂ and R₁₁ to R₁₄ are optionally linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
R₁₀ₐ is the same as defined in connection with R₁₄,
* and *' in Formulae 2A and 2B each indicate a binding site to M in Formula 1, asubstituent(s) of the substituted C₁-C₆₀ alkyl group, the substituted C₂-C₆₀ alkenyl group, the substituted C₂-C₆₀ alkynyl group, the substituted C₁-C₆₀ alkoxy group, the substituted C₁-C₆₀ alkylthio group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted monovalent non-aromatic condensed heteropolycyclic group is(are):
deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, or a C₁-C₆₀ alkoxy group;
a C₁-C₆₀ alkyl group, a C₂-C₆₀ alkenyl group, a C₂-C₆₀ alkynyl group, or a C₁-C₆₀ alkoxy group, each substituted with deuterium, -F, -Cl, -Br, -I, -CD₃, -CD₂H, -CDH₂, -CF₃, - CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), - Ge(Q₁₃)(Q₁₄)(Q₁₅), -B(Q₁₆)(Q₁₇), -P(=O)(Q₁₈)(Q₁₉), -P(Q₁₈)(Q₁₉), or any combination thereof;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group, each unsubstituted or substituted with deuterium, -F, -Cl, -Br, -I, -CD₃, -CD₂H, -CDH₂, -CF₃, - CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), - Ge(Q₂₃)(Q₂₄)(Q₂₅), -B(Q₂₆)(Q₂₇), -P(=O)(Q₂₈)(Q₂₉), -P(Q₂₈)(Q₂₉), or any combination thereof; -N(Q₃₁)(Q₃₂), -Ge(Q₃₃)(Q₃₄)(Q₃₅), -B(Q₃₆)(Q₃₇), -P(=O)(Q₃₈)(Q₃₉), or -P(Q₃₈)(Q₃₉); or any combination thereof;
Q₁ to Q₉, Q₁₁ to Q₁₉, Q₂₁ to Q₂₉, and Q₃₁ to Q₃₉ are each independently hydrogen; deuterium; -F; -Cl; -Br; -I; a hydroxyl group; a cyano group; a nitro group; an amino group; an amidino group; a hydrazine group; a hydrazone group; a carboxylic acid group or a salt thereof; a sulfonic acid group or a salt thereof; a phosphoric acid group or a salt thereof; a C₁-C₆₀ alkyl group unsubstituted or substituted with deuterium, a C₁-C₆₀ alkyl group, a C₆-C₆₀ aryl group, or any combination thereof; a C₂-C₆₀ alkenyl group; a C₂-C₆₀ alkynyl group; a C₁-C₆₀ alkoxy group; a C₃-C₁₀ cycloalkyl group; a C₁-C₁₀ heterocycloalkyl group; a C₃-C₁₀ cycloalkenyl group; a C₁-C₁₀ heterocycloalkenyl group; a C₆-C₆₀ aryl group unsubstituted or substituted with deuterium, a C₁-C₆₀ alkyl group, a C₆-C₆₀ aryl group, or any combination thereof; a C₆-C₆₀ aryloxy group; a C₆-C₆₀ arylthio group; a C₁-C₆₀ heteroaryl group, a monovalent non-aromatic condensed polycyclic group; or a monovalent non-aromatic condensed heteropolycyclic group.

2. The organometallic compound of claim 1, wherein
M in Formula 1 is Ir, and the sum of n1 and n2 is 3, or
M in Formula 1 is Pt, and the sum of n1 and n2 is 2.

3. The organometallic compound of claims 1 or 2, wherein
X₂₁ in Formula 2A is O or S; and/or
wherein
Z₁ in Formula 2A is:
hydrogen, deuterium, -F, or a cyano group;
a C₁-C₂₀ alkyl group unsubstituted or substituted with deuterium, -F, a cyano group, a C₃-C₁₀ cycloalkyl group, a deuterated C₃-C₁₀ cycloalkyl group, a (C₁-C₂₀ alkyl)C₃-C₁₀ cycloalkyl group, a C₁-C₁₀ heterocycloalkyl group, a deuterated C₁-C₁₀ heterocycloalkyl group, a (C₁-C₂₀ alkyl)C₁-C₁₀ heterocycloalkyl group, or any combination thereof; or
a C₃-C₁₀ cycloalkyl group or a C₁-C₁₀ heterocycloalkyl group, unsubstituted or substituted with deuterium, -F, a cyano group, a C₁-C₂₀ alkyl group, a deuterated C₁-C₂₀ alkyl group, or any combination thereof.

4. The organometallic compound of any of claims 1-3, wherein
a number of carbon included in R₁₂ of Formula 2B is at least two; and/or
wherein
the organometallic compound represented by Formula 1 satisfies at least one of <Condition (1)> to <Condition (3)> below:
<Condition (1)>
In Formula 2A, Z₁ is not hydrogen, and a1 is an integer of 1 to 20.
<Condition (2)>
In Formula 2B, R₁₄ is not hydrogen, and b1 is an integer of 1 to 20.
<Condition (3)>
In Formula 2A, Z₂ is not hydrogen, and a2 is an integer of 1 to 6.

5. The organometallic compound of any of claims 1-4, wherein
the organometallic compound represented by Formula 1 comprises at least one deuterium, at least one fluoro group (-F), at least one cyano group (-CN), or any combination thereof; and/or
wherein
Z₂ in Formula 2A is not hydrogen,
a2 is an integer from 1 to 3, and
at least one of Z₂(s) in number of a2 are each independently a substituted or unsubstituted C₁-C₆₀ alkyl group, a substituted or unsubstituted C₁-C₆₀ alkoxy group, a substituted or unsubstituted C₁-C₆₀ alkylthio group, a substituted or unsubstituted C₃-C₁₀ cycloalkyl group, a substituted or unsubstituted C₁-C₁₀ heterocycloalkyl group, a substituted or unsubstituted C₆-C₆₀ aryl group, a substituted or unsubstituted C₁-C₆₀ heteroaryl group, a substituted or unsubstituted monovalent non-aromatic condensed polycyclic group, or a substituted or unsubstituted monovalent non-aromatic condensed heteropolycyclic group.

6. The organometallic compound of any of claims 1-5, wherein a group represented by 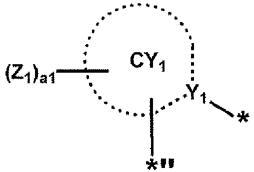 in Formula 2A is represented by one of Formulae CY1-1 to CY1-28: 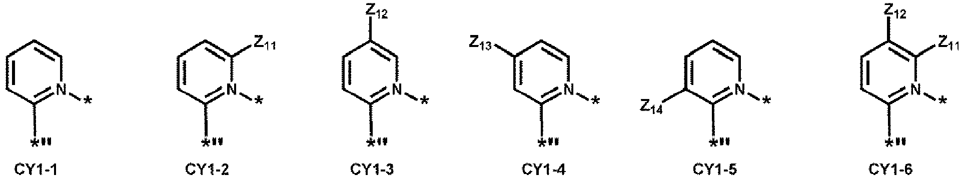 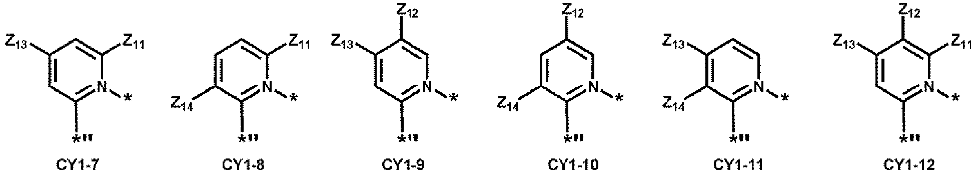 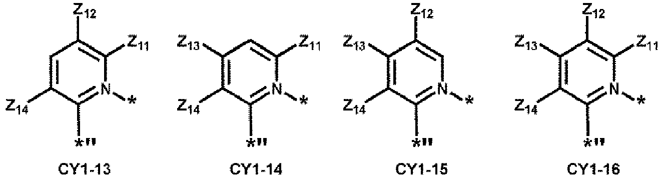 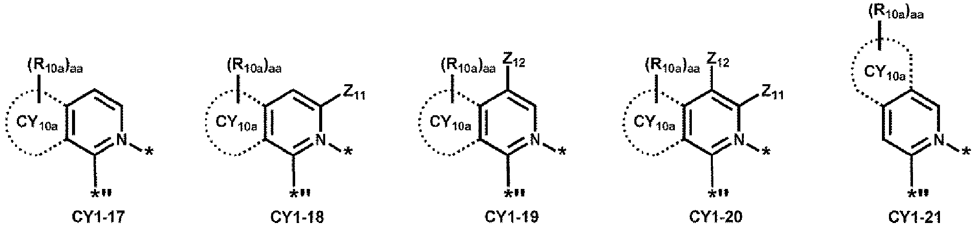 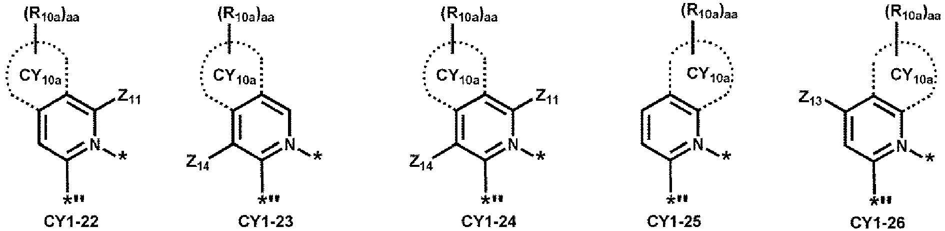 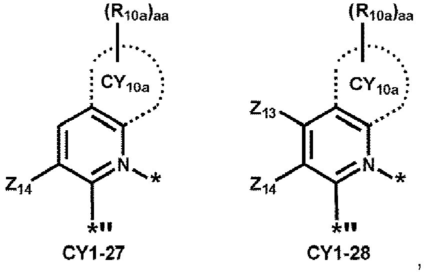 wherein, in Formulae CY1-1 to CY1-28,
Z₁₁ to Z₁₄ are each independently the same as defined in connection with Z₁ in claim 1, wherein each of Z₁₁ to Z₁₄ are not hydrogen,
ring CY₁₀ₐ is a C₅-C₃₀ carbocyclic group or a C₁-C₃₀ heterocyclic group,
R₁₀ₐ is the same as described in claim 1,
aa is an integer from 0 to 10, and
* indicates a binding site to M in Formula 1, and *" indicates a binding site to one of T₁ to T₄ in Formula 2A.

7. The organometallic compound of claim 6, wherein a group represented by 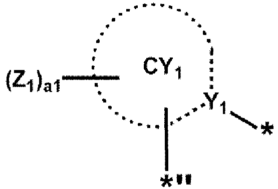 in Formula 2A is represented by one of Formulae CY1-4, CY1-7, CY1-9, CY1-11, CY1-12, and CY1-14 to CY1-24.

8. The organometallic compound of any of claims 1-7, wherein a group represented by 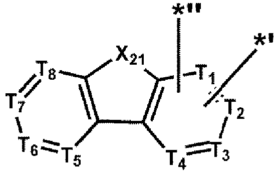 in Formula 2A is represented by one of Formulae CY2-1 to CY2-6: 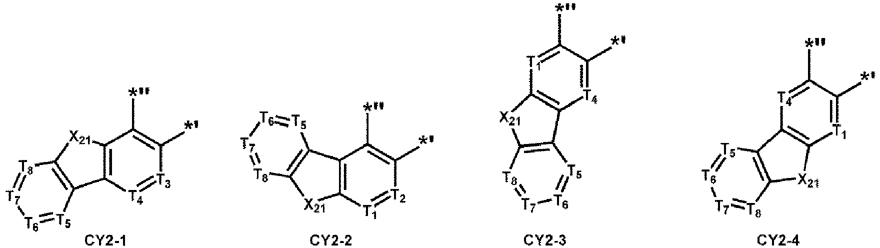 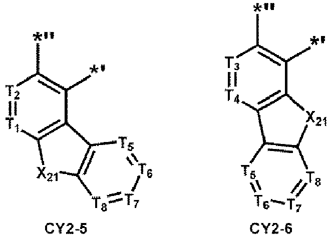 wherein, in Formulae CY2-1 to CY2-6,
T₁ to T₈ are each independently C or N,
X₂₁ is the same as described in claim 1,
*" indicates a binding site to ring CY₁ in Formula 2A, and
*' indicates a binding site to M in Formula 1.

9. The organometallic compound of claim 8, wherein
a) X₁ in Formula 2B is Ge, and each of T₁ to T₈ in Formulae CY2-1 to CY2-6 is C,
b) X₁ in Formula 2B is Si or Ge, and at least one of T₃ to T₈ in Formulae CY2-1 and CY2-6 is N,
c) X₁ in Formula 2B is Si or Ge, and at least one of T₁, T₂, and T₅ to T₈ in Formulae CY2-2 and CY2-5 is N,
d) X₁ in Formula 2B is Si or Ge, and at least one of T₁ and T₄ to T₈ in Formulae CY2-3 and CY2-4 is N.

10. The organometallic compound of claims 8 or 9, wherein
1) T₁ to T₈ in Formulae CY2-1 to CY2-6 are C;
2) one of T₃ to T₈ in Formula CY2-1 is N, and the remaining T₃ to T₈ that are not N in Formula CY2-1 are C;
3) T₃ and T₈ in Formula CY2-1 are N, and T₄ to T₇ in Formula CY2-1 are C;
4) T₆ and T₈ in Formula CY2-1 are N, and T₃ to T₅ and T₇ in Formula CY2-1 are C;
5) one of T₁, T₂ and T₈ in Formula CY2-2 is N, and the remaining T₁, T₂ and T₅ to T₈ that are not N in Formula CY2-2 are C;
6) T₁ and T₈ in Formula CY2-2 are N, and T₂ and T₅ to T₇ in Formula CY2-2 are C;
7) T₂ and T₈ in Formula CY2-2 are N, and T₁ and T₅ to T₇ in Formula CY2-2 are C;
8) one of T₁, T₄ and T₈ in Formulae CY2-3 and CY2-4 is N, and the remaining T₁, T₄ and T₅ to T₈ that are not N in Formulae CY2-3 and CY2-4 are C;
9) T₁ and T₈ in Formulae CY2-3 and CY2-4 are N, and T₄ and T₅ to T₇ in Formulae CY2-3 and CY2-4 are C;
10) T₄ and T₈ in Formulae CY2-3 and CY2-4 are N, and T₁ and T₅ to T₇ in Formulae CY2-3 and CY2-4 are C;
11) one of T₁ and T₈ in Formula CY2-5 is N, and the remaining T₁, T₂ and T₅ to T₈ that are not N in Formula CY2-5 are C;
12) T₁ and T₈ in Formula CY2-5 are N, and T₂ and T₅ to T₇ in Formula CY2-5 are C;
13) one of T₄ and T₈ in Formula CY2-6 is N, and the remaining T₃ to T₈ that are not N in Formula CY2-6 are C; or
14) T₄ and T₈ in Formula CY2-6 are N, and T₃ and T₅ to T₇ in Formula CY2-6 are C.

11. The organometallic compound of any of claims 1-10, wherein a group represented by 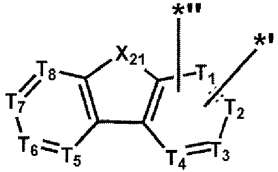 in Formula 2A is represented by one of Formulae CY2-1001 to CY2-1141, CY2-2001 to CY2-2092, CY2-3001 to CY2-3092, CY2-4001 to CY2-4092, CY2-5001 to CY2-5065 and CY2-6001 to CY2-6065: 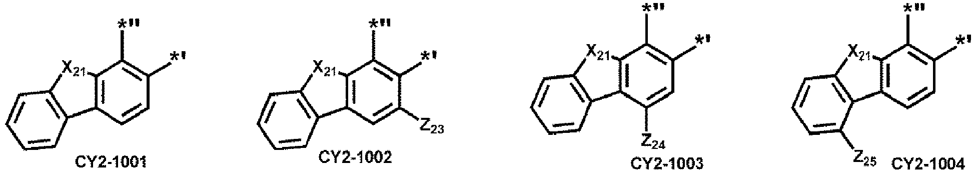 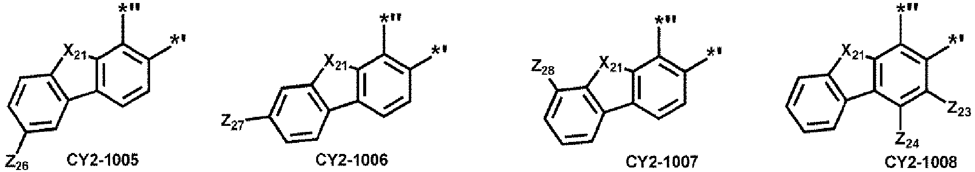 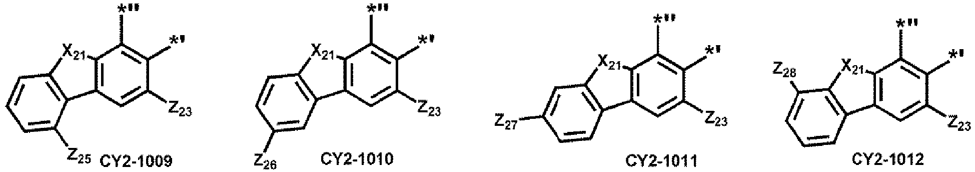 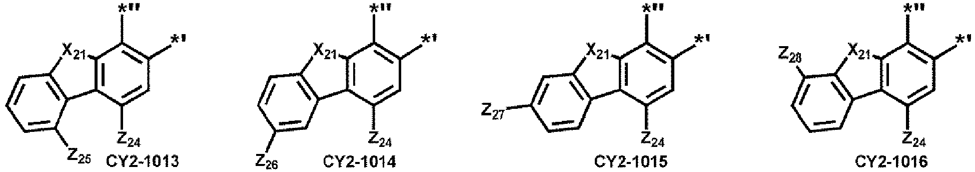 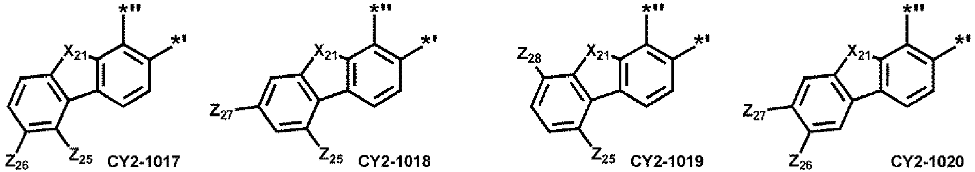 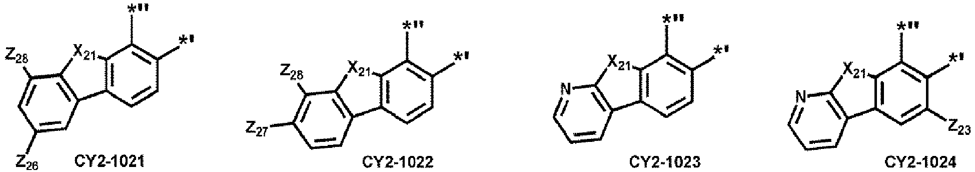 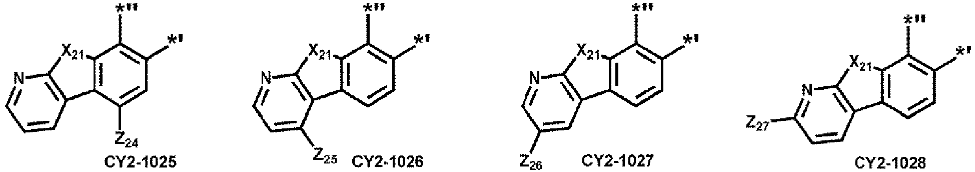 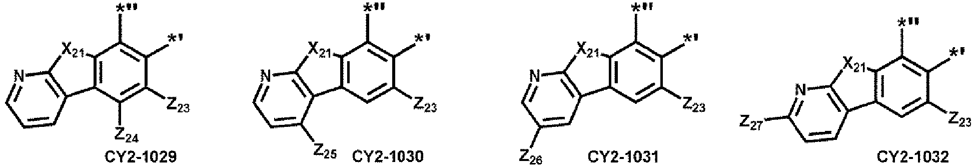 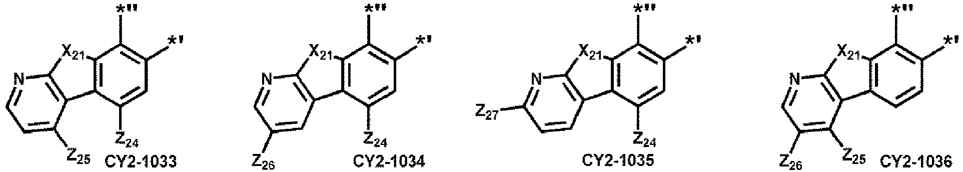 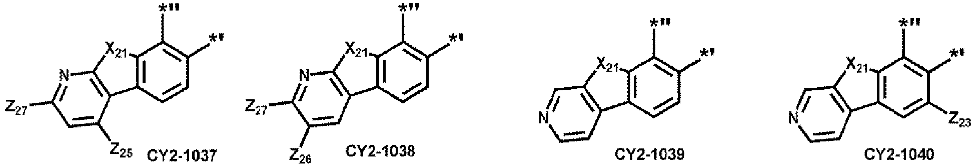 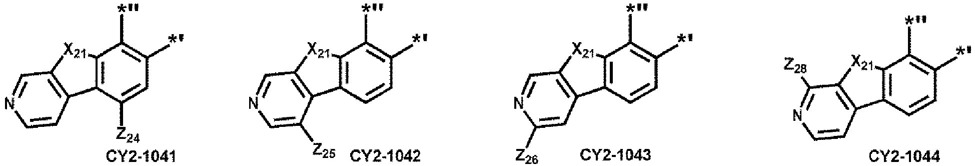 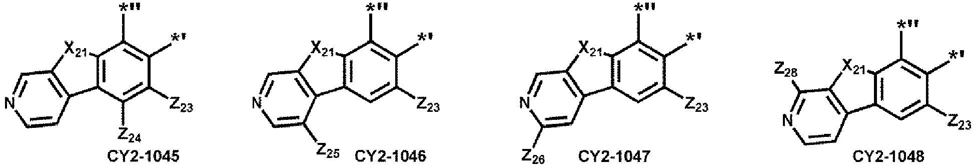 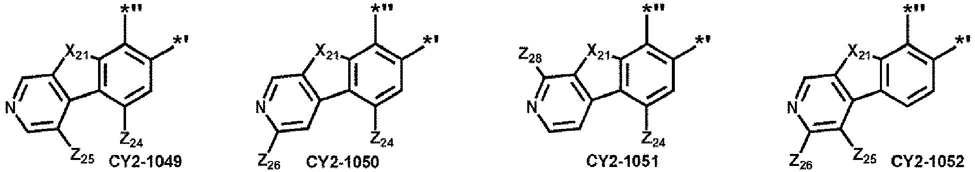 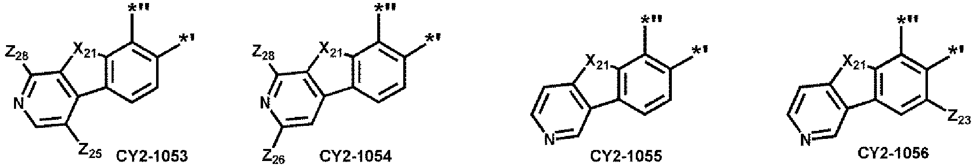 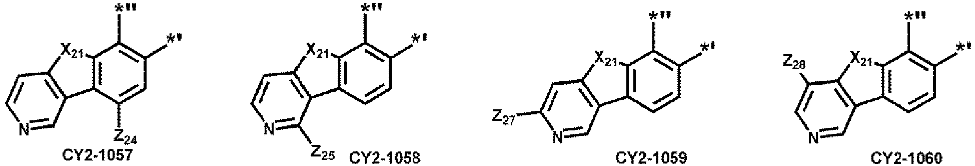 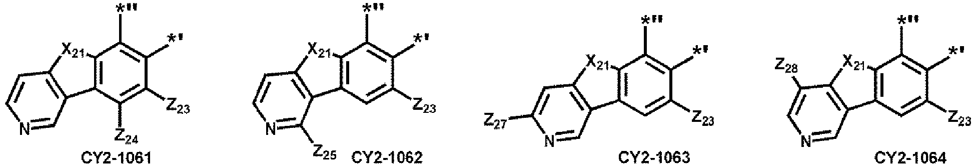 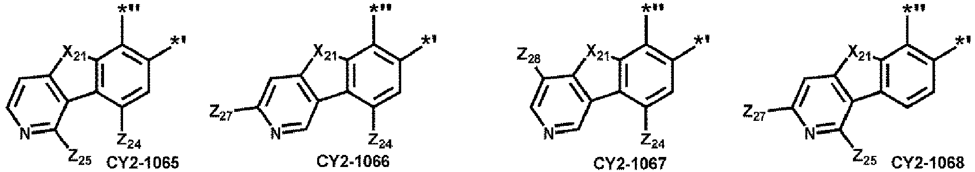 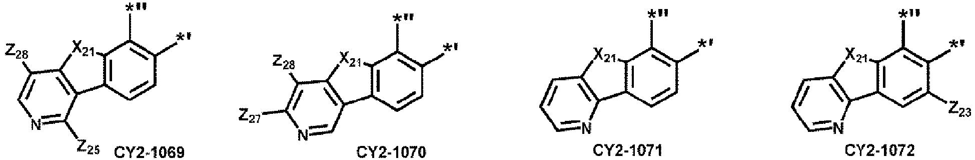 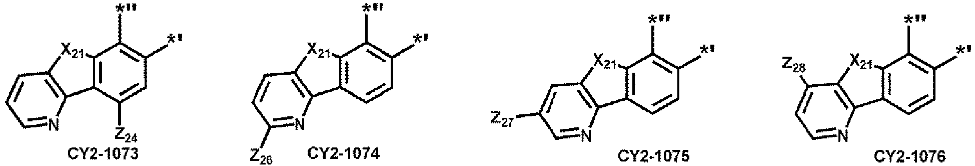 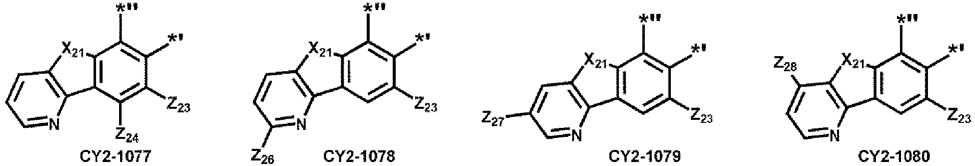 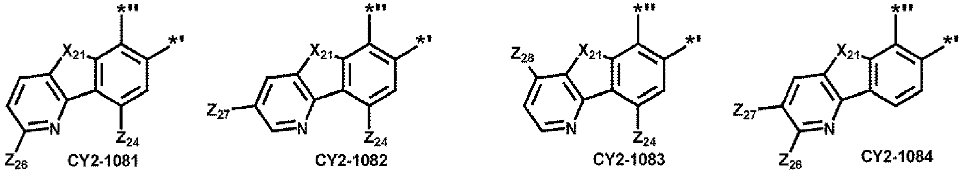 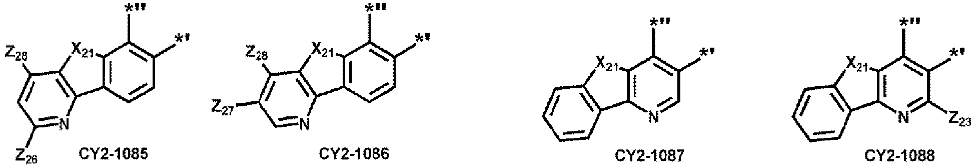 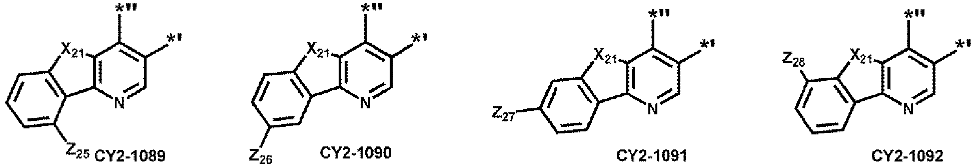 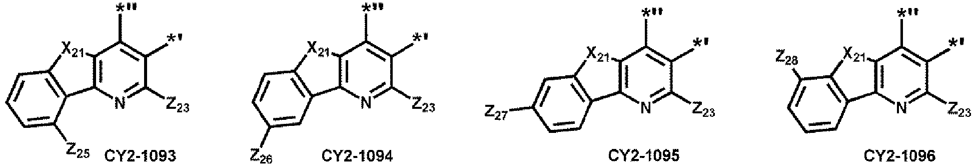 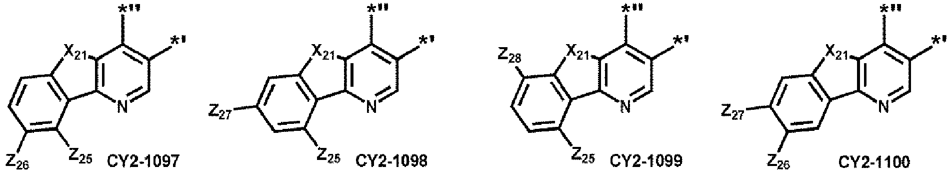 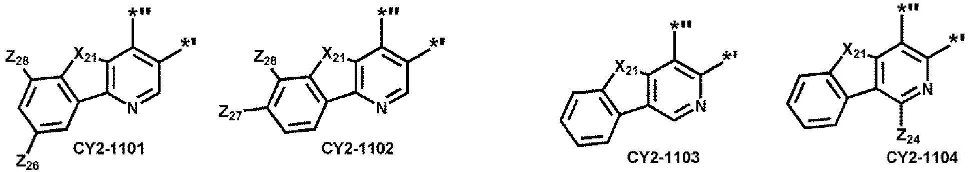 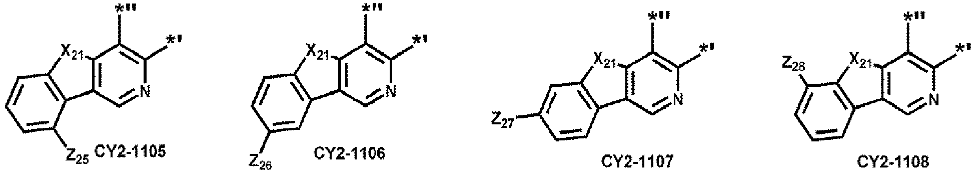 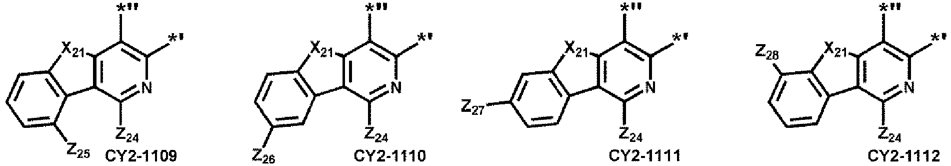 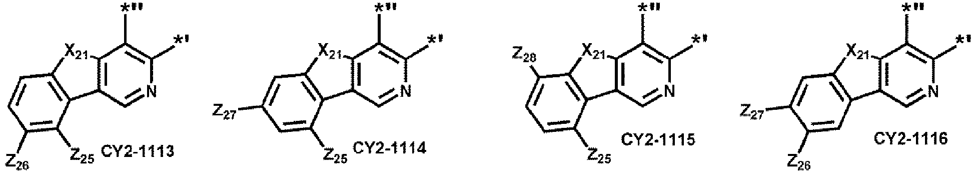 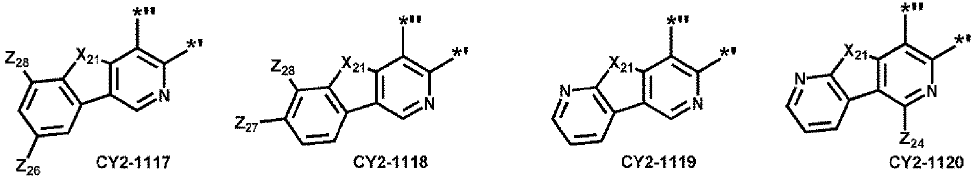 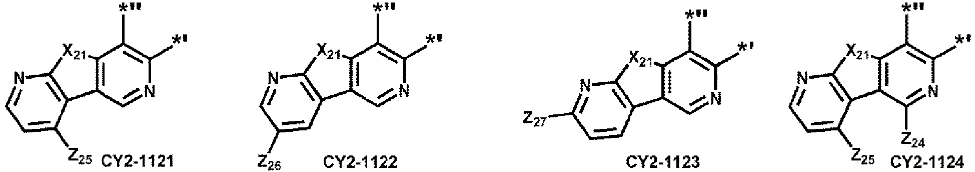 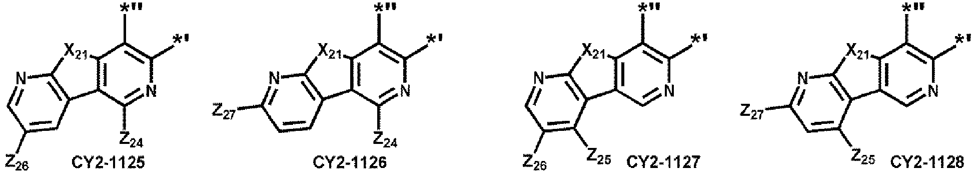 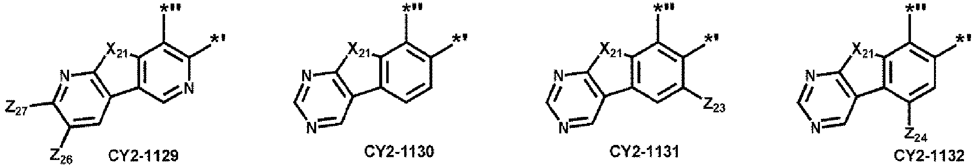 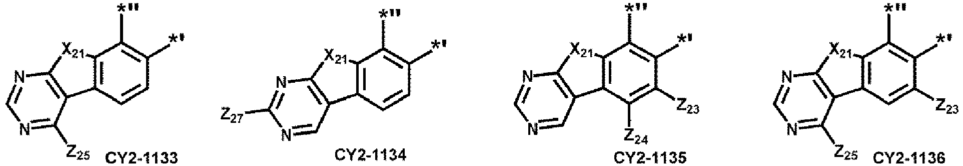 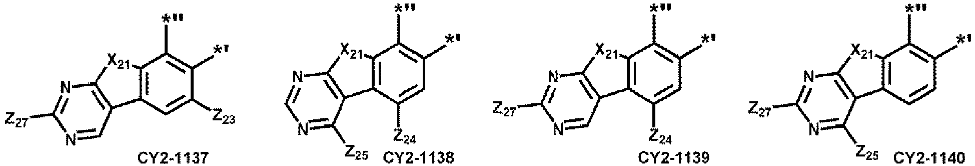 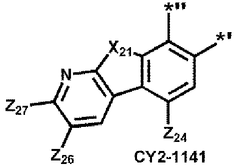 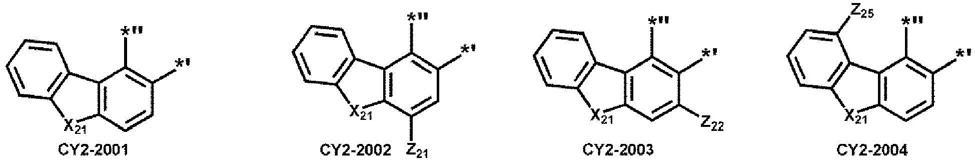 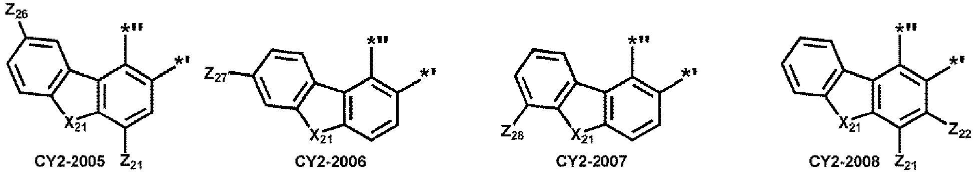 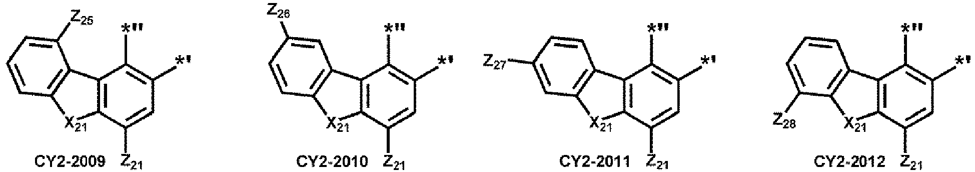 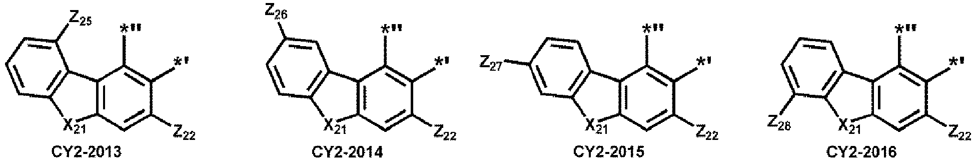 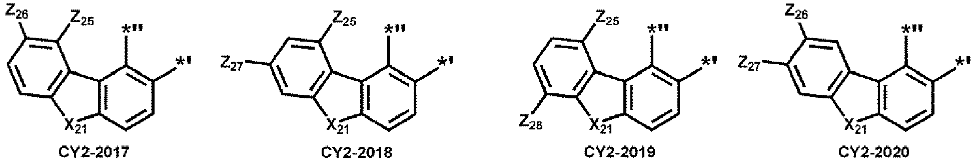 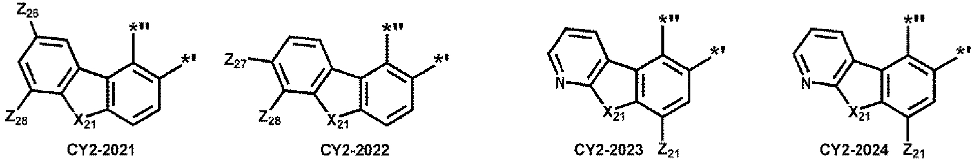 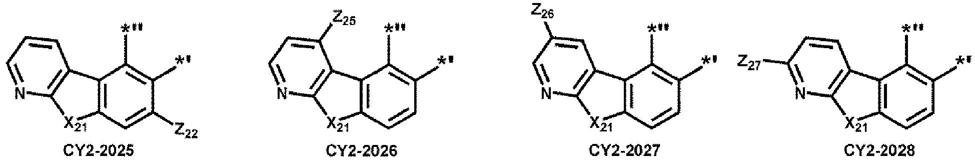 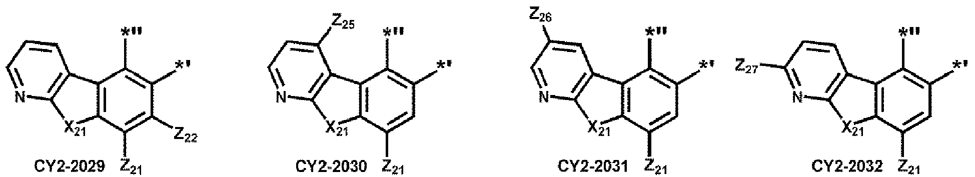 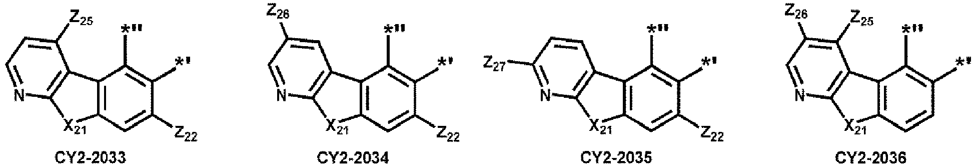 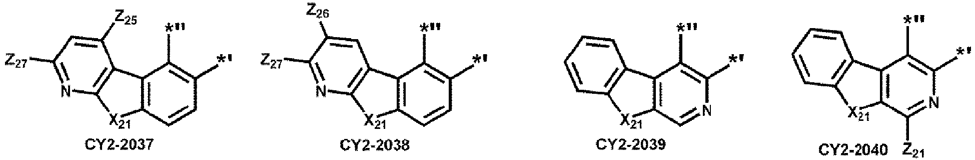 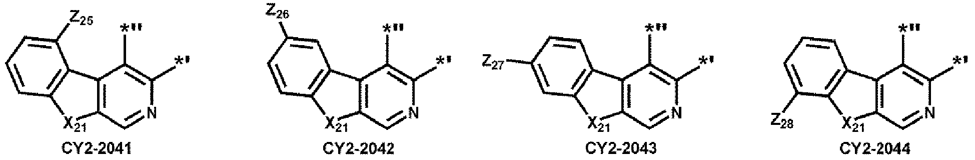 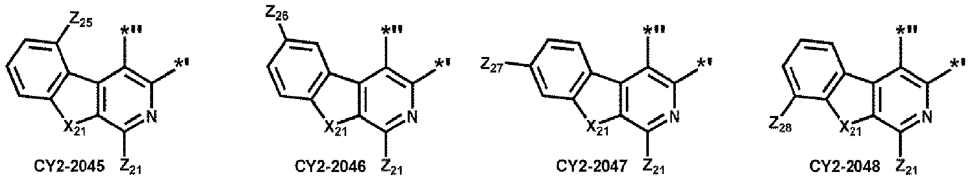 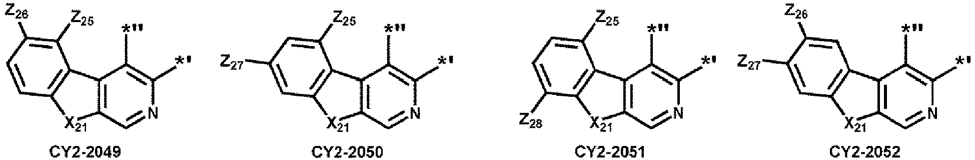 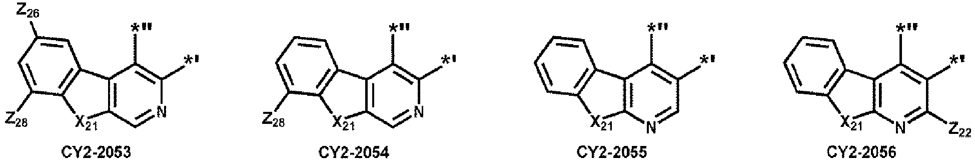 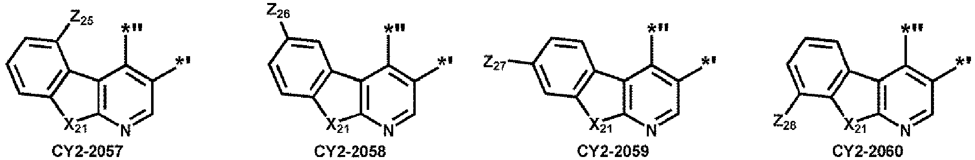 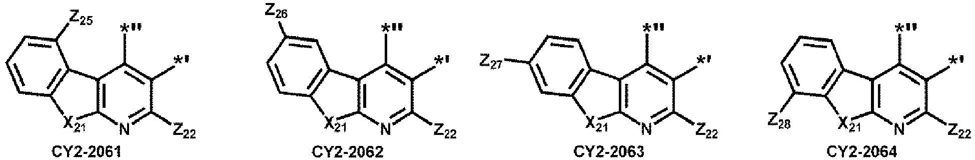 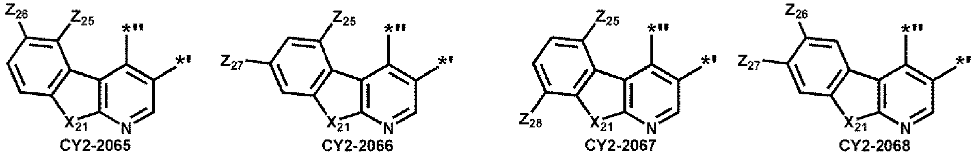 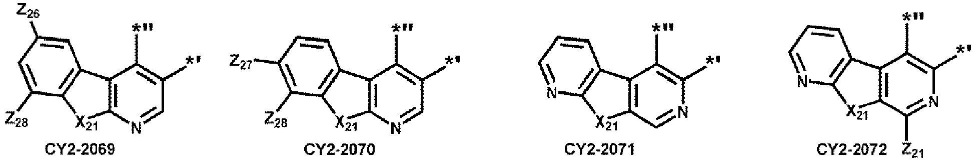 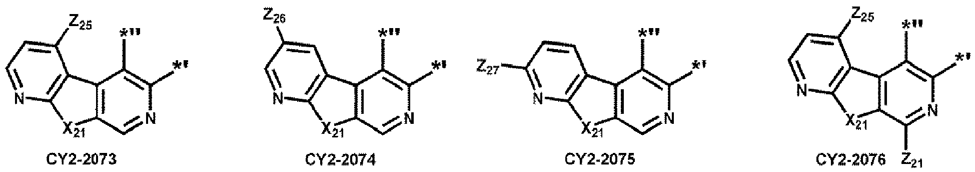 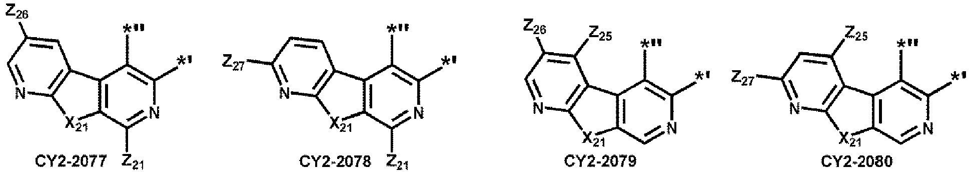 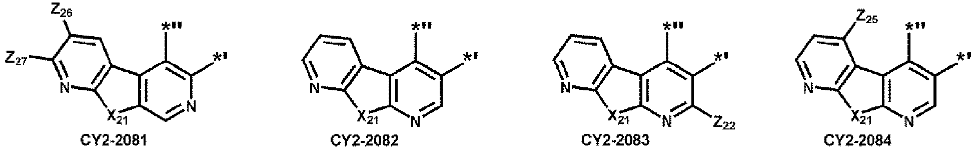 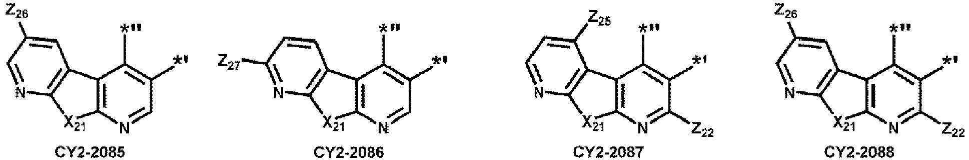 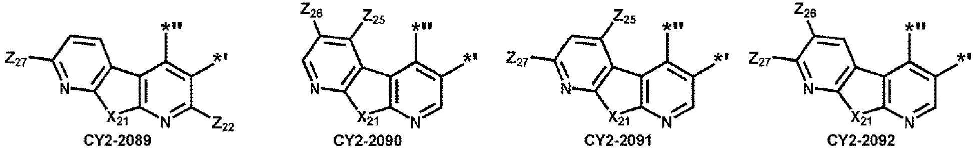 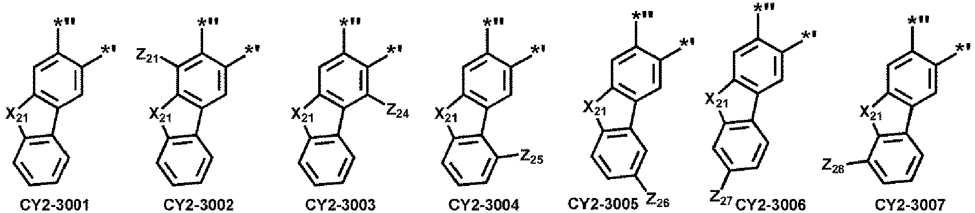 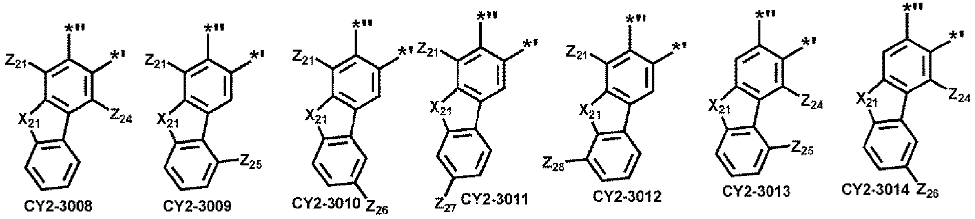 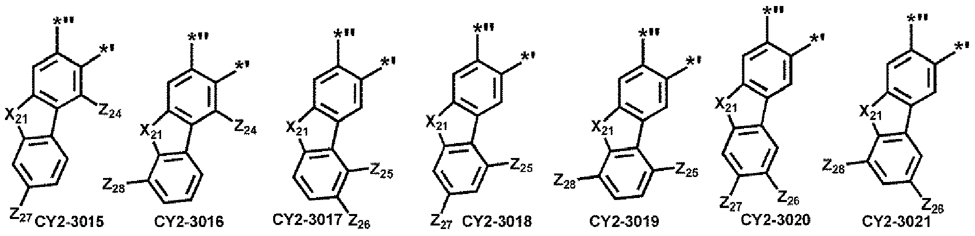 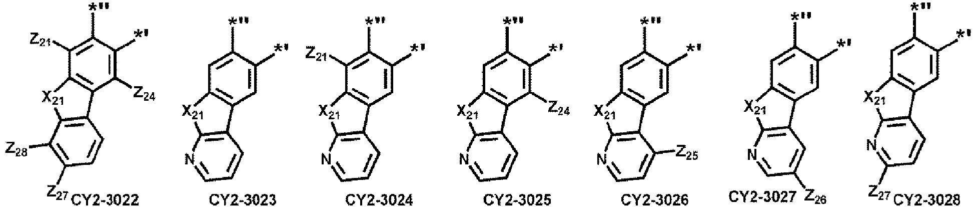 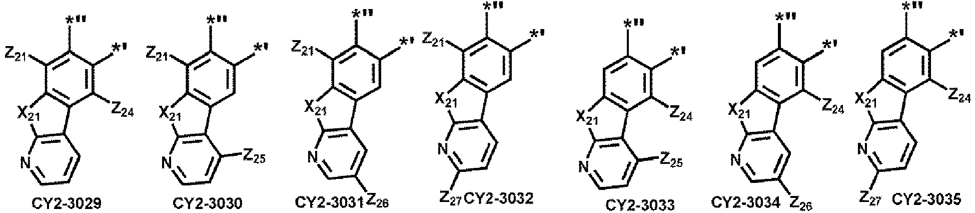 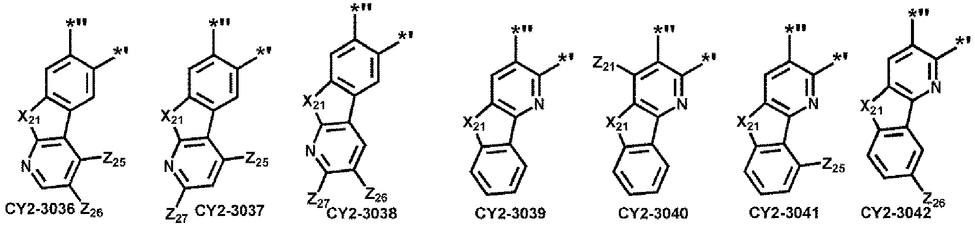 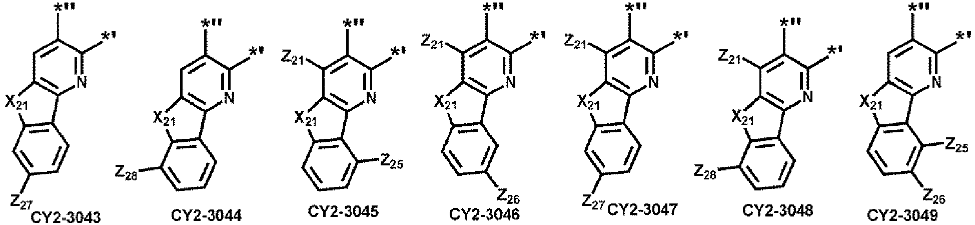 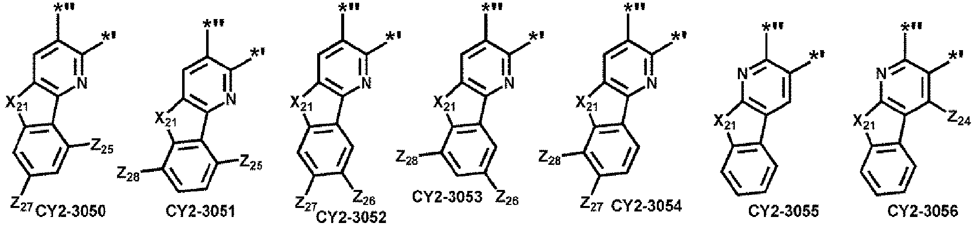 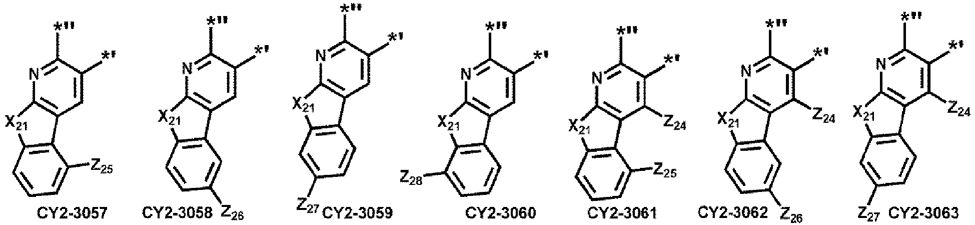 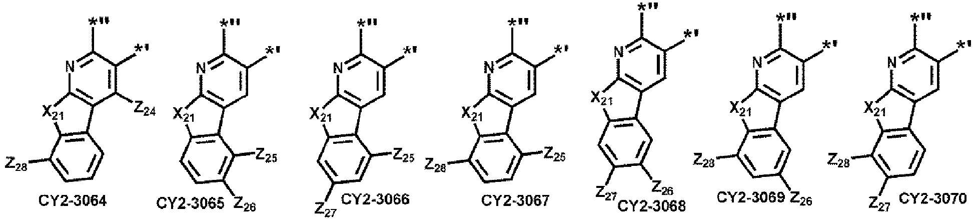 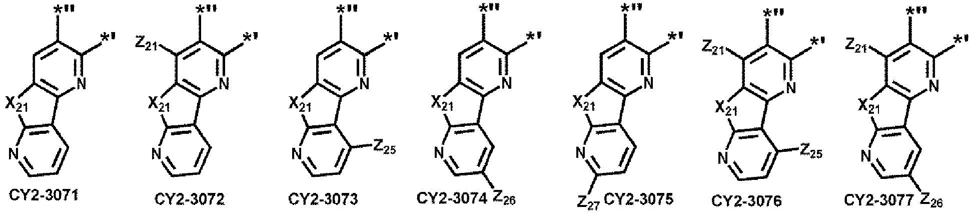 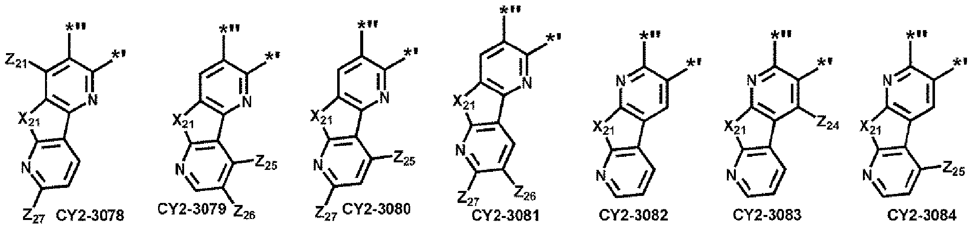 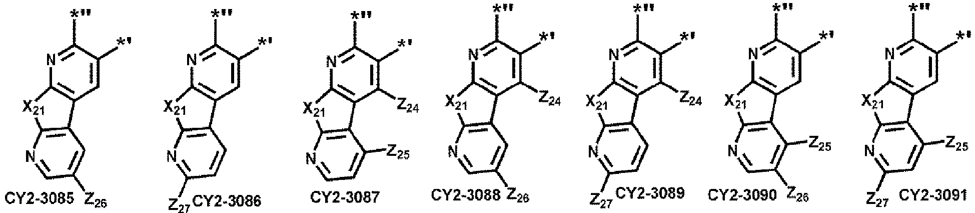 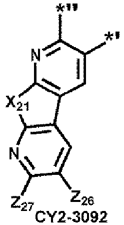 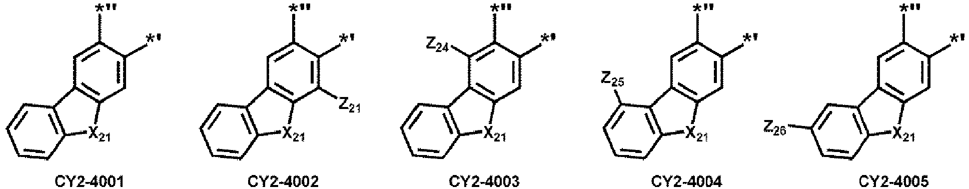 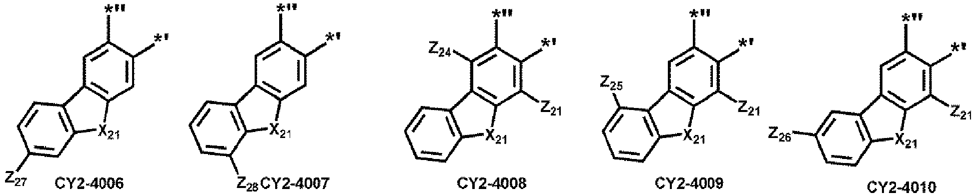 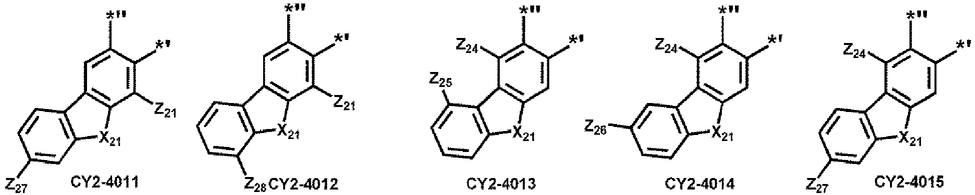 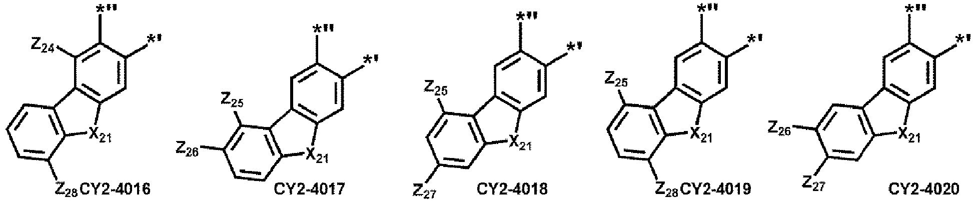 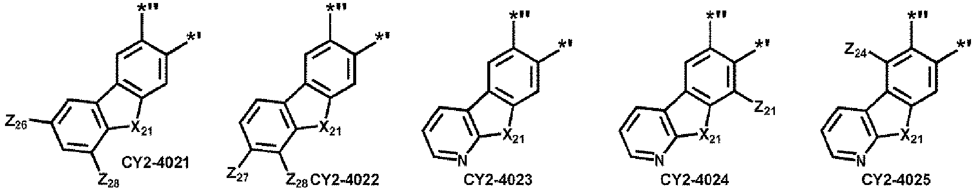 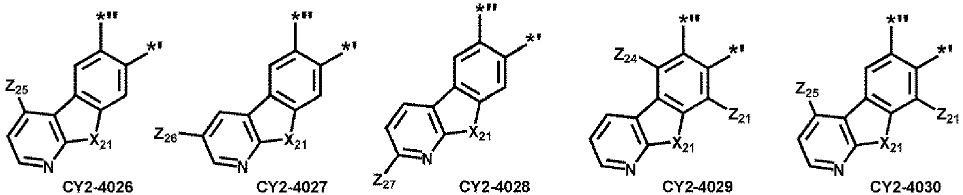 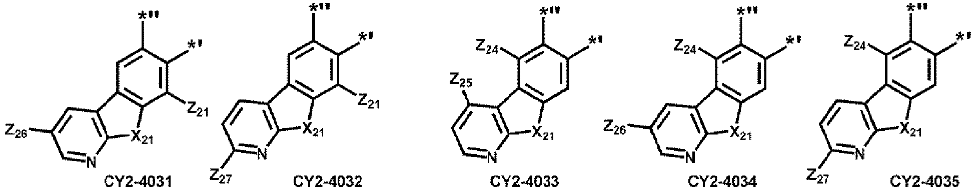 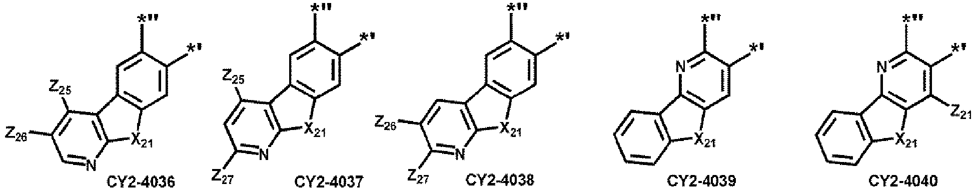 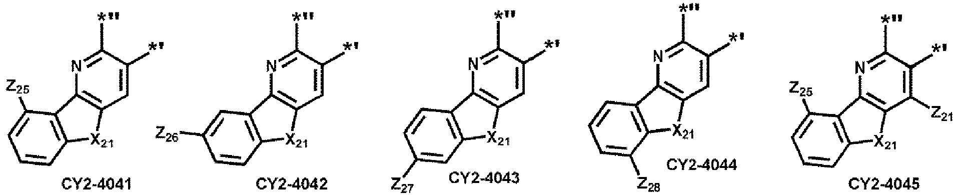 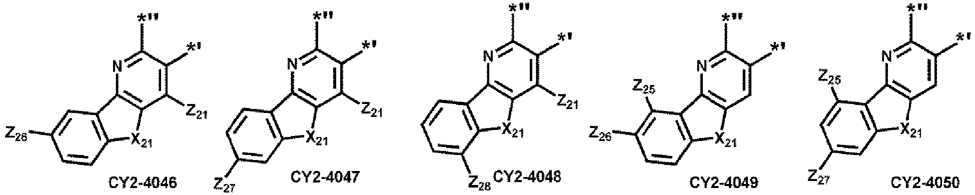 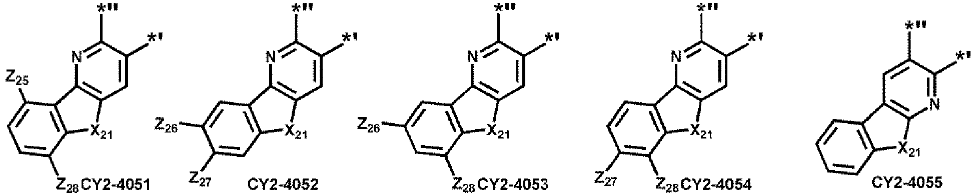 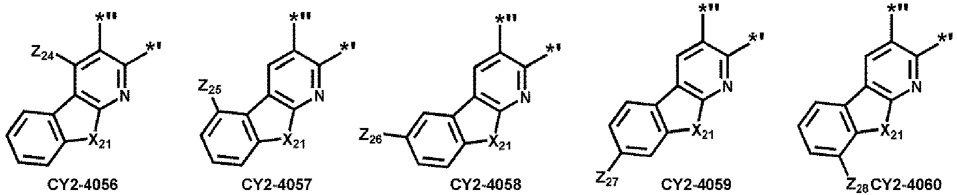 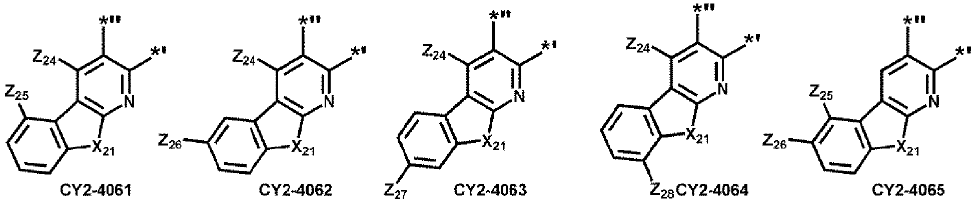 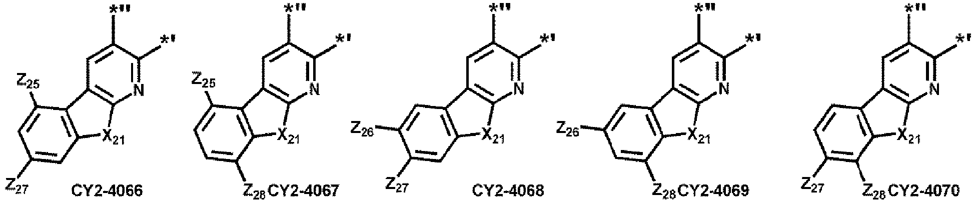 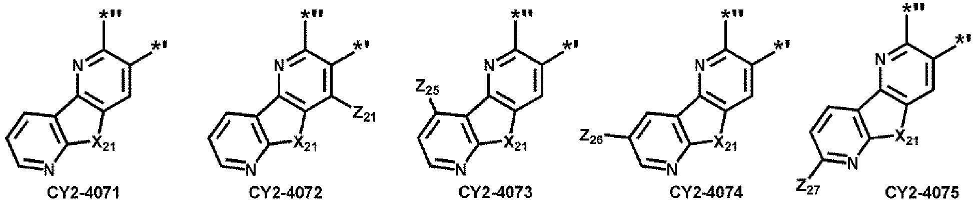 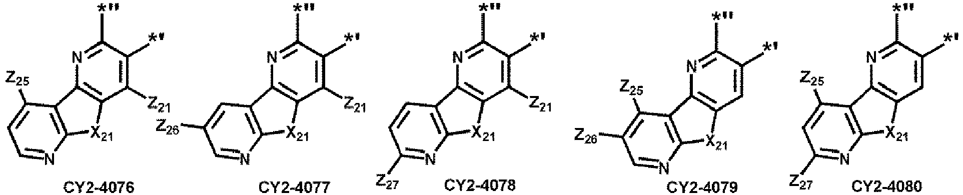 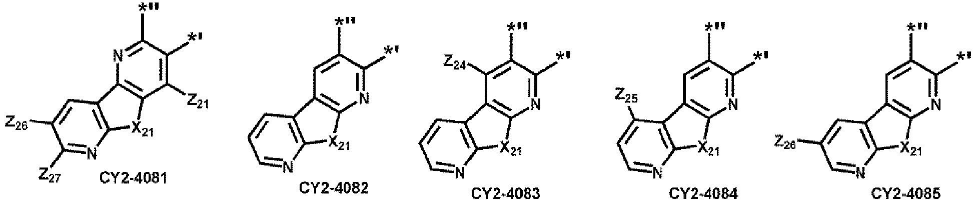 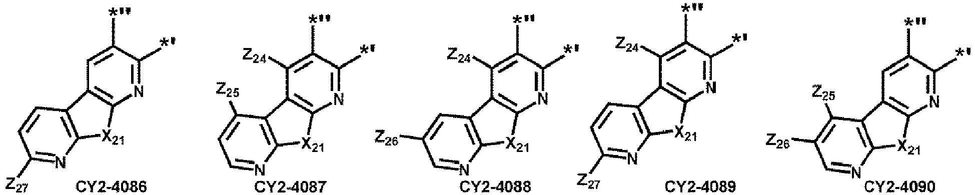 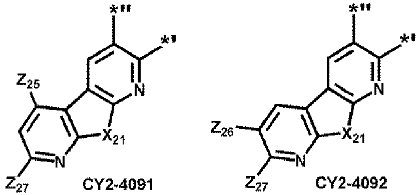 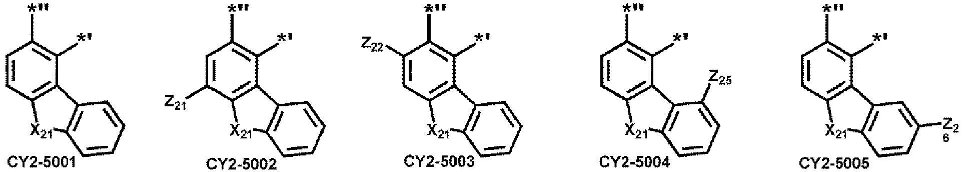 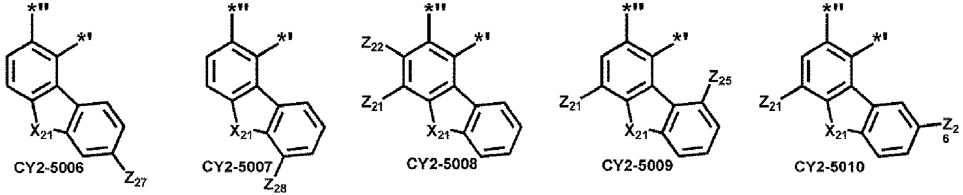 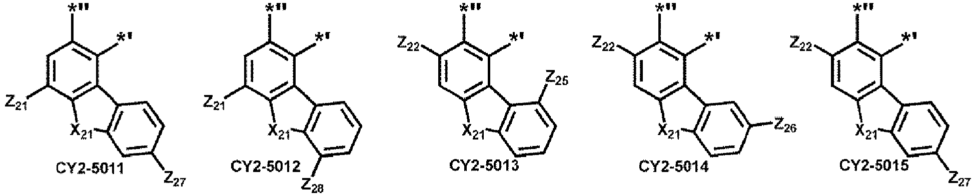 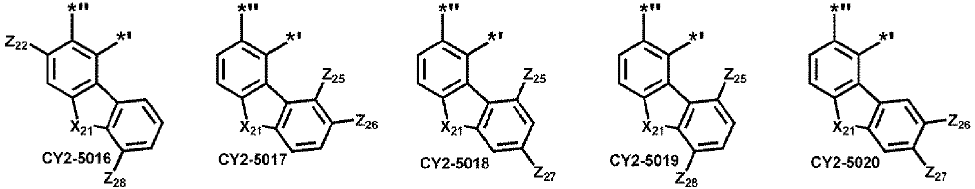 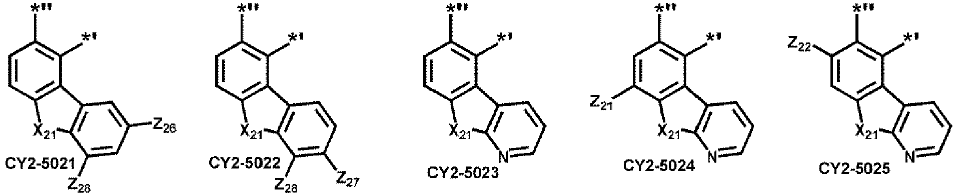 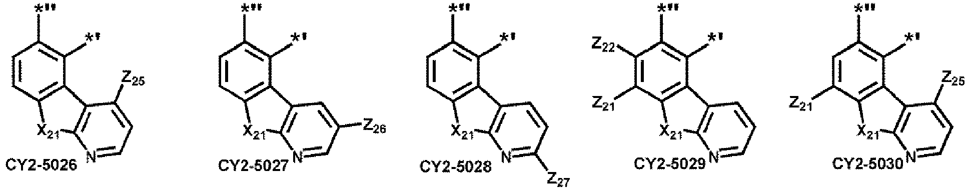 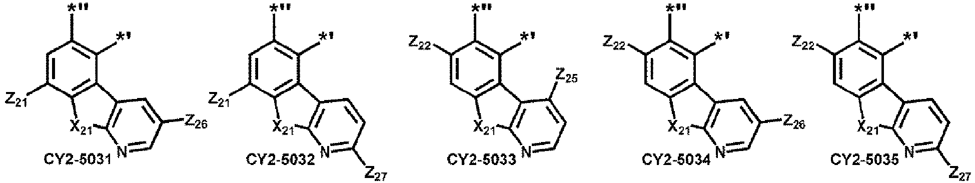 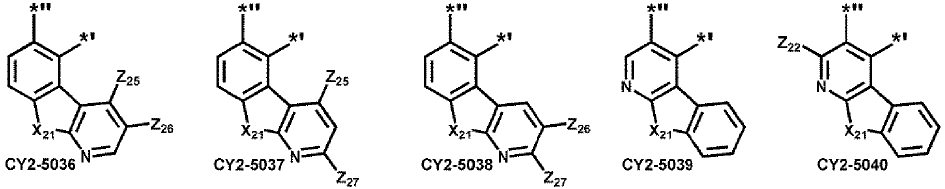 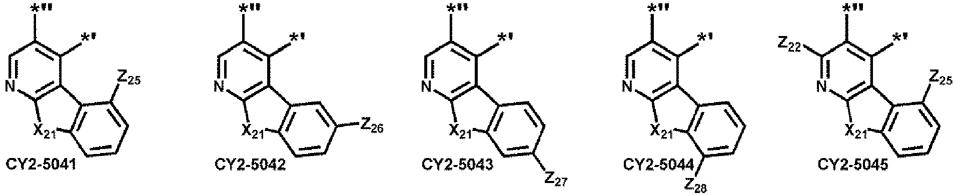 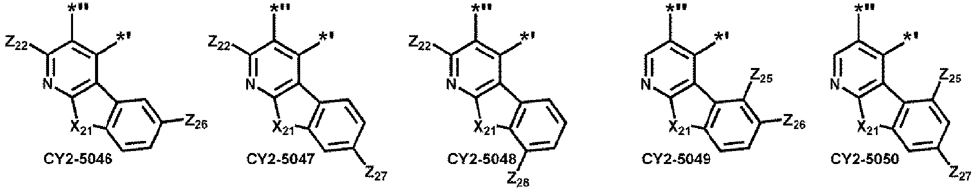 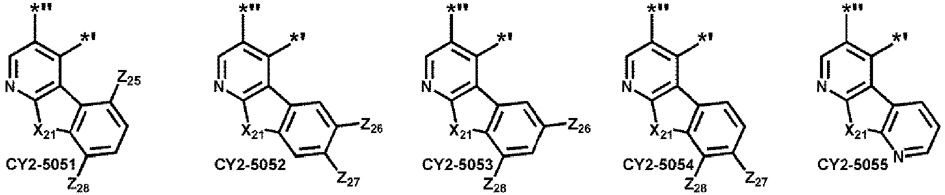 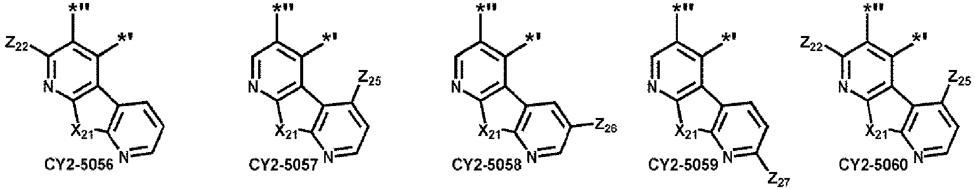 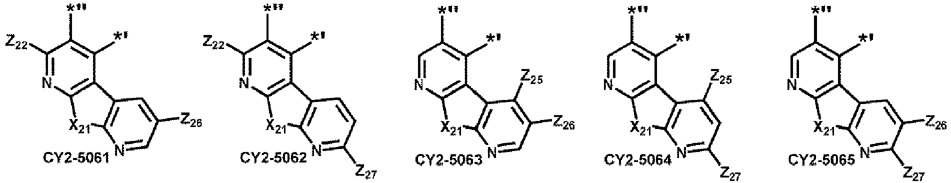 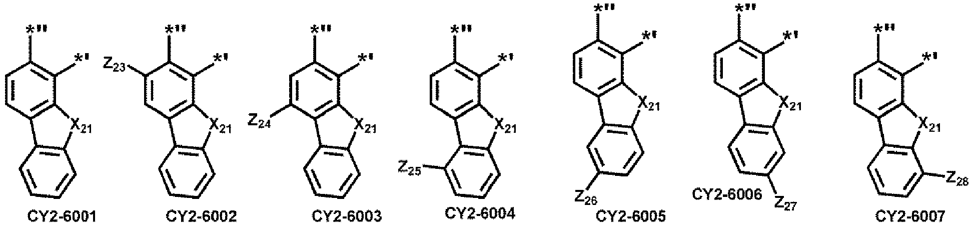 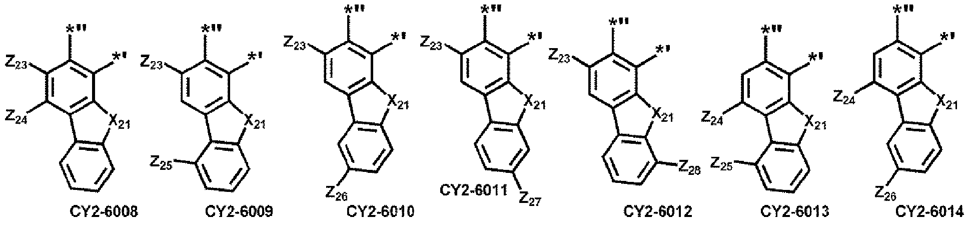 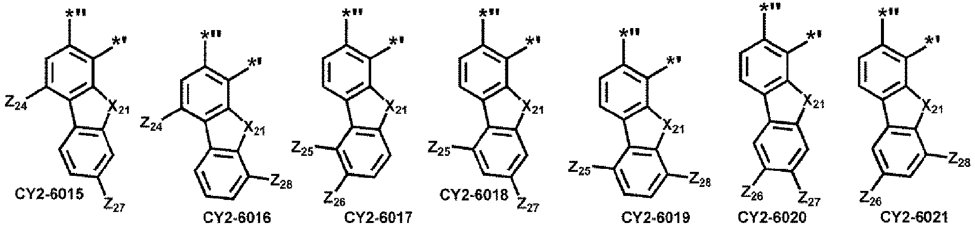 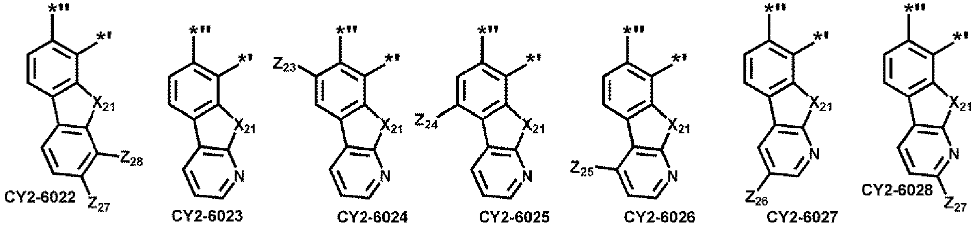 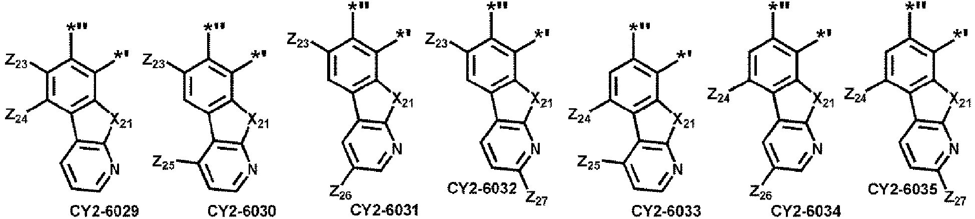 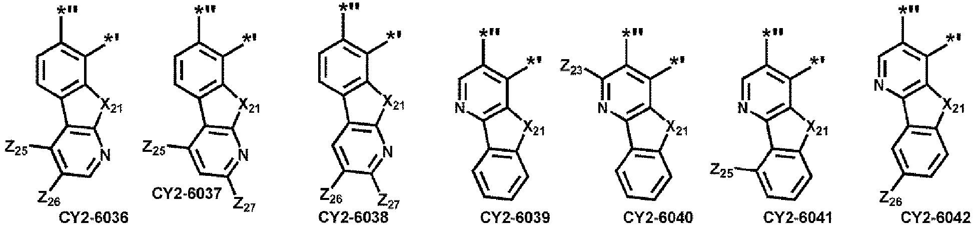 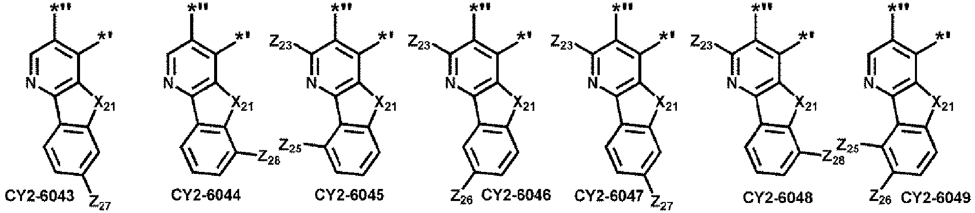 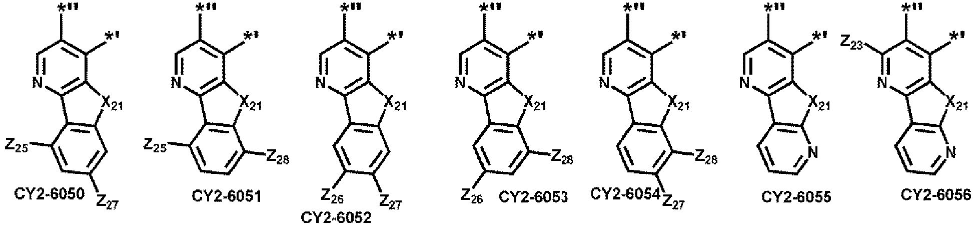 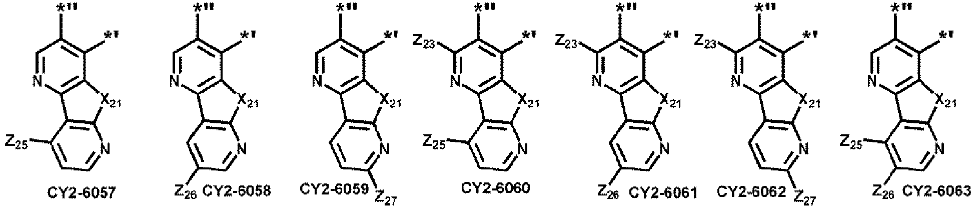 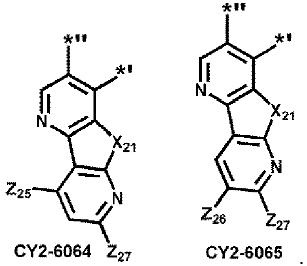 wherein, in Formulae CY2-1001 to CY2-1141, CY2-2001 to CY2-2092, CY2-3001 to CY2-3092, CY2-4001 to CY2-4092, CY2-5001 to CY2-5065 and CY2-6001 to CY2-6065,
X₂₁ is the same as described in claim 1,
Z₂₁ to Z₂₈ are each independently the same as defined in connection with Z₂ in claim 1, wherein each of Z₂₁ to Z₂₈ are not hydrogen,
*" indicates a binding site to ring CY₁ in Formula 2A, and
*' indicates a binding site to M in Formula 1.

12. The organometallic compound of any of claims 1-11, wherein a group represented by 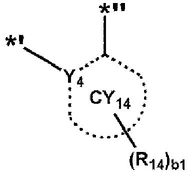 in Formula 2B is represented by one of Formulae CY14(1) to CY14(63): 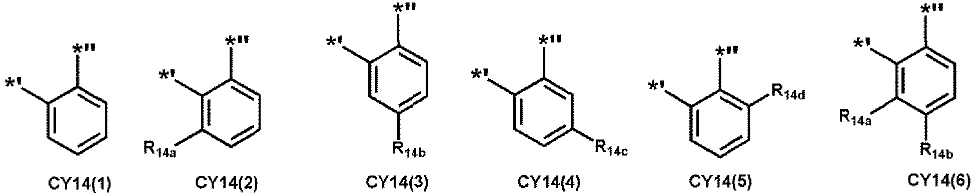 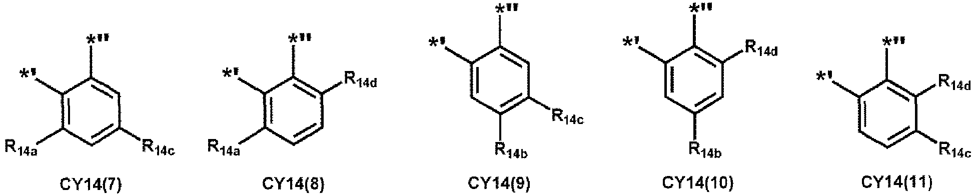 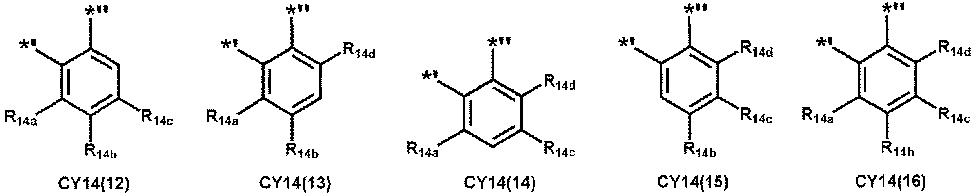 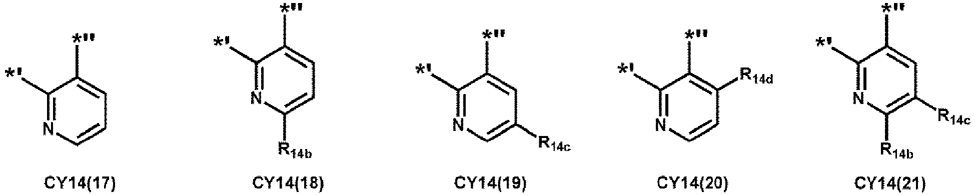 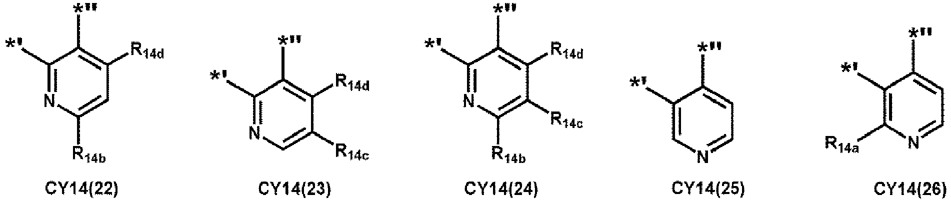 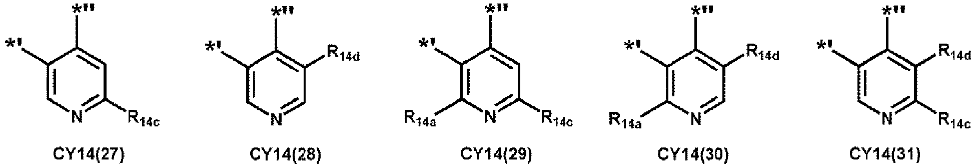 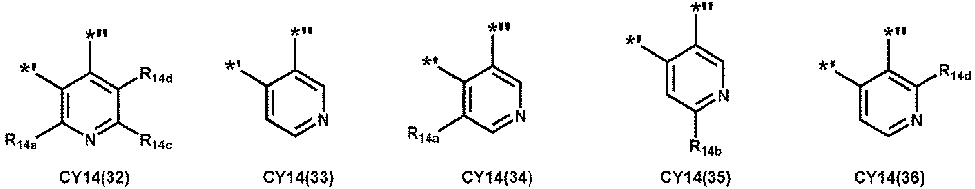 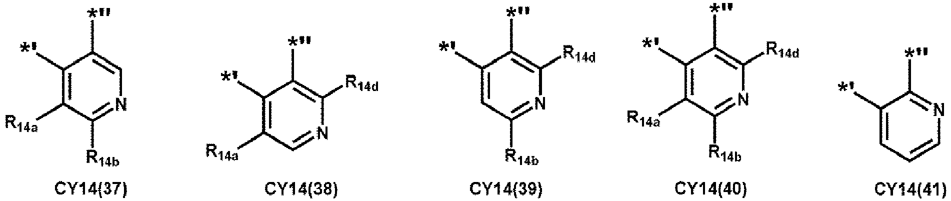 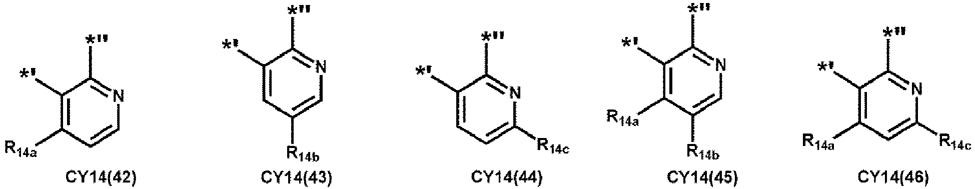 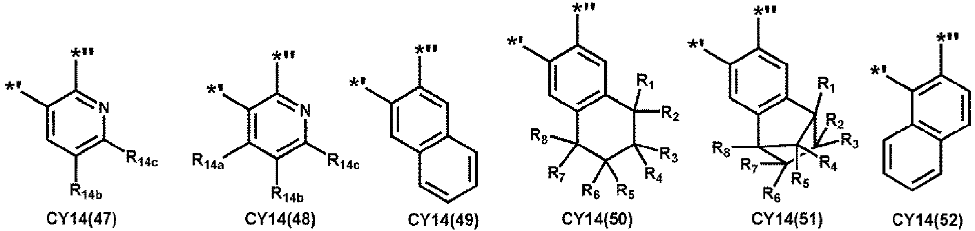 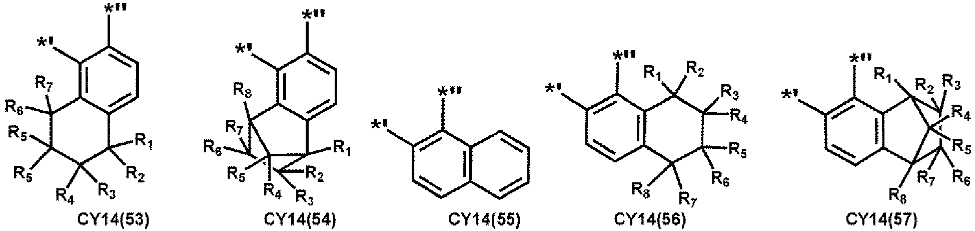 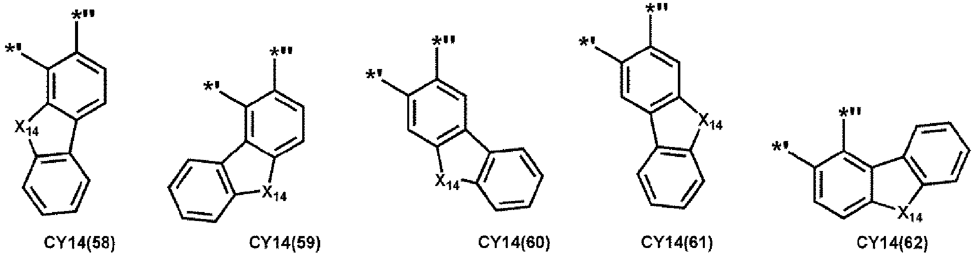 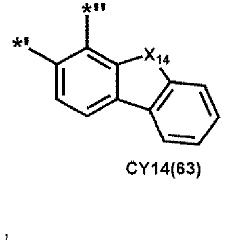
wherein, in Formulae CY14(1) to CY14(63),
R₁₄ₐ to R_{14d} are each independently the same as defined in connection with R₁₄ in claim 1, wherein each of R₁₄ₐ to R_{14d} are not hydrogen,
X₁₄ is C(R₁)(R₂), N(R₁), O, S, or Si(R₁)(R₂),
R₁ to R₈ are each the same as defined in connection with R₁₄ in claim 1,
*" indicates a binding site to a carbon atom of a neighboring pyridine ring in Formula 2B, and
*' indicates a binding site to M in Formula 1.

13. An organic light-emitting device comprising:
a first electrode,
a second electrode; and
an organic layer disposed between the first electrode and the second electrode and comprising an emission layer,
wherein the organic layer comprises at least one of the organometallic compound of any of claims 1-12; preferably
wherein
the first electrode is an anode,
the second electrode is a cathode, and
the organic layer further comprises a hole transport region disposed between the first electrode and the emission layer and an electron transport region disposed between the emission layer and the second electrode,
wherein the hole transport region comprises a hole injection layer, a hole transport layer, an electron blocking layer, a buffer layer, or any combination thereof, and
the electron transport region comprises a hole blocking layer, an electron transport layer, an electron injection layer, or any combination thereof.

14. The organic light-emitting device of claim 13, wherein
the emission layer comprises the at least one of the organometallic compound;
preferably wherein
the emission layer further comprises a host, and an amount of the host is greater than that of the organometallic compound.

15. An electronic apparatus comprising the organic light-emitting device of claims 13 or 14.

## Patentansprüche

1. Metallorganische Verbindung, dargestellt durch Formel 1:
<Formel 1> M(L₁)ₙ₁(L₂)ₙ₂
wobei, in Formel 1,
M ein Übergangsmetall ist;
L₁ ein Ligand dargestellt durch Formel 2A ist,
L₂ ein Ligand dargestellt durch Formel 2B ist,
n1 und n2 jeweils unabhängig 1 oder 2 sind, wobei, wenn n1 2 ist, zwei Li(s) gleich oder verschieden voneinander sind und wenn n2 2 ist, zwei L₂(s) gleich oder voneinander verschieden sind,
die Summe von n1 und n2 2 oder 3 ist, und L₁ und L₂ voneinander verschieden sind: 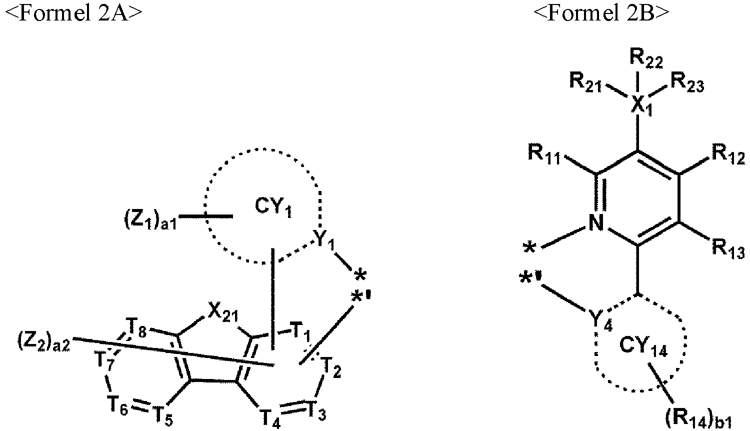 wobei in Formeln 2A und 2B,
Y₁ und Y₄ jeweils unabhängig C oder N sind,
X₁ Si oder Ge ist,
**X₂₁** O, S, S(=O), N(Z₂₉), C(Z₂₉)(Z₃₀) oder Si(Z₂₉)(Z₃₀) ist,
T₁ bis T₄ jeweils unabhängig C, N, ein mit Ring CY₁ verbundener Kohlenstoff oder ein mit M verbundener Kohlenstoff in Formel 1 sind, wobei eines von T₁ bis T₄ ein mit M in Formel 1 verbundener Kohlenstoff ist, und eines der verbleibenden T₁ bis T₄ die nicht mit M in Formel 1 verbunden sind, Kohlenstoff ist, der mit Ring CY₁ verbunden ist,
T₅ bis T₈ jeweils unabhängig C oder N sind,
wenn X₁ Si ist, mindestens eines der übrigen T₁ bis T₈, die nicht mit M und dem Ring CY₁ verbunden sind, N ist,
Ring CY₁ und Ring CY₁₄ jeweils unabhängig eine C₅-C₃₀-carbocyclische Gruppe oder eine C₁-C₃₀-heterocyclische Gruppe sind,
R₂₁ bis R₂₃ jeweils unabhängig eine C₁-C₆₀-Alkylgruppe oder eine C₆-C₆₀-Arylgruppe, jeweils unsubstituiert oder substituiert mit Deuterium, -F, -Cl, -Br, -I, -CD₃, -CD₂H, -CDH₂, -CF₃, -CF₂H, -CFH₂, eine Hydroxylgruppe, eine Cyanogruppe, eine Nitrogruppe, eine Aminogruppe, eine Amidinogruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, eine C₁-C₆₀-Alkylgruppe, eine C₃-C₁₀-Cycloalkylgruppe, eine Phenylgruppe oder eine beliebige Kombination davon sind,
Z₁, Z₂ und R₁₁ bis R₁₄ jeweils unabhängig Wasserstoff, Deuterium, -F, -Cl, -Br, -I, - SF₅, eine Hydroxylgruppe, eine Cyanogruppe, eine Nitrogruppe, eine Aminogruppe, eine Amidinogruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, eine substituierte oder unsubstituierte C₁-C₆₀-Alkylgruppe, eine substituierte oder unsubstituierte C₂-C₆₀-Alkenylgruppe, eine substituierte oder unsubstituierte C₂-C₆₀-Alkinylgruppe, eine substituierte oder unsubstituierte C₁-C₆₀-Alkoxygruppe, eine substituierte oder unsubstituierte C₁-C₆₀-Alkylthiogruppe, eine substituierte oder unsubstituierte C₃-C₁₀-Cycloalkylgruppe, eine substituierte oder unsubstituierte C₁-C₁₀-Heterocycloalkylgruppe, eine substituierte oder unsubstituierte C₃-C₁₀-Cycloalkenylgruppe, eine substituierte oder unsubstituierte C₁-C₁₀-Heterocycloalkenylgruppe, eine substituierte oder unsubstituiertes C₆-C₆₀-Arylgruppe, eine substituierte oder unsubstituierte C₆-C₆₀-Aryloxygruppe, eine substituierte oder unsubstituierte C₆-C₆₀-Arylthiogruppe, eine substituierte oder unsubstituierte C₁-C₆₀-Heteroarylgruppe, eine substituierte oder unsubstituierte monovalente nicht-aromatische kondensierte polyzyklische Gruppe, eine substituierte oder unsubstituierte monovalente nicht-aromatische kondensierte heteropolyzyklische Gruppe, -N(Q₁)(Q₂), -Ge(Q₃)(Q₄)(Q₅), -B(Q₆)(Q₇), -P(=O)(Q₈)(Q₉) oder -P(Q₈)(Q₉), wobei R₁₂ weder Wasserstoff noch an Methylgruppe ist,
a1 und b1 jeweils unabhängig eine ganze Zahl von 0 bis 20 sind, wobei, wenn a1 2 oder mehr ist, zwei oder mehrere Z₁(s) gleich oder verschieden sind, und wenn b 1 2 oder mehr ist, zwei oder mehrere R₁₄(s) gleich oder verschieden sind,
a2 eine ganze Zahl von 0 bis 6 ist, wobei, wenn a2 2 oder mehr ist, zwei oder mehrere Z₂(s) gleich oder verschieden sind,
zwei oder mehrere ausgewählt aus R₂₁ bis R₂₃ optional verbunden sind, um eine C₅-C₃₀-carbocyclische Gruppe, die unsubstituiert oder mit mindestens einem R₁₀ₐ substituiert ist oder eine C₁-C₃₀-heterocyclische Gruppe, die unsubstituiert oder mit mindestens einem R₁₀ₐ substituiert ist, zu bilden,
zwei oder mehrere ausgewählt aus einer Vielzahl von Z₁(s) optional verbunden sind, um eine C₅-C₃₀-carbocyclische Gruppe, die unsubstituiert oder mit mindestens einem R₁₀ₐ substituiert ist oder eine C₁-C₃₀-heterocyclische Gruppe, die unsubstituiert oder mit mindestens einem R₁₀ₐ substituiert ist, zu bilden,
zwei oder mehrere ausgewählt aus einer Vielzahl von Z₂(s) optional verbunden sind, um eine C₅-C₃₀-carbocyclische Gruppe, die unsubstituiert oder mit mindestens einem R₁₀ₐ substituiert ist oder eine C₁-C₃₀-heterocyclische Gruppe, die unsubstituiert oder mit mindestens einem R₁₀ₐ substituiert ist, zu bilden,
R₁₂ und R₁₃ optional verbunden sind, um eine C₅-C₃₀-carbocyclische Gruppe, die unsubstituiert oder mit mindestens einem R₁₀ₐ substituiert ist oder eine C₁-C₃₀-heterocyclische Gruppe, die unsubstituiert oder mit mindestens einem R₁₀ₐ substituiert ist, zu bilden,
zwei oder mehrere ausgewählt aus einer Vielzahl von R₁₄(s) optional verbunden sind, um eine C₅-C₃₀-carbocyclische Gruppe, die unsubstituiert oder mit mindestens einem R₁₀ₐ substituiert ist oder eine C₁-C₃₀-heterocyclische Gruppe, die unsubstituiert oder mit mindestens einem R₁₀ₐ substituiert ist, zu bilden,
zwei oder mehrere ausgewählt aus Z₁, Z₂ und R₁₁ bis R₁₄ optional verbunden sind, um eine C₅-C₃₀-carbocyclische Gruppe, die unsubstituiert oder mit mindestens einem R₁₀ₐ substituiert ist oder eine C₁-C₃₀-heterocyclische Gruppe, die unsubstituiert oder mit mindestens einem R₁₀ₐ substituiert ist, zu bilden,
R₁₀ₐ gleich wie definiert in Verbindung mit R₁₄ ist,
^{∗} und ^{∗}' in Formeln 2A und 2B jeweils eine Bindungsstelle an M in Formel 1 angibt, ein Substituent(en) der substituierten C₁-C₆₀-Alkylgruppe, der substituierten C₂-C₆₀-Alkenylgruppe, der substituierten C₂-C₆₀-Alkinylgruppe, der substituierten C₁-C₆₀-Alkoxygruppe, der substituierten C₁-C₆₀-Alkylthiogruppe, der substituierten C₃-C₁₀-Cycloalkylgruppe, der substituierten C₁-C₁₀-Heterocycloalkylgruppe,der substituierten C₃-C₁₀-Cycloalkenylgruppe, der substituierten C₁-C₁₀-Heterocycloalkenylgruppe, der substituierten C₆-C₆₀-Arylgruppe, der substituierten C₆- C₆₀-Aryloxygruppe, der substituierten C₆-C₆₀-Arylthiogruppe, der substituierten C₁-C₆₀-Heteroarylgruppe, der substituierten monovalenten nicht-aromatischen kondensierten polycyclischen Gruppe, und der substituierten monovalenten nicht-aromatischen kondensierten heteropolycyclischen Gruppe, wie folgt ist (sind):
Deuterium, -F, -Cl, -Br, -I, -CD₃, -CD₂H, -CDH₂, -CF₃, -CF₂H, -CFH₂, eine Hydroxylgruppe, eine Cyanogruppe, eine Nitrogruppe, eine Aminogruppe, eine Amidinogruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, eine C₁-C₆₀-Alkylgruppe, eine C₂-C₆₀-Alkenylgruppe, eine C₂-C₆₀-Alkinylgruppe oder eine C₁-C₆₀-Alkoxygruppe;
eine C₁-C₆₀-Alkylgruppe, eine C₂-C₆₀-Alkenylgruppe, eine C₂-C₆₀-Alkinylgruppe oder eine C₁-C₆₀-Alkoxygruppe, jeweils substituiert mit Deuterium, -F, -Cl, -Br, -I, -CD₃, -CD₂H, -CDH₂, -CF₃, -CF₂H, -CFH₂, eine Hydroxylgruppe, eine Cyanogruppe, eine Nitrogruppe, eine Aminogruppe, eine Amidinogruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, eine C₃-C₁₀-Cycloalkylgruppe, eine C₁-C₁₀-Heterocycloalkylgruppe,eine C₃-C₁₀-Cycloalkenylgruppe, eine C₁-C₁₀-Heterocycloalkenylgruppe, eine C₆-C₆₀-Arylgruppe, eine C₆-C₆₀-Aryloxygruppe, eine C₆-C₆₀-Arylthiogruppe, eine C₁-C₆₀-Heteroarylgruppe, eine monovalente nicht-aromatische kondensierte polyzyklische Gruppe, eine monovalente nicht-aromatische kondensierte heteropolyzyklische Gruppe, -N(Q₁₁)(Q₁₂), -Ge(Q₁₃)(Q₁₄)(Q₁₅), -B(Q₁₆)(Q₁₇), -P(=O)(Q₁₈)(Q₁₉), -P(Q₁₈)(Q₁₉) oder eine beliebige Kombination davon;
eine C₃-C₁₀-Cycloalkylgruppe, eine C₁-C₁₀-Heterocycloalkylgruppe,eine C₃-C₁₀-Cycloalkenylgruppe, eine C₁-C₁₀-Heterocycloalkenylgruppe, eine C₆-C₆₀-Arylgruppe, eine C₆-C₆₀-Aryloxygruppe, eine C₆-C₆₀-Arylthiogruppe, eine C₁-C₆₀-Heteroarylgruppe, eine monovalenten nichtaromatischen kondensierten polycyclischen Gruppe oder eine monovalenten nichtaromatischen kondensierten heteropolycyclischen Gruppe, jeweils unsubstituiert oder substituiert mit Deuterium, -F, -Cl, -Br, -I, -CD₃, -CD₂H, -CDH₂, -CF₃, -CF₂H, -CFH₂, eine Hydroxylgruppe, eine Cyanogruppe, eine Nitrogruppe, eine Aminogruppe, eine Amidinogruppe, eine Hydrazingruppe, eine Hydrazongruppe, eine Carbonsäuregruppe oder einem Salz davon, eine Sulfonsäuregruppe oder einem Salz davon, eine Phosphorsäuregruppe oder einem Salz davon, eine C₁-C₆₀-Alkylgruppe, eine C₂-C₆₀-Alkenylgruppe, eine C₂-C₆₀-Alkynylgruppe, eine C₁-C₆₀-Alkoxygruppe, eine C₃-C₁₀-Cycloalkylgruppe, eine C₁-C₁₀-Heterocycloalkylgruppe,eine C₃-C₁₀-Cycloalkenylgruppe, eine C₁-C₁₀-Heterocycloalkenylgruppe, eine C₆-C₆₀-Arylgruppe, eine C₆-C₆₀-Aryloxygruppe, eine C₆-C₆₀-Arylthiogruppe, eine C₁-C₆₀-Heteroarylgruppe, eine monovalenten nichtaromatischen kondensierten polycyclischen Gruppe und eine monovalenten nichtaromatischen kondensierten heteropolycyclischen Gruppe, -N(Q₂₁)(Q₂₂),-Ge(Q₂₃)(Q₂₄)(Q₂₅), -B(Q₂₆)(Q₂₇), -P(=O)(Q₂₈)(Q₂₉), -P(Q₂₈)(Q₂₉) oder eine beliebige Kombination davon;
-N(Q₃₁)(Q₃₂), -Ge(Q₃₃)(Q₃₄)(Q₃₅), -B(Q₃₆)(Q₃₇), -P(=O)(Q₃₈)(Q₃₉) oder -P(Q₃₈)(Q₃₉); oder eine beliebige Kombination davon;
Q₁ bis Q₉, Q₁₁ bis Q₁₉, Q₂₁ bis Q₂₉ und Q₃₁ bis Q₃₉ jeweils unabhängig Wasserstoff; Deuterium; -F; -Cl; -Br; -I; eine Hydroxylgruppe; eine Cyanogruppe; eine Nitrogruppe; eine Aminogruppe; eine Amidinogruppe; eine Hydrazingruppe; eine Hydrazongruppe; eine Carbonsäuregruppe oder ein Salz davon; eine Sulfonsäuregruppe oder ein Salz davon; eine Phosphorsäuregruppe oder ein Salz davon; eine C₁-C₆₀-Alkylgruppe unsubstituiert oder substituiert mit Deuterium, eine C₁-C₆₀-Alkylgruppe, eine C₆-C₆₀-Arylgruppe oder eine beliebige Kombination davon; eine C₂-C₆₀-Alkenylgruppe, eine C₂-C₆₀-Alkinylgruppe, eine C₁-C₆₀-Alkoxygruppe; eine C₃-C₁₀-Cycloalkylgruppe; eine C₁-C₁₀-Heterocycloalkylgruppe; eine C₃-C₁₀-Cycloalkenylgruppe; eine C₁-C₁₀-Heterocycloalkenylgruppe; eine C₆-C₆₀-Arylgruppe unsubstituiert oder substituiert mit Deuterium, eine C₁-C₆₀-Alkylgruppe, eine C₆-C₆₀-Arylgruppe oder eine beliebige Kombination davon; eine C₆-C₆₀-Aryloxygruppe, eine C₆-C₆₀-Arylthiogruppe, eine C₁-C₆₀-Heteroarylgruppe; eine monovalente nicht-aromatische kondensierte polycyclische Gruppe; oder eine monovalente nicht-aromatische kondensierte heteropolycyclische Gruppe sind.

2. Metallorganische Verbindung nach Anspruch 1, wobei
M in Formel 1 Ir ist und die Summe von n1 und n2 3 ist oder
M in Formel 1 Pt ist und die Summe von n1 und n2 2 ist.

3. Metallorganische Verbindung nach Anspruch 1 oder 2, wobei
X₂₁ in Formel 2A O oder S ist; und/oder
wobei
Z₁ in Formel 2A wie folgt ist:
Wasserstoff, Deuterium, -F oder eine Cyanogruppe;
eine C₁-C₂₀-Alkylgruppe unsubstituiert oder substituiert mit Deuterium, -F, eine Cyanogruppe, eine C₃-C₁₀-Cycloalkylgruppe, eine deuterierte C₃-C₁₀-Cycloalkylgruppe, eine (C₁-C₂₀-Alkyl)C₃-C₁₀-Cycloalkylgruppe, eine C₁-C₁₀-Heterocycloalkylgruppe,eine deuterierte C₁-C₁₀-Heterocycloalkylgruppe,eine (C₁-C₂₀-Alkyl)C₁-C₁₀-Heterocycloalkylgruppe oder eine beliebige Kombination davon; oder
eine C₃-C₁₀-Cycloalkylgruppe oder eine C₁-C₁₀-Heterocycloalkylgruppe,unsubstituiert oder substituiert mit Deuterium, -F, eine Cyanogruppe, eine C₁-C₂₀-Alkylgruppe, eine deuterierte C₁-C₂₀-Alkylgruppe oder eine beliebige Kombination davon.

4. Metallorganische Verbindung nach einem der Ansprüche 1-3, wobei eine Anzahl von Kohlenstoff, die in R₁₂ von Formel 2B beinhaltet ist, mindestens zwei ist; und/oder wobei
die metallorganische Verbindung, dargestellt durch Formel 1, mindestens eine von <Bedingung (1)> bis <Bedingung (3)> unten erfüllt:
<Bedingung (1)>
In Formel 2A ist Z₁ nicht Wasserstoff ist und a1 ist eine ganze Zahl von 1 bis 20
<Bedingung (2)>
In Formel 2B ist R₁₄ nicht Wasserstoff, und b1 ist eine ganze Zahl von 1 bis 20
<Bedingung (3)>
In Formel 2A ist Z₂ nicht Wasserstoff ist und a2 ist eine ganze Zahl von 1 bis 6.

5. Metallorganische Verbindung nach einem der Ansprüche 1-4, wobei die durch Formel 1 dargestellte metallorganische Verbindung mindestens ein Deuterium, mindestens eine Fluorgruppe (-F), mindestens eine Cyanogruppe (-CN) oder eine beliebige Kombination davon umfasst; und/oder wobei
Z₂ in Formel 2A nicht Wasserstoff ist, a2 eine ganze Zahl von 1 bis 3 ist, und
mindestens eines von Z₂(s) in Anzahl von a2 jeweils unabhängig eine substituierte oder unsubstituierte C₁-C₆₀-Alkylgruppe, eine substituierte oder unsubstituierte C₁-C₆₀-Alkoxygruppe, eine substituierte oder unsubstituierte C₁-C₆₀-Alkylthiogruppe, eine substituierte oder unsubstituierte C₃-C₁₀-Cycloalkylgruppe, eine substituierte oder unsubstituierte C₁-C₁₀-Heterocycloalkylgruppe,eine substituierte oder unsubstituierte C₆-C₆₀-Arylgruppe, eine substituierte oder unsubstituierte C₁-C₆₀-Heteroarylgruppe, eine substituierte oder unsubstituierte monovalente nicht-aromatische kondensierte polyzyklische Gruppe, eine substituierte oder unsubstituierte monovalente nicht-aromatische kondensierte heteropolyzyklische Gruppe ist.

6. Metallorganische Verbindung nach einem der Ansprüche 1-5, wobei eine Gruppe, dargestellt durch 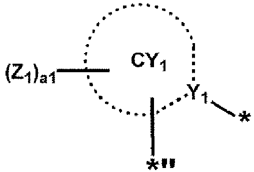 in Formel 2A dargestellt ist durch eine von Formeln CY1-1 bis CY1-28: 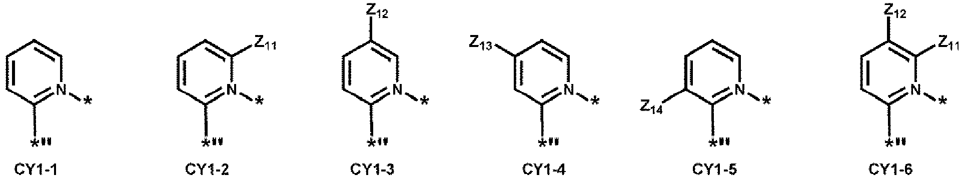 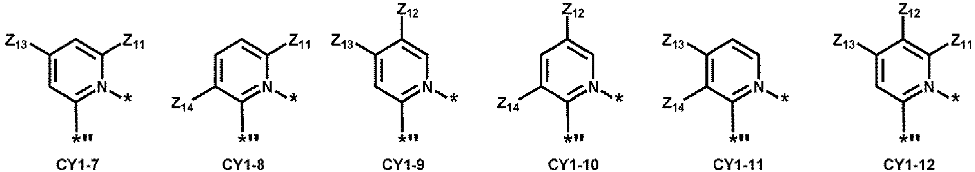 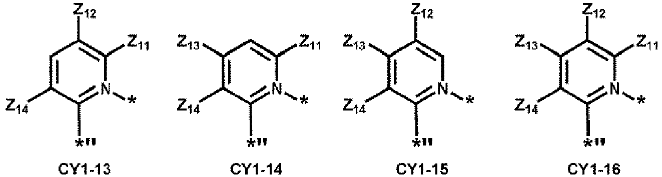 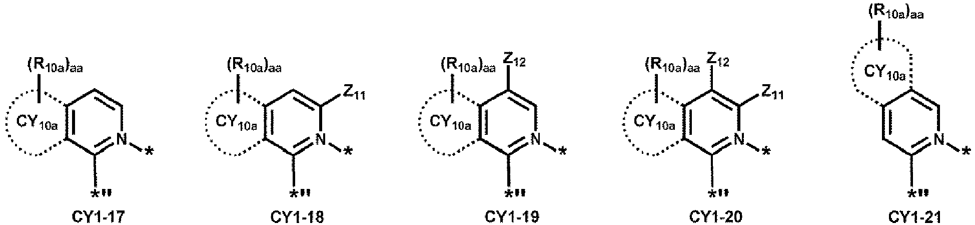 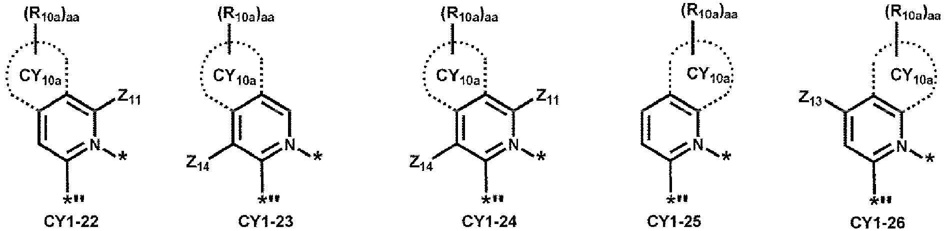 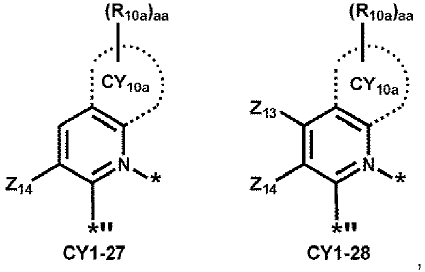 wobei, in Formeln CY1-1 bis CY1-28,
Zu bis Z₁₄ jeweils unabhängig gleich sind wie definiert in Verbindung mit Z₁ in Anspruch 1, wobei jedes von Z₁₁ bis Z₁₄ nicht Wasserstoff ist,
Ring CY₁₀ₐ eine C₅-C₃₀ -carbocyclische Gruppe oder eine C₁-C₃₀-heterocyclische Gruppe ist,
R₁₀ₐ gleich ist wie beschrieben in Anspruch 1, aa eine ganze Zahl von 0 bis 10 ist, und
^{∗} eine Bindungsstelle an M in Formel 1 angibt, und ^{∗}" eine Bindungsstelle eine eines von T₁ bis T₄ in Formel 2A angibt.

7. Metallorganische Verbindung nach Anspruch 6, wobei eine Gruppe dargestellt durch 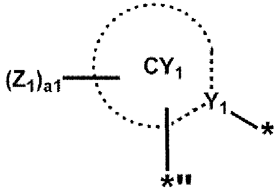 in Formel 2A durch eine von Formeln CY1-4, CY1-7, CY1-9, CY1-11, CY1-12 und CY1-14 bis CY1-24 dargestellt ist.

8. Metallorganische Verbindung nach einem der Ansprüche 1-7, wobei eine Gruppe, dargestellt durch 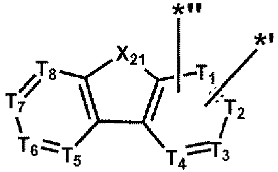 in Formel 2A, durch eine von Formeln CY2-1 bis CY2-6 dargestellt ist: 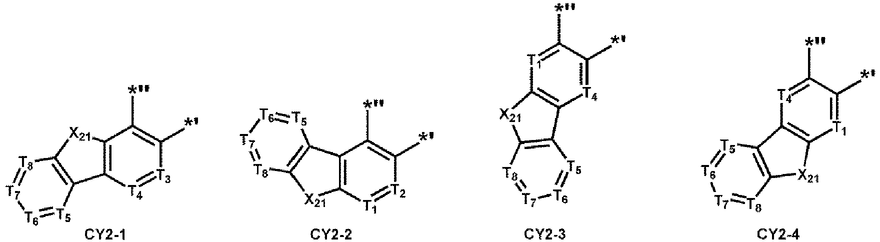 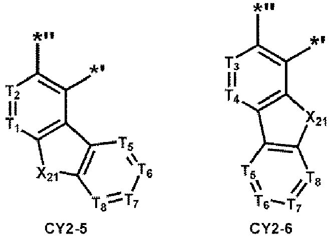 wobei, in den Formeln CY2-1 bis CY2-6,
T₁ bis T₈ jeweils unabhängig C oder N sind,
X₂₁ gleich ist wie beschrieben in Anspruch 1,
^{∗}" eine Bindungsstelle an Ring CY₁ in Formel 2A angibt, und ^{∗}' eine Bindungsstelle an M in Formel 1 angibt.

9. Metallorganische Verbindung nach Anspruch 8, wobei
a) X₁ in Formel 2B Ge ist, und jedes von T₁ bis T₈ in Formeln CY2-1 bis CY2-6 C ist,
b) X₁ in Formel 2B Si oder Ge ist, und mindestens eines von T₃ bis T₈ in Formeln CY2-1 und CY2-6 N ist,
c) X₁ in Formel 2B Si oder Ge ist, und mindestens eines von T₁, T₂ und T₅ bis T₈ in Formeln CY2-2 und CY2-5 N ist,
d) X₁ in Formel 2B Si oder Ge ist, und mindestens eines von T₁ und T₄ bis T₈ in Formeln CY2-3 und CY2-4 N ist.

10. Metallorganische Verbindung nach Anspruch 8 oder 9, wobei
1) T₁ bis T₈ in Formeln CY2-1 bis CY2-6 C sind;
2) eines von T₃ bis T₈ in Formel CY2-1 N ist, und das restliche T₃ bis T₈ die in Formel CY2-1 nicht N sind, C sind;
3) T₃ und T₈ in Formel CY2-1 N sind, und T₄ bis T₇in Formel CY2-1 C sind;
4) T₆ und T₈ in Formel CY2-1 N sind, und T₃ bis T₅ und T₇ in Formel CY2-1 C sind;
5) eines von T₁, T₂ und T₈ in Formel CY2-2 N ist, und die restlichen T₁, T₂ und T₅ bis T₈, die in Formel CY2-2 nicht N sind, C sind;
6) T₁ und T₈ in Formel CY2-2 N sind, und T₂ und T₅ bis T₇ in Formel CY2-2 C sind;
7) T₂ und T₈ in Formel CY2-2 N sind, und T₁ und T₅ bis T₇ in Formel CY2-2 C sind;
8) eines von T₁, T₄ und T₈ in Formeln CY2-3 und CY2-4 N ist, und die restlichen T₁, T₄ und T₅ bis T₈, die in Formeln CY2-3 und CY2-4 nicht N sind, C sind;
9) T₁ und T₈ in Formeln CY2-3 und CY2-4 N sind, und T₄ und T₅ bis T₇ in Formeln CY2-3 und CY2-4 C sind;
10) T₄ und T₈ in Formeln CY2-3 und CY2-4 N sind, und T₁ und T₅ bis T₇ in Formeln CY2-3 und CY2-4 C sind;
11) eines von T₁ und T₈ in Formel CY2-5 N ist, und die restlichen T₁, T₂ und T₅ bis T₈, die in Formel CY2-5 nicht N sind, C sind;
12) T₁ und T₈ in Formel CY2-5 N sind, und T₂ und T₅ bis T₇ in Formel CY2-5 C sind;
13) eines von T₄ und T₈ in Formel CY2-6 N ist, und die restlichen T₃ bis T₈, die in Formel CY2-6 nicht N sind, C sind; oder
14) T₄ und T₈ in Formel CY2-6 N sind, und T₃ und T₅ bis T₇ in Formel CY2-6 C sind.

11. Metallorganische Verbindung nach einem der Ansprüche 1-10, wobei eine Gruppe, dargestellt durch 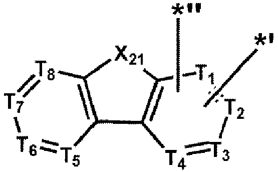 in Formel 2A, durch eine von Formeln CY2-1001 bis CY2-1141, CY2-2001 bis CY2-2092, CY2-3001 bis CY2-3092, CY2-4001 bis CY2-4092, CY2-5001 bis CY2-5065 und CY2-6001 bis CY2-6065 dargestellt ist: 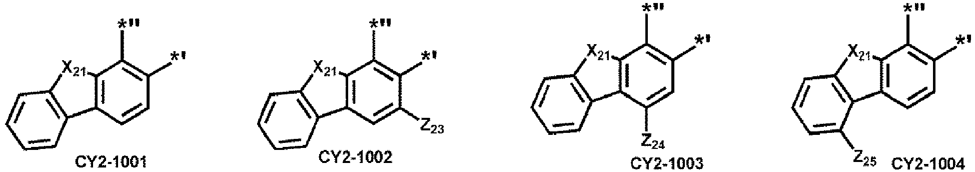 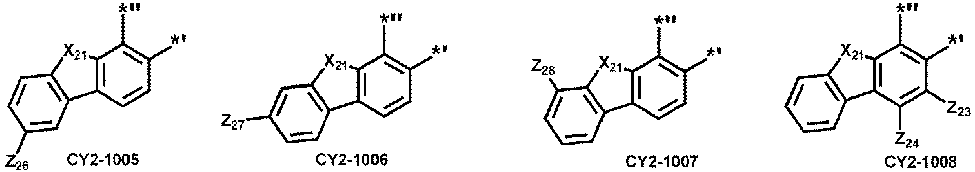 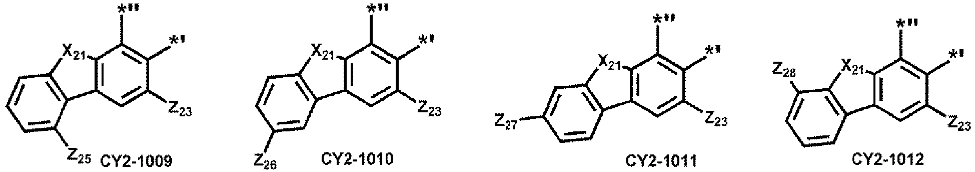 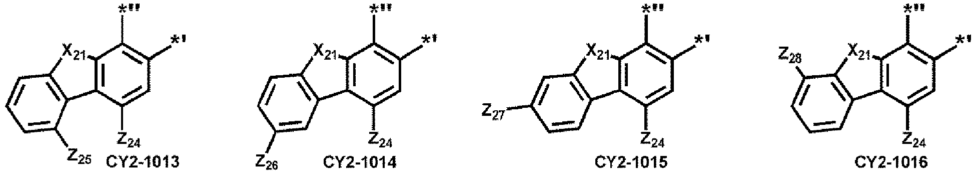 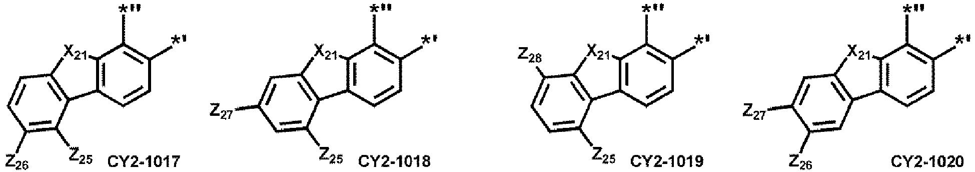 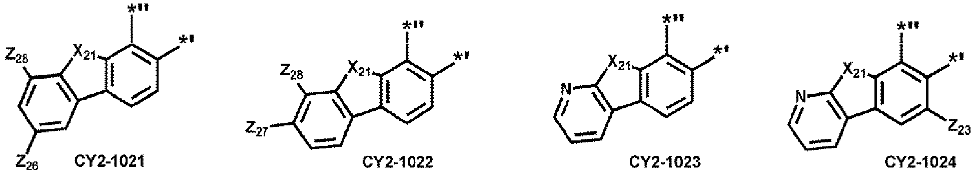 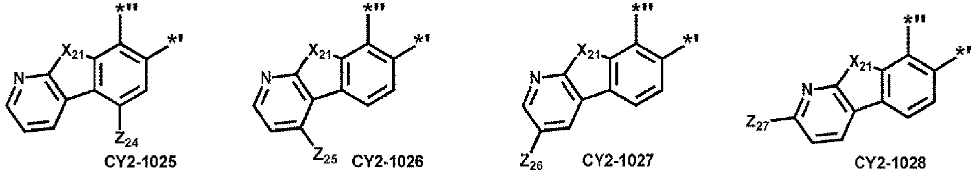 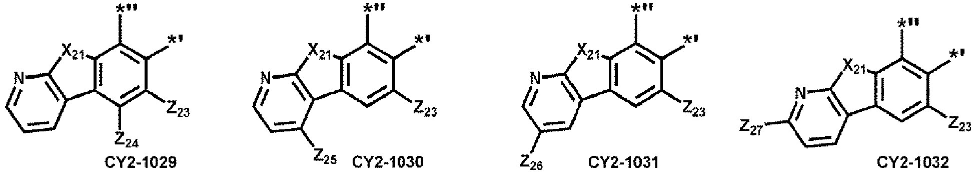 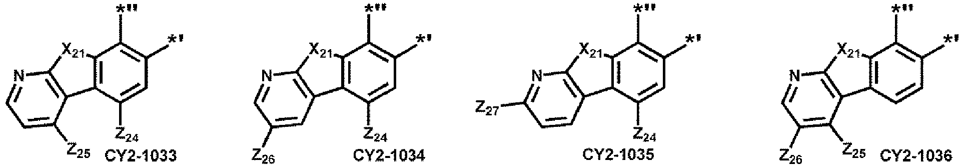 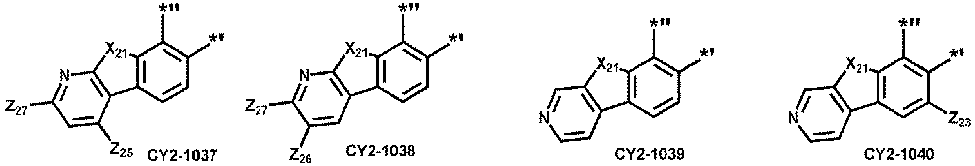 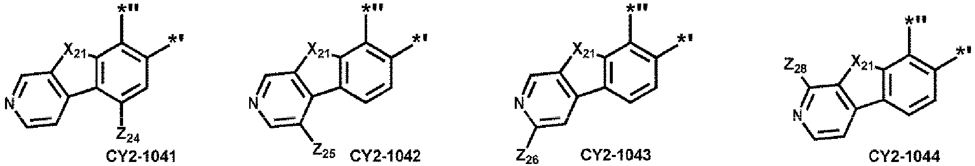 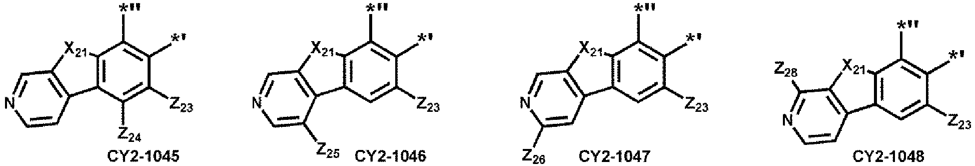 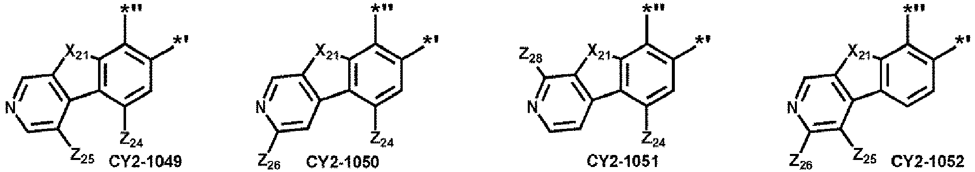 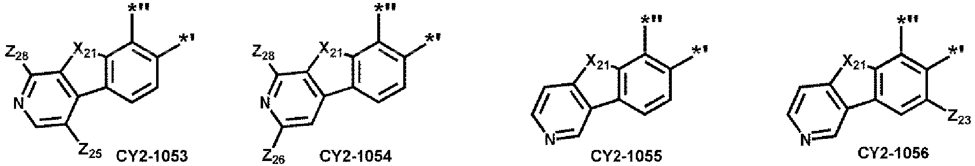 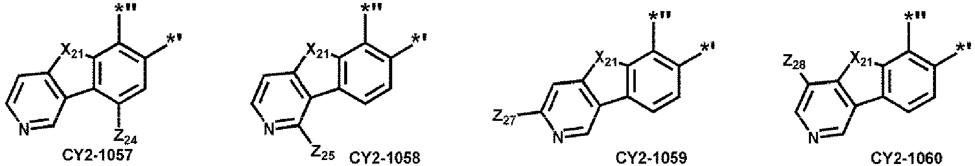 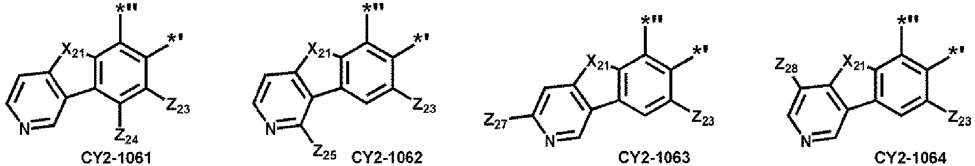 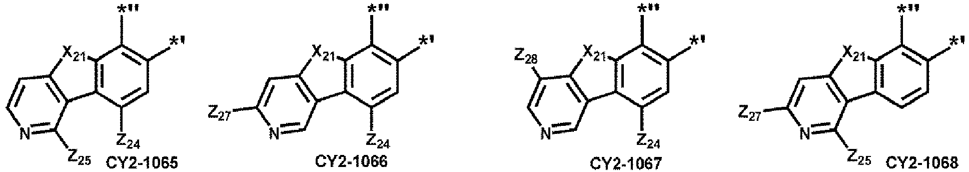 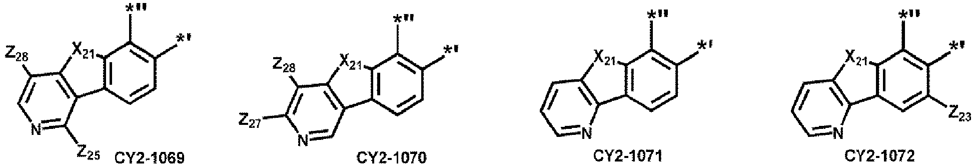 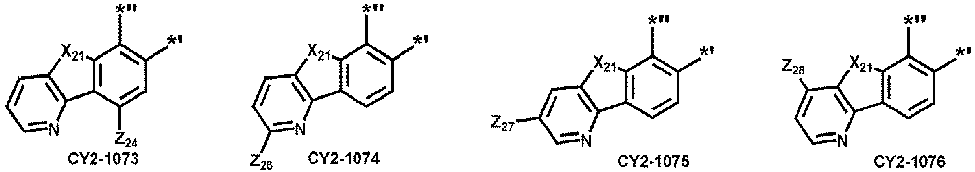 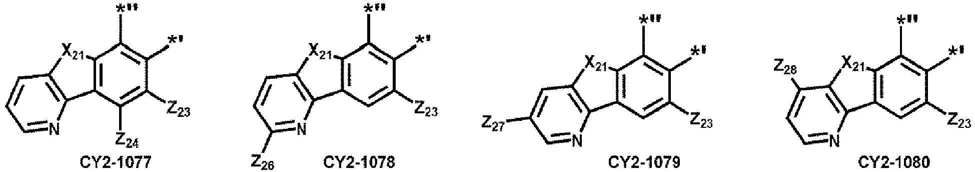 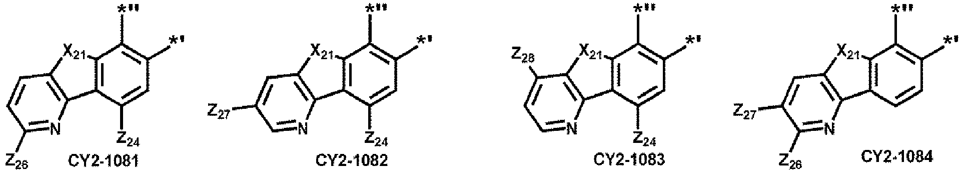 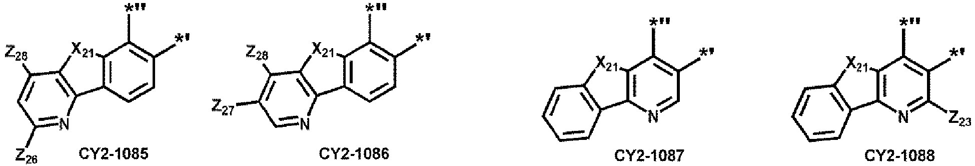 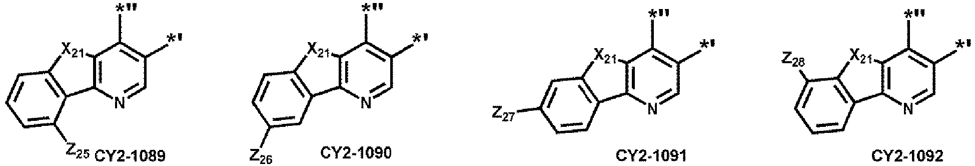 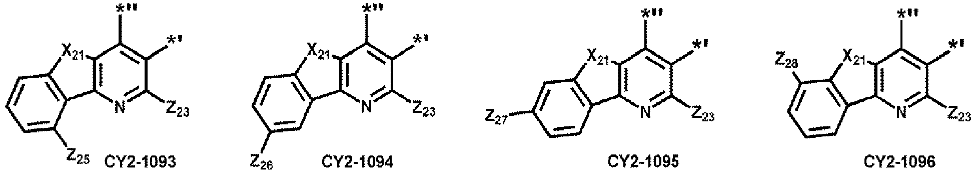 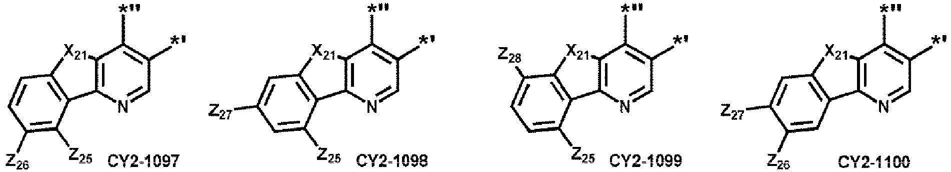 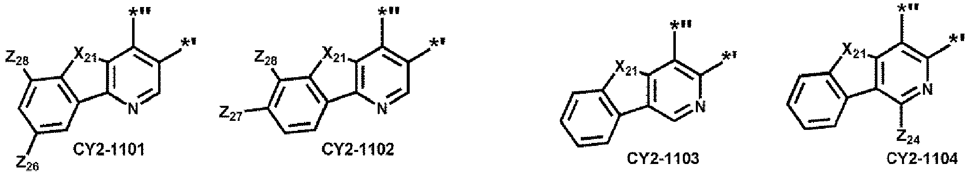 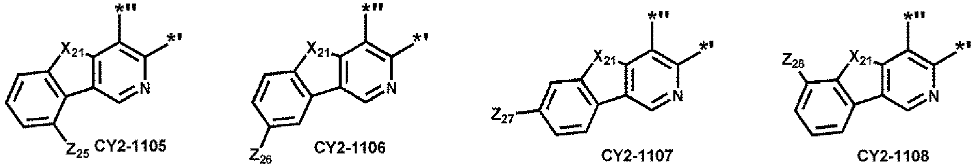 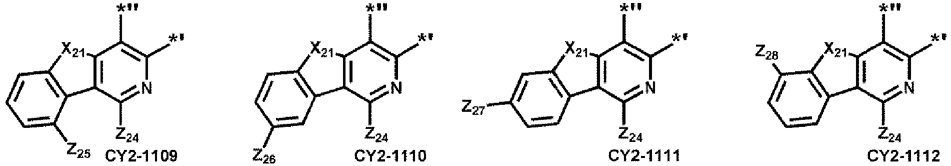 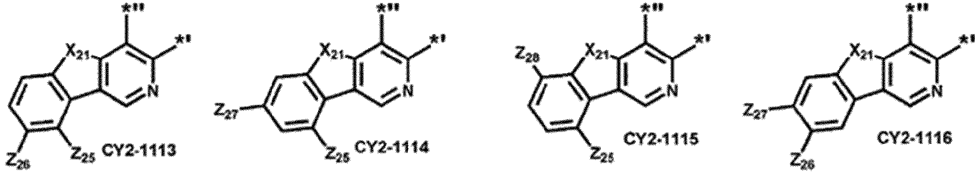 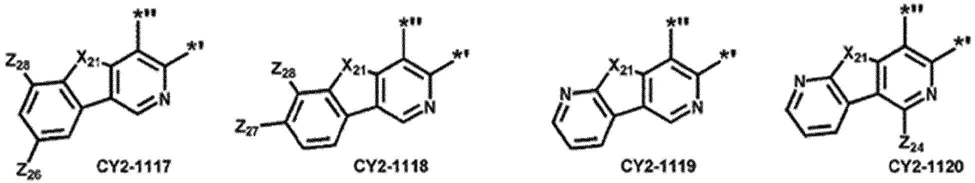 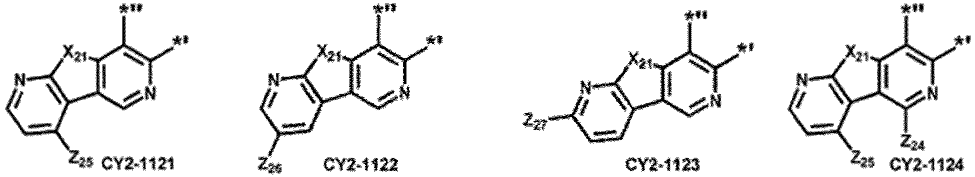 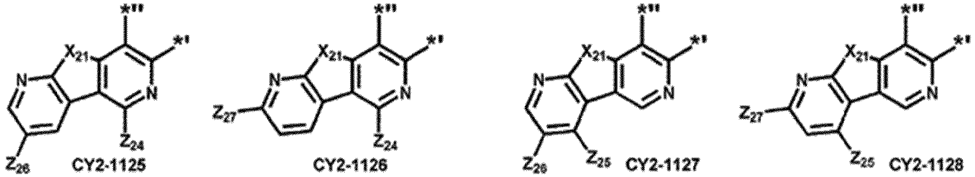 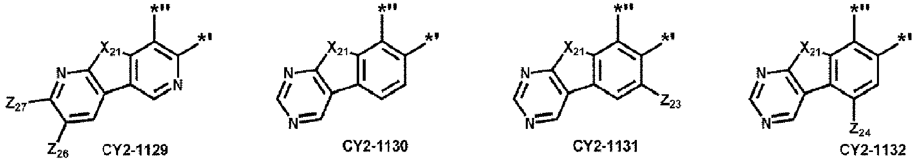 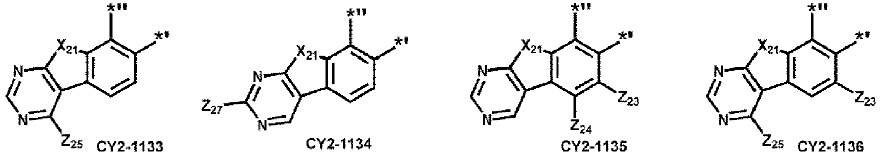 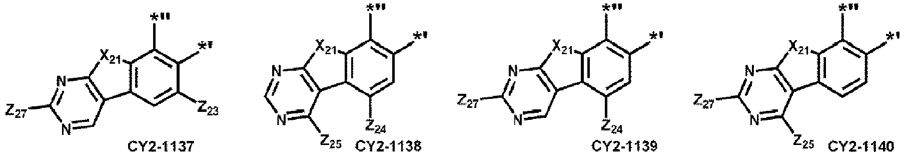 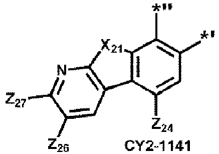 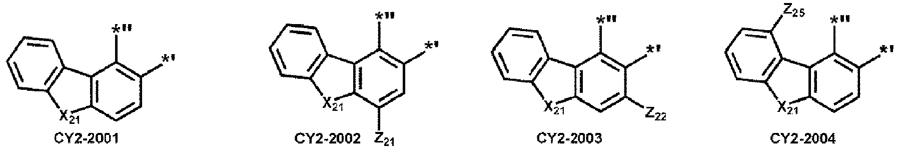 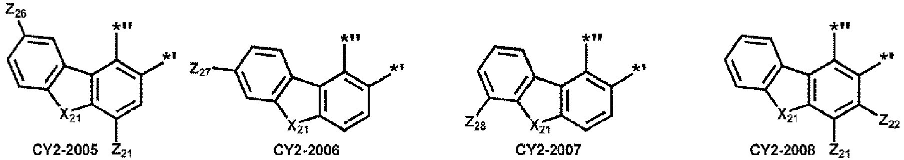 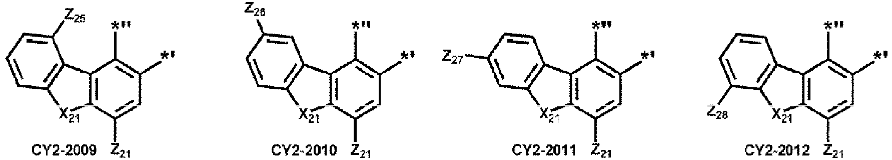 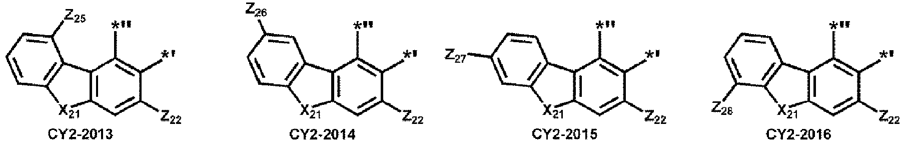 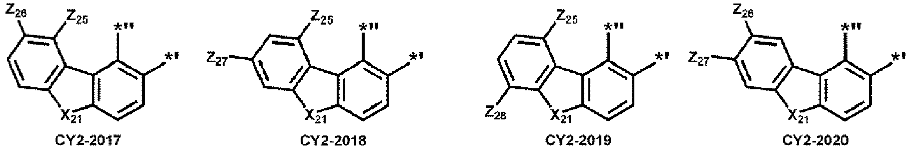 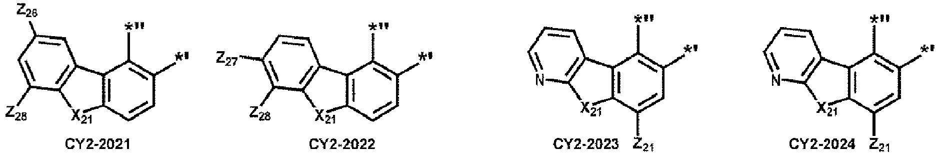 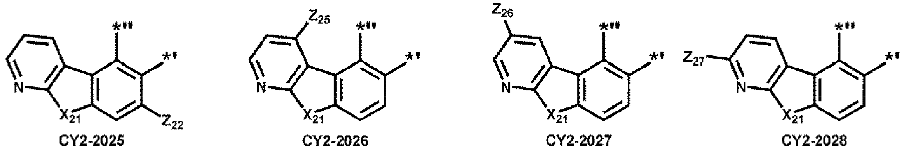 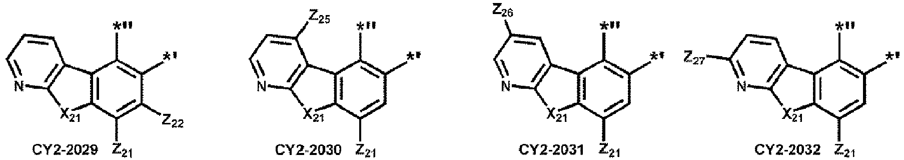 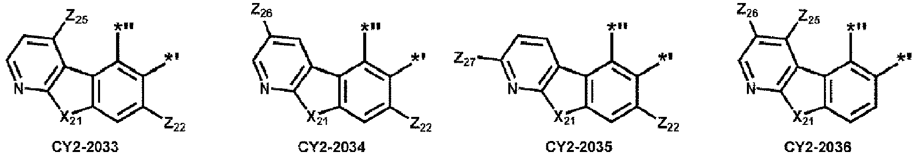 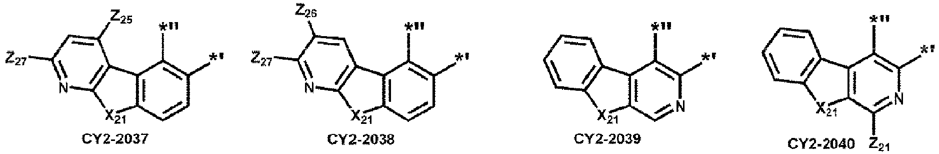 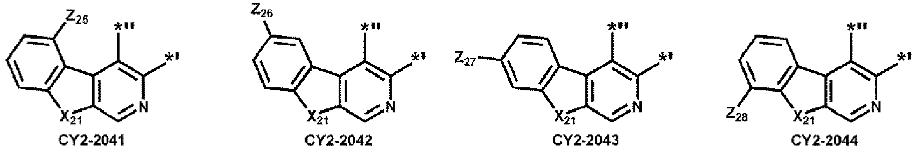 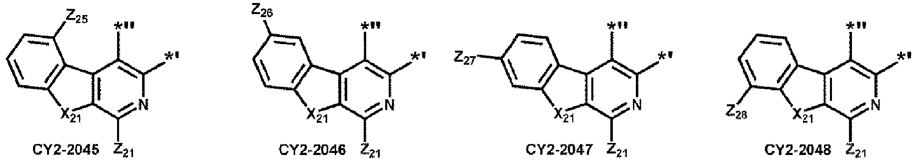 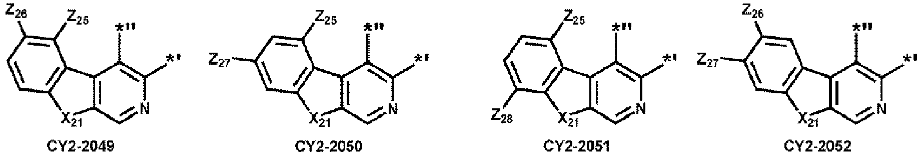 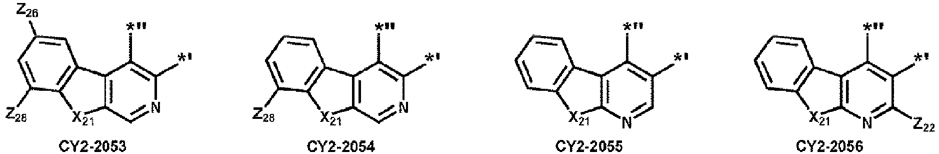 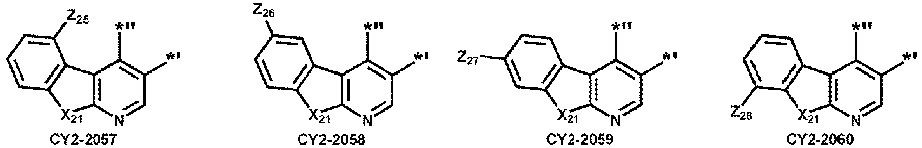 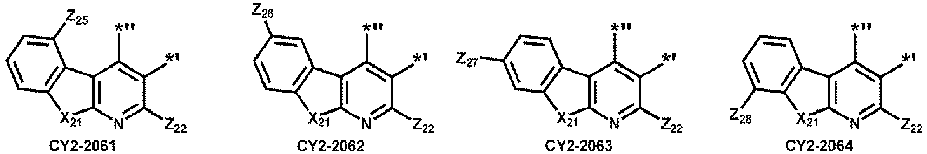 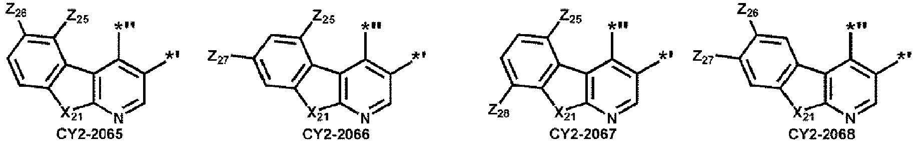 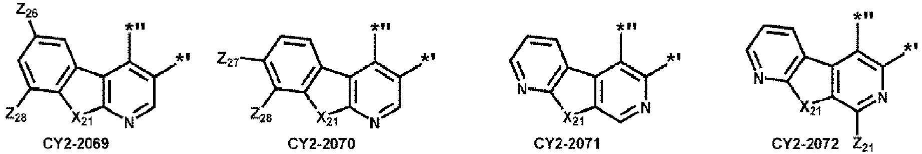 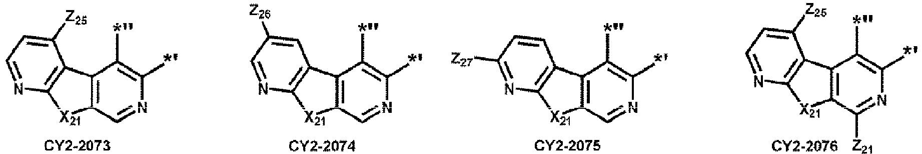 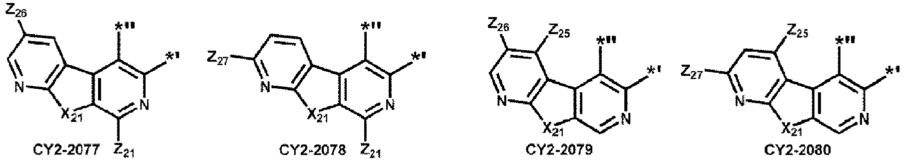 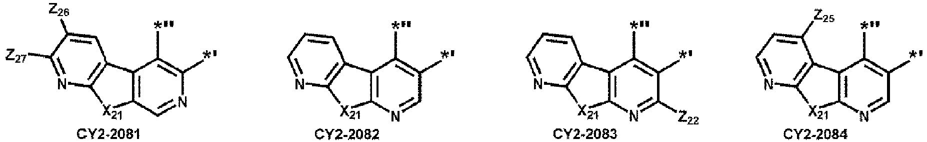 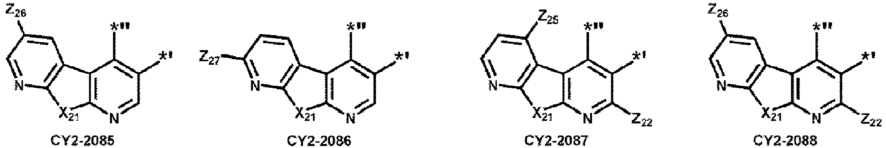 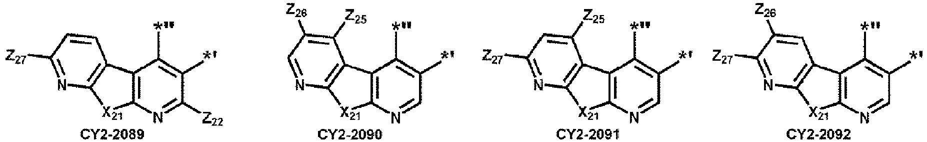 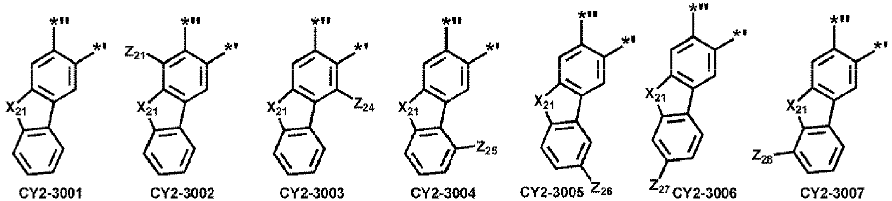 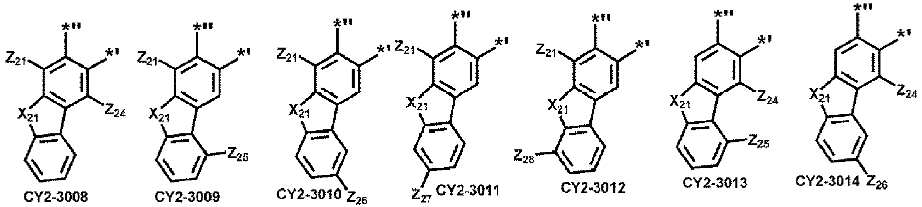 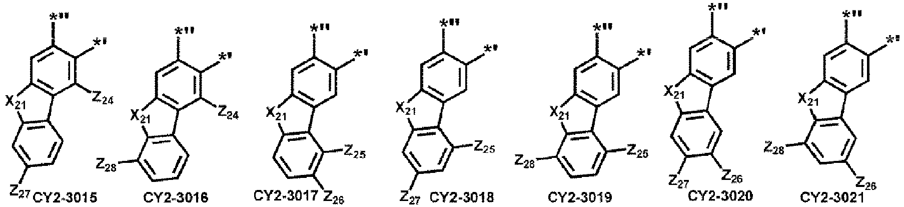 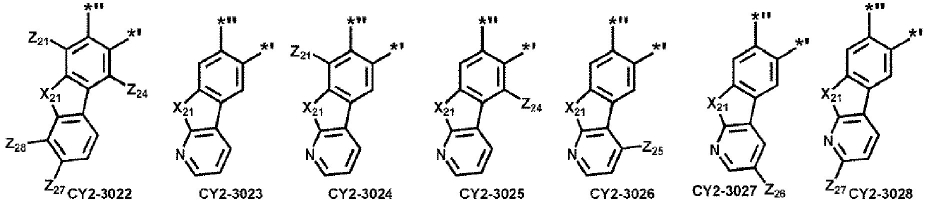 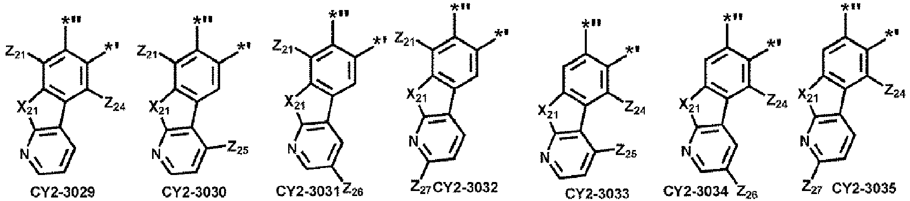 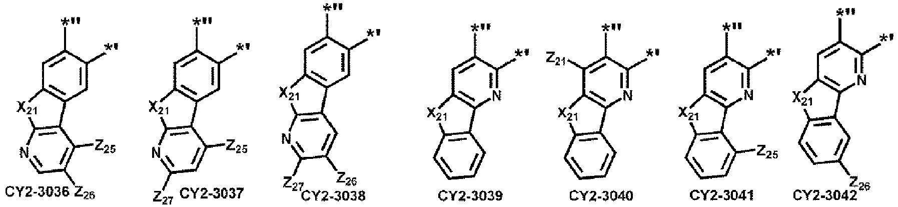 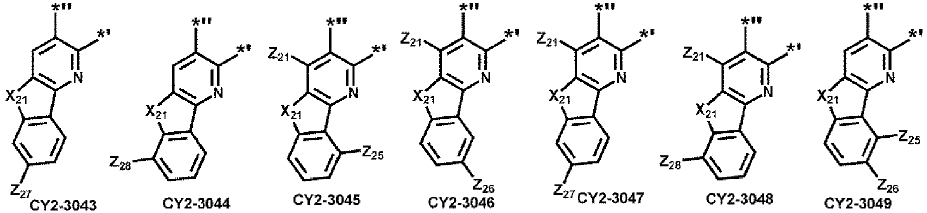 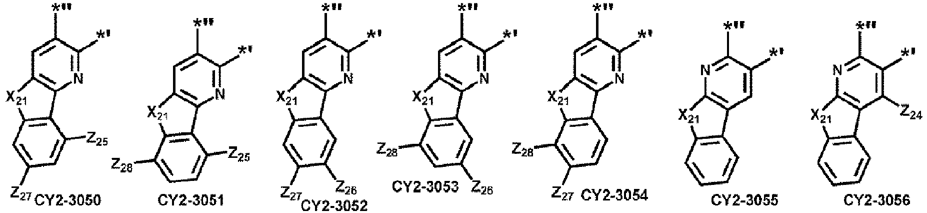 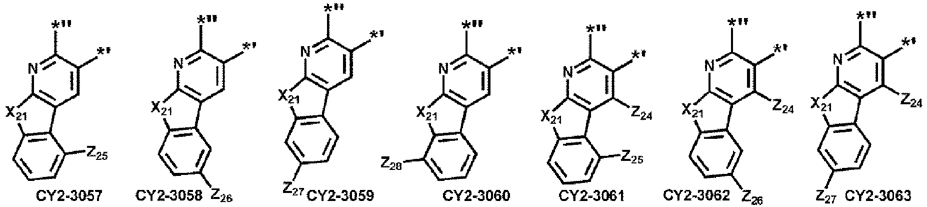 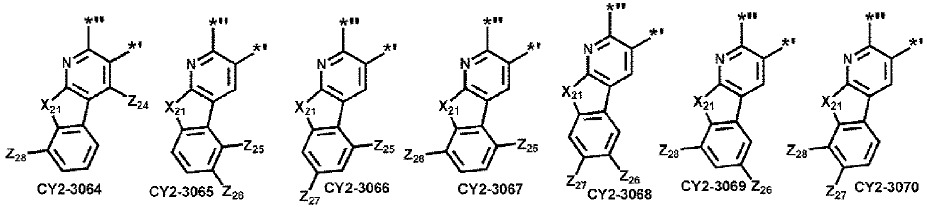 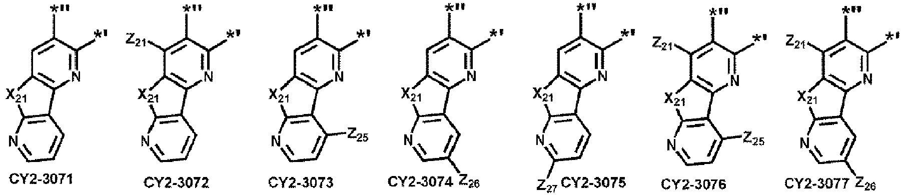 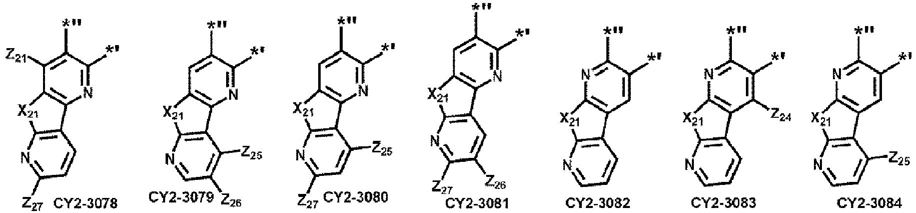 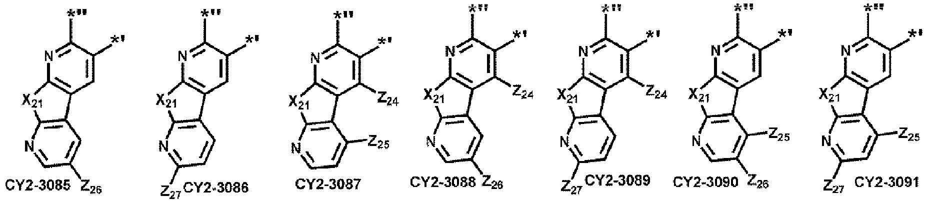 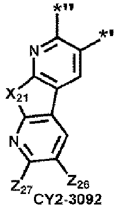 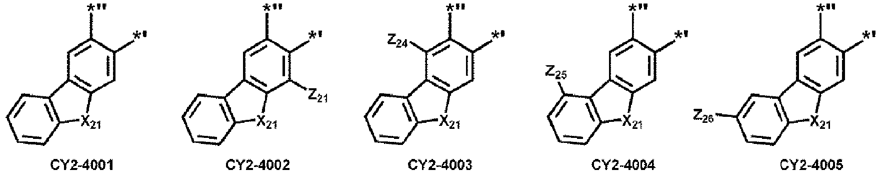 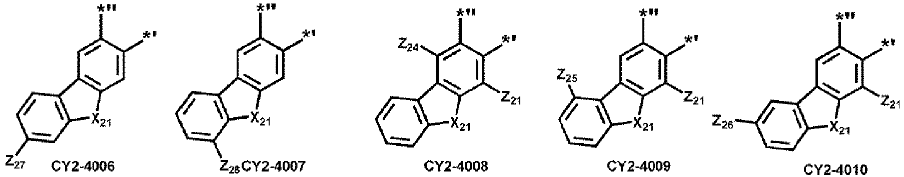 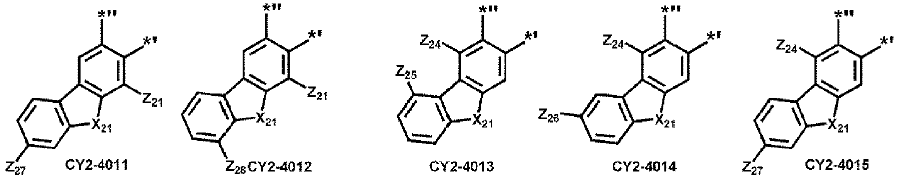 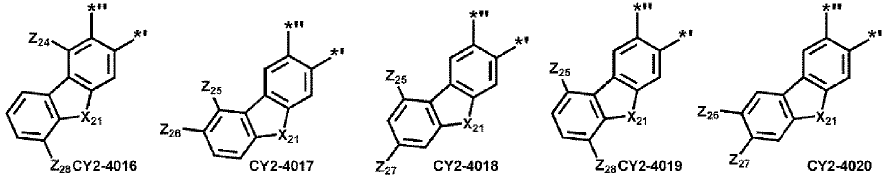 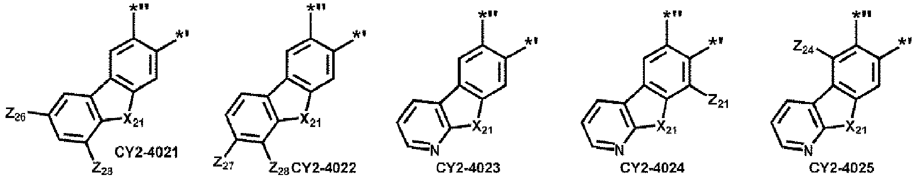 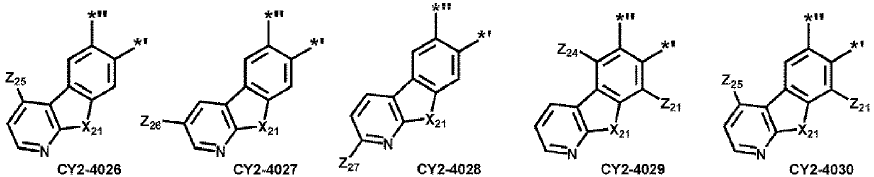 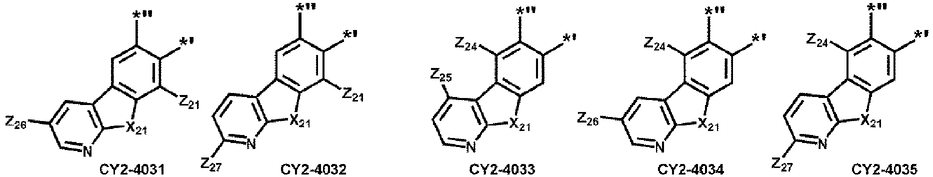 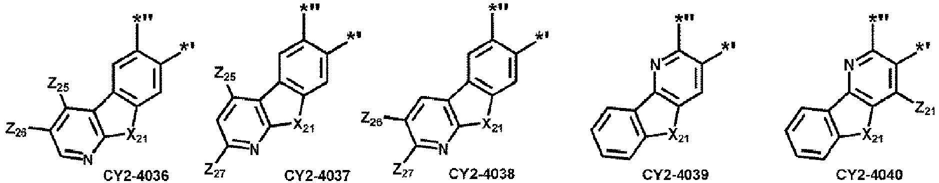 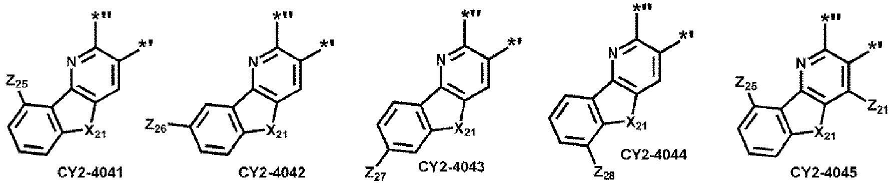 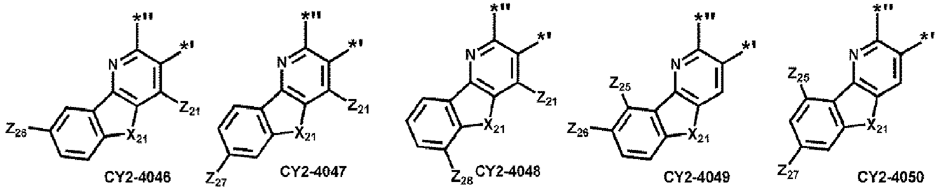 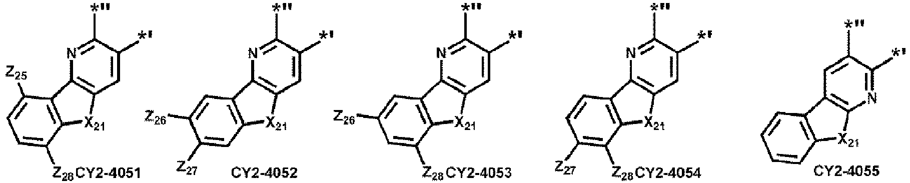 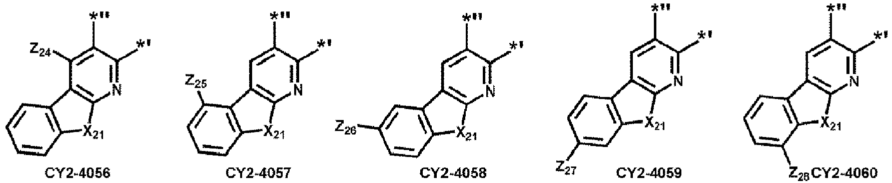 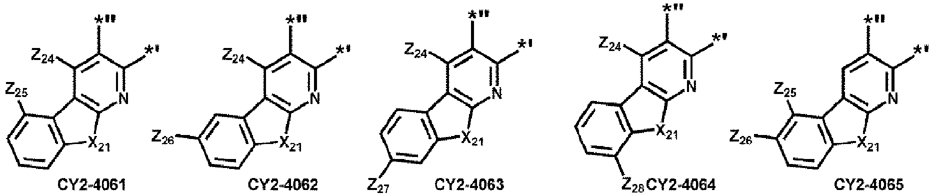 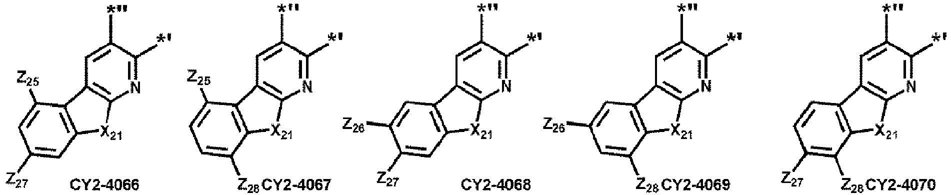 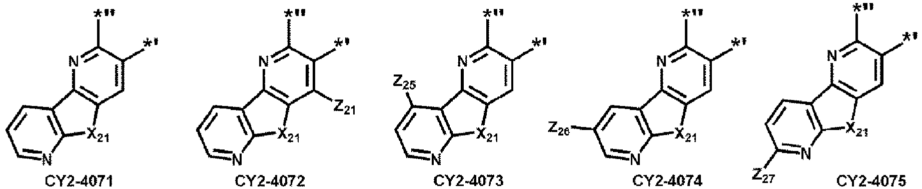 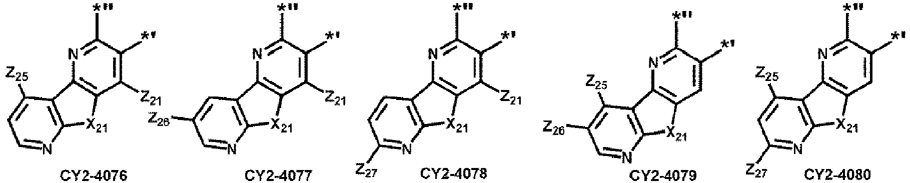 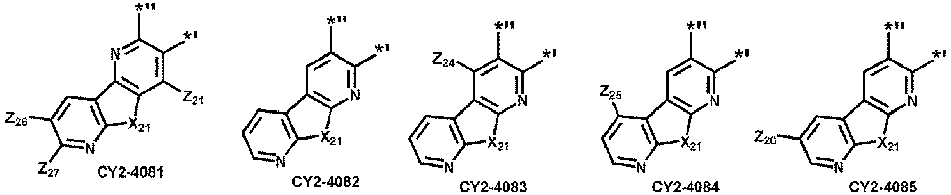 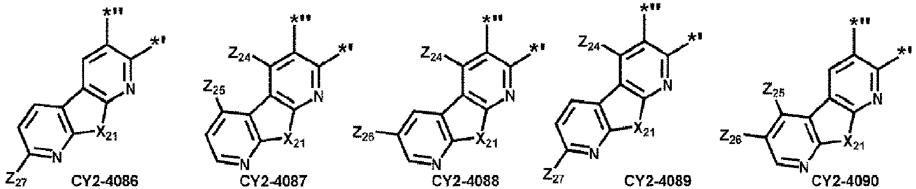 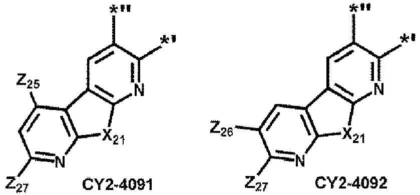 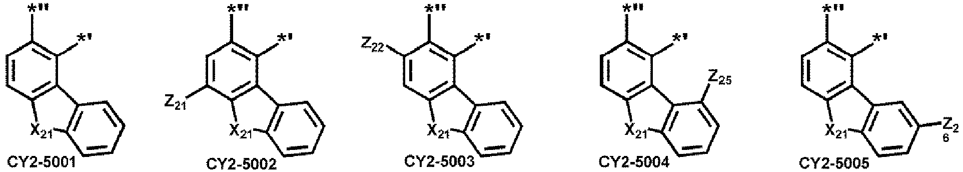 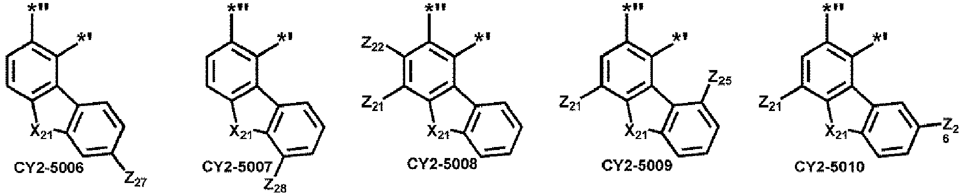 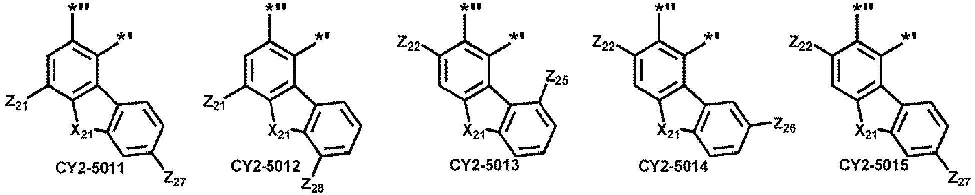 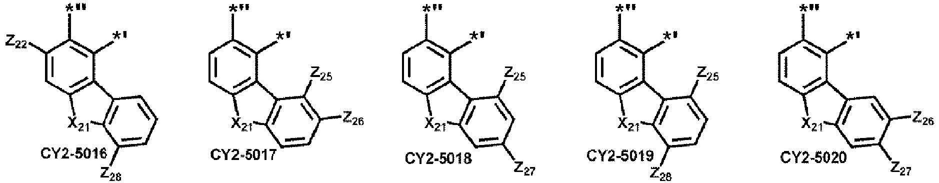 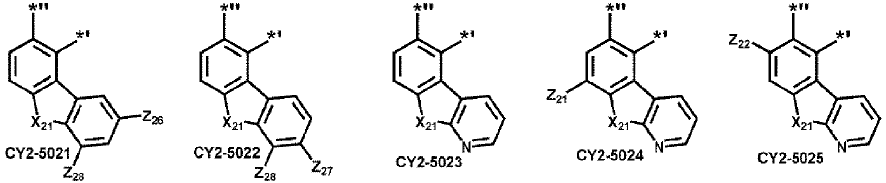 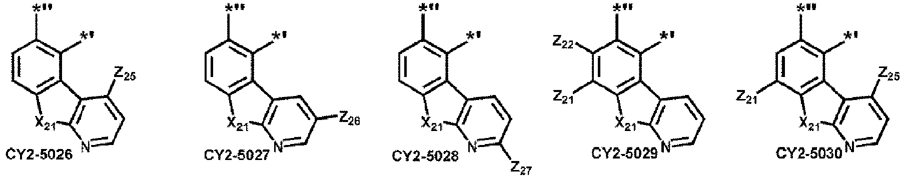 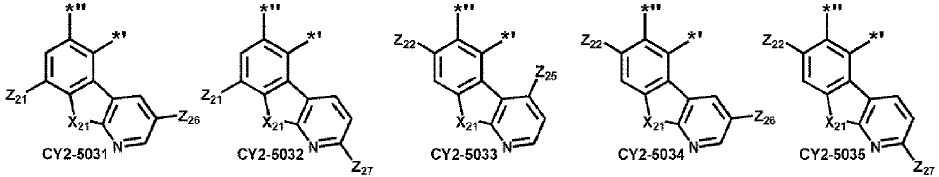 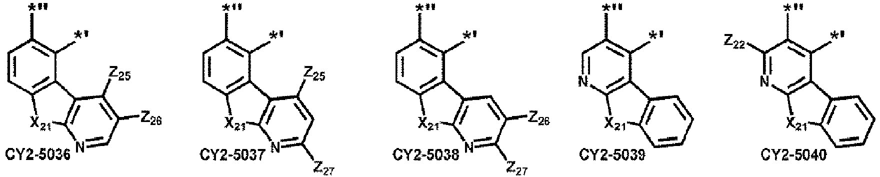 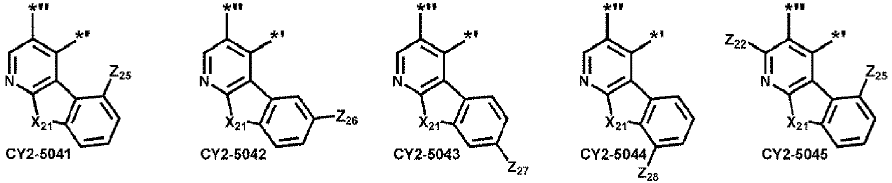 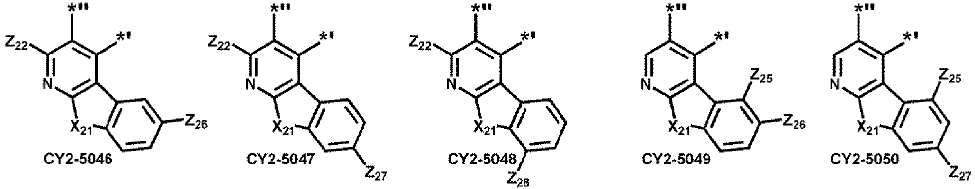 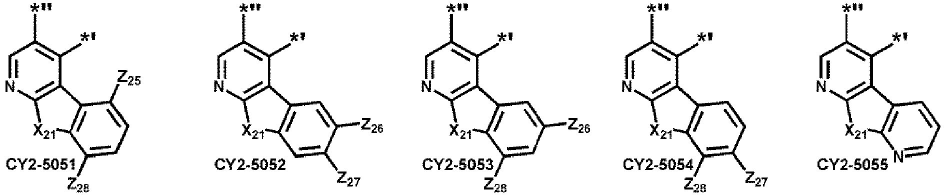 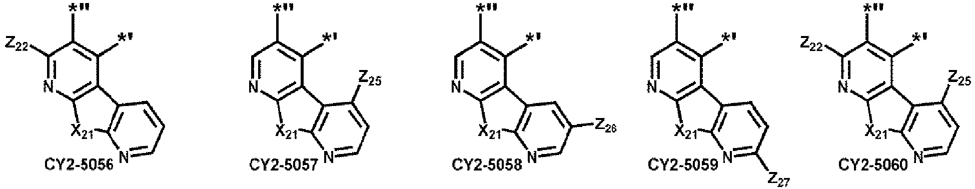 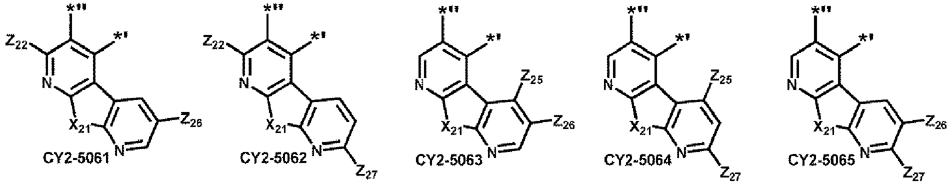 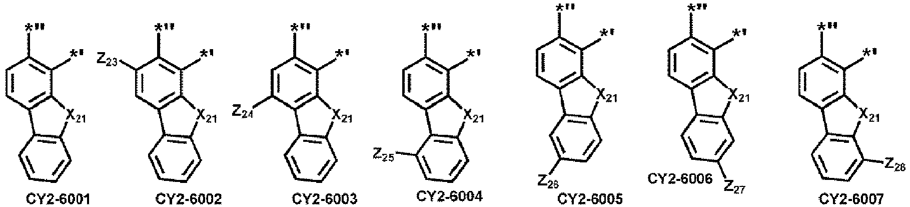 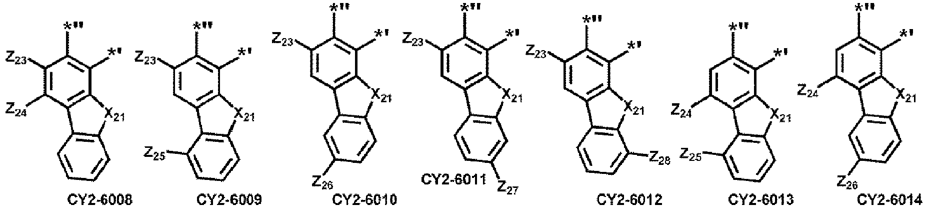 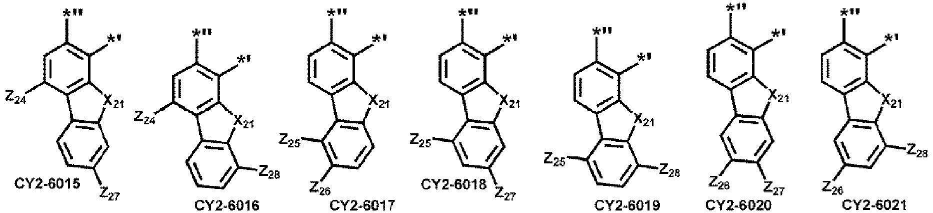 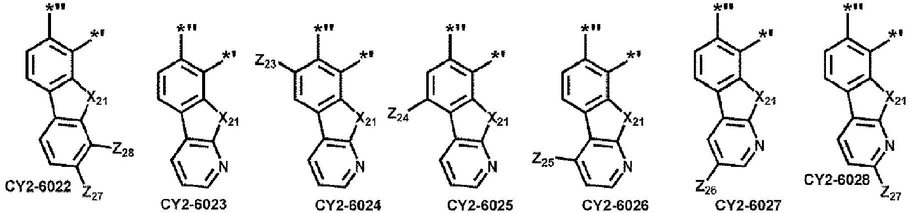 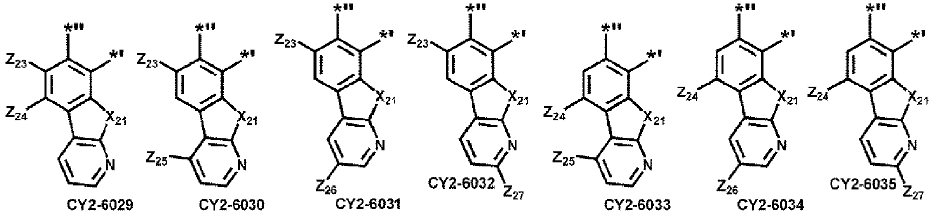 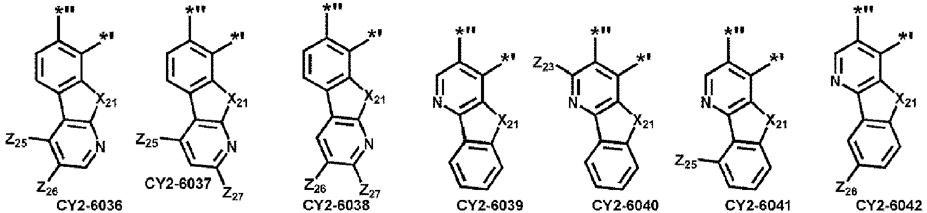 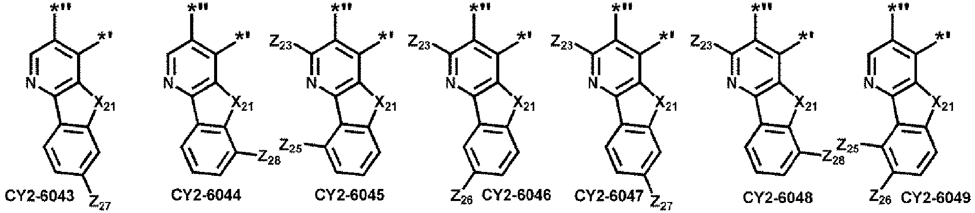 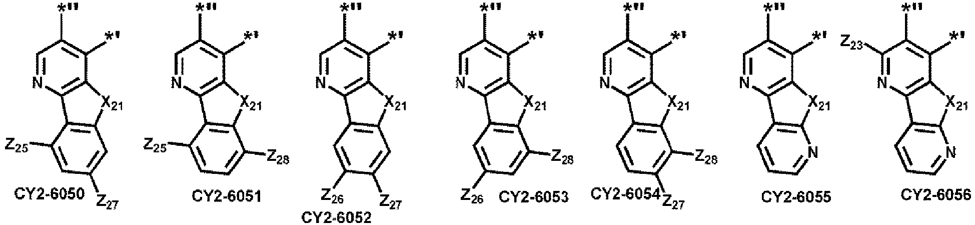 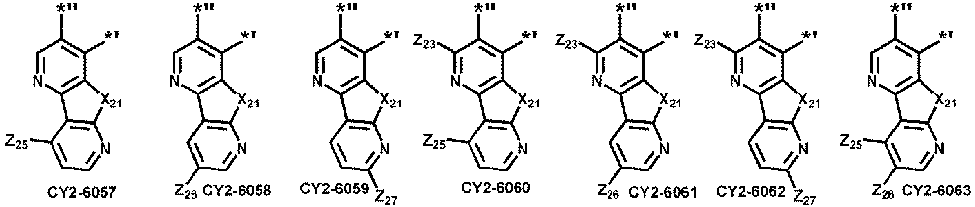 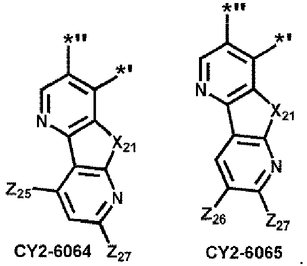 wobei in Formeln CY2-1001 bis CY2-1141, CY2-2001 bis CY2-2092, CY2-3001 bis CY2-3092, CY2-4001 bis CY2-4092, CY2-5001 bis CY2-5065 und CY2-6001 bis CY2-6065, X₂₁ gleich ist wie beschrieben in Anspruch 1,
Z₂₁ bis Z₂₈ jeweils unabhängig gleich sind wie definiert in Verbindung mit Z₂ in Anspruch 1, wobei jedes von Z₂₁ bis Z₂₈ nicht Wasserstoff ist,
^{∗}" eine Bindungsstelle an Ring CY₁ in Formel 2A angibt, und ^{∗}' eine Bindungsstelle an M in Formel 1 angibt.

12. Metallorganische Verbindung nach einem der Ansprüche 1-11, wobei eine Gruppe, dargestellt durch 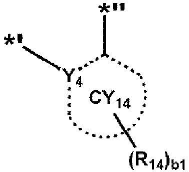 in Formel 2B, durch eine von Formeln CY14(1) bis CY14(63) dargestellt ist: 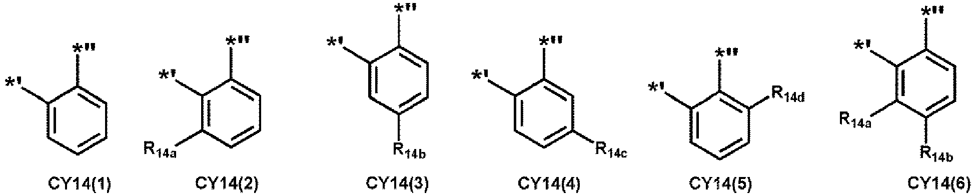 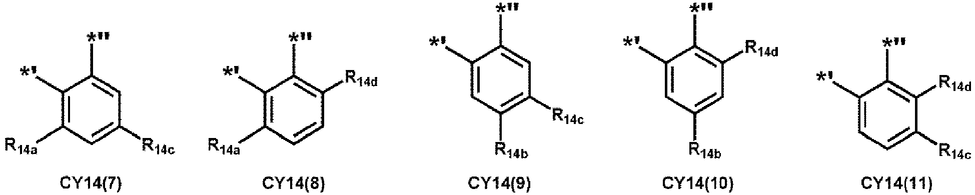 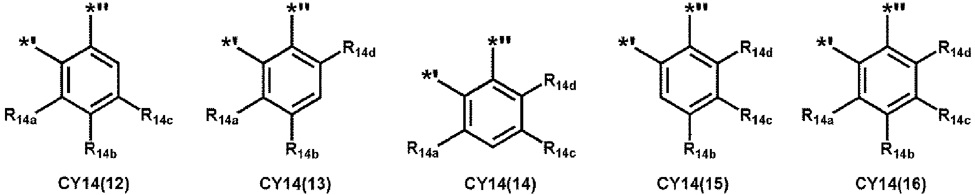 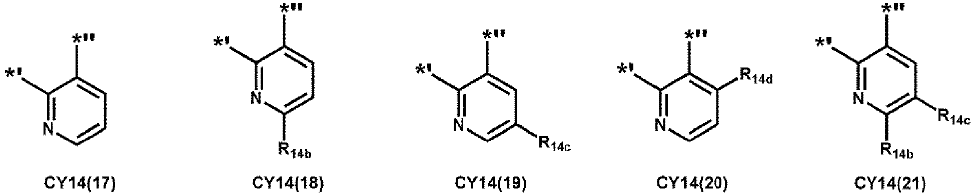 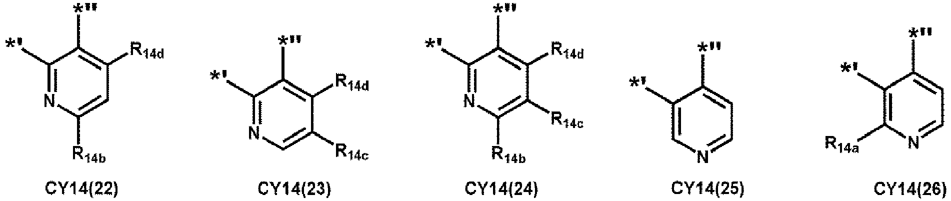 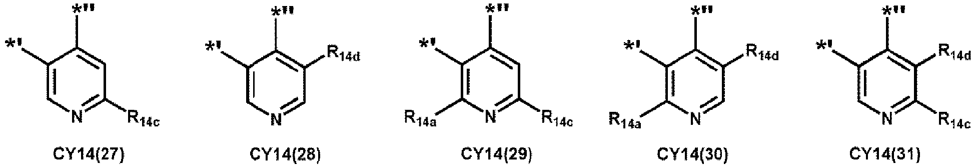 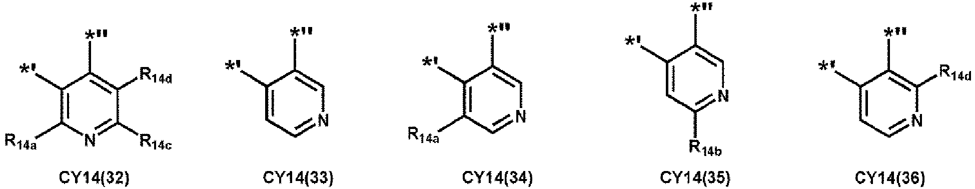 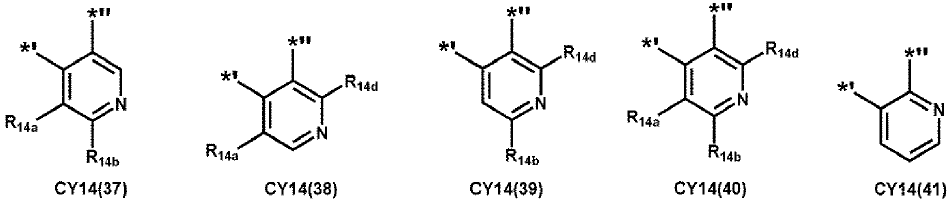 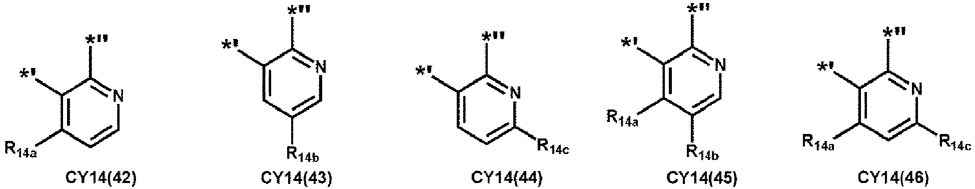 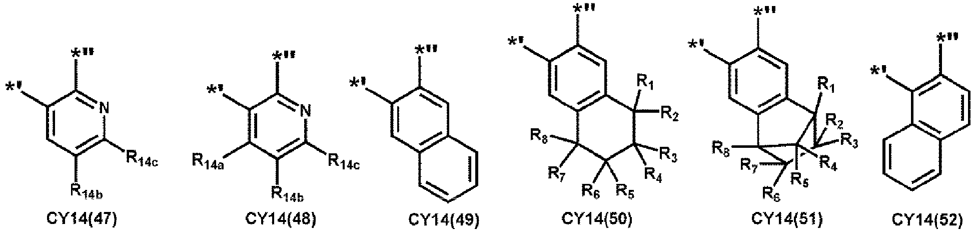 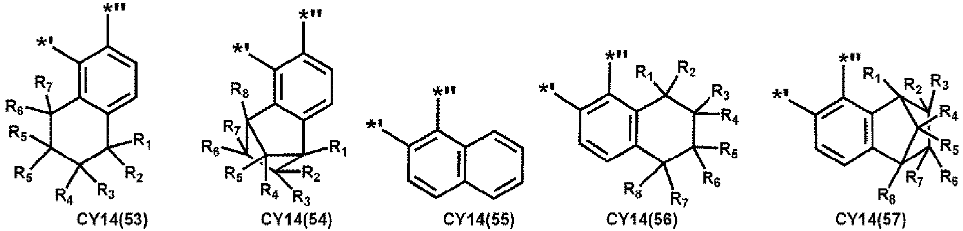 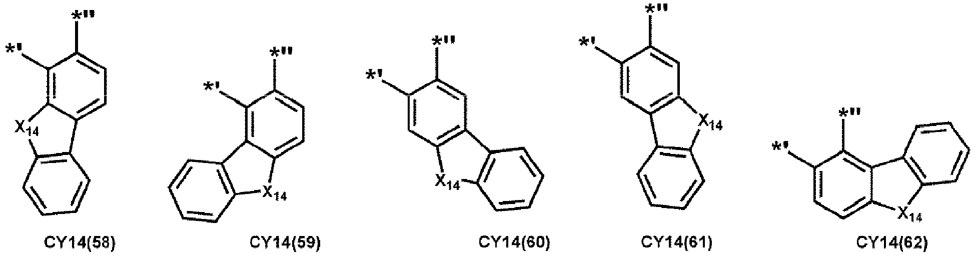 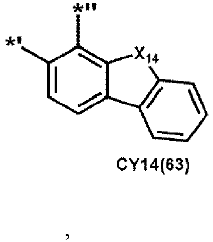 wobei in Formeln CY14(1) bis CY14(63),
R₁₄ₐ bis R_{14d} jeweils unabhängig gleich sind wie definiert in Verbindung mit R₁₄ in Anspruch 1, wobei jedes von R₁₄ₐ bis R_{14d} nicht Wasserstoff ist,
X₁₄ C(R₁)(R₂), N(R₁), O, S oder Si(R₁)(R₂) ist,
R₁ bis R₈ sind gleich sind wie definiert in Verbindung mit R₁₄ in Anspruch 1,
^{∗}" eine Bindungsstelle an ein Kohlenstoffatom eines benachbarten Pyridinrings in Formel 2B angibt, und
^{∗} eine Bindungsstelle an M in Formel 1 angibt.

13. Organische lichtemittierende Vorrichtung, umfassend: eine erste Elektrode,
eine zweite Elektrode; und
eine organische Schicht, die zwischen der ersten Elektrode und der zweiten Elektrode angeordnet ist und umfassend eine Emissionsschicht,
wobei die organische Schicht mindestens eine der metallorganischen Verbindung nach einem der Ansprüche 1-12 umfasst; vorzugsweise wobei
die erste Elektrode eine Anode ist,
die zweite Elektrode eine Kathode ist, und
die organische Schicht ferner einen Lochtransportbereich, angeordnet zwischen der ersten Elektrode und der Emissionsschicht, und einen Elektronentransportbereich, angeordnet zwischen der Emissionsschicht und der zweiten Elektrode, umfasst,
wobei der Lochtransportbereich eine Lochinjektionsschicht, eine Lochtransportschicht, eine Elektronensperrschicht, eine Pufferschicht oder eine beliebige Kombination davon umfasst, und
der Elektronentransportbereich eine Lochsperrschicht, eine Elektronentransportschicht, eine Elektroneninjektionsschicht oder eine beliebige Kombination davon umfasst.

14. Organische lichtemittierende Vorrichtung nach Anspruch 13, wobei
die Emissionsschicht die mindestens eine der metallorganischen Verbindung umfasst;
vorzugsweise wobei
die Emissionsschicht ferner einen Wirt umfasst, und eine Menge des Wirts größer ist als die der metallorganischen Verbindung.

15. Elektronische Vorrichtung, umfassend die organische lichtemittierende Vorrichtung nach Anspruch 13 oder 14.

## Revendications

1. Composé organométallique représenté par la Formule 1 :
<Formule 1> M(L₁)ₙ₁(L₂)ₙ₂
où, dans la Formule 1,
M est un métal de transition,
L₁ est un ligand représenté par la Formule 2A,
L₂ est un ligand représenté par la Formule 2B,
n1 et n2 sont chacun indépendamment 1 ou 2, où, quand n1 est 2, deux L₁ sont identiques ou différents l'un de l'autre et quand n2 est 2, deux L₂ sont identiques ou différents l'un de l'autre,
la somme de n1 et n2 est 2 ou 3, et
L₁ et L₂ sont différents l'un de l'autre : 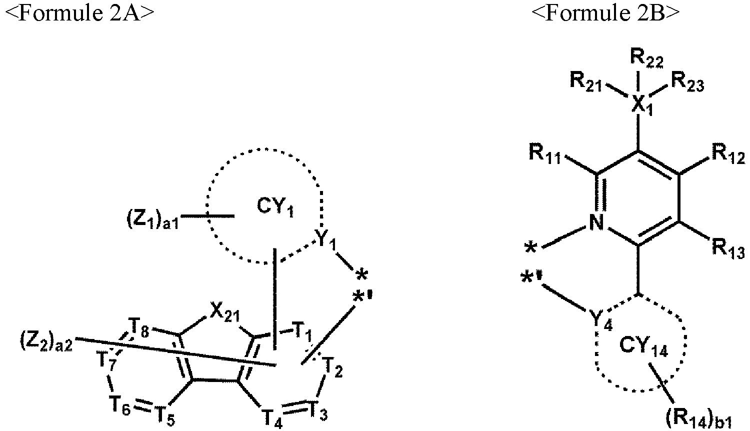 où, dans les Formules 2A et 2B,
Y₁ et Y₄ sont chacun indépendamment C ou N,
X₁ est Si ou Ge,
**X₂₁** est O, S, S(=O), N(Z₂₉), C(Z₂₉)(Z₃₀), ou Si(Z₂₉)(Z₃₀),
T₁ à T₄ sont chacun indépendamment C, N, à liaison carbone au cycle CYi, ou à liaison carbone à M dans la Formule 1, où l'un de T₁ à T₄ a une liaison carbone à M dans la Formule 1, et l'un des T₁ à T₄ restants non liés à M dans la Formule 1 a une liaison carbone au cycle CYi,
T₅ à T₈ sont chacun indépendamment C ou N,
où X₁ est Si, au moins l'un des T₁ à T₈ restants sans liaison carbone à M et au cycle CY₁ est N,
le cycle CY₁ et le cycle CY₁₄ sont chacun indépendamment un groupe carbocyclique en C₅-C₃₀ ou un groupe hétérocyclique en C₁- **C₃₀**,
R₂₁ à R₂₃ sont chacun indépendamment un groupe alkyle en C₁-C₆₀ ou un groupe aryle en C₆-C₆₀, chacun étant non substitué ou substitué par deutérium, -F, -Cl, -Br, -I, -CD₃, -CD₂H, -CDH₂, -CF₃, - CF₂H, -CFH₂, un groupe hydroxyle, un groupe cyano, un groupe nitro, un groupe amino, un groupe amidino, un groupe hydrazine, un groupe hydrazone, un groupe acide carboxylique ou un sel de celui, un groupe acide sulfonique ou un sel de celui-ci, un groupe acide phosphorique ou un sel de celui-ci, un groupe alkyle en C₁-C₆₀, un groupe cycloalkyle en C₃-C₁₀, un groupe phényle ou toute combinaison de ce qui précède,
Z₁, Z₂, et R₁₁ à R₁₄ sont chacun indépendamment hydrogène, deutérium, -F, -Cl, -Br, -I, - SF₅, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁-C₆₀ substitué ou non substitué, un groupe alkényle en C₂-C₆₀ substitué ou non substitué, un groupe alkynyle en C₂-C₆₀ substitué ou non substitué, un groupe alkoxy en C₁-C₆₀ substitué ou non substitué, un groupe alkylthio en C₁-C₆₀ substitué ou non substitué, un groupe cycloalkyle en C₃-C₁₀ substitué ou non substitué, un groupe hétérocycloalkyle en C₁-C₁₀ substitué ou non substitué, un groupe cycloalkényle en C₃-C₁₀ substitué ou non substitué, un groupe hétérocycloalkényle en C₁-C₁₀ substitué ou non substitué, un groupe aryle en C₆-C₆₀ substitué ou non substitué, un groupe aryloxy en C₆-C₆₀ substitué ou non substitué, un groupe arylthio en C₆-C₆₀ substitué ou non substitué, un groupe hétéroaryle en C₁-C₆₀ substitué ou non substitué, un groupe polycyclique condensé non aromatique monovalent substitué ou non substitué, un groupe hétéropolycyclique condensé non aromatique monovalent substitué ou non substitué, -N(Q₁)(Q₂), -Ge(Q₃)(Q₄)(Q₅), -B(Q₆)(Q₇), -P(=O)(Q₈)(Q₉), ou - P(Q₈)(Q₉), où R₁₂ n'est ni hydrogène ni un groupe méthyle,
a1 et b1 sont chacun indépendamment un nombre entier compris entre 0 et 20, où, quand a1 est 2 ou plus, au moins deux Z₁ sont identiques ou différents l'un de l'autre, et quand b1 est 2 ou plus, au moins deux R₁₄ sont identiques ou différents l'un de l'autre,
a2 est un nombre entier compris entre 0 et 6, où, quand a2 est 2 ou plus, au moins deux Z₂ sont identiques ou différents l'un de l'autre,
au moins deux éléments choisis parmi R₂₁ à R₂₃ sont éventuellement liés pour former un groupe carbocyclique en C₅-C₃₀ qui est non substitué ou substitué par au moins un R₁₀ₐ ou un groupe hétérocyclique en C₁-C₃₀ qui est non substitué ou substitué par au moins un R₁₀ₐ,
au moins deux éléments choisis parmi une pluralité de Z₁ sont éventuellement liés pour former un groupe carbocyclique en C₅-C₃₀ qui est non substitué ou substitué par au moins un R₁₀ₐ ou un groupe hétérocyclique en C₁-C₃₀ qui est non substitué ou substitué par au moins un R₁₀ₐ,
au moins deux éléments choisis parmi une pluralité de Z₂ sont éventuellement liés pour former un groupe carbocyclique en C₅-C₃₀ qui est non substitué ou substitué par au moins un R₁₀ₐ ou un groupe hétérocyclique en C₁-C₃₀ qui est non substitué ou substitué par au moins un R₁₀ₐ,
R₁₂ et R₁₃ sont éventuellement liés pour former un groupe carbocyclique en C₅-C₃₀ qui est non substitué ou substitué par au moins un R₁₀ₐ ou un groupe hétérocyclique en C₁-C₃₀ qui est non substitué ou substitué par au moins un R₁₀ₐ,
au moins deux éléments choisis parmi une pluralité de R₁₄ sont éventuellement liés pour former un groupe carbocyclique en C₅-C₃₀ qui est non substitué ou substitué par au moins un R₁₀ₐ ou un groupe hétérocyclique en C₁-C₃₀ qui est non substitué ou substitué par au moins un R₁₀ₐ,
au moins deux éléments choisis parmi Z₁, Z₂ et R₁₁ à R₁₄ sont éventuellement liés pour former un groupe carbocyclique en C₅-C₃₀ qui est non substitué ou substitué par au moins un R₁₀ₐ ou un groupe hétérocyclique en C₁-C₃₀ qui est non substitué ou substitué par au moins un R₁₀ₐ,
R₁₀ₐ est le même que défini en relation avec R₁₄,
^{∗} et ^{∗}' dans les Formules 2A et 2B indique chacun un site de liaison à M dans la Formule 1,
un ou plusieurs substituants du groupe alkyle en C₁-C₆₀ substitué, groupe alkényle en C₂-C₆₀ substitué, groupe alkynyle en C₂-C₆₀ substitué, groupe alkoxy en C₁-C₆₀ substitué, groupe alkylthio en C₁-C₆₀ substitué, groupe cycloalkyle en C₃-C₁₀ substitué, groupe hétérocycloalkyle en C₁-C₁₀ substitué, groupe cycloalkényle en C₃-C₁₀ substitué, groupe hétérocycloalkényle en C₁-C₁₀ substitué, groupe aryle en C₆-C₆₀ substitué, groupe aryloxy en C₆-C₆₀ substitué, groupe arylthio en C₆-C₆₀ substitué, groupe hétéroaryle en C₁-C₆₀ substitué, groupe polycyclique condensé non aromatique monovalent substitué, et groupe hétéropolycyclique condensé non aromatique monovalent substitué est(sont) :
deutérium, -F, -Cl, -Br, -I, -CD₃, -CD₂H, -CDH₂, -CF₃, -CF₂H, -CFH₂, groupe hydroxyle, groupe cyano, groupe nitro, groupe amino, groupe amidino, groupe hydrazine, groupe hydrazone, groupe acide carboxylique ou un sel de celui-ci, groupe acide sulfonique ou un sel de celui-ci, groupe acide phosphorique ou un sel de celui-ci, groupe alkyle en C₁-C₆₀, groupe alkényle en C₂-C₆₀, groupe alkynyle en C₂-C₆₀, ou groupe alkoxy en C₁-C₆₀ ;
groupe alkyle en C₁-C₆₀, groupe alkényle en C₂-C₆₀, groupe alkynyle en C₂-C₆₀, ou groupe alkoxy en C₁-C₆₀, chacun substitué par deutérium, -F, -Cl, -Br, -I, -CD₃, -CD₂H, -CDH₂, -CF₃, -CF₂H, -CFH₂, groupe hydroxyle, groupe cyano, groupe nitro, groupe amino, groupe amidino, groupe hydrazine, groupe hydrazone, groupe acide carboxylique ou un sel de celui-ci, groupe acide sulfonique ou un sel de celui-ci, groupe acide phosphorique ou un sel de celui-ci, groupe cycloalkyle en C₃-C₁₀,groupe hétérocycloalkyle en C₁-C₁₀, groupe cycloalkényle en C₃-C₁₀, groupe hétérocycloalkényle en C₁-C₁₀, groupe aryle en C₆-C₆₀, groupe aryloxy en C₆-C₆₀, groupe arylthio en C₆-C₆₀, groupe hétéroaryle en C₁-C₆₀, groupe polycyclique condensé non aromatique monovalent, groupe hétéropolycyclique condensé non aromatique monovalent, -N(Q₁₁)(Q₁₂), -Ge(Q₁₃)(Q₁₄)(Q₁₅), -B(Q₁₆)(Q₁₇), -P(=O)(Q₁₈)(Q₁₉), -P(Q₁₈)(Q₁₉), ou toute combinaison de ce qui précède ;
groupe cycloalkyle en C₃-C₁₀,groupe hétérocycloalkyle en C₁-C₁₀, groupe cycloalkényle en C₃-C₁₀,groupe hétérocycloalkényle en C₁-C₁₀, groupe aryle en C₆-C₆₀, groupe aryloxy en C₆-C₆₀, groupe arylthio en C₆-C₆₀, groupe hétéroaryle en C₁-C₆₀, groupe polycyclique condensé non aromatique monovalent, ou groupe hétéropolycyclique condensé non aromatique monovalent, chacun non substitué ou substitué par deutérium, -F, -Cl, -Br, -I, -CD₃, -CD₂H, -CDH₂, -CF₃, -CF₂H, -CFH₂, groupe hydroxyle, groupe cyano, groupe nitro, groupe amino, groupe amidino, groupe hydrazine, groupe hydrazone, groupe acide carboxylique ou un sel de celui-ci, groupe acide sulfonique ou un sel de celui-ci, groupe acide phosphorique ou un sel de celui-ci, groupe alkyle en C₁-C₆₀, groupe alkényle en C₂-C₆₀, groupe alkynyle en C₂-C₆₀, groupe alkoxy en C₁-C₆₀, groupe cycloalkyle en C₃- C₁₀, groupe hétérocycloalkyle en C₁-C₁₀, groupe cycloalkényle en C₃-C₁₀,groupe hétérocycloalkényle en C₁- C₁₀, groupe aryle en C₆-C₆₀, groupe aryloxy en C₆-C₆₀, groupe arylthio en C₆-C₆₀, groupe hétéroaryle en C₁-C₆₀, groupe polycyclique condensé non aromatique monovalent, groupe hétéropolycyclique condensé non aromatique monovalent, -N(Q₂₁)(Q₂₂),-Ge(Q₂₃)(Q₂₄)(Q₂₅), -B(Q₂₆)(Q₂₇), -P(=O)(Q₂₈)(Q₂₉), -P(Q₂₈)(Q₂₉), ou toute combinaison de ce qui précède ;
-N(Q₃₁)(Q₃₂), -Ge(Q₃₃)(Q₃₄)(Q₃₅), -B(Q₃₆)(Q₃₇), -P(=O)(Q₃₈)(Q₃₉), ou -P(Q₃₈)(Q₃₉) ; ou toute combinaison de ce qui précède ;
Q₁ à Q₉, Q₁₁ à Q₁₉, Q₂₁ à Q₂₉, et Q₃₁ à Q₃₉ sont chacun indépendamment hydrogène ; deutérium ; -F; -Cl; -Br; -I; groupe hydroxyle ; groupe cyano ; groupe nitro ; groupe amino ; groupe amidino ; groupe hydrazine ; groupe hydrazone ; groupe acide carboxylique ou un sel de celui-ci ; groupe acide sulfonique ou un sel de celui-ci ; groupe acide phosphorique ou un sel de celui-ci ; groupe alkyle en C₁-C₆₀ non substitué ou substitué par deutérium, groupe alkyle en C₁-C₆₀, groupe aryle en C₆-C₆₀, ou toute combinaison de ce qui précède ; groupe alkényle en C₂-C₆₀ ; groupe alkynyle en C₂-C₆₀ ; groupe alkoxy en C₁-C₆₀ ; groupe cycloalkyle en C₃-C₁₀ ; groupe hétérocycloalkyle en C₁-C₁₀ ; groupe cycloalkényle en C₃-C₁₀ ; groupe hétérocycloalkényle en C₁-C₁₀ ; groupe aryle en C₆-C₆₀ non substitué ou substitué par deutérium, groupe alkyle en C₁-C₆₀, groupe aryle en C₆-C₆₀, ou toute combinaison de ce qui précède ; groupe aryloxy en C₆-C₆₀ ; groupe arylthio en C₆-C₆₀ ; groupe hétéroaryle en C₁-C₆₀, groupe polycyclique condensé non aromatique monovalent ; ou groupe hétéropolycyclique condensé non aromatique monovalent.

2. Composé organométallique selon la revendication 1, dans lequel
M dans la Formule 1 est Ir, et la somme de n1 et n2 est 3, ou
M dans la Formule 1 est Pt, et la somme de n1 et n2 est 2.

3. Composé organométallique selon la revendication 1 ou 2, dans lequel
X₂₁ dans la Formule 2A est O ou S ; et/ou
dans lequel
Z₁ dans la Formule 2A est :
hydrogène, deutérium, -F, ou groupe cyano ;
groupe alkyle en C₁-C₂₀ non substitué ou substitué par deutérium, -F, groupe cyano, groupe cycloalkyle en C₃-C₁₀,groupe cycloalkyle en C₃-C₁₀ deutéré, groupe cycloalkyle en (C₁-C₂₀ alkyl)C₃-C₁₀, groupe hétérocycloalkyle en C₁-C₁₀, groupe hétérocycloalkyle en C₁-C₁₀ deutéré, groupe hétérocycloalkyle en (C₁-C₂₀ alkyl)C₁-C₁₀, ou toute combinaison de ce qui précède ; ou
groupe cycloalkyle en C₃-C₁₀ ou groupe hétérocycloalkyle en C₁-C₁₀, non substitué ou substitué par deutérium, -F, groupe cyano, groupe alkyle en C₁-C₂₀, groupe alkyle en C₁-C₂₀ deutéré, ou toute combinaison de ce qui précède.

4. Composé organométallique selon l'une quelconque des revendications 1 à 3, dans lequel
le nombre d'atomes de carbone compris dans R₁₂ de la Formule 2B est au moins deux ; et/ou
dans lequel
le composé organométallique représenté par la Formule 1 satisfait au moins une condition de la <Condition (1)> à la <Condition (3)> ci-dessous :
<Condition (1)>
Dans la Formule 2A, Z₁ n'est pas hydrogène, et a1 est un nombre entier compris entre 1 et 20
<Condition (2)>
Dans la Formule 2B, R₁₄ n'est pas hydrogène, et b1 est un nombre entier compris entre 1 et 20
<Condition (3)>
Dans la Formule 2A, Z₂ n'est pas hydrogène, et a2 un nombre entier compris entre 1 et 6.

5. Composé organométallique selon l'une quelconque des revendications 1 à 4, dans lequel le composé organométallique représenté par la Formule 1 comprend au moins un atome de deutérium, au moins un groupe fluoro (-F), au moins un groupe cyano (-CN), ou toute combinaison de ce qui précède ; et/ou
dans lequel
Z₂ dans la Formule 2A n'est pas hydrogène,
a2 est un nombre entier de 1 à 3, et
un ou plusieurs Z₂ en nombre de a2 sont chacun indépendamment un groupe alkyle en C₁-C₆₀ substitué ou non substitué, groupe alkoxy en C₁-C₆₀ substitué ou non substitué, groupe alkythio en C₁-C₆₀ substitué ou non substitué, groupe cycloalkyle en C₃-C₁₀ substitué ou non substitué, groupe hétérocycloalkyle en C₁-C₁₀ substitué ou non substitué, groupe aryle en C₆-C₆₀ substitué ou non substitué, groupe hétéroaryle en C₁-C₆₀ substitué ou non substitué, groupe polycyclique condensé non aromatique monovalent substitué ou non substitué, ou groupe hétéropolycyclique condensé non aromatique monovalent substitué ou non substitué.

6. Composé organométallique selon l'une quelconque des revendications 1 à 5, dans lequel un groupe représenté par 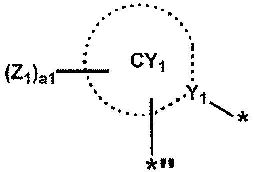 dans la Formule 2A est représenté par l'une des Formules CY1-1 à CY1-28 : 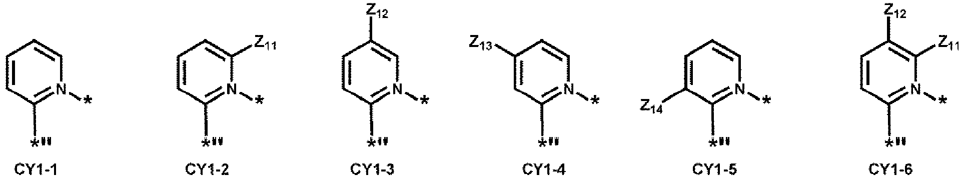 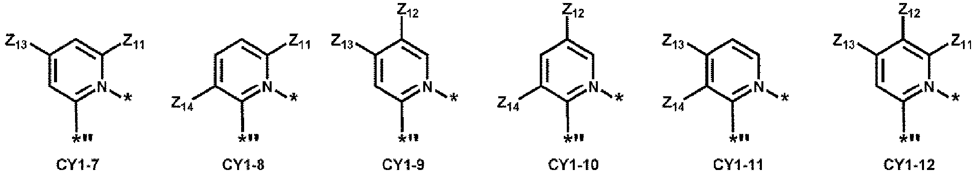 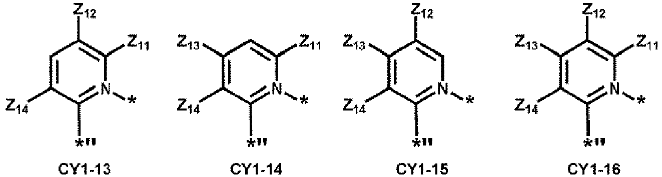 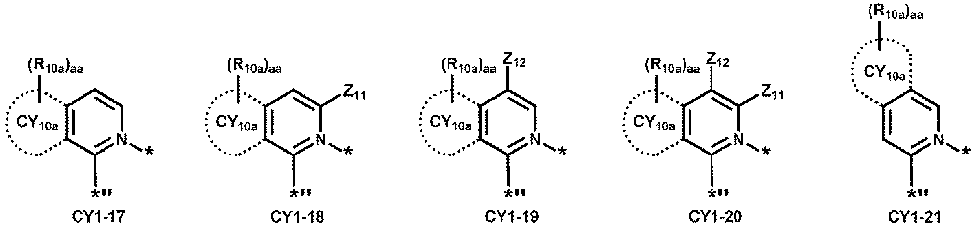 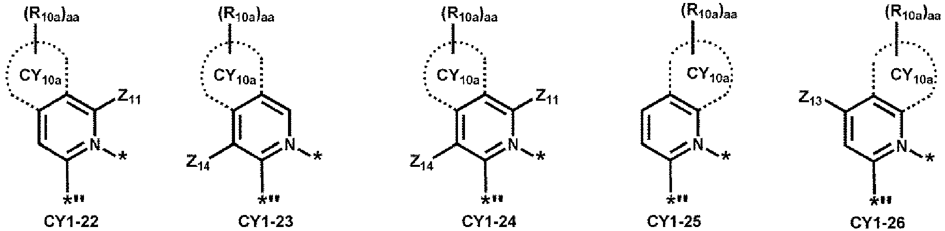 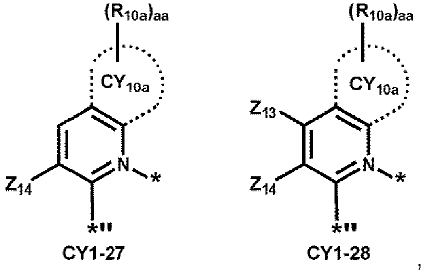 où, dans les Formules CY1-1 à CY1-28,
Z₁₁ à Z₁₄ sont chacun indépendamment les mêmes que définis en relation avec Z₁ dans la revendication 1, chaque Z₁₁ à Z₁₄ n'étant pas hydrogène,
le cycle CY₁₀ₐ est un groupe carbocyclique en C₅-C₃₀ ou un groupe hétérocyclique en C₁-C₃₀,
R₁₀ₐ est le même que décrit dans la revendication 1,
aa est un nombre entier compris entre 0 et 10, et
^{∗} indique un site de liaison à M dans la Formule 1, et ^{∗}" indique un site de liaison à l'un de T₁ à T₄ dans la Formule 2A.

7. Composé organométallique selon la revendication 6, dans lequel un groupe représenté par 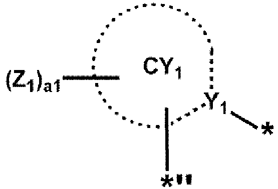 dans la Formule 2A est représenté par l'une des Formules CY1-4, CY1-7, CY1-9, CY1-11, CY1-12, et CY1-14 à CY1-24.

8. Composé organométallique selon l'une quelconque des revendications 1 à 7, dans lequel un groupe représenté par 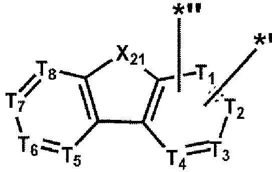 dans la Formule 2A est représenté par l'une des Formules CY2-1 à CY2-6 : 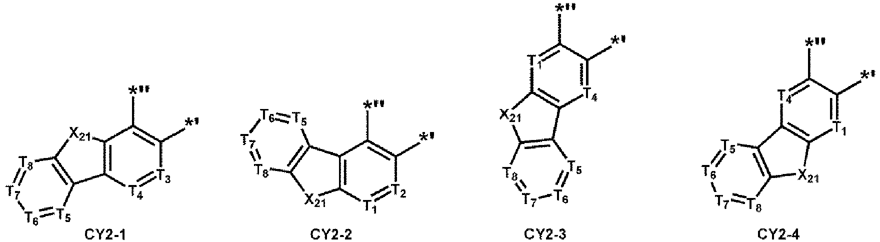 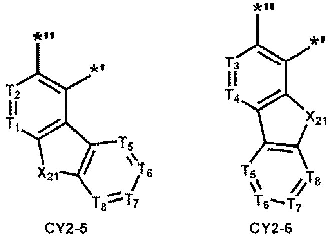 où, dans les Formules CY2-1 à CY2-6,
T₁ à T₈ sont chacun indépendamment C ou N,
X₂₁ est le même que décrit dans la revendication 1,
^{∗}" indique un site de liaison au cycle CY₁ dans la Formule 2A, et
^{∗}' indique un site de liaison à M dans la Formule 1.

9. Composé organométallique selon la revendication 8, dans lequel
a) X₁ dans la Formule 2B est Ge, et chacun de T₁ à T₈ dans les Formules CY2-1 à CY2-6 est C,
b) X₁ dans la Formule 2B est Si ou Ge, et au moins l'un de T₃ à T₈ dans les Formules CY2-1 et CY2-6 est N,
c) X₁ dans la Formule 2B est Si ou Ge, et au moins l'un de T₁, T₂, et T₅ à T₈ dans les Formules CY2-2 et CY2-5 est N,
d) X₁ dans la Formule 2B est Si ou Ge, et au moins l'un de T₁ et T₄ à T₈ dans les Formules CY2-3 et CY2-4 est N.

10. Composé organométallique selon la revendication 8 ou 9, dans lequel
1) T₁ à T₈ dans les Formules CY2-1 à CY2-6 sont C ;
2) l'un de T₃ à T₈ dans la Formule CY2-1 est N, et les T₃ à T₈ restants qui ne sont pas N dans la Formule CY2-1 sont C ;
3) T₃ et T₈ dans la Formule CY2-1 sont N, et T₄ à T₇ dans la Formule CY2-1 sont C ;
4) T₆ et T₈ dans la Formule CY2-1 sont N, et T₃ à T₅ et T₇ dans la Formule CY2-1 sont C ;
5) l'un de T₁, T₂ et T₈ dans la Formule CY2-2 est N, et les T₁, T₂ et T₅ à T₈ restants qui ne sont pas N dans la Formule CY2-2 sont C ;
6) T₁ et T₈ dans la Formule CY2-2 sont N, et T₂ et T₅ à T₇ dans la Formule CY2-2 sont C ;
7) T₂ et T₈ dans la Formule CY2-2 sont N, et T₁ et T₅ à T₇ dans la Formule CY2-2 sont C ;
8) l'un de T₁, T₄ et T₈ dans les Formules CY2-3 et CY2-4 est N, et les T₁, T₄ et T₅ à T₈ restants qui ne sont pas N dans les Formules CY2-3 et CY2-4 sont C ;
9) T₁ et T₈ dans les Formules CY2-3 et CY2-4 sont N, et T₄ et T₅ à T₇ dans les Formules CY2-3 et CY2-4 sont C ;
10) T₄ et T₈ dans les Formules CY2-3 et CY2-4 sont N, et T₁ et T₅ à T₇ dans les Formules CY2-3 et CY2-4 sont C ;
11) l'un de T₁ et T₈ dans la Formule CY2-5 est N, et les T₁, T₂ et T₅ à T₈ restants qui ne sont pas N dans la Formule CY2-5 sont C ;
12) T₁ et T₈ dans la Formule CY2-5 sont N, et T₂ et T₅ à T₇ dans la Formule CY2-5 sont C ;
13) l'un de T₄ à T₈ dans la Formule CY2-6 est N, et les T₃ à T₈ restants qui ne sont pas N dans la Formule CY2-6 sont C ; ou
14) T₄ et T₈ dans la Formule CY2-6 sont N, et T₃ et T₅ à T₇ dans la Formule CY2-6 sont C.

11. Composé organométallique selon l'une quelconque des revendications 1 à 10, dans lequel un groupe représenté par 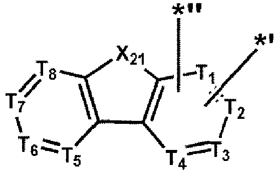 dans la Formule 2A est représenté par l'une des Formules CY2-1001 à CY2-1141, CY2-2001 à CY2-2092, CY2-3001 à CY2-3092, CY2-4001 à CY2-4092, CY2-5001 à CY2-5065 et CY2-6001 à CY2-6065 : 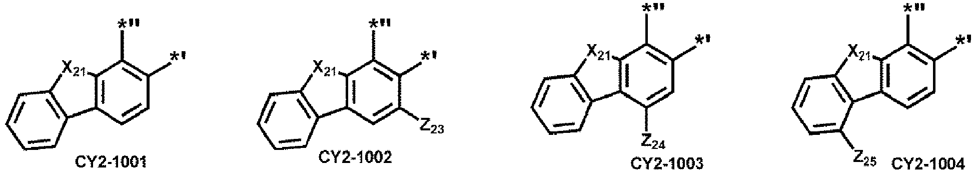 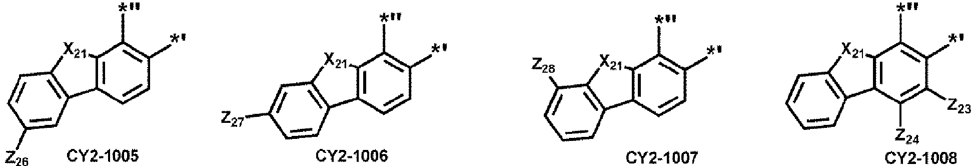 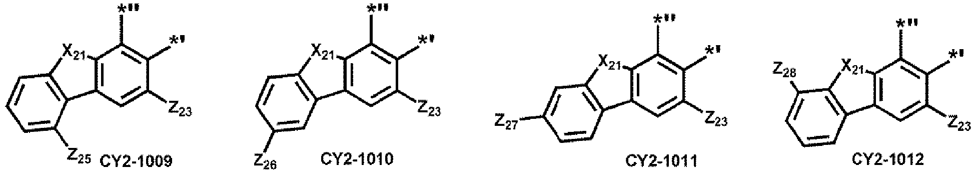 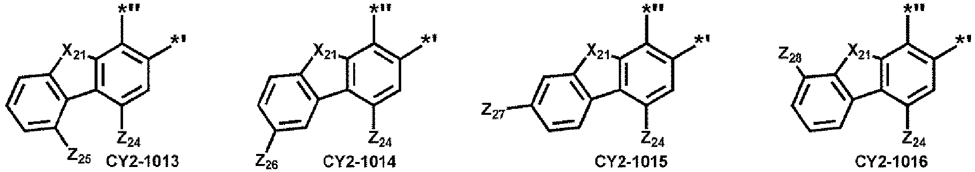 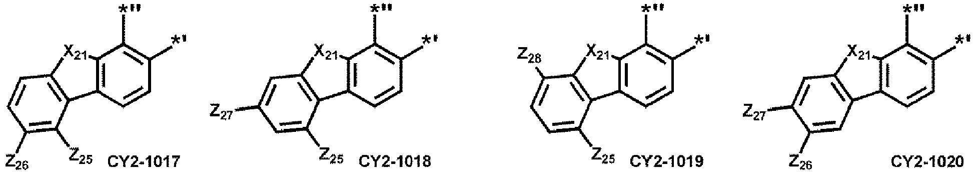 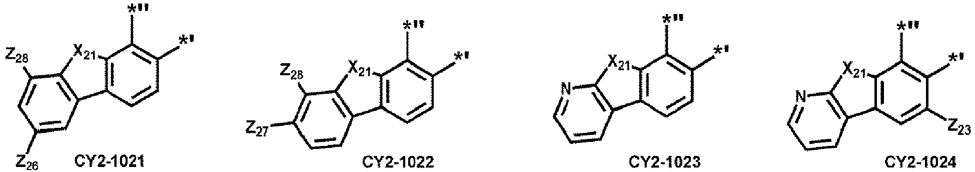 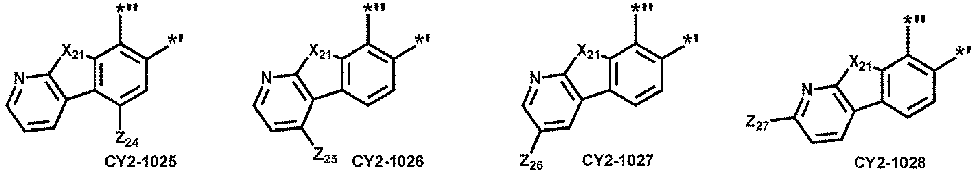 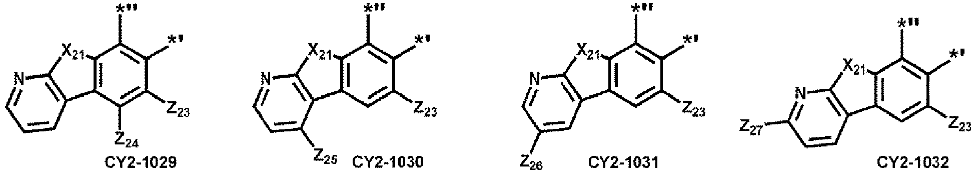 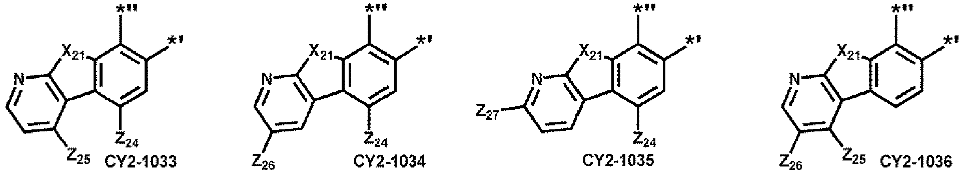 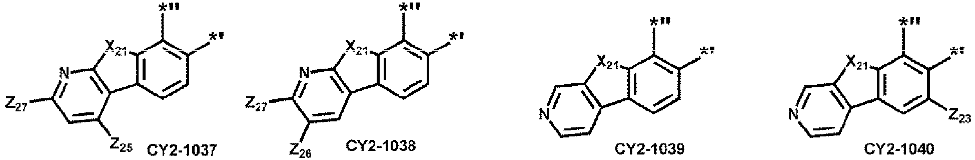 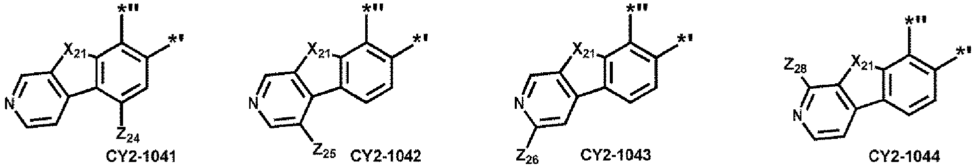 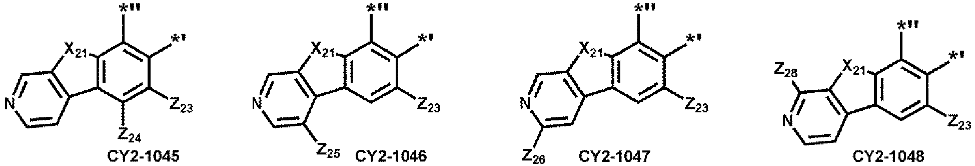 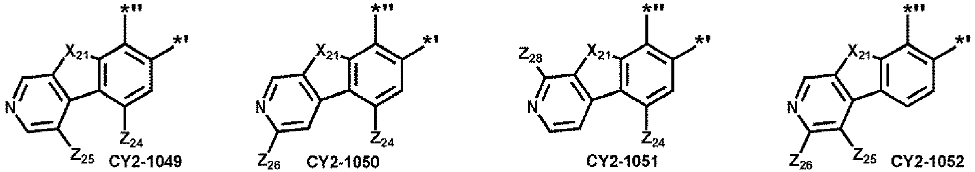 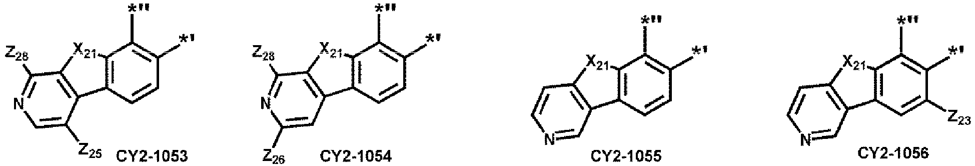 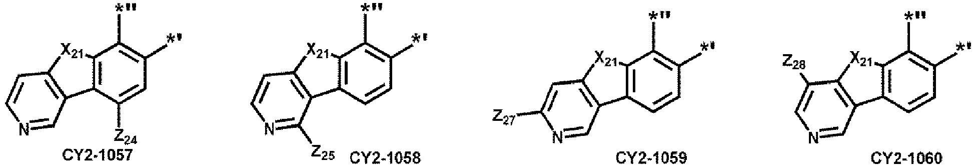 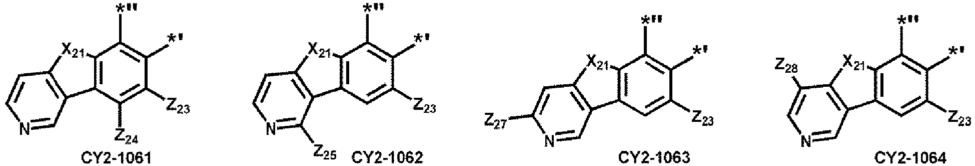 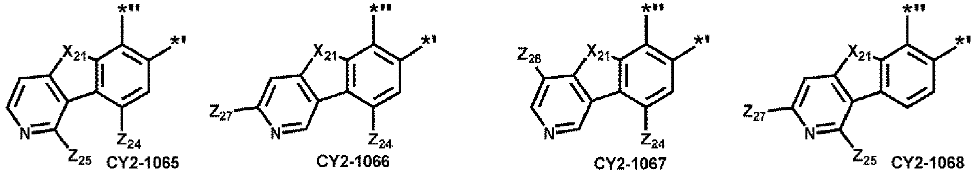 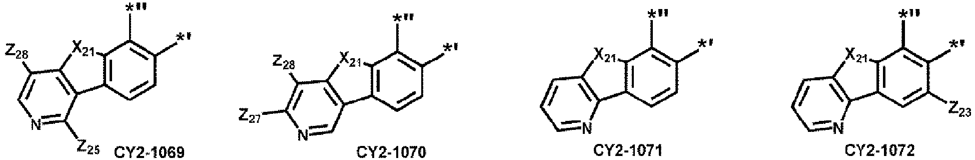 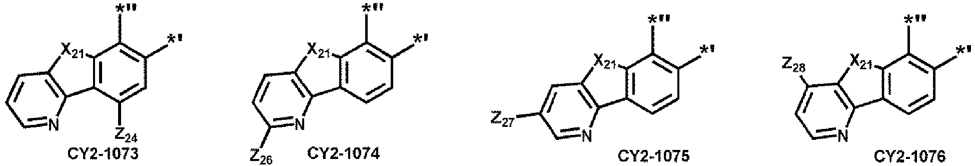 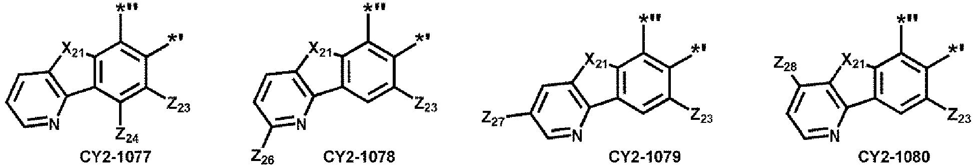 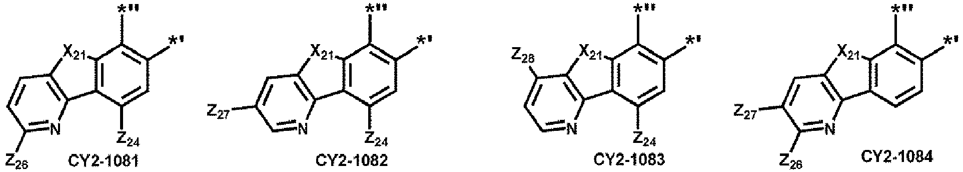 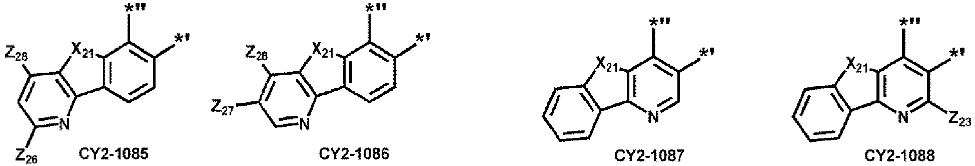 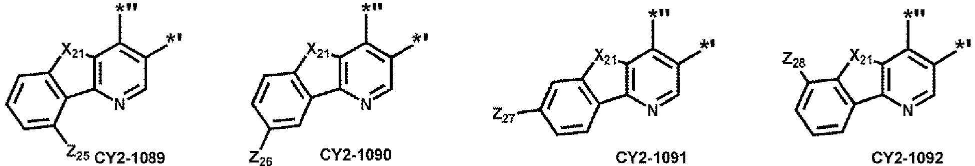 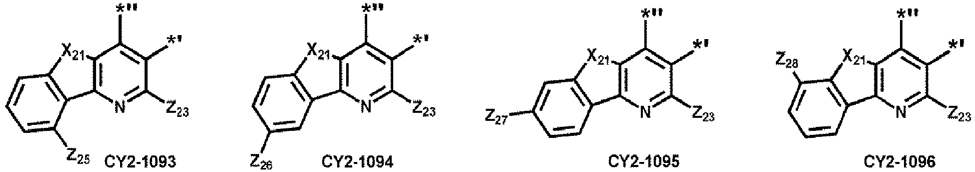 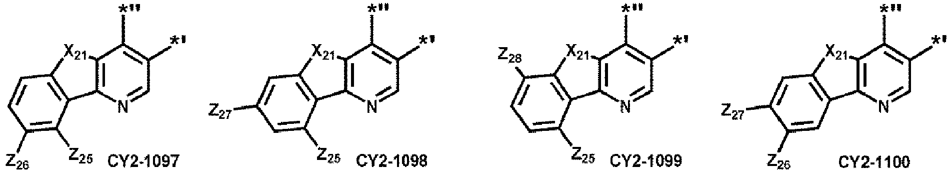 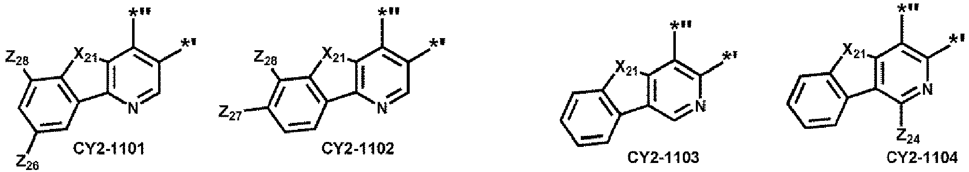 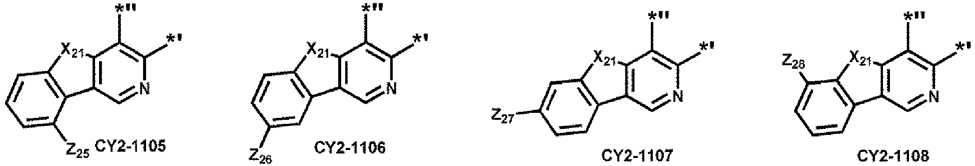 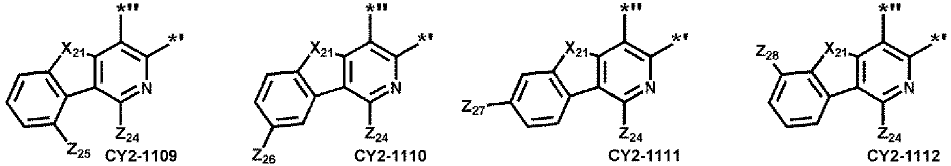 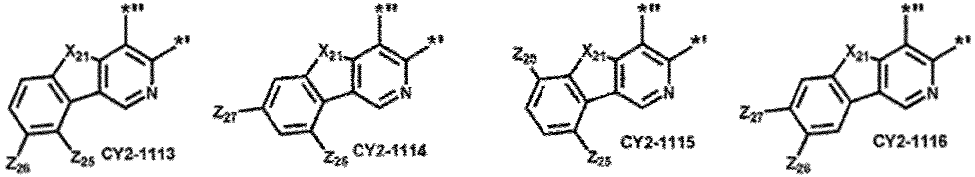 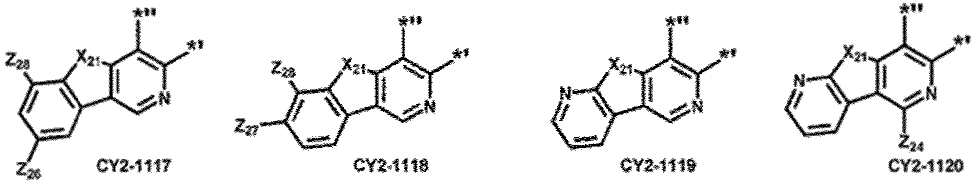 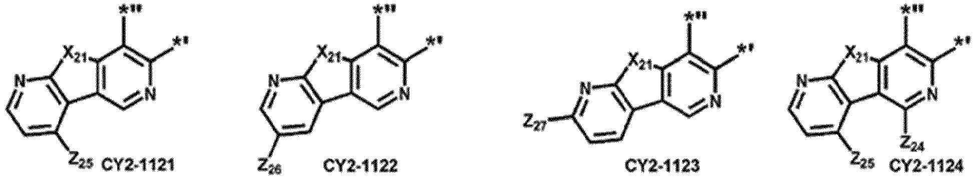 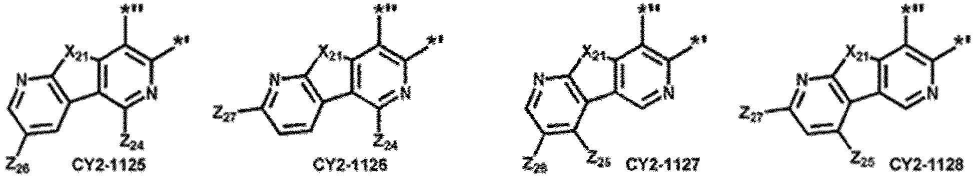 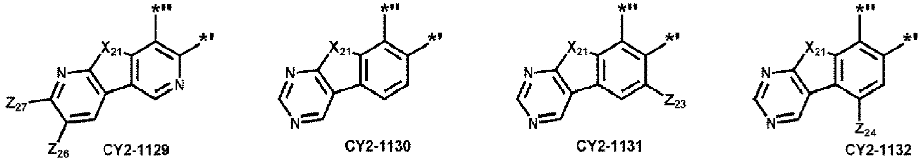 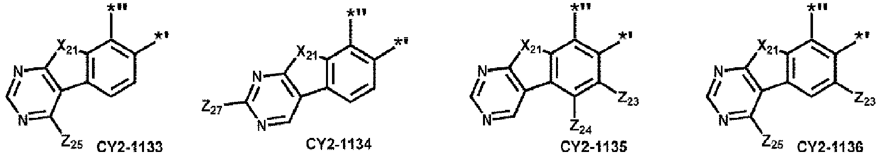 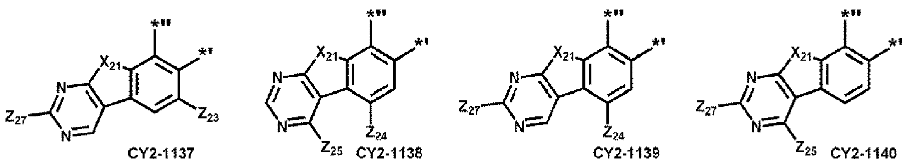 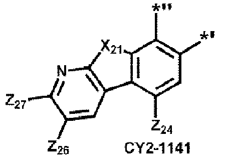 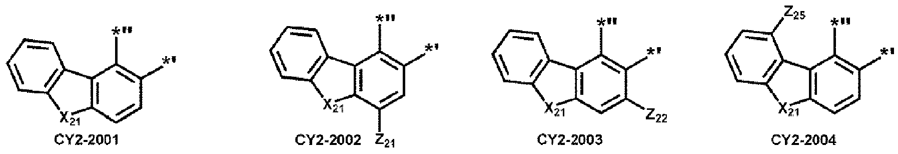 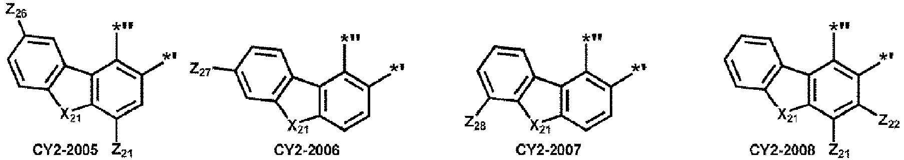 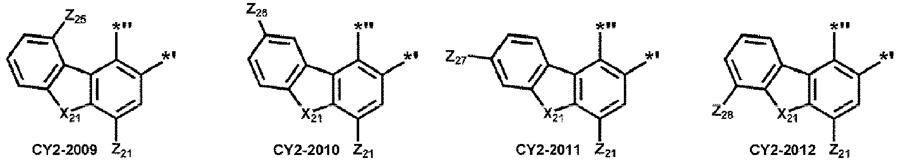 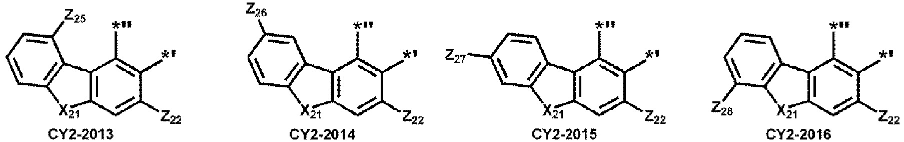 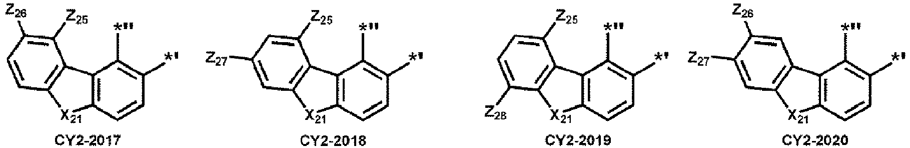 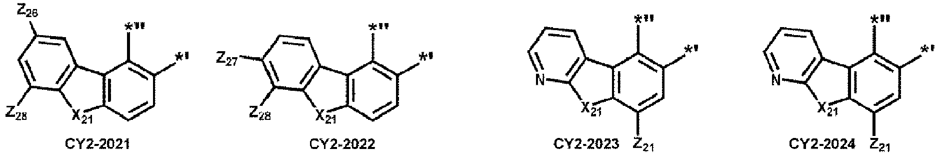 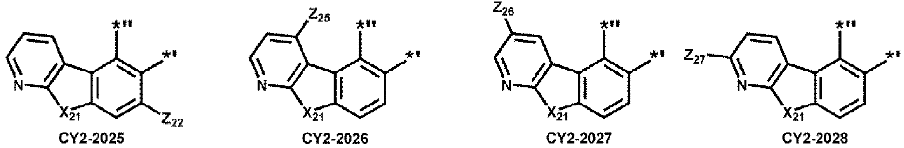 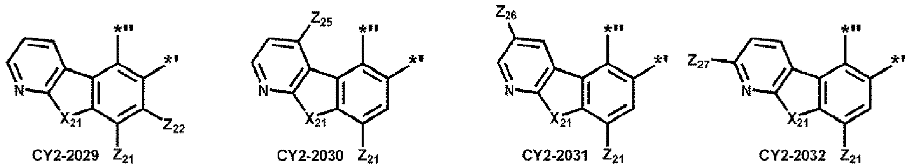 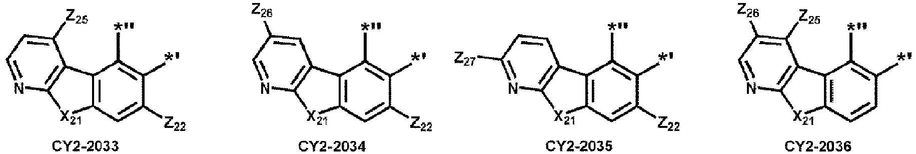 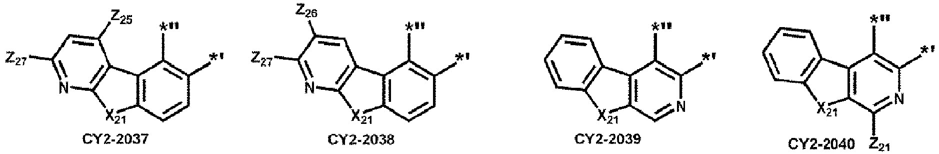 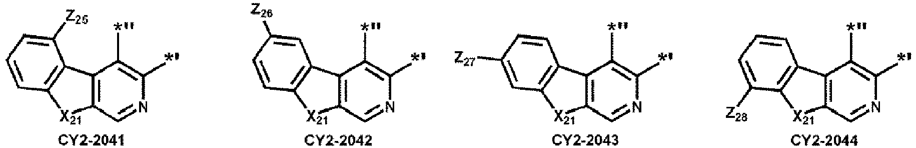 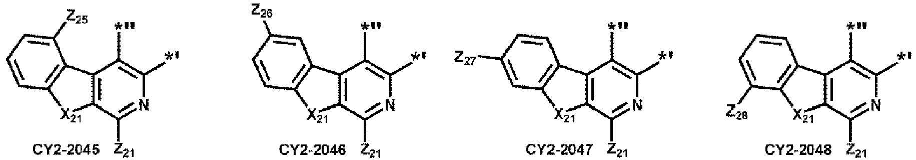 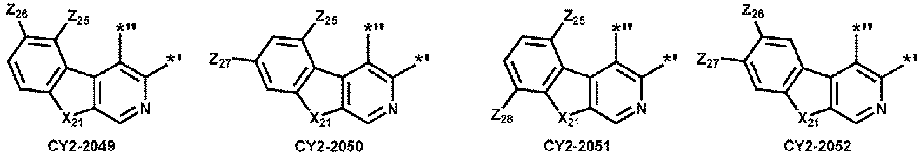 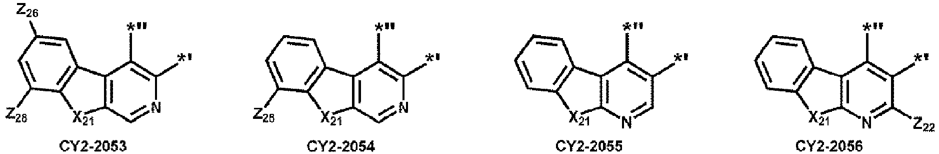 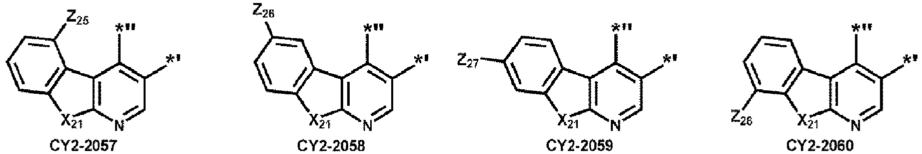 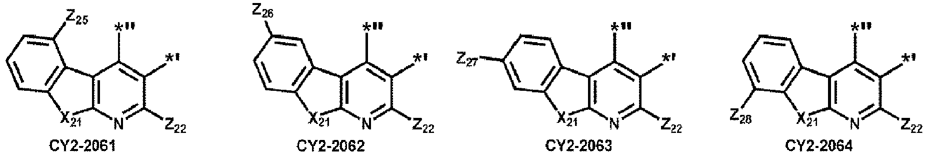 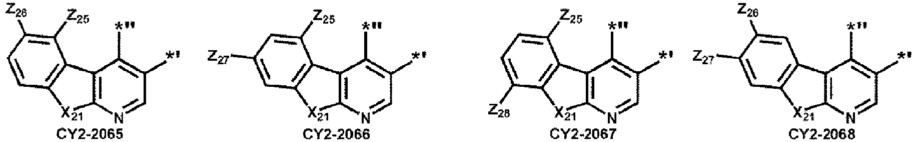 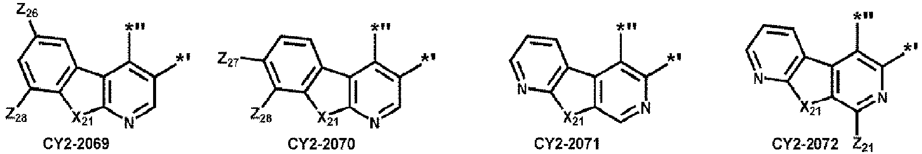 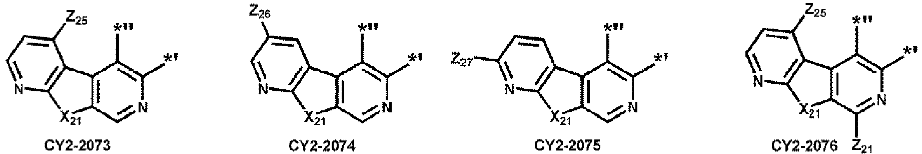 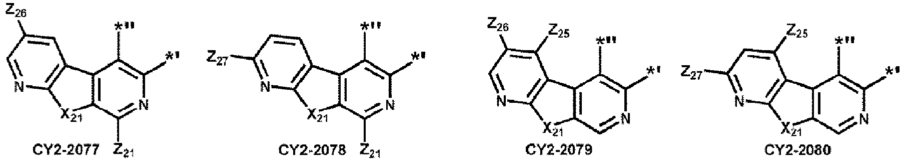 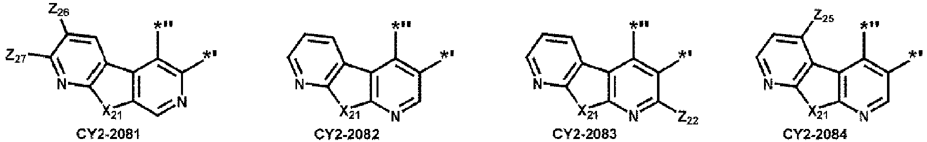 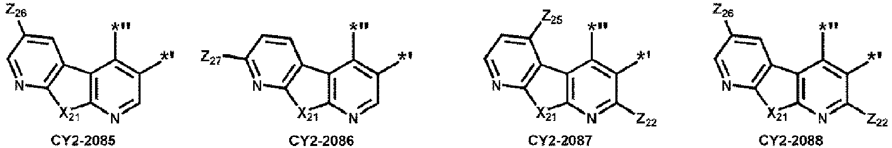 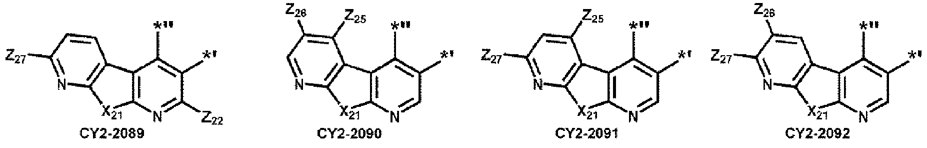 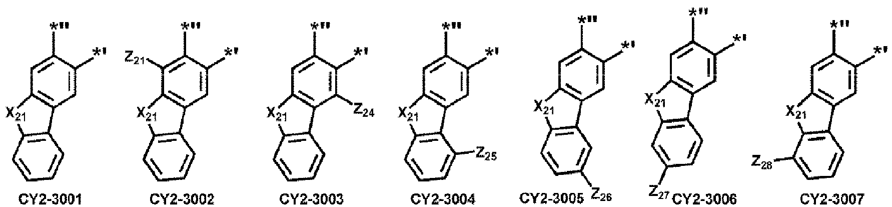 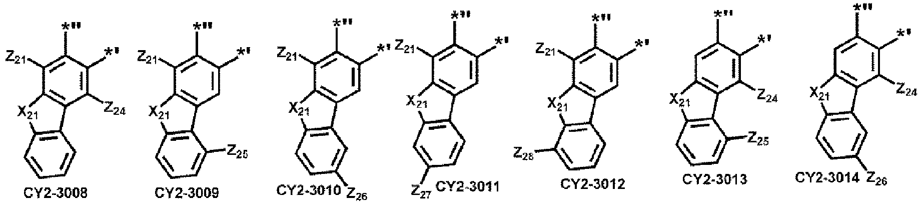 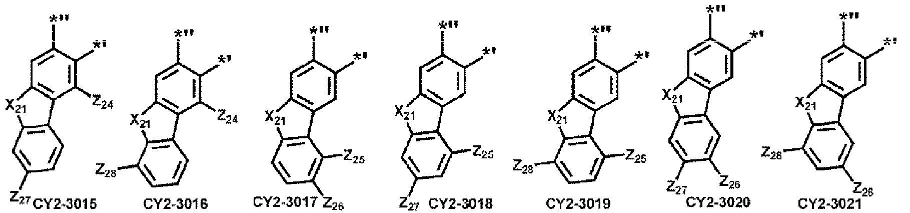 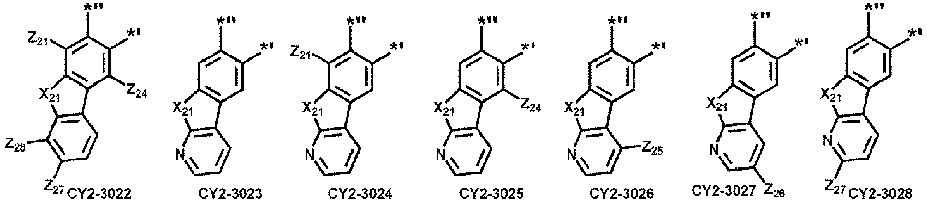 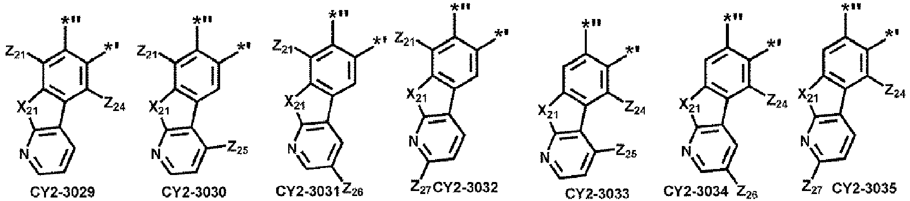 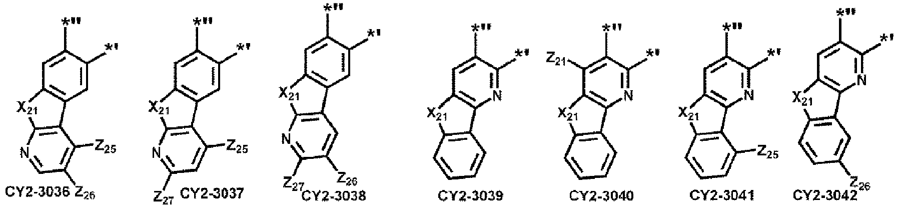 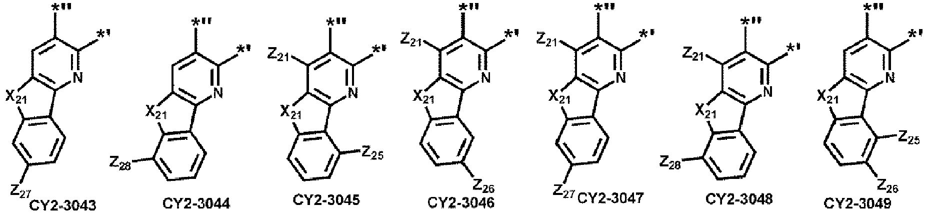 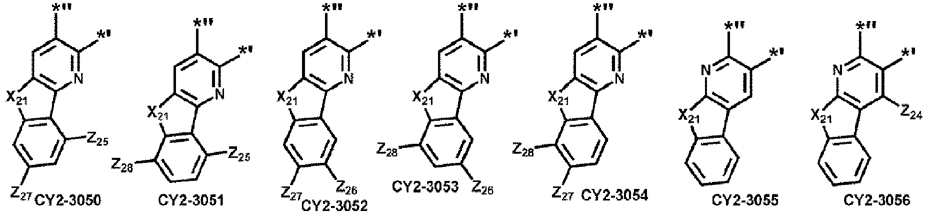 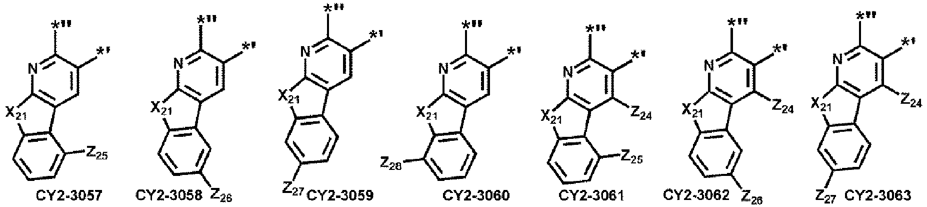 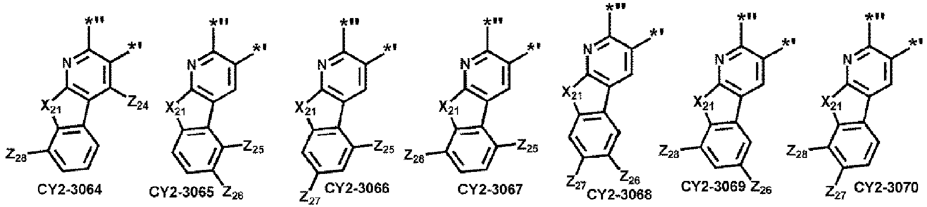 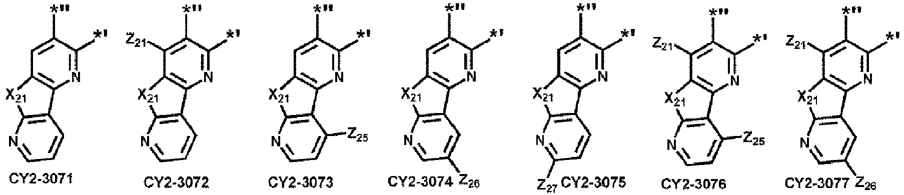 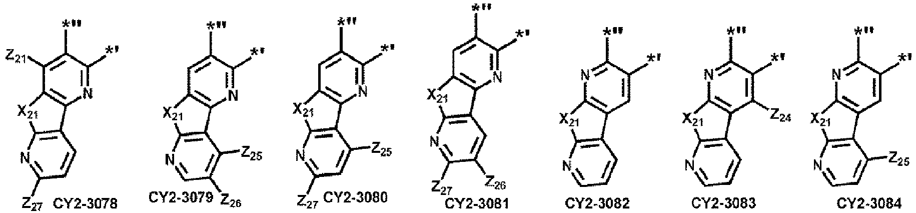 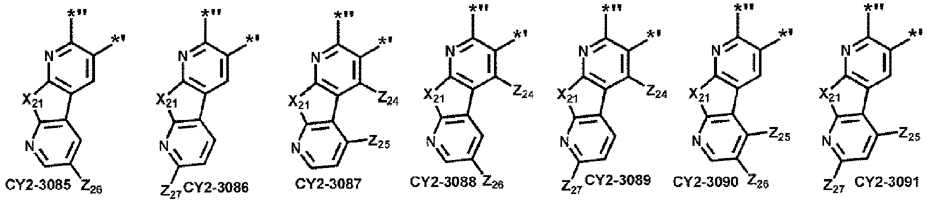 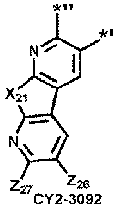 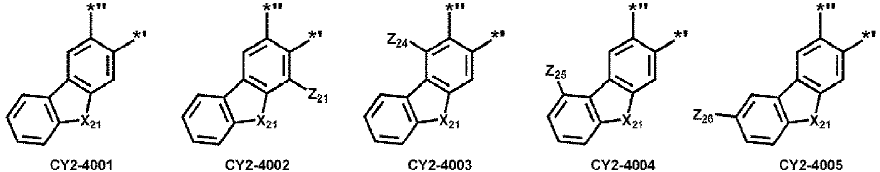 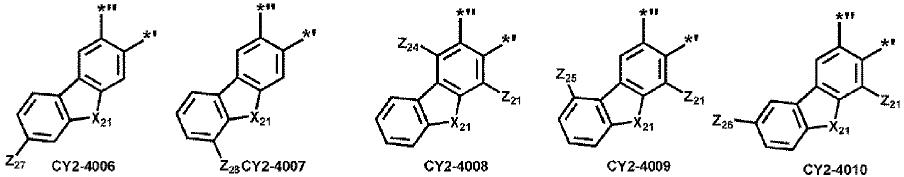 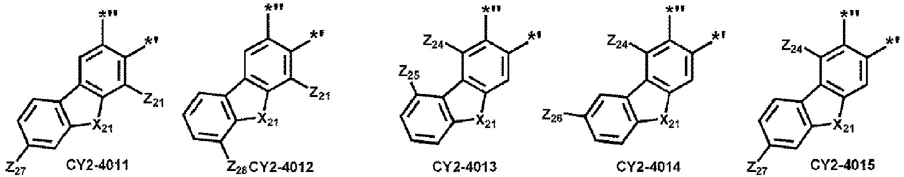 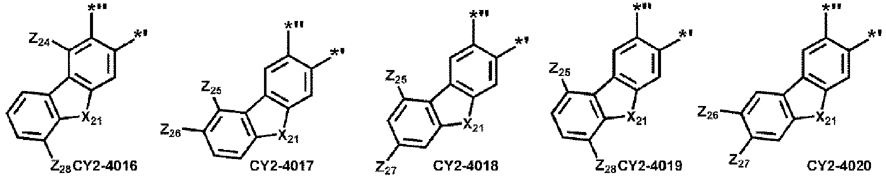 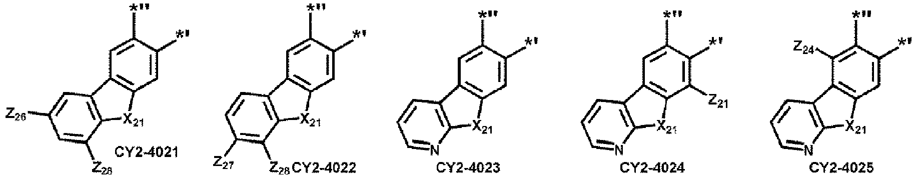 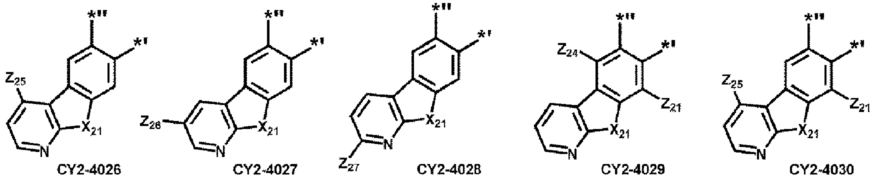 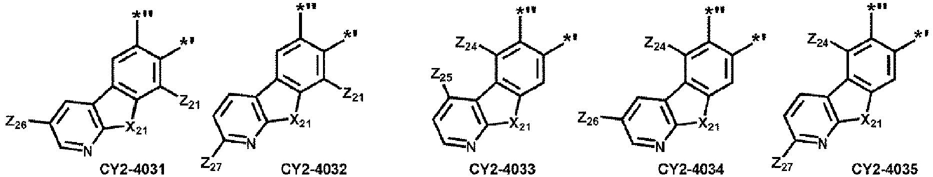 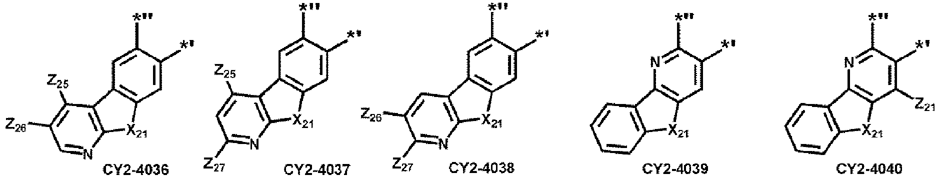 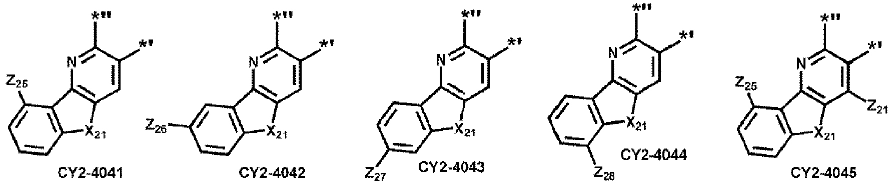 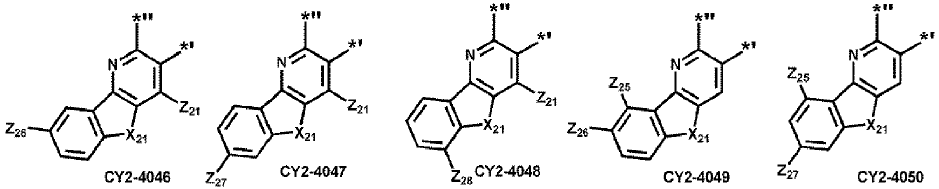 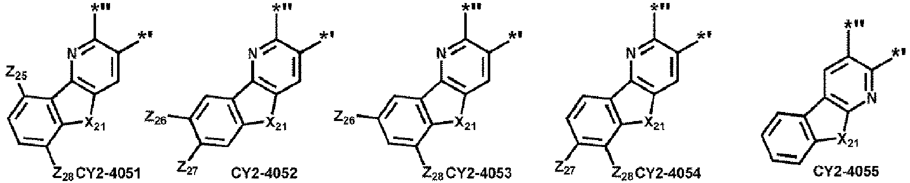 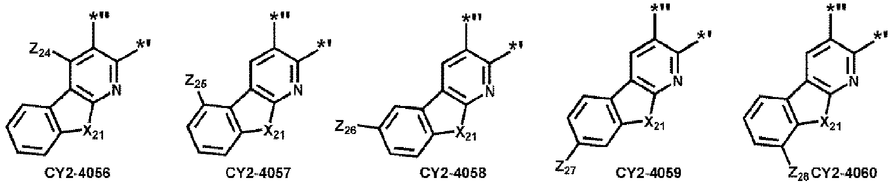 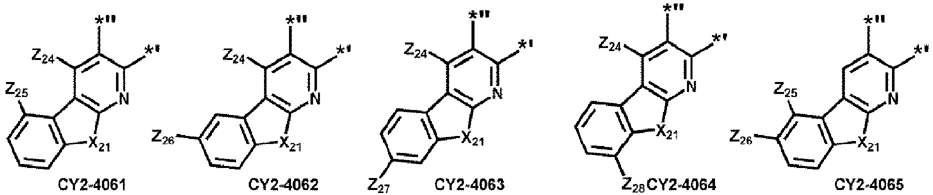 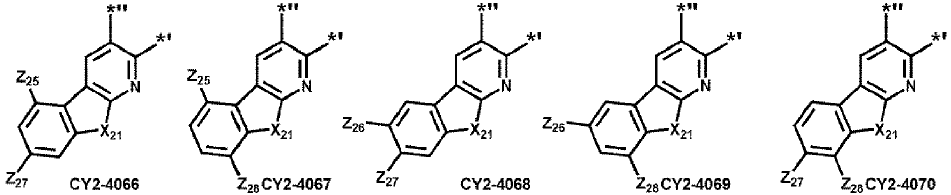 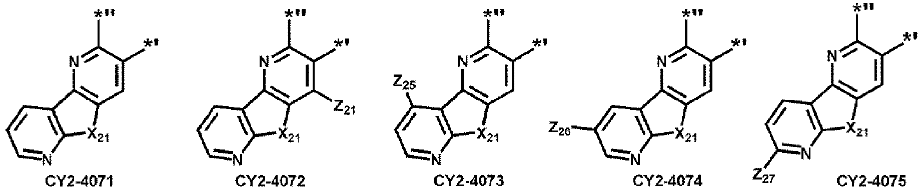 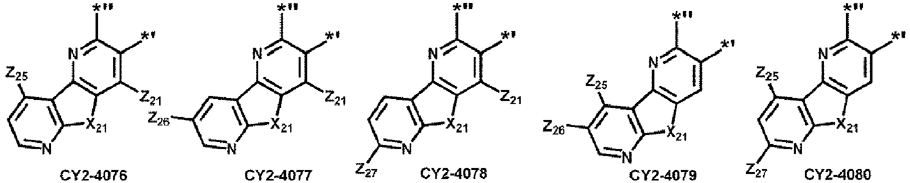 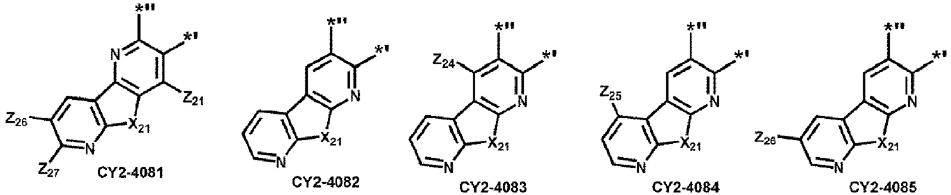 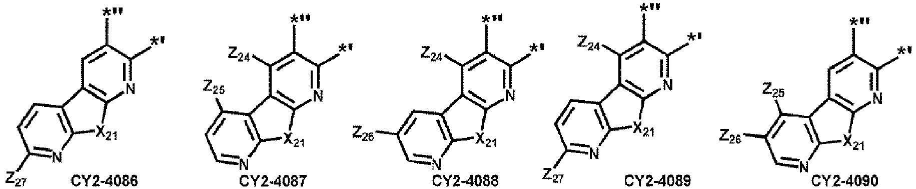 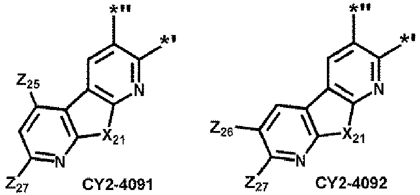 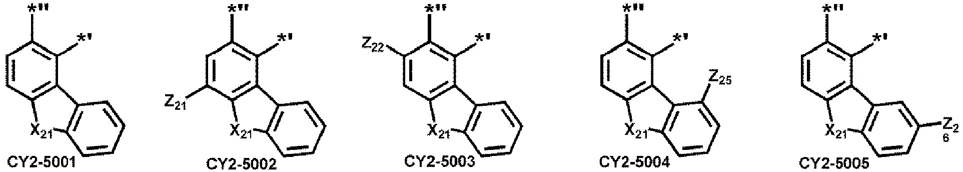 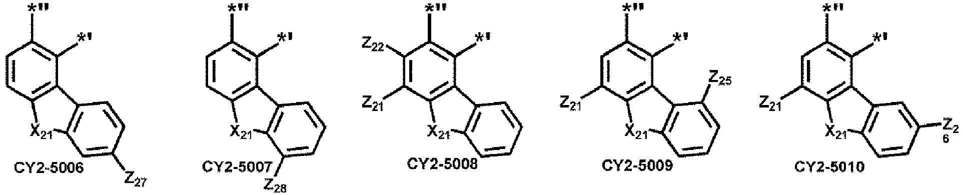 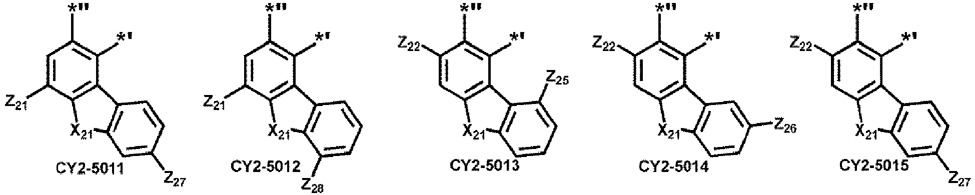 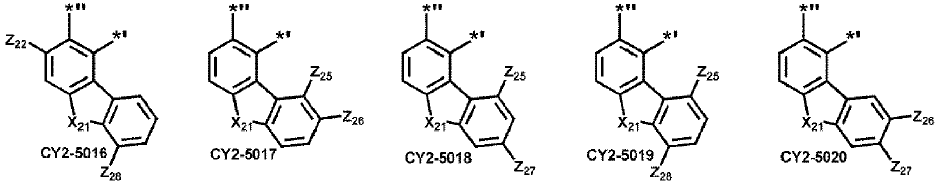 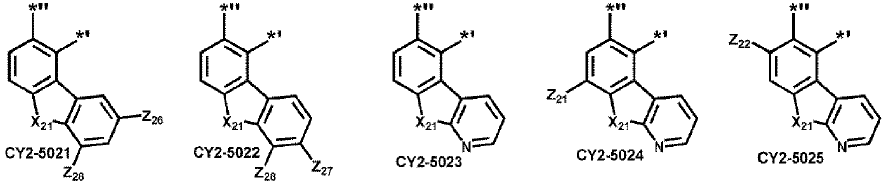 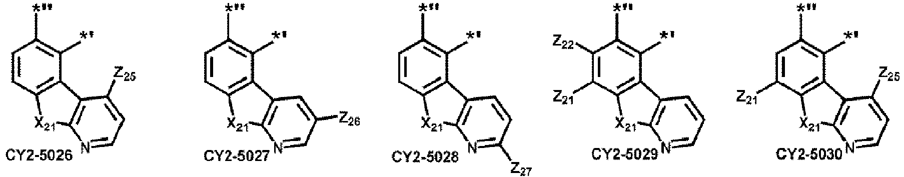 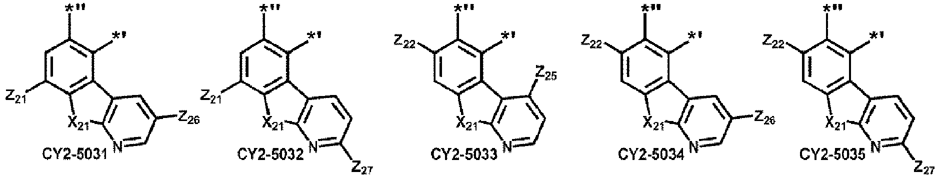 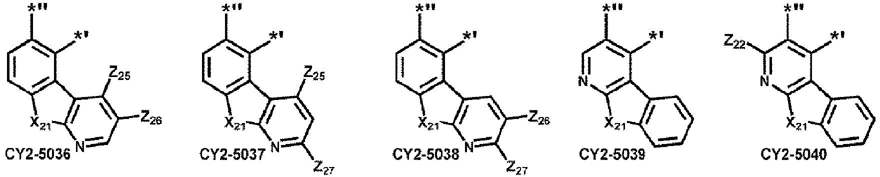 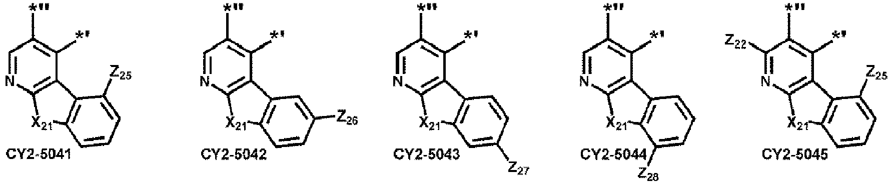 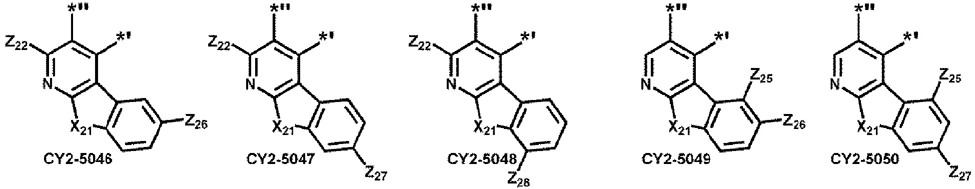 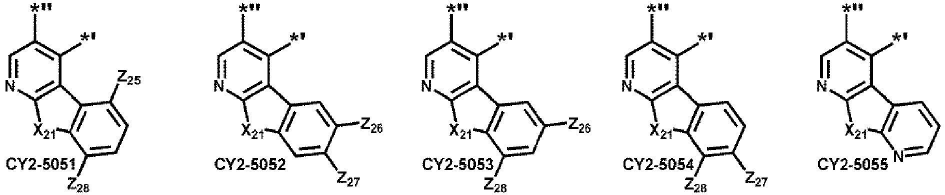 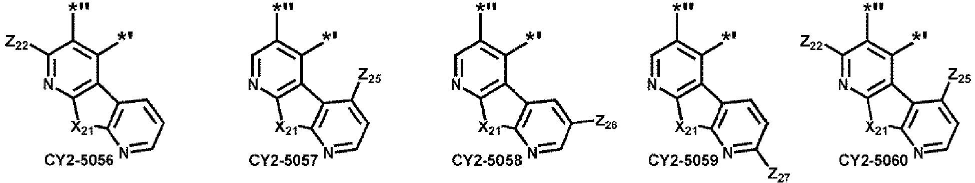 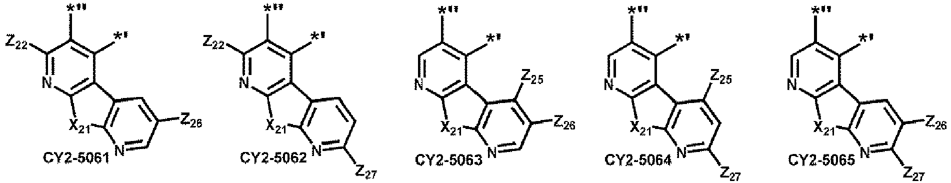 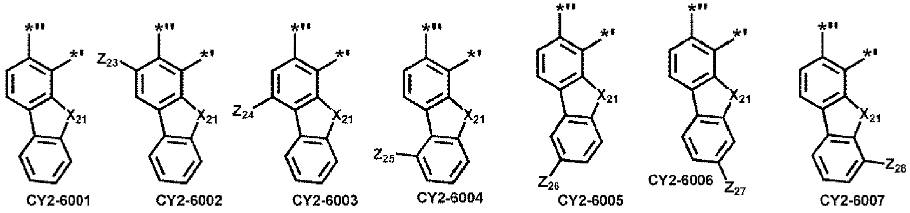 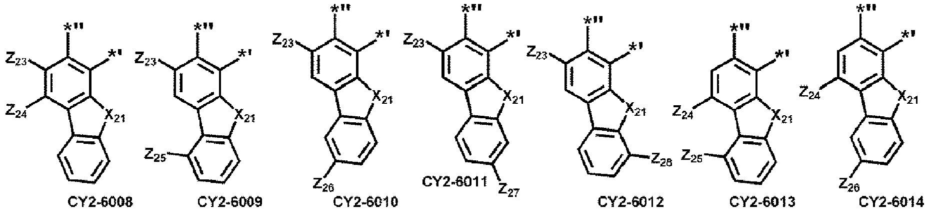 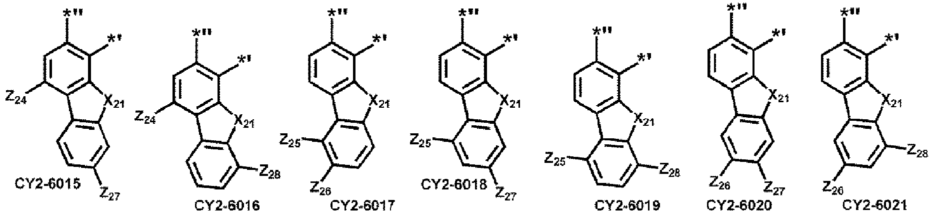 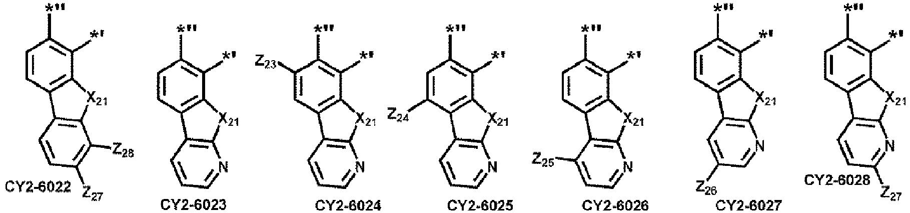 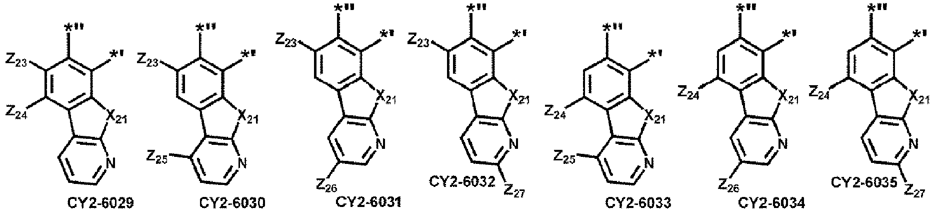 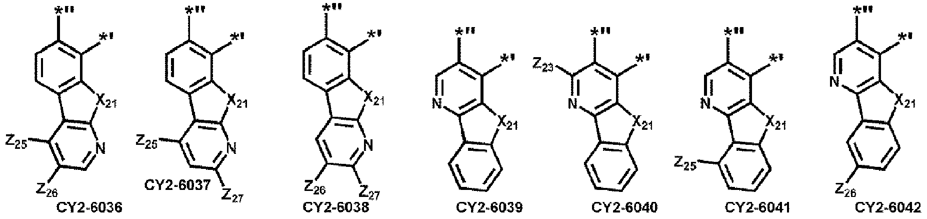 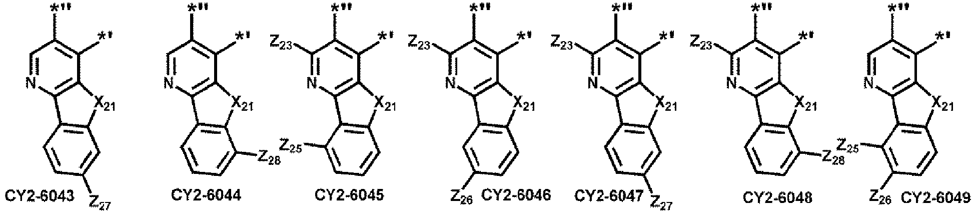 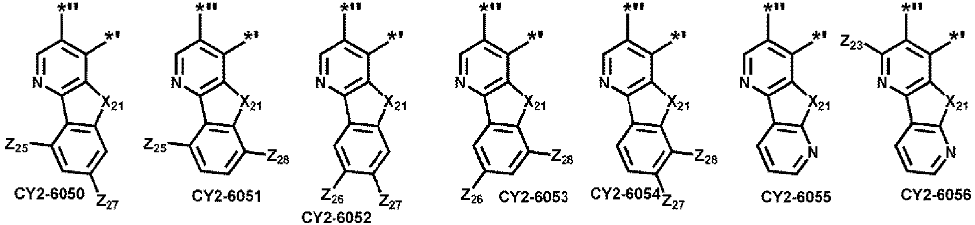 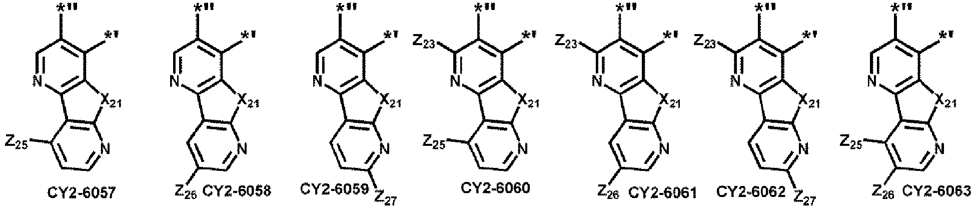 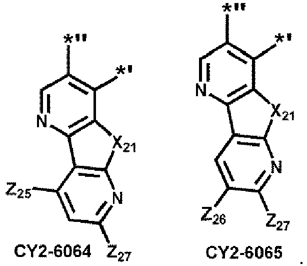
où, dans les Formules CY2-1001 à CY2-1141, CY2-2001 à CY2-2092, CY2-3001 à CY2-3092, CY2-4001 à CY2-4092, CY2-5001 à CY2-5065 et CY2-6001 à CY2-6065,
X₂₁ est le même que décrit dans la revendication 1,
Z₂₁ à Z₂₈ sont chacun indépendamment les mêmes que définis en relation avec Z₂ dans la revendication 1, chacun de Z₂₁ à Z₂₈ n'étant pas hydrogène,
^{∗}" indique un site de liaison au cycle CY₁ dans la Formule 2A, et
^{∗}' indique un site de liaison à M dans la Formule 1.

12. Composé organométallique selon l'une quelconque des revendications 1 à 11, dans lequel un groupe représenté par 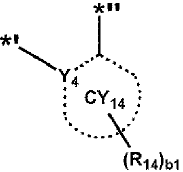 dans la Formule 2B est représenté par l'une des Formules CY14(1) à CY14(63) : 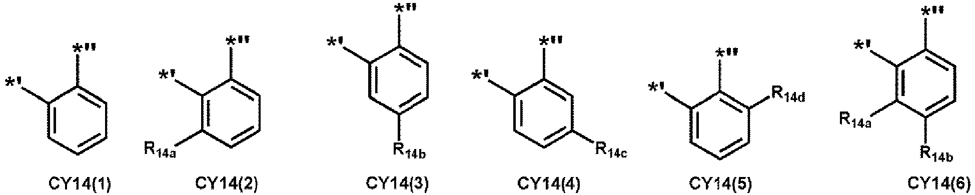 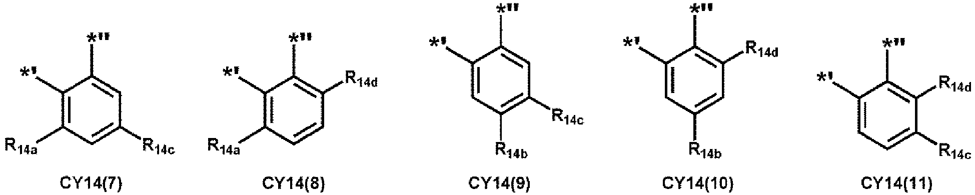 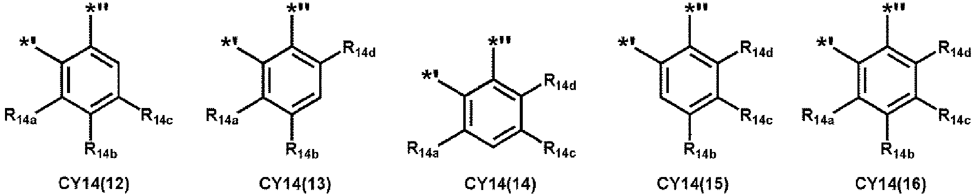 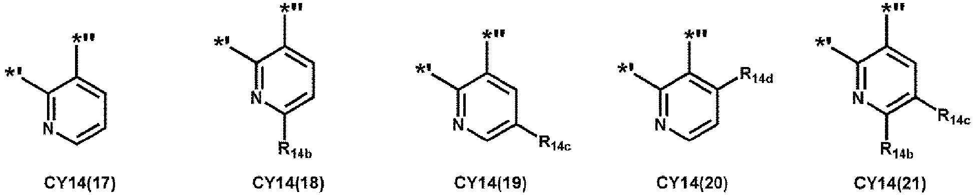 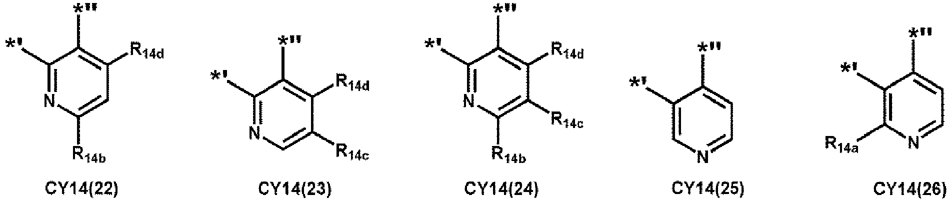 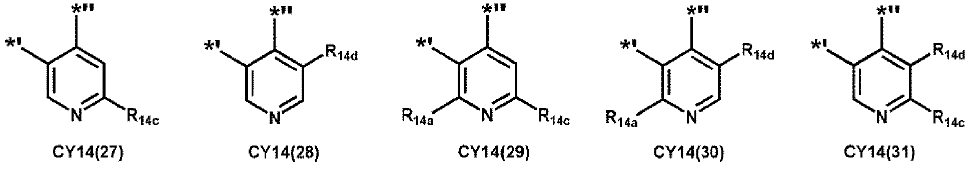 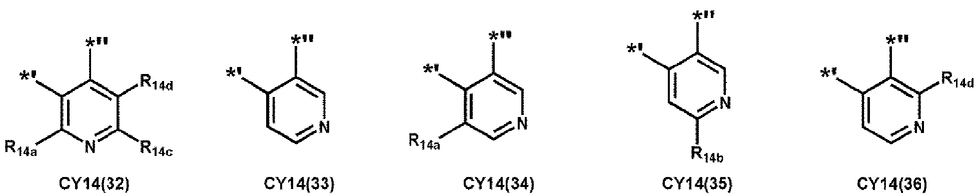 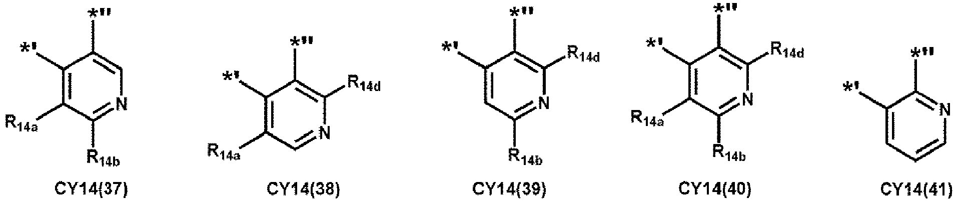 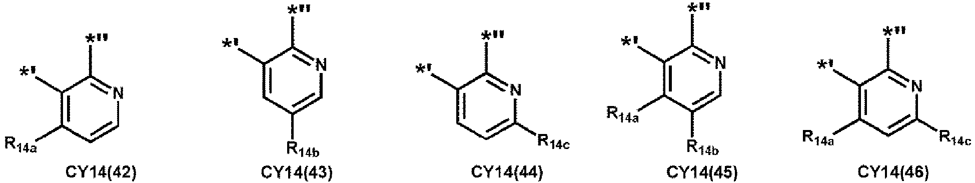 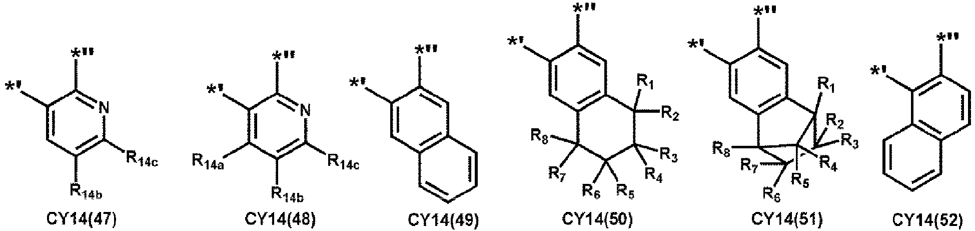 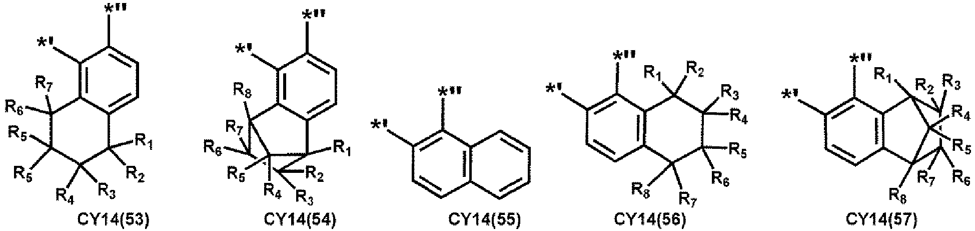 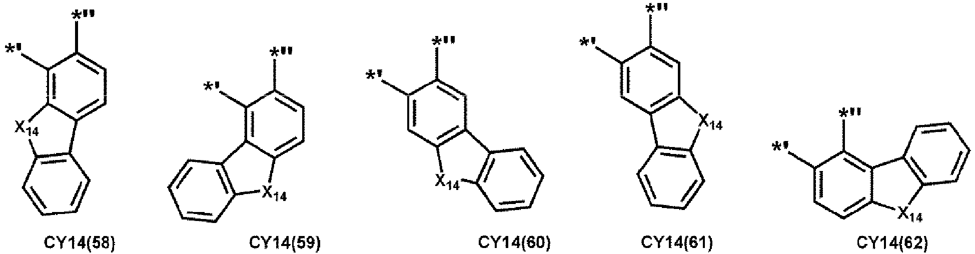 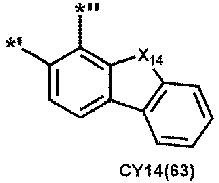
où, dans les Formules CY14(1) to CY14(63),
R₁₄ₐ à R_{14d} sont chacun indépendamment les mêmes que définis en relation avec R₁₄ dans la revendication 1, chacun de R₁₄ₐ à R_{14d} n'étant pas hydrogène,
X₁₄ est C(R₁)(R₂), N(R₁), O, S, ou Si(R₁)(R₂),
R₁ à R₈ sont chacun les mêmes que définis en relation avec R₁₄ dans la revendication 1,
^{∗}" indique un site de liaison à un atome de carbone d'un cycle de pyridine voisin dans la Formule 2B, et
^{∗}' indique un site de liaison à M dans la Formule 1.

13. Dispositif électroluminescent organique comprenant :
une première électrode,
une seconde électrode ; et
une couche organique disposée entre la première électrode et la seconde électrode et comprenant une couche d'émission,
la couche organique comprenant au moins un composé organométallique de l'une quelconque des revendications 1 à 12 ; de préférence
où
la première électrode est une anode,
la seconde électrode est une cathode, et
la couche organique comprend en outre une région de transport de trous disposée entre la première électrode et la couche d'émission et une région de transport d'électrons disposée entre la couche d'émission et la seconde électrode,
où la région de transport de trous comprend une couche d'injection de trous, une couche de transport de trous, et une couche de blocage d'électrons, une couche tampon, ou toute combinaison de ce qui précède, et
la région de transport d'électrons comprend une couche de blocage de trous, une couche de transport d'électrons, et une couche d'injection d'électrons, ou toute combinaison de ce qui précède.

14. Dispositif électroluminescent organique selon la revendication 13, dans lequel
la couche d'émission comprend le ou les composés organométalliques ;
de préférence où
la couche d'émission comprend en outre un hôte, et une quantité de l'hôte est supérieure à celle du composé organométallique.

15. Appareil électronique comprenant le dispositif électroluminescent organique selon la revendication 13 ou 14.
